# EUROPEAN PATENT SPECIFICATION

(11) **EP 2 187 743 B1**
(45) Date of publication and mention of the grant of the patent: **30.07.2014**
(21) Application number: 08827362.8
(22) Date of filing: 18.08.2008
(51) Int. Cl.: A61K 9/00, A61K 31/4184, A61K 31/575, A61K 39/395, A61P 27/02

(54) **Rapamycin formulations for treatment of age related macular degeneration**
Rapamycinformulierungen zur Behandlung der altersbedingten Makuladegeneration
Formulations de rapamycine pour le traitement de la dégénérescence maculaire liée à l'âge

(30) Priority: 16.08.2007 US 965282 P; 16.08.2007 US 965258 P
(43) Date of publication of application: 26.05.2010
(73) Proprietor: Santen Pharmaceutical Co., Ltd, Osaka 533-8651 (JP)
(72) Inventor: DOR, Philippe, Jm, Union City, CA 94587 (US); MUDUMBA, Sreenivasu, Union City, CA 94587 (US); NIVAGGIOLI, Thierry, Union City, CA 94587 (US); WEBER, David, A., Union City, CA 94587 (US); FAROOQ, Sidiq, Union City, CA 94587 (US); TAKHAR, Sudeep, Kaur, Union City, CA 94587 (US)
(74) Representative: Holmberg, Martin Tor
(86) International application number: PCT/US2008/073520
(87) International publication number: WO 2009/023877

(56) References cited:
- WO-A1-03/007944
- WO-A1-2005/044259
- US-A1- 2005 025 810
- US-A1- 2005 187 241
- US-A1- 2006 182 783
- US-A1- 2006 216 288
- US-A1- 2006 258 698
- ENG K ET AL: "Ranibizumab in neovascular age-related macular degeneration" CLINICAL INTERVENTIONS IN AGING, DOVE MEDICAL PRESS, NZ, vol. 1, no. 4, 1 January 2006 (2006-01-01) , pages 451-466, XP007911685 ISSN: 1176-9092
- CHUN D W ET AL: "A Pilot Study of Multiple Intravitreal Injections of Ranibizumab in Patients with Center-Involving Clinically Significant Diabetic Macular Edema" OPHTHALMOLOGY, J. B. LIPPINCOTT CO., PHILADELPHIA, PA, US LNKD- DOI:10.1016/J.OPHTHA.2006.04.033, vol. 113, no. 10, 1 October 2006 (2006-10-01), pages 1706-1712, XP025037682 ISSN: 0161-6420 [retrieved on 2006-10-01]
- JAISSLE G B ET AL: "Bevacizumab for treatment of macular edema secondary to retinal vein occlusion" DER OPHTHALMOLOGE, SPRINGER, BERLIN, DE LNKD- DOI:10.1007/S00347-006-1355-2, vol. 103, no. 6, 1 June 2006 (2006-06-01), pages 471-475, XP019424758 ISSN: 1433-0423
- BRADLEY JOHN ET AL: "Combination therapy for the treatment of ocular neovascularization" ANGIOGENESIS, KLUWER, DORDRECHT, NL LNKD- DOI:10.1007/S10456-007-9069-X, vol. 10, no. 2, 1 January 2007 (2007-01-01), pages 141-148, XP002505079 ISSN: 0969-6970 [retrieved on 2007-03-13]
- LAL.: 'Drop Volume of Commercial Anti-Glaucoma Eye Drops.' IND. J. PHARMACOL. vol. 25, 1993, pages 163 - 164, XP008131361

## Description

### FIELD

Described herein are liquid formulations for treatment, prevention, inhibition, delaying onset of, or causing regression of a disease or condition by delivery of therapeutic agents to a subject, such as a human subject, for use in the treatment of age-related macular degeneration (AMD) by delivery of a liquid formulation comprising a therapeutic agent, such as rapamycin (sirolimus), to the eye of the subject,. Examples of liquid formulations include solutions, suspensions, and in situ gelling formulations.

### BACKGROUND

The retina of the eye contains the cones and rods that detect light. In the center of the retina is the macula lutea, which is about 1/3 to 1/2 cm in diameter. The macula provides detailed vision, particularly in the center (the fovea), because the cones are higher in density. Blood vessels, ganglion cells, inner nuclear layer and cells, and the plexiform layers are all displaced to one side (rather than resting above the cones), thereby allowing light a more direct path to the cones.

Under the retina are the choroid, comprising a collection of blood vessels embedded within a fibrous tissue, and the deeply pigmented epithelium, which overlays the choroid layer. The choroidal blood vessels provide nutrition to the retina (particularly its visual cells).

There are a variety of retinal disorders for which there is currently no treatment or for which the current treatment is not optimal. Retinal disorders such as uveitis (an inflammation of the uveal tract: iris, ciliary body, and choroid), central retinal vein occlusive diseases (CRVO), branch retinal venous occlusion (BRVO), macular degeneration, macular edema, proliferative diabetic retinopathy, and retinal detachment generally are all retinal disorders that are difficult to treat with conventional therapies.

Age-related macular degeneration (AMD) is the major cause of severe visual loss in the United States for individuals over the age of 60. AMD occurs in either an atrophic or less commonly an exudative form. The atrophic form of AMD is also called "dry AMD," and the exudative form of AMD is also called "wet AMD."

In exudative AMD, blood vessels grow from the choriocapillaris through defects in Bruch's membrane, and in some cases the underlying retinal pigment epithelium. Organization of serous or hemorrhagic exudates escaping from these vessels results in fibrovascular scarring of the macular region with attendant degeneration of the neuroretina, detachment and tears of the retinal pigment epithelium, vitreous hemorrhage and permanent loss of central vision. This process is responsible for more than 80% of cases of significant visual loss in subjects with AMD. Current or forthcoming treatments include laser photocoagulation, photodynamic therapy, treatment with VEGF antibody fragments, treatment with pegylated aptamers, and treatment with certain small molecule agents.

Several studies have recently described the use of laser photocoagulation in the treatment of initial or recurrent neovascular lesions associated with AMD (Macular Photocoagulation Study Groups (1991) in Arch. Ophthal. 109:1220; Arch. Ophthal. 109:1232; Arch. Ophthal. 109:1242). Unfortunately, AMD subjects with subfoveal lesions subjected to laser treatment experienced a rather precipitous reduction in visual acuity (mean 3 lines) at 3 months follow-up. Moreover, at two years post-treatment treated eyes had only marginally better visual acuity than their untreated counterparts (means of 20/320 and 20/400, respectively). Another drawback of the procedure is that vision after surgery is immediately worse.

Photodynamic therapy (PDT) is a form of phototherapy, a term encompassing all treatments that use light to produce a beneficial reaction in a subject. Optimally, PDT destroys unwanted tissue while sparing normal tissue. Typically, a compound called a photosensitizer is administered to the subject. Usually, the photosensitizer alone has little or no effect on the subject. When light, often from a laser, is directed onto a tissue containing the photosensitizer, the photosensitizer is activated and begins destroying targeted tissue. Because the light provided to the subject is confined to a particularly targeted area, PDT can be used to selectively target abnormal tissue, thus sparing surrounding healthy tissue. PDT is currently used to treat retinal diseases such as AMD. PDT is currently the mainstay of treatment for subfoveal choroidal neovascularization in subjects with AMD (Photodynamic Therapy for Subfoveal Choroidal Neovascularization in Age Related Macular Degeneration with Verteporfin (TAP Study Group) Arch Ophthalmol. 1999 117:1329-1345.

Choroidal neovascularization (CNV) has proven to be recalcitrant to treatment in most cases. Conventional laser treatment can ablate CNV and help to preserve vision in selected cases not involving the center of the retina, but this is limited to only about 10% of the cases. Unfortunately, even with successful conventional laser photocoagulation, the neovascularization recurs in about 50-70% of eyes (50% over 3 years and >60% at 5 years). (Macular Photocoagulation Study Group, Arch. Ophthalmol. 204:694-701 (1986)). In addition, many subjects who develop CNV are not good candidates for laser therapy because the CNV is too large for laser treatment, or the location cannot be determined so that the physician cannot accurately aim the laser. Photodynamic therapy, although utilized in up to 50% of new cases of subfoveal CNV has only marginal benefits over natural history, and generally delays progression of visual loss rather than improving vision which is already decreased secondary to the subfoveal lesion. PDT is neither preventive nor definitive. Several PDT treatments are usually required per subject and additionally, certain subtypes of CNV fare less well than others.

Thus, there remains a long-felt need for methods, compositions, and formulations that may be used to optimally prevent or significantly inhibit choroidal neovascularization and to prevent and treat wet AMD.

In addition to AMD, choroidal neovascularization is associated with such retinal disorders as presumed ocular histoplasmosis syndrome, myopic degeneration, angioid streaks, idiopathic central serous chorioretinopathy, inflammatory conditions of the retina and or choroid, and ocular trauma. Angiogenic damage associated with neovascularization occurs in a wide range of disorders including diabetic retinopathy, venous occlusions, sickle cell retinopathy, retinopathy of prematurity, retinal detachment, ocular ischemia and trauma.

Uveitis is another retinal disorder that has proven difficult to treat using existing therapies. Uveitis is a general term that indicates an inflammation of any component of the uveal tract. The uveal tract of the eye consists of the iris, ciliary body, and choroid. Inflammation of the overlying retina, called retinitis, or of the optic nerve, called optic neuritis, may occur with or without accompanying uveitis.

Uveitis is most commonly classified anatomically as anterior, intermediate, posterior, or diffuse. Posterior uveitis signifies any of a number of forms of retinitis, choroiditis, or optic neuritis. Diffuse uveitis implies inflammation involving all parts of the eye, including anterior, intermediate, and posterior structures.

The symptoms and signs of uveitis may be subtle, and vary considerably depending on the site and severity of the inflammation. Regarding posterior uveitis, the most common symptoms include the presence of floaters and decreased vision. Cells in the vitreous humor, white or yellow-white lesions in the retina and/or underlying choroid, exudative retinal detachments, retinal vasculitis, and optic nerve edema may also be present in a subject suffering from posterior uveitis.

Ocular complications of uveitis may produce profound and irreversible loss of vision, especially when unrecognized or treated improperly. The most frequent complications of posterior uveitis include retinal detachment; neovascularization of the retina, optic nerve, or iris; and cystoid macular edema.

Macular edema (ME) can occur if the swelling, leaking, and hard exudates noted in background diabetic retinopathy (BDR) occur within the macula, the central 5% of the retina most critical to vision. Background diabetic retinopathy (BDR) typically consists of retinal microaneurisms that result from changes in the retinal microcirculation. These microaneurisms are usually the earliest visible change in retinopathy seen on exam with an ophthalmoscope as scattered red spots in the retina where tiny, weakened blood vessels have ballooned out. The ocular findings in background diabetic retinopathy progress to cotton wool spots, intraretinal hemorrhages, leakage of fluid from the retinal capillaries, and retinal exudates. The increased vascular permeability is also related to elevated levels of local growth factors such as vascular endothelial growth factor. The macula is rich in cones, the nerve endings that detect color and upon which daytime vision depends. When increased retinal capillary permeability affects the macula, blurring occurs in the middle or just to the side of the central visual field, rather like looking through cellophane. Visual loss may progress over a period of months, and can be very annoying because of the inability to focus clearly. ME is a common cause of severe visual impairment.

There have been many attempts to treat CNV and its related diseases and conditions, as well as other conditions such as macular edema and chronic inflammation, with pharmaceuticals. For example, use of rapamycin to inhibit CNV and wet AMD has been described in U.S. Patent Publication No. 2005/018724. The use of rapamycin to treat inflammatory diseases of the eye has been described in US patent number 5,387,589. The previously mentioned U.S. Patent Publication 2005/187241 and Clinical Interventions in Aging, 2006, vol. 1, no. 4, pages 451-466 (K Eng et al.) disclose rapamycin in the treatment of macular edema or age related macular degeneration. WO 2005/044259, WO 03/007944, J. Ophthamol., 2006, vol. 113, no. 10, pages 1706-1702 (D W Chun et al.) and Der Ophthalmologe, 2006, vol. 103, no. 6, pages 471-475 (G B Jaissle et al.) disclose the uses of verteporfin, ranibizumab and bevacizumab in the treatment of age-related macular degeneration. However, none of these documents disclose that a combination therapy with formulations of rapamycin and an additional agent can be advantageous. Angiogenesis, 2007, vol.10, no. 2 (J Bradley et al.) gives general reference, without any specific examples related to rapamycin, that diseases involving neovascularization in the eye may benefit from combination therapy as used in cancer treatment.

Particularly for chronic diseases, including those described herein, there is a great need for long acting methods for delivering therapeutic agents to the eye, such as to the posterior segment to treat CNV in such diseases as AMD, macular edema, proliferative retinopathies, and chronic inflammation. Formulations with extended delivery of therapeutic agent are more comfortable and convenient for a subject, due to a diminished frequency of ocular injections of the therapeutic agent.

Direct delivery of therapeutic agents to the eye rather than systemic administration may be advantageous because the therapeutic agent concentration at the site of action is increased relative to the therapeutic agent concentration in a subject's circulatory system. Additionally, therapeutic agents may have undesirable side effects when delivered systemically to treat posterior segment disease. Thus, localized drug delivery may promote efficacy while decreasing side effects and systemic toxicity.

### SUMMARY

The invention is set out in the appended claims. The embodiments of the description that do not fall within the scope of said claims are provided for illustrative purposes only and do not fom part of the present invention. The compositions, and liquid formulations described herein allow delivery of a therapeutic agent to a human subject or to the eye(s) of a human subject. Described herein are compositions, and liquid formulations for delivering a variety of therapeutic agents for extended periods of time which can be used for the treatment, prevention, inhibition, delaying onset of, or causing regression of a number of conditions or diseases, such as diseases or conditions of the eye. The liquid formulations may be solutions, suspensions, or in situ gelling formulations.

Described herein are compositions and liquid formulations for administering to a human subject an amount of rapamycin effective to treat, prevent, inhibit, delay onset of, or cause regression of wet AMD, dry AMD, or macular edema (such as diabetic macular edema).

As described in further detail in the Detailed Description section, the compositions and liquid formulations may also be used for delivery to a human subject or to the eye(s) of a human subject of therapeutically effective amounts of rapamycin for the treatment, prevention, inhibition, delaying of the onset of, or causing the regression of wet AMD, dry AMD, or macular edema. In some variations, compositions, and liquid formulations are used to treat wet AMD. In some variations, the compositions, and liquid formulations are used to prevent wet AMD. In some variations, compositions, and liquid formulations are used to treat dry AMD. In some variations, the compositions, and liquid formulations are used to prevent dry AMD. In some variations, the formulations described herein are used to prevent the transition from dry AMD to wet AMD. In some variations, the formulations described herein are used to treat macular edema (including, diabetic macular edema). In some variations, the formulations described herein are used to prevent macular edema. The compositions and liquid formulations may also be used for delivery to a human subject or to the eye of a human subject of therapeutically effective amounts of rapamycin for the treatment, prevention, inhibition, delaying of the onset of, or causing the regression of CNV. In some variations, the compositions and liquid formulations are used to treat CNV. The compositions and liquid formulations may also be used for delivery to a human subject or to the eye of a human subject of therapeutically effective amounts of rapamycin for the treatment, prevention, inhibition, delaying of the onset of, or causing the regression of angiogenesis in the eye. In some variations, the compositions and liquid formulations are used to treat angiogenesis. Other diseases and conditions that may be treated, prevented, inhibited, have onset delayed, or caused to regress using rapamycin are described in the *Diseases and Conditions* section of the *Detailed Description.*

As described in further detail in the *Detailed Description,* the compositions and liquid formulations may also be used for delivery to a human subject or to the eye of a human subject of therapeutically effective amounts of therapeutic agents other than rapamycin for the treatment, prevention, inhibition, delaying of the onset of, or causing the regression of wet AMD or macular edema. In some variations, the compositions and liquid formulations are used to treat wet AMD. In some variations, the compositions and liquid formulations are used to treat macular edema. Therapeutic agents that may be used are described in detail in the *Therapeutic Agents* section. Such therapeutic agents include rapamycin. The compositions and liquid formulations may also be used for delivery to a human subject or to the eye of a subject of therapeutically effective amounts of therapeutic agents for the treatment, prevention, inhibition, delaying of the onset of, or causing the regression of CNV. In some variations, compositions and liquid formulations are used to treat CNV. The compositions and liquid formulations may also be used for delivery to a human subject or to the eye of a subject of therapeutically effective amounts of therapeutic agents for the treatment, prevention, inhibition, delaying of the onset of, or causing the regression of angiogenesis in the eye. In some variations, compositions and liquid formulations are used to treat angiogenesis. Other diseases and conditions that may be treated, prevented, inhibited, have onset delayed, or caused to regress using therapeutic agents other than rapamycin are described in the *Diseases and Conditions* section of the *Detailed Description.*

One liquid formulation described herein comprises a solution that includes a therapeutic agent dissolved in a solvent. Generally, any solvent that has the desired effect may be used in which the therapeutic agent dissolves and which can be administered to a human subject or an eye of a subject. Generally, any concentration of therapeutic agent that has the desired effect can be used. The formulation in some variations is a solution which is unsaturated, a saturated or a supersaturated solution. The solvent may be a pure solvent or may be a mixture of liquid solvent components. In some variations the solution formed is an in situ gelling formulation. Solvents and types of solutions that may be used are well known to those versed in such drug delivery technologies.

The liquid formulations described herein may form a non-dispersed mass when placed into a rabbit eye, for example the vitreous of a rabbit eye. In some variations the non-dispersed mass comprises a gel. In some variations, the liquid formulation comprises a therapeutic agent and a plurality of polymers. In some variations one of the polymers is polyacrylate or polymethacrylate. In some variations one of the polymers is polyvinylpyrrolidone.

The liquid formulations described herein may form a non-dispersed mass when placed into a human eye, for example injected into the vitreous of a human eye or injected between the sclera and conjunctiva of a human eye.

In some variations, the non-dispersed mass comprises a depot. In some variations, the non-dispersed mass consists of a depot.

For liquid formulations which form a non-dispersed mass, the non-dispersed mass may generally be any geometry or shape. The non-dispersed mass-forming liquid formulations may, for instance, appear as a compact spherical mass when placed in the vitreous. In some variations the liquid formulations described herein form a milky or whitish colored semi-contiguous or semi-solid non-dispersed mass relative to the medium in which it is placed, when placed in the vitreous.

The liquid formulations described herein may generally be administered in any volume that has the desired effect. In one method a volume of a liquid formulation is administered to the vitreous and the liquid formulation is less than one half the volume of the vitreous.

Routes of administration that may be used to administer a liquid formulation described herein are for example (1) placement of the liquid formulation by placement, including by injection, into a medium, including to an aqueous medium in the body, including to intraocular or periocular injection; or (2) oral administration of the liquid formulation. The liquid formulation as described herein may be administered systemically, including for example the following delivery routes: rectal, vaginal, infusion, intramuscular, intraperitoneal, intraarterial, intrathecal, intrabronchial, intracisternal, cutaneous, subcutaneous, intradermal, transdermal, intravenous, intracervical, intraabdominal, intracranial, intrapulmonary, intrathoracic, intratracheal, nasal, buccal, sublingual, oral, parenteral, or nebulised or aerosolized using aerosol propellants. In some variations, the liquid formulation is administered subconjunctivally. In some variations, the liquid formulation is administered intravitreally. In some alternative variations, the liquid formulation described herein is administered by subtenon injection. In some variations, the liquid formulation described herein is administered topically.

The liquid formulations described herein may be delivered to any medium of a human subject, including to an aqueous medium of a subject.

One liquid formulation described herein comprises a liquid formulation of rapamycin or other therapeutic agent. The liquid formulations may comprise a solution, suspension, an in situ gelling formulation, or an emulsion. The droplets in the emulsion may generally be of any size, including up to about 5,000 nm.

In some formulations described herein, the liquid formulations may comprise a therapeutic agent, for example rapamycin, and one or more solubilizing agents or solvents. The solubilizing agent or solvent may be glycerin, DMSO, DMA, N-methylpyrrolidone, ethanol, benzyl alcohol, isopropyl alcohol, polyethylene glycol of various molecular weights, for example PEG 300 and PEG 400, or propylene glycol or a mixture of one or more thereof.

In some formulations described herein, the liquid formulation includes hyaluronic acid.

The liquid formulations described herein may deliver a therapeutic agent or agents for an extended period of time. One example of such an extended release delivery system is a liquid formulation that delivers a therapeutic agent or agents to a human subject or to the eye of a human subject in an amount sufficient to maintain an amount effective to treat, prevent, inhibit, delay onset of, or cause regression of a disease or condition in a subject for an extended period of time. In some variations, the liquid formulation is used to treat a disease or condition in a human subject. In some variations, the liquid formulation delivers the therapeutic agent for at least about one, about two, about three, about six, about nine, or about twelve months.

The liquid formulations described herein may deliver rapamycin or other therapeutic agents for an extended period of time. One example of such an extended release delivery system is a liquid formulation that delivers rapamycin to a human subject or to the eye of a human subject in an amount sufficient to maintain an amount effective to treat, prevent, inhibit, delay onset of, or cause regression of wet age-related macular degeneration for an extended period of time. In some variations, the liquid formulation is used to treat wet age-related macular degeneration for an extended period of time. In some variations, the liquid formulation is used to prevent wet age-related macular degeneration for an extended period of time. In some variations, the liquid formulation is used to prevent transition of dry AMD to wet AMD for an extended period of time. In one example, the liquid formulation delivers the rapamycin to the vitreous, sclera, retina, choroid, macula, or other tissues of a human subject in an amount sufficient to treat, prevent, inhibit, delay onset of, or cause regression of wet age-related macular degeneration for at least about three, about six, about nine, or about twelve months. In some variations, the level of rapamycin is sufficient to treat AMD. In some variations, the level of rapamycin is sufficient to prevent onset of wet AMD.

Other extended periods of release are described in the Detailed Description.

Described herein is a liquid formulation comprising a therapeutic agent, wherein the liquid formulation contains less than about 90% (w/w) of any material having a hygroscopicity that is about equal to or greater than that of polyethylene glycol (PEG 400). The liquid formulation may contain less than about 80%, less than about 70%, less than about 60%, less than about 50%, or less than about 40% of the material having a hygroscopicity that is about equal to or greater than that of polyethylene glycol (PEG 400). The material may be a solvent. Methods are provided for treating an ocular disease in a human subject comprising administering to the subject by intraocular or periocular delivery a volume of the liquid formulation containing an amount of the therapeutic agent effective to treat an ocular disease in the subject. Methods for preventing an ocular disease in a human subject comprising administering to the subject by intraocular or periocular delivery a volume of the liquid formulation containing an effective amount of the therapeutic agent are also described herein.

Also described herein is a formulation for use in a method of treating an ocular disease in a human subject comprising administering to the human subject a volume of a liquid formulation by placement between the sclera and conjunctiva of the subject, wherein the liquid formulation comprises: (a) an effective amount of a therapeutic agent; and (b) a material selected from the group consisting of polyethylene glycol 400 (PEG 400), a material about as hygroscopic as PEG 400, or a material more hygroscopic than PEG 400. The liquid formulation may comprise less than about 80 µl, less than about 60 µl, or less than about 40 µl of such a material. In some variations, the material is a solvent.

Also described herein is a formulation for use in a method of preventing an ocular disease in a human subject comprising administering to the human subject a volume of a liquid formulation by placement between the sclera and conjunctiva of the subject, wherein the liquid formulation comprises: (a) an effective amount of a therapeutic agent; and (b) a material selected from the group consisting of polyethylene glycol 400 (PEG 400), a material about as hygroscopic as PEG 400, or a material more hygroscopic than PEG 400. The liquid formulation may comprise less than about 80 µl, less than about 60 µl, or less than about 40 µl of such a material. In some variations, the material is a solvent.

Further described herein is a formulation for use in method of treating an ocular disease in a human subject, comprising administering periocularly to the subject a volume of formulation comprising an amount of therapeutic agent and a volume of an excipient, wherein the amount of therapeutic agent is sufficient to treat the ocular disease, and wherein the excipient is PEG 400 or a material having a hygroscopicity about equal to or less than PEG 400 and the volume of the excipient is less than or equal to about 100 µl. The volume of the excipient may be less than or equal to about 80 µl, less than or equal to about 60 µl, or less than or equal to about 40 µl. The volume of formulation may be administered between the sclera and conjunctiva of the subject. The ocular disease may be dry age-related macular degeneration, wet age-related macular degeneration or macular edema. The excipient may be a solvent. The excipient is for example PEG 400.

Further described herein is a formulation for use in a method of preventing an ocular disease in a human subject, comprising administering periocularly to the subject a volume of formulation comprising an amount of therapeutic agent and a volume of an excipient, wherein the amount of therapeutic agent is sufficient to prevent the ocular disease, and wherein the excipient is PEG 400 or a material having a hygroscopicity about equal to or less than PEG 400 and the volume of the excipient is less than or equal to about 100 µl. In some variations, the volume of the excipient is less than or equal to about 80 µl, less than or equal to about 60 µl, or less than or equal to about 40 µl. The volume of formulation may be administered between the sclera and conjunctiva of the subject. The ocular disease is for example dry age-related macular degeneration, wet age-related macular degeneration or macular edema. The excipient may be a solvent. The excipient is for example PEG 400.

Described herein is also a liquid formulation comprising a therapeutic agent, water, and one or more excipients selected from the group consisting of N-methyl pyrrolidone (NMP), dimethyl acetamine (DMA), dimethyl sulfoxide (DMSO), propylene glycol (PG), polyethylene glycol 600 (PEG 600), and polyethylene glycol 400. In some examples, the excipient is a solvent. The formulation may for example comprise at least about five percent (w/w) of water. In addition to the therapeutic agent and water, the formulation may for example comprise both (i) an excipient selected from the group consisting of N-methyl pyrrolidone (NMP), dimethyl acetamine (DMA), and dimethyl sulfoxide (DMSO); and (ii) an excipient selected from the group consisting of propylene glycol (PG), polyethylene glycol 600 (PEG 600), and polyethylene glycol 400. The formulation may comprise up to about five percent (w/w) therapeutic agent. The formulation may further comprise ethanol. Methods are provided for treating an ocular disease in a human subject comprising administering to the subject by intraocular or periocular delivery a volume of the liquid formulation containing an amount of the therapeutic agent effective to treat the ocular disease in the subject. Formulations are provided for use in methods for preventing an ocular disease in a human subject comprising administering to the subject by intraocular or periocular delivery a volume of the liquid formulation containing an effective amount of the therapeutic agent are also described herein.

In some examples of each of the aforementioned formulations, as well as other formulations described herein, the liquid formulation, when injected between the sclera and conjunctiva of a rabbit eye delivers an amount of the therapeutic agent sufficient to achieve, for a period of time of at least 30 days following administration of the liquid formulation, one or both of the following: (a) an average concentration of therapeutic agent in the vitreous of the rabbit eye equivalent to a rapamycin concentration of at least 0.01 ng/ml; or (b) an average concentration of therapeutic agent in the retina choroid tissues of the rabbit eye equivalent to a rapamycin concentration of at least 0.001 ng/mg.

In some examples of each of the aforementioned formulations, as well as other formulations described herein, the liquid formulation, when injected into the vitreous of a rabbit eye delivers an amount of the therapeutic agent sufficient to achieve, for a period of time of at least 30 days following administration of the liquid formulation, one or both of the following: (a) an average concentration of therapeutic agent in the retina choroid tissues of the rabbit eye equivalent to a rapamycin concentration of at least 0.01 ng/mg; or (b) an average concentration of therapeutic agent in the vitreous of the rabbit eye equivalent to a rapamycin concentration of at least 1000 ng/ml.

Described herein is a liquid formulation comprising a therapeutic agent, wherein the liquid formulation when administered by subtenon injection to a rabbit eye delivers an amount of the therapeutic agent sufficient to achieve, for a period of time of at least 30 days following administration of the liquid formulation, one or both of the following: (a) an average concentration of therapeutic agent in the vitreous of the rabbit eye equivalent to a rapamycin concentration of at least 0.01 ng/ml; or (b) an average concentration of therapeutic agent in the retina choroid tissues of the rabbit eye equivalent to a rapamycin concentration of at least 0.001 ng/mg. Formulations are provided for use in methods of treating an ocular disease in a human subject comprising administering to the subject by intraocular or periocular delivery a volume of the liquid formulation containing an amount of the therapeutic agent effective to treat an ocular disease in the subject. Formulations for use in methods of preventing an ocular disease in a human subject comprising administering to the subject by intraocular or periocular delivery a volume of the liquid formulation containing an effective amount of the therapeutic agent are also described herein.

The liquid formulation described herein is for example administered by intravitreal, subconjunctival, topical, or subtenon administration.

Also described herein is a liquid formulation comprising: (a) a therapeutic agent; (b) a first component selected from the group consisting of ethoxlyated p-tert-octylphenol formaldehyde polymer, polyoxyl 35 castor oil, propylene glycol monolaurate, propylene glycol dicaprylate/dicaprate, and macrogol 15 hydroxystearate; and (c) a second component comprising monoglycerides and/or diglycerides of caprylate. The formulation may further comprise ethanol and/or water. Formultions for use in methods of treating an ocular disease in a human subject, comprising administering to the subject by topical delivery a volume of the liquid formulation containing an amount of the therapeutic agent effective to treat the ocular disease in the human subject are also described. Formulations for use in methods of preventing an ocular disease in a human subject, comprising administering to the subject by topical delivery a volume of the liquid formulation containing an amount of the therapeutic agent effective to prevent the ocular disease in the human subject are also described. The liquid formulation may be applied topically to an eye of the subject about once or less a day, about once or less every 5 days, or about once or less every 10 days. As described herein, the methods may be methods of treatment. The disease to be treated is for example a disease of the retina. Also disclosed is that the disease is AMD or macular edema.

In addition, described herein is a formulation for use in a method of treating an ocular disease in a human subject, comprising administering to the human subject by topical delivery a volume of a liquid formulation containing an amount of a therapeutic agent effective to treat the ocular disease in the human subject, wherein the liquid formulation when topically applied to a rabbit eye in a volume of 80 µl a day for six days delivers an amount of the therapeutic agent sufficient to achieve: (a) an average concentration of the therapeutic agent in the cornea of the rabbit eye at day 6 equivalent to a rapamycin concentration of at least 0.01 ng/mg; or (b) an average concentration of therapeutic agent in the retina choroid tissues of the rabbit eye at day 6 equivalent to a rapamycin concentration of at least 0.001 ng/mg. The liquid formulation may be applied topically to an eye of the subject about once or less a day, about once or less every 5 days, or about once or less every 10 days. It is described herein that the method may be a method of treatment. The liquid formulation may consist essentially of about 2% (w/w) rapamycin, about 4% (w/w) ethanol, and about 94% (w/w) PEG 400.

In addition, described herein is a formulation for use in a method of preventing an ocular disease in a human subject, comprising administering to the human subject by topical delivery a volume of a liquid formulation containing an amount of a therapeutic agent effective to prevent the ocular disease in the human subject, wherein the liquid formulation when topically applied to a rabbit eye in a volume of 80 µl a day for six days delivers an amount of the therapeutic agent sufficient to achieve: (a) an average concentration of the therapeutic agent in the cornea of the rabbit eye at day 6 equivalent to a rapamycin concentration of at least 0.01 ng/mg; or (b) an average concentration of therapeutic agent in the retina choroid tissues of the rabbit eye at day 6 equivalent to a rapamycin concentration of at least 0.001 ng/mg. As exemplified, the liquid formulation may be applied topically to an eye of the subject about once or less a day, about once or less every 5 days, or about once or less every 10 days.. The liquid formulation may consist essentially of about 2% (w/w) rapamycin, about 4% (w/w) ethanol, and about 94% (w/w) PEG 400.

Further described herein is a liquid formulation comprising a therapeutic agent and water, wherein the liquid formulation when applied to a rabbit eye in a volume of 80µl a day for six days delivers an amount of the therapeutic agent sufficient to achieve: (a) an average concentration of the therapeutic agent in the cornea of the rabbit eye at day 6 equivalent to a rapamycin concentration of at least 0.01 ng/mg; or (b) an average concentration of therapeutic agent in the retina choroid tissues of the rabbit eye at day 6 equivalent to a rapamycin concentration of at least 0.001 ng/mg. Formulations for use in methods of treating an ocular disease in a human subject, comprising administering to the subject by topical delivery a volume of the liquid formulation containing an amount of the therapeutic agent effective to treat the ocular disease in the human subject are also described. Formulations for use in methods of preventing an ocular disease in a human subject, comprising administering to the subject by topical delivery a volume of the liquid formulation containing an amount of the therapeutic agent effective to prevent the ocular disease in the human subject are also described. The liquid formulation may for example be applied topically to an eye of the subject about once or less a day, about once or less every 5 days, or about once or less every 10 days. As described herein, the methods may be methods of treatment.

Also described herein is a formulation for use in a method of treating an ocular disease in a human subject, comprising topically administering to the subject a liquid formulation comprising a therapeutic agent, wherein the liquid formulation, when a volume of about 40µl is applied topically to a rabbit eye, delivers an amount of the therapeutic agent sufficient to achieve: (a) an average concentration of the therapeutic agent in the cornea of the rabbit eye equivalent to an average rapamycin concentration of at least 0.01 ng/mg for at least about ten days after administration of the liquid formulation; or (b) an average concentration of therapeutic agent in the retina choroid tissues of the rabbit eye equivalent to an average rapamycin concentration of at least 0.001 ng/mg for at least about ten days after administration of the liquid formulation. The liquid formulation may for example further comprise polyethylene glycol. The liquid formulation may be applied topically to an eye of the subject about once or less a day, about once or less every 5 days, or about once or less every 10 days. Described herein is that the methods may be methods of treatment. The liquid formulation may consist essentially of about 2% (w/w) rapamycin, about 4% (w/w) ethanol, and about 94% (w/w) PEG 400.

Also described herein is a formulations for use in a method of preventing an ocular disease in a human subject, comprising topically administering to the subject a liquid formulation comprising a therapeutic agent, wherein the liquid formulation, when a volume of about 40µl is applied topically to a rabbit eye, delivers an amount of the therapeutic agent sufficient to achieve: (a) an average concentration of the therapeutic agent in the cornea of the rabbit eye equivalent to an average rapamycin concentration of at least 0.01 ng/mg for at least about ten days after administration of the liquid formulation; or (b) an average concentration of therapeutic agent in the retina choroid tissues of the rabbit eye equivalent to an average rapamycin concentration of at least 0.001 ng/mg for at least about ten days after administration of the liquid formulation. The liquid formulation may further comprise polyethylene glycol. The liquid formulation may be applied topically to an eye of the subject about once or less a day, about once or less every 5 days, or about once or less every 10 days. The liquid formulation may consist essentially of about 2% (w/w) rapamycin, about 4% (w/w) ethanol, and about 94% (w/w) PEG 400.

In the aforementioned formulations, as well as other formulations described herein, the therapeutic agent may for example be rapamycin.

In formulations described herein, the therapeutic agent may be rapamycin, or a pharmaceutically acceptable salt or ester thereof.

Further described herein is a liquid formulation consisting essentially of about 2% (w/w) rapamycin, about 4% (w/w) ethanol, and about 94% (w/w) PEG 400, as well as a liquid formulation consisting essentially of about 4% (w/w) rapamycin, about 4% (w/w) ethanol, and about 92% (w/w) PEG 400. A liquid formulation consisting essentially of about 1.47 % (w/w) rapamycin, about 2.93 % (w/w) ethanol, about 26.6 % (w/w) normal saline, and about 69 % (w/w) PEG 400 is also described.

Methods of using such formulations in the treatment of an ocular disease are also described. Uses of such formulations in methods of preventing an ocular disease are also described. The uses may comprise administering the liquid formulation by intravitreal, subconjunctival, topical, or subtenon administration.

A formulation for use in a method of treating an ocular disease in a human subject, comprising administering to the human subject a volume of a liquid formulation by placement in the vitreous of the subject, wherein the volume of the liquid formulation comprises between about 20 µg and about 750 µg of rapamycin, or a pharmaceutically acceptable salt or ester thereof, is also described herein. For example, the volume of the liquid formulation maybe about 30 µl or less, about 20 µl or less, or about 10 µl or less. In certain examples the volume of the liquid formulation contains between about 30 µg and about 200 µg of rapamycin. The liquid formulation, in some examples, consists essentially of about 2% (w/w) rapamycin, about 4% (w/w) ethanol, and about 94% (w/w) PEG 400.

A formulation for use in a method of preventing an ocular disease in a human subject, comprising administering to the human subject a volume of a liquid formulation by placement in the vitreous of the subject, wherein the volume of the liquid formulation comprises between about 20 µg and about 750 µg of rapamycin, or a pharmaceutically acceptable salt or ester thereof, is also described herein. The volume of the liquid formulation may for example be about 30 µl or less, about 20 µl or less, or about 10 µl or less. In certain examples, the volume of the liquid formulation contains between about 30 µg and about 200 µg of rapamycin. The liquid formulation may consist essentially of about 2% (w/w) rapamycin, about 4% (w/w) ethanol, and about 94% (w/w) PEG 400.

In addition, described herein is formulation for use in a method of treating an ocular disease in a human subject, comprising administering to the human subject a volume of a liquid formulation by placement between the sclera and conjunctiva of the subject, wherein the liquid formulation comprises between about 50 µg and about 3 mg of rapamycin, or a pharmaceutically acceptable salt or ester thereof. In some examples, the volume of the liquid formulation is about 150 µl or less, about 100 µl or less, or about 40 µl or less. In some examples, the volume of the liquid formulation comprises between about 300 µg and about 1000 µg of rapamycin. In some examples, the amount of rapamycin in the volume of the formulation that is administered is about 400 µg to about 900 µg, about 500 µg to about 800 µg, or about 600 µg to about 700 µg of rapamycin. The volume of the formulation that is administered to the subject may for example be about 10 µl to about 50 µl, about 15 µl to about 45 µl, about 20 µl to about 40 µl, or about 25 µl to about 35 µl. In some variations, the volume of the formulation that is administered comprises about 440 µg of rapamycin in about 20 µl. In some variations, the volume of the formulation that is administered comprises about 660 µg of rapamycin in about 30 µl. In some variations, the volume of the formulation that is administered comprises about 880 µg of rapamycin in about 40 µl. The liquid formulation, in some variations, consists essentially of about 2% (w/w) rapamycin, about 4% (w/w) ethanol, and about 94% (w/w) PEG 400. In some examples, the liquid formulation comprises less than about 40 µl of polyethylene glycol. In some alternative examples, the formulation comprises less than about 80 µl of polyethylene glycol (including PEG 400), less than about 60 µl of polyethylene glycol, or less than about 40 µl of polyethylene glycol. In some examples, the formulation comprises no polyethylene glycol.

In addition, described herein is formulation for use in a method of preventing an ocular disease in a human subject, comprising administering to the human subject a volume of a liquid formulation by placement between the sclera and conjunctiva of the subject, wherein the liquid formulation comprises between about 50 µg and about 3 mg of rapamycin, or a pharmaceutically acceptable salt or ester thereof. In some examples, the volume of the liquid formulation is about 150 µl or less, about 100 µl or less, or about 40 µl or less. In some examples, the volume of the liquid formulation comprises between about 300 µg and about 1000 µg of rapamycin. In some examples, the amount of rapamycin in the volume of the formulation that is administered is about 400 µg to about 900 µg, about 500 µg to about 800 µg, or about 600 µg to about 700 µg of rapamycin. In some examples, the volume of the formulation that is administered to the subject is about 10 µl to about 50 µl, about 15 µl to about 45 µl, about 20 µl to about 40 µl, or about 25 µl to about 35 µl. In some examples, the volume of the formulation that is administered comprises about 440 µg of rapamycin in about 20 µl. In some examples, the volume of the formulation that is administered comprises about 660 µg of rapamycin in about 30 µl. In some examples, the volume of the formulation that is administered comprises about 880 µg of rapamycin in about 40 µl. The liquid formulation, in some examples, consists essentially of about 2% (w/w) rapamycin, about 4% (w/w) ethanol, and about 94% (w/w) PEG 400. In some examples, the liquid formulation comprises less than about 40 µl of polyethylene glycol. In some alternative examples, the formulation comprises less than about 80 µl of polyethylene glycol (including PEG 400), less than about 60 µl of polyethylene glycol, or less than about 40 µl of polyethylene glycol. In some examples, the formulation comprises no polyethylene glycol.

Further described herein is formulation for use in a method of treating an ocular disease in a human subject, comprising administering by subtenon injection to the subject a volume of a liquid formulation comprising between about 50 µg and about 3 mg of a therapeutic agent. In some variations, the therapeutic agent is selected from the group consisting of rapamycin or dasatinib, or a pharmaceutically acceptable salt or ester of either agent. In some variations, the liquid formulation comprises greater than about 30 µl, greater than about 60 µl, or greater than about 80 µl of polyethylene glycol 400 (PEG 400), or a material having a hygroscopicity equal to or greater than PEG 400. In some variations, the material is a solvent. In some variations, the liquid formulation consists essentially of about 2% (w/w) rapamycin, about 4% (w/w) ethanol, and about 94% (w/w) PEG 400.

Further described herein is a formulation for use in a method of preventing an ocular disease in a human subject, comprising administering by subtenon injection to the subject a volume of a liquid formulation comprising between about 50 µg and about 3 mg of a therapeutic agent. In some examples, the therapeutic agent is selected from the group consisting of rapamycin or dasatinib, or a pharmaceutically acceptable salt or ester of either agent. In some examples, the liquid formulation comprises greater than about 30 µl, greater than about 60 µl, or greater than about 80 µl of polyethylene glycol 400 (PEG 400), or a material having a hygroscopicity equal to or greater than PEG 400. In some examples, the material is a solvent. In some examples, the liquid formulation consists essentially of about 2% (w/w) rapamycin, about 4% (w/w) ethanol, and about 94% (w/w) PEG 400.

Also described herein are a formulations for use in a method of treating an ocular disease in a human subject comprising: (a) administering to the human subject a volume of a first liquid formulation by placement in the vitreous of the subject, wherein the first liquid formulation comprises a first therapeutic agent; and (b) administering to the human subject a volume of a second liquid formulation by placement between the sclera and conjunctiva of the subject, wherein the second liquid formulation comprises rapamycin, or a pharmaceutically acceptable salt or ester thereof; wherein an amount of the first and second therapeutic agents effective to treat the ocular disease is administered to the subject. In some variations, step (b) is subsequent to step (a). In some variations, the first therapeutic agent is rapamycin, or a pharmaceutically acceptable salt or ester thereof. The first liquid formulation may be identical to the second liquid formulation or different from the second liquid formulation. As an examples disclosed herein, the first therapeutic agent is selected from the group consisting of ranibizumab and bevacizumab. In some variations, both step (a) and step (b) are performed during a single administration session (such as a single visit with a physician or other medical professional or other single session in which the administrations are performed by the subject, a physician, or other medical professional). In some variations, the first and/or second liquid formulation is a liquid formulation consisting essentially of about 2% (w/w) rapamycin, about 4% (w/w) ethanol, and about 94% (w/w) PEG 400.

Also described herein are formulations for use in a method of preventing an ocular disease in a human subject comprising: (a) administering to the human subject a volume of a first liquid formulation by placement in the vitreous of the subject, wherein the first liquid formulation comprises a first therapeutic agent; and (b) administering to the human subject a volume of a second liquid formulation by placement between the sclera and conjunctiva of the subject, wherein the second liquid formulation comprises rapamycin, or a pharmaceutically acceptable salt or ester thereof; wherein an amount of the first and second therapeutic agents effective to prevent the ocular disease is administered to the subject. In some examples, step (b) is subsequent to step (a). In some examples, the first therapeutic agent is rapamycin, or a pharmaceutically acceptable salt or ester thereof. The first liquid formulation may be identical to the second liquid formulation or different from the second liquid formulation. In some examples, the first therapeutic agent is selected from the group consisting of ranibizumab and bevacizumab. In some examples, both step (a) and step (b) are performed during a single administration session (including a single visit with a physician or other medical professional or other single session in which the administrations are performed by the subject, a physician, or other medical professional). In some, the first and/or second liquid formulation is a liquid formulation consisting essentially of about 2% (w/w) rapamycin, about 4% (w/w) ethanol, and about 94% (w/w) PEG 400.

In some variations of each of the aforementioned formulations, as well as other formulations described herein, the liquid formulation comprises between about 0.001% (w/w) to about 10% (w/w) therapeutic agent. In some variations, the formulation comprises between about 0.001 % (w/w) to about 1.0% (w/w) the therapeutic agent. In some variations, the formulation comprises between about 0.01% (w/w) to about 0.1 % (w/w) therapeutic agent. In further variations, the formulation comprises between about 0.1 % (w/w) to about 6 % (w/w) therapeutic agent. In some variations, the formulation contains up to about 5% rapamycin.

Further described herein are formulations for use in a method of treating an ocular disease in a human subject, comprising: (a) administering to the human subject a volume of a first therapeutic agent; and (b) administering to the human subject by intraocular or periocular delivery a volume of a liquid formulation (including, for example, by intravitreal, subconjunctival, topical, or subtenon administration), wherein the liquid formulation consists essentially of about 2% (w/w) rapamycin, about 4% (w/w) ethanol, and about 94% (w/w) PEG 400, and wherein an amount of the first therapeutic agent and rapamycin effective to treat the ocular disease is administered to the subject. The first therapeutic agent may for examples be selected from the group consisting of ranibizumab, bevacizumab, and verteporfin. The first therapeutic agent may, in some variations, be administered to the subject by intraocular delivery.

Further described herein is are formulations for use in a method of preventing an ocular disease in a human subject, comprising: (a) administering to the human subject a volume of a first therapeutic agent; and (b) administering to the human subject by intraocular or periocular delivery a volume of a liquid formulation (e.g., by intravitreal, subconjunctival, topical, or subtenon administration), wherein the liquid formulation consists essentially of about 2% (w/w) rapamycin, about 4% (w/w) ethanol, and about 94% (w/w) PEG 400, and wherein an amount of the first therapeutic agent and rapamycin effective to prevent the ocular disease is administered to the subject. In some examples, the first therapeutic agent is selected from the group consisting of ranibizumab, bevacizumab, and verteporfin. The first therapeutic agent may, in some examples, be administered to the subject by intraocular delivery.

Also described herein is a formulation for use in a method of treating an ocular disease in a human subject,: (a) diluting a liquid formulation consisting essentially of about 2% (w/w) rapamycin, about 4% (w/w) ethanol, and about 94% (w/w) PEG 400 with aqueous liquid to produce a diluted liquid formulation; and (b) administering to the human subject by intraocular or periocular delivery a volume of the diluted liquid formulation, wherein an amount of rapamycin effective to treat the ocular disease is administered to the subject. In some variations, the formulation is a clear solution. In some variations, the dilution is up to about a 1.4-fold dilution. In some variations, the liquid formulation is diluted up to about 1.2 fold. In some variations, the aqueous liquid is saline. In some variations, the diluted liquid formulation is administered by intravitreal, subconjunctival, topical, or subtenon administration.

Also described herein is a formulation for use in a method of preventing an ocular disease in a human subject, comprising: (a) diluting a liquid formulation consisting essentially of about 2% (w/w) rapamycin, about 4% (w/w) ethanol, and about 94% (w/w) PEG 400 with aqueous liquid to produce a diluted liquid formulation; and (b) administering to the human subject by intraocular or periocular delivery a volume of the diluted liquid formulation, wherein an amount of rapamycin effective to prevent the ocular disease is administered to the subject. In some examples, the formulation is a clear solution. In some examples, the dilution is up to about a 1.4-fold dilution. In some examples, the liquid formulation is diluted up to about 1.2 fold. In some examples, the aqueous liquid is saline. In some examples, the diluted liquid formulation is administered by intravitreal, subconjunctival, topical, or subtenon administration.

In formulations described herein, the ocular disease is a disease of the retina. In some variations, the ocular disease is wet age-related macular degeneration. In some variations, the ocular disease is dry age-related macular degeneration. Also described us that the ocular disease is macular edema (e.g., diabetic macular edema).

The present disclosure describes formulations for use in methods of treating macular edema in a human subject by repeated administration of rapamycin, comprising: administering two or more doses of a rapamycin formulation to an eye of the human subject, wherein the period between consecutive doses is at least 8 weeks, and the cumulative amount of rapamycin in the two or more doses is effective to treat macular edema in the human subject for an extended period of at least 16 weeks. The period between consecutive doses may be at least 12 weeks, and the cumulative amount of rapamycin in the two or more doses is effective to treat macular edema in the human subject for an extended period of at least 24 weeks. The two or more doses of the rapamycin formulation may be administered by subconjunctival placement. The rapamycin formulation may be a solution of rapamycin dissolved in a solvent system. The solvent system may comprise polyethylene glycol. The solvent system may further comprise ethanol. The rapamycin formulation may comprise a suspension of particles of rapamycin.

The present disclosure also describes a formulation for use in methods of treating diabetic macular edema in a human subject by repeated administration of rapamycin, the comprising: administering two or more doses of a rapamycin formulation to an eye of the human subject, wherein the rapamycin formulation is a solution comprising rapamycin and polyethylene glycol, the rapamycin formulation is administered by placement of each dose between a sclera and a conjunctiva of the eye of the subject, the period between consecutive doses is at least 8 weeks, and wherein the cumulative amount of rapamycin in the two or more doses is effective to treat diabetic macular edema in the human subject for an extended period of at least 16 weeks. The period between consecutive doses may for example be at least 12 weeks and the cumulative amount of rapamycin in the two or more doses is effective to treat diabetic macular edema in the human subject for an extended period of at least 24 weeks. The rapamycin formulation may for example further comprise ethanol. In a subset of these examples, each dose of the rapamycin formulation may contain between about 200 µg and about 2,000 µg of rapamycin, while in some specific examples, each dose of the rapamycin formulation contains about 220 µg, about 440 µg, about 880 µg, or about 1320 µg of rapamycin. In specific examples, the rapamycin formulation comprises: (a) about 2% (w/w) rapamycin, about 4% (w/w) ethanol, and about 94% (w/w) polyethylene glycol 400 (PEG 400); (b) about 4% (w/w) rapamycin, about 4% (w/w) ethanol, and about 92% (w/w) polyethylene glycol 400 (PEG 400); or (c) about 1% (w/w) rapamycin, about 4% (w/w) ethanol, and about 95% (w/w) polyethylene glycol 400 (PEG 400). In some examples, the rapamycin formulation consists essentially of: (a) about 2% (w/w) rapamycin, about 4% (w/w) ethanol, and about 94% (w/w) polyethylene glycol 400 (PEG 400); (b) about 4% (w/w) rapamycin, about 4% (w/w) ethanol, and about 92% (w/w) polyethylene glycol 400 (PEG 400); or (c) about 1% (w/w) rapamycin, about 4% (w/w) ethanol, and about 95% (w/w) polyethylene glycol 400 (PEG 400).

Moreover, the present disclosure provides liquid formulations comprising: (a) between about 0.001 % (w/w) to about 1.0% (w/w) rapamycin; (b) a first component selected from the group consisting of ethoxlyated p-tert-octylphenol formaldehyde polymer, and polyoxyl 35 castor oil; and (c) a second component selected from the group consisting of glycerol caprylate, and glycerol caprate. The liquid formulations may for example further comprise (d) a third component selected from the group consisting of ethanol, phosphatidylcholine in propylene glycol/ethanol carrier, and propylene glycol monolaurate. The liquid formulations may for example further comprise water. The liquid formulations may for example comprise between about 0.01% (w/w) to about 0.1 % (w/w) rapamycin. In addition the present disclosure provides methods for treating an ocular disease in a human subject, the method comprising administering to the human subject by topical delivery to an eye a volume of the liquid formulation containing an amount of rapamycin effective to treat the ocular disease in the human subject. The ocular disease may for example be selected from the group consisting of age-related macular degeneration, macular edema, dry eye, allergic conjunctivitis, scleritis, uveitis, and corneal transplant. In a subset of these examples, the age-related macular degeneration is wet age-related macular degeneration. The period of time between consecutive administrations of the liquid formulation may for example be at least about 1 day, while in others the period of time between consecutive administrations of the liquid formulation is at least about 7 days or at least about 14 days.

In addition the present disclosure provides formulation for use in methods of treating an ocular disease in a human subject, comprising administering to the human subject by topical delivery a volume of a liquid formulation containing an amount of a therapeutic agent effective to prevent or treat the ocular disease in the human subject, wherein the liquid formulation when topically applied to a rabbit eye in a volume of at least about 40 µl a day for six days delivers an amount of the therapeutic agent sufficient to achieve one or more of: (a) an average concentration of the therapeutic agent in the cornea of the rabbit eye at day 8 equivalent to a rapamycin concentration of at least 0.01 ng/g; (b) an average concentration of the therapeutic agent in the retina choroid of the rabbit eye at day 8 equivalent to a rapamycin concentration of at least 0.01 ng/g; and (c) an average concentration of the therapeutic agent in the sclera of the rabbit eye at day 8 equivalent to a rapamycin concentration of at least 0.01 ng/g. The liquid formulation when topically applied may achieve one or more of (a), (b), and (c), and further achieve: (d) an average concentration of therapeutic agent in the blood of the rabbit at day 8 less than the average concentration of the therapeutic agent in one or more of the cornea, the retina choroid and the sclera. The therapeutic agent may for example be rapamycin, or a pharmaceutically acceptable salt or ester thereof, while in others the therapeutic agent is dasatinib, or a pharmaceutically acceptable salt or ester thereof. In some specific examples, the ocular disease is selected from the group consisting of age-related macular degeneration, macular edema, dry eye, allergic conjunctivitis, scleritis, uveitis, and corneal transplant. In some specific examples, the age-related macular degeneration is wet age-related macular degeneration. In some examples, the period of time between consecutive administrations of the liquid formulation is at least about 1 day, while in others the period of time between consecutive administrations of the liquid formulation is at least about 7 days, or at least about 14 days. In some specific examples, the liquid formulation consists essentially of (a) about 2% (w/w) rapamycin, about 4% (w/w) ethanol, and about 94% (w/w) PEG 400; (b) about 4% (w/w) rapamycin, about 4% (w/w) ethanol, and about 92% (w/w) PEG 400; or (c) about 1% (w/w) rapamycin, about 4% (w/w) ethanol, and about 95% (w/w) PEG 400.

The present disclosure also provides formulation for use in methods of treating an ocular disease in a human subject, the method comprising topically administering to the subject a liquid formulation comprising a therapeutic agent, wherein the liquid formulation, when a volume of at least about 40µL is applied topically to a rabbit eye, delivers an amount of the therapeutic agent sufficient to achieve one or more of: (a) an average concentration of the therapeutic agent in the cornea of the rabbit eye equivalent to an average rapamycin concentration of at least 0.01 ng/g for at least about seven days after administration of the liquid formulation; (b) an average concentration of therapeutic agent in the retina choroid of the rabbit eye equivalent to an average rapamycin concentration of at least 0.01 ng/g for at least about seven days after administration of the liquid formulation; and (c) an average concentration of the therapeutic agent in the sclera of the rabbit eye equivalent to an average rapamycin concentration of at least 0.01 ng/g for at least about seven days after administration of the liquid formulation. In some examples, the liquid formulation when topically applied achieves one or more of (a), (b), and (c), and further achieves (d) an average concentration of therapeutic agent in the blood of the rabbit at day seven less than the average concentration of the therapeutic agent in one or more of the cornea, the retina choroid and the sclera. In some embodiments, the therapeutic agent is rapamycin or a pharmaceutically acceptable salt or ester thereof. The ocular disease may be selected from the group consisting of age-related macular degeneration, macular edema, dry eye, allergic conjunctivitis, scleritis, uveitis, and corneal transplant. In some particularly preferred embodiments, the age-related macular degeneration is wet age-related macular degeneration. In some embodiments, the liquid formulation comprises between about 0.001 % (w/w) to about 10% (w/w) rapamycin. In a subset of these embodiments, the liquid formulation comprises between about 0.01% (w/w) to about 1.0% (w/w) rapamycin. In some embodiments, the liquid formulation further comprises polyethylene glycol. In some specific examples, the period of time between consecutive administrations of the liquid formulation is at least about 1 day, while in others the period of time between consecutive administrations of the liquid formulation is at least about 7 days or at least about 14 days.

Moreover the present description provides formulation for use in methods of administration by a periocular route a liquid formulation to an eye of a subject without causing chemosis of the eye, comprising: administering less than 40 µl of the liquid formulation between a sclera and a conjunctiva of the eye of the subject, wherein the liquid formulation comprises a therapeutic agent and a solvent, and wherein the liquid formulation has a hygroscopicity that is 80% or greater than the hygroscopicity of a liquid formulation consisting essentially of about 2% (w/w) rapamycin, about 4% (w/w) ethanol, and about 94% (w/w) polyethylene glycol 400 (PEG 400). As used herein, the term "chemosis" refers to edema of the conjunctiva of the eye, forming a swelling partly or fully around the iris of the eye. In some embodiments, the therapeutic agent is rapamycin. In some embodiments, the solvent comprises polyethylene glycol, while in a subset of these embodiments, the polyethylene glycol is polyethylene glycol 400 (PEG 400). About 1 µg to about 5,000 µg rapamycin may be administered to the subject. In a subset of these embodiments, about 220 µg, about 440 µg, about 880 µg, or about 1320 µg rapamycin is administered to the subject. Also disclosed are formulations for use in methods of treating an ocular disease in a human subject, wherein the ocular disease is selected from the group consisting of age-related macular degeneration, macular edema and dry eye, the method comprising administering to the subject, an amount of the therapeutic agent effective to treat the ocular disease in the human subject. In some embodiments, the ocular disease is age-related macular degeneration, which in preferred embodiments is wet age-related macular degeneration. The ocular disease may be macular edema, which in specific examples is diabetic macular edema. In specific examples, the ocular disease is dry eye.

In addition, the present disclosure provides formulations for use in methods of treating an ocular disease in a human subject, the method comprising: (a) administering to the human subject a volume of a first formulation by intraocular or periocular delivery to the subject, wherein the volume of the first formulation comprises an amount of ranibizumab or bevacizumab; and (b) administering to the human subject a volume of a second formulation by periocular delivery to the subject, wherein the volume of the second formulation comprises an amount of rapamycin, or a pharmaceutically acceptable salt or ester thereof; wherein the amount of ranibizumab or bevacizumab and the amount of rapamycin or a pharmaceutically acceptable salt or ester thereof, together are effective to treat the ocular disease when administered to the human subject. The first formulation may be administered by intravitreal placement, and the second formulation may be administered by subconjunctival placement. In specific examples, step (a) is coincident with step (b) (same doctor visit or occuring on the same day). In other examples, step (b) is subsequent to step (a) (different doctor visits or occuring on different days). The ocular disease may be age-related macular degeneration, or macular edema. In specific examples, the age-related macular degeneration is wet age-related macular degeneration, or the macular edema is diabetic macular edema. The volume of the first formulation may for example contain between about 100 µg and about 500 µg of ranibizumab or bevacizumab. The volume of the second formulation may as another example contain between about 200 µg and about 2000 µg of rapamycin.

The present disclosure also provides formulations for use in methods of treating an ocular disease in a human subject, comprising: (a) administering to the human subject by intraocular or periocular delivery a volume of a first formulation comprising an amount of a first therapeutic agent; and (b) administering to the human subject by periocular delivery a volume of a second formulation comprising an amount of rapamycin, wherein the second formulation consists essentially of rapamycin, ethanol, and polyethylene glycol 400 (PEG 400), and wherein the amount of the first therapeutic agent and the amount of rapamycin together are effective to treat the ocular disease when administered to the human subject. The first therapeutic agent may for example be selected from the group consisting of ranibizumab, bevacizumab, and verteporfin. The second formulation may for example consist essentially of: (a) about 2% (w/w) rapamycin, about 4% (w/w) ethanol, and about 94% (w/w) PEG 400; (b) about 4% (w/w) rapamycin, about 4% (w/w) ethanol, and about 92% (w/w) PEG 400; or (c) about 1% (w/w) rapamycin, about 4% (w/w) ethanol, and about 95% (w/w) PEG 400. The administration of the first formulation may be by a route selected from the group consisting of intravitreal, subconjunctival, and subtenon placement, and the administration of the second formulation by subconjunctival or subtenon placement. In some instances, step (a) is coincident with step (b) (same doctor visit or occuring on the same day). In other instances, step (b) is subsequent to step (a) (different doctor visits or occuring on different days). The ocular disease may be age-related macular degeneration or macular edema. In some specific examples, the age-related macular degeneration is wet age-related macular degeneration. In some instances, the ocular disease is macular edema. In some specific examples, the macular edema is diabetic macular edema.

Furthermore the present disclosure provides formulations for use in methods of treating wet age-related macular degeneration or diabetic macular edema in a human subject, the method comprising: (a) administering to the human subject a volume of a first formulation by intravitreal placement, wherein the volume of the first formulation comprises an amount of ranibizumab or bevacizumab; and (b) administering to the human subject a volume of a second formulation by subconjunctival placement, wherein the volume of the second formulation comprises an amount of rapamycin, or a pharmaceutically acceptable salt or ester thereof; wherein the amount of ranibizumab or bevacizumab and the amount of rapamycin or a pharmaceutically acceptable salt or ester thereof, together are effective to treat wet age-related macular degeneration or diabetic macular edema when administered to the human subject. In some instances, step (a) is coincident with step (b) (same doctor visit or occuring on the same day). In other instances, step (b) is subsequent to step (a) (different doctor visits or occuring on different days). The volume of the first formulation may contain between about 100 µg and about 500 µg of ranibizumab or bevacizumab. The volume of the second formulation may contain between about 200 µg and about 2000 µg of rapamycin. In some instances, the second formulation consists essentially of (a) about 2% (w/w) rapamycin, about 4% (w/w) ethanol, and about 94% (w/w) polyethylene glycol 400 (PEG 400); (b)about 4% (w/w) rapamycin, about 4% (w/w) ethanol, and about 92% (w/w) polyethylene glycol 400 (PEG 400); or (c) about 1% (w/w) rapamycin, about 4% (w/w) ethanol, and about 95% (w/w) polyethylene glycol 400 (PEG 400).

Moreover the present disclosure provides liquid formulations comprising (i) a solvent selected from the group consisting of N-methyl pyrrolidone (NMP), dimethyl acetamine (DMA), and dimethyl sulfoxide (DMSO); (ii) poly(D,L-lactide-co-glycolide) (PLGA), or a solvent selected from the group consisting of propylene glycol (PG), polyethylene glycol 600 (PEG 600), polyethylene glycol 400 (PEG 400); and (iii) a therapeutic agent. The liquid formulation when: (a) injected between the sclera and conjunctiva of a rabbit eye may deliver an amount of the therapeutic agent sufficient to achieve, for a period of time of at least 30 days following administration of the liquid formulation, one or both of the following: (i) an average concentration of therapeutic agent in the vitreous of the rabbit eye equivalent to a rapamycin concentration of at least 0.01 ng/ml; or (ii) an average concentration of therapeutic agent in the retina choroid tissues of the rabbit eye equivalent to a rapamycin concentration of at least 0.01 ng/g; (b) administered by subtenon injection to a rabbit eye delivers an amount of the therapeutic agent sufficient to achieve, for a period of time of at least 30 days following administration of the liquid formulation, one or both of the following: (i) an average concentration of therapeutic agent in the vitreous of the rabbit eye equivalent to a rapamycin concentration of at least 0.01 ng/ml; or (ii) an average concentration of therapeutic agent in the retina choroid tissues of the rabbit eye equivalent to a rapamycin concentration of at least 0.01 ng/g; or (c) injected into the vitreous of a rabbit eye delivers an amount of the therapeutic agent sufficient to achieve, for a period of time of at least 30 days following administration of the liquid formulation, one or both of the following: (a) an average concentration of therapeutic agent in the vitreous of the rabbit eye equivalent to a rapamycin concentration of at least 100 ng/ml; or (b) an average concentration of therapeutic agent in the retina choroid tissues of the rabbit eye equivalent to a rapamycin concentration of at least 0.01 ng/g. The liquid formulation may comprise between about 0.001% (w/w) to about 10% (w/w) of the therapeutic agent. The therapeutic agent may be rapamycin, or a pharmaceutically acceptable salt or ester thereof. The therapeutic agent may also be dasatinib, or a pharmaceutically acceptable salt or ester thereof. In some instances, the liquid formulation consists essentially of (a) about 4% (w/w) rapamycin, about 4% (w/w) ethanol, and about 92% (w/w) PEG 400; or (b) about 1.0-4.0% (w/w) rapamycin, about 2.0-4.0 % (w/w) ethanol, about 20-30 % (w/w) normal saline, and quantity of PEG 400 sufficient for the total formula weight percentage to equal 100%. The present disclosure further provides methods for treating an ocular disease in a human subject, the method comprising administering to the human subject by intraocular or periocular delivery a volume of the liquid formulation containing an amount of the therapeutic agent effective to treat the ocular disease in the human subject. The ocular disease may be age-related macular degeneration, macular edema, or dry eye. In some specific examples, the age-related macular degeneration is wet age-related macular degeneration. In other specific examples, the macular edema is diabetic macular edema. In 1 further specific examples, the ocular disease is dry eye. In particular, the administration of the liquid formulation is by intravitreal, subconjunctival, or subtenon administration.

Additionally, the present description provides formulation for use in methods of treating an ocular disease in a human subject, the method comprising administering to the human subject a volume of a liquid formulation comprising between about 200 µg and about 5000 µg of rapamycin, or a pharmaceutically acceptable salt or ester thereof. In some embodiments, the volume of the liquid formulation contains between about 200 µg and about 1500 µg of rapamycin. In some embodiments, when administration is by placement of the liquid formulation in the vitreous of the subject, the volume of the liquid formulation is about 100 µl or less, preferably about 25 µl or less. In some embodiments, when administration is by placement of the liquid formulation between the sclera and conjunctiva of the subject, the volume of the liquid formulation is about 1,000 µl or less, preferably about 50 µl or less. In some embodiments, when administration is by placement of the liquid formulation in the subtenon space of the subject, the volume of the liquid formulation is about 5,000 µl or less, preferably about 100 µl or less. The ocular disease may be age-related macular degeneration, macular edema, or dry eye. In some preferred embodiments, the age-related macular degeneration is wet age-related macular degeneration. If the ocular disease is macular edema, the macular edema may be diabetic macular edema.

The present disclosure also provides a solid formulation comprising rapamycin, polyvinylpyrrolidone, a phospholipid solution, and polyethylene glycol. In some embodiments, the formulation further comprises one or both of gamma tocopherol and ascorbyl palmatate. Methods are disclosed for treating an ocular disease in a human subject, the method comprising administering to an eye of the human subject by topical or periocular delivery a volume of the solid formulation containing an amount of rapamycin effective to treat the ocular disease in the human subject. The ocular disease may be age-related macular degeneration, macular edema, or dry eye. The amount of rapamycin in the solid formulation may for example be effective to treat the ocular disease in the human subject for an extended period of at least two weeks, at least four weeks, at least six weeks or at least eight weeks.

### BRIEF DESCRIPTION OF THE DRAWINGS

FIGURE 1 depicts the average level of rapamycin in the retina choroid tissue (ng/g) of rabbit eyes at 14, 30, 60, and 90 days after subconjunctival injection of 40 µl of 2% solution of rapamycin in ethanol and PEG 400, 1.47% solution of rapamycin in ethanol and PEG 400, or 1.38% solution of rapamycin in ethanol and PEG 400 or 20 µl of a 4% solution of rapamycin in ethanol and PEG 400.
FIGURE 2 depicts the level of rapamycin in the retina choroid tissue (ng/g) of rabbit eyes at 3, 14, 30, 45, and 60 days after intravitreal injection of 6 µl of 2% solution of rapamycin in ethanol and PEG 400 or 8.5 µl of 1.47% solution of rapamycin in ethanol and PEG 400.
FIGURES 3A-3D depict the concentration of rapamycin present in (A) whole blood (ng/ml), (B) sclera (ng/g), (C) retina choroid (ng/g), and (D) vitreous fluid (ng/mL) at 3, 7, 14, 30, 60, and 90 days after either subconjunctival or subtenous injection of 30 µl of 2% solution of rapamycin in ethanol and PEG 400.
FIGURE 4 depicts the concentration of rapamycin present in tissue or fluid of rabbit eyes after topical administration of one eye drop (about 40 µL) of a 2% rapamycin solution.
FIGURE 5 depicts the concentration of rapamycin present in tissue or fluid of rabbit eyes after topical administration by eye drops of a 0.07% rapamycin solution.
FIGURE 6 depicts the concentration of rapamycin present in eye tissue (ng/g) of rabbit eyes after topical administration by eye drops of a 0.09% rapamycin solution.
FIGURE 7 depicts the concentration of rapamycin present in eye tissue or fluid (ng/g or ng/ml) after topical administration by eye drops of a 0.08% rapamycin solution comprising phosal, capmul, tyloxapol and water.
FIGURE 8 depicts the concentration of rapamycin present in eye tissue or fluid (ng/g or ng/ml) after topical administration by eye drops of a 0.17% rapamycin solution comprising cremophor, capmul, ethanol, and water.
FIGURE 9 depicts the concentration of rapamycin present in eye tissue or blood (ng/g or ng/ml) after topical administration by eye drops of a non-aqueous solution comprising 0.5%, 1.0% or 4.0% rapamycin.
FIGURES 10A-B depict the concentration of rapamycin present in eye tissue or blood (ng/g or ng/ml) after topical administration by eye drops of a non-aqueous solution or an aqueous solution comprising about 0.2% rapamycin.
FIGURE 11 depicts the concentration of rapamycin present in eye tissue or fluid (ng/g or ng/ml) after topical administration by eye drops of a non-aqueous solution comprising about 0.2% rapamycin.
FIGURE 12 depicts the concentration of rapamycin present in eye tissue or fluid (ng/g or ng/ml) after intravitreal administration of liquid formulation comprising about 0.6% rapamycin.
FIGURE 13 depicts the concentration of rapamycin present in eye tissue or fluid (ng/g or ng/ml) after periocular administration of solid formulation comprising about 10% rapamycin.
FIGURE 14 depicts the concentration of dasatanib present in eye tissue or fluid (ng/g or ng/ml) after intravitreal administration of liquid formulation comprising about 5% dasatanib.

### DETAILED DESCRIPTION

Described herein are compositions, liquid formulations and methods relating to delivery of therapeutic agents to a subject, including to a human subject or to the eye of a human subject. These compositions, liquid formulations, and methods may be used for the treatment, prevention, inhibition, delaying onset of, or causing regression of diseases and conditions of the eye including for example diseases or conditions of the posterior segment, including for example choroidal neovascularization; macular degeneration; age-related macular degeneration, including wet AMD and dry AMD; retinal angiogenesis; chronic uveitis; and other retinoproliferative conditions. In some variations, the compositions, liquid formulations, and methods are used for the treatment of the aforementioned diseases or conditions of the eye.

Herein are described (1) the therapeutic agents that may be delivered to a subject, including to a human subject or an eye of a subject using the compositions, liquid formulations, and methods described herein, (2) the diseases and conditions that may be treated, prevented, inhibited, onset delayed, or regression caused by delivery of the therapeutic agents, (3) liquid formulations that may be used to deliver the therapeutic agents, (4) routes of administration for delivery of the liquid formulations, (5) extended delivery of therapeutic agents including for example to rapamycin, and (6) description of the treatment of CNV and wet AMD by delivery of rapamycin to a subject, including to a human subject or to the eye of a subject for an extended period of time using the described compositions and liquid formulations.

The term "about," as used herein, refers to the level of accuracy that is obtained when the methods described herein, such as the methods in the examples, are used. However, by "about" a certain amount of a component of a formulation is meant 90-110% of the amount stated.

### Therapeutic Agents

Examples of therapeutic agents that may be used include compounds that act by binding members of the immunophilin family of cellular proteins. Such compounds are known as "immunophilin binding compounds." Immunophilin binding compounds include the "limus" family of compounds.

An example of a limus compound that may be used is sirolimus (rapamycin).

Therapeutic agents described herein also include analogs, prodrugs, salts and esters of limus compounds.

The terms rapamycin, rapa, and sirolimus are used interchangeably herein.

The limus family of compounds may be used in the compositions, liquid formulations and methods for the treatment, prevention, inhibition, delaying the onset of, or causing the regression of angiogenesis-mediated diseases and conditions of the eye, including choroidal neovascularization. The limus family of compounds may be used to prevent, treat, inhibit, delay the onset of, or cause regression of AMD, including wet AMD. Rapamycin and rapamycin derivatives and analogs may be used to prevent, treat, inhibit, delay the onset of, or cause regression of angiogenesis-mediated diseases and conditions of the eye, including choroidal neovascularization. Rapamycin may be used to prevent, treat, inhibit, delay the onset of, or cause regression of AMD, including wet AMD. In some variations, a member of the limus family of compounds or rapamycin is used to treat wet AMD or angiogenesis-mediated diseases and conditions of the eye including choroidal neovascularization.

Examples of other therapeutic agents that may be used include those disclosed in the following patents and publications: PCT Publication WO 2004/027027, assigned to Trustees of the University of Pennsylvania; US Patent 5,387,589, assigned to University of Louisville Research Foundation; US Patent 6,376,517, assigned to GPI NIL Holdings, Inc; PCT Publication WO 2004/028477, assigned to Innorx Inc; US Patent 6,416,777, assigned to Alcon Universal Ltd; US Patent 6,713,081, assigned to Department of Health and Human Services; and US Patent number 5,100,899.

Examples of other therapeutic agents that may be used according to the present description are pyrrolidine, dithiocarbamate (NFκB inhibitor); squalamine; TPN 470 analogue and fumagillin; PKC (protein kinase C) inhibitors; Tie-1 and Tie-2 kinase inhibitors; inhibitors of VEGF receptor kinase; proteosome inhibitors such as Velcade™ (bortezomib, for injection; ranibuzumab (Lucentis™) and other antibodies directed to the same target; pegaptanib (Macugen™); vitronectin receptor antagonists, such as cyclic peptide antagonists of vitronectin receptor-type integrins; α-v/β-3 integrin antagonists; α-v/β-1 integrin antagonists; thiazolidinediones such as rosiglitazone or troglitazone; interferon, including γ-interferon or interferon targeted to CNV by use of dextran and metal coordination; pigment epithelium derived factor (PEDF); endostatin; angiostatin; tumistatin; canstatin; anecortave acetate; acetonide; triamcinolone; tetrathiomolybdate; RNA silencing or RNA interference (RNAi) of angiogenic factors, including ribozymes that target VEGF expression; Accutane™ (13-*cis* retinoic acid); ACE inhibitors, including for example quinopril, captopril, and perindozril; inhibitors of mTOR (mammalian target of rapamycin); 3-aminothalidomide; pentoxifylline; 2-methoxyestradiol; colchicines; AMG-1470; cyclooxygenase inhibitors such as nepafenac, rofecoxib, diclofenac, rofecoxib, NS398, celecoxib, vioxx, and (E)-2-alkyl-2(4-methanesulfonylphenyl)-1-phenylethene; t-RNA synthase modulator; metalloprotease 13 inhibitor; acetylcholinesterase inhibitor; potassium channel blockers; endorepellin; purine analog of 6-thioguanine; cyclic peroxide ANO-2; (recombinant) arginine deiminase; epigallocatechin-3-gallate; cerivastatin; analogues of suramin; VEGF trap molecules; apoptosis inhibiting agents; Visudyne™, snET2 and other photo sensitizers, which may be used with photodynamic therapy (PDT); inhibitors of hepatocyte growth factor (antibodies to the growth factor or its receptors, small molecular inhibitors of the c-met tyrosine kinase, truncated versions of HGF e.g. NK4).

According to the present description, the therapeutic agent may be a BCR/ABL inhibitor. According to the present description the therapeutic agent may be a Src family tyrosine kinases inhibitor. According to the present description the therapeutic agent may be a dual BCR/ABL and Src family tyrosine kinases inhibitor. According to the present description the dual BCR/ABL and Src family tyrosine kinases inhibitor may be dasatinib (*i.e.,* BMS-354825, Sprycel®, or N-(2-chloro-6-methylphenyl)-2-[[6-[4-(2-hydroxyethyl)-1-piperazinyl]-2-methyl-4-pyrimidinyl]amino]-5-thiazole carboxamide monohydrate) or a derivative, an analog, or a pharmaceutically acceptable salt or ester thereof. According to the present description dasatinib may be used to treat, prevent, inhibit, or delay onset or regression of an ocular disease. In some variations, the ocular disease is age-related macular degeneration (AMD). In some variations, the AMD is wet AMD. In some variations, the AMD is dry AMD. According to the present description the ocular disease may be macular edema (e.g., diabetic macular edema).

Other therapeutic agents that may be used according to the present description include anti-inflammatory agents, for example nonsteroidal anti-inflammatory agents and steroidal anti-inflammatory agents. According to the present description active agents that may be used in the liquid formulations are ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, antibacterials, antihypertensives, pressors, antiprotozoal agents, antiviral agents, antifungal agents, anti-infective agents, antitumor agents, antimetabolites, and antiangiogenic agents.

Steroidal therapeutic agents that may be used according to the present description include for example 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, and any of their derivatives.

According to the present description cortisone, dexamethasone, fluocinolone, hydrocortisone, methylprednisolone, prednisolone, prednisone, and triamcinolone, or their derivatives, may be used. The liquid formulation may include a combination of two or more steroidal therapeutic agents.

In one example, the steroidal therapeutic agents may constitute from about 0.05% to about 50% by weight of the liquid formulation. In another example, the steroid constitutes from about 0.05% to about 10%, between about 10% to about 20%; between about 30% to about 40%; or between about 40% to about 50% by weight of the liquid formulation.

Other examples of therapeutic agents that may be used according to the present description include anaesthetics, analgesics, cell transport/mobility impending agents such as colchicines, vincristine, cytochalasin B and related compounds; carbonic anhydrase inhibitors such as acetazolamide, methazolamide, dichlorphenamide, diamox and neuroprotectants such as nimodipine and related compounds; antibiotics such as tetracycline, chlortetracycline, bacitracin, neomycin, polymyxin, gramicidin, cephalexin, oxytetracycline, chloramphenicol, rifampicin, ciprofloxacin, aminosides, gentamycin, erythromycin and penicillin, quinolone, ceftazidime, vancomycine imipeneme; antifungals such as amphotericin B, fluconazole, ketoconazole and miconazole; antibacterials such as sulfonamides, sulfadiazine, sulfacetamide, sulfamethizole and sulfisoxazole, nitrofurazone and sodium propionate; antivirals, such as idoxuridine, trifluorothymidine, trifluorouridine, acyclovir, ganciclovir, cidofovir, interferon, DDI, AZT, foscamet, vidarabine, irbavirin, protease inhibitors and anti-cytomegalovirus agents; antiallergenics such as sodium cromoglycate, antazoline, methapyriline, chlorpheniramine, cetirizine, pyrilamine and prophenpyridamine; synthetic glucocorticoids and mineralocorticoids and more generally hormones forms derivating from the cholesterol metabolism (DHEA, progesterone, estrogens); non-steroidal anti-inflammatories such as salicylate, indomethacin, ibuprofen, diclofenac, flurbiprofen, piroxicam and COX2 inhibitors; antineoplastics such as carmustine, cisplatin, fluorouracil; adriamycin, asparaginase, azacitidine, azathioprine, bleomycin, busulfan, carboplatin, carmustine, chlorambucil, cyclophosphamide, cyclosporine, cytarabine, dacarbazine, dactinomycin, daunorubicin, doxorubicin, estramustine, etoposide, etretinate, filgrastin, floxuridine, fludarabine, fluorouracil, florxymesterone, flutamide, goserelin, hydroxyurea, ifosfamide, leuprolide, levamisole, limustine, nitrogen mustard, melphalan, mercaptopurine, methotrexate, mitomycin, mitotane, pentostatin, pipobroman, plicamycin, procarbazine, sargramostin, streptozocin, tamoxifen, taxol, teniposide, thioguanine, uracil mustard, vinblastine, vincristine and vindesine; immunological drugs such as vaccines and immune stimulants; insulin, calcitonin, parathyroid hormone and peptide and vasopressin hypothalamus releasing factor; beta adrenergic blockers such as timolol, levobunolol and betaxolol; cytokines, interleukins and growth factors epidermal growth factor, fibroblast growth factor, platelet derived growth factor, transforming growth factor beta, ciliary neurotrophic growth factor, glial derived neurotrophic factor, NGF, EPO, PLGF, brain nerve growth factor (BNGF), vascular endothelial growth factor (VEGF) and monoclonal antibodies or fragments thereof directed against such growth factors; anti-inflammatories such as hydrocortisone, dexamethasone, fluocinolone, prednisone, prednisolone, methylprednisolone, fluorometholone, betamethasone and triamcinolone; decongestants such as phenylephrine, naphazoline and tetrahydrazoline; miotics and anti-cholinesterases such as pilocarpine, carbachol, di-isopropyl fluorophosphate, phospholine iodine and demecarium bromide; mydriatics such as atropine sulphate, cyclopentolate, homatropine, scopolamine, tropicamide, eucatropine; sympathomimetics such as epinephrine and vasoconstrictors and vasodilators, anticlotting agents such as heparin, antifibrinogen, fibrinolysin, anticlotting activase, antidiabetic agents include acetohexamide, chlorpropamide, glipizide, glyburide, tolazamide, tolbutamide, insulin and aldose reductase inhibitors, hormones, peptides, nucleic acids, saccharides, lipids, glycolipids, glycoproteins and other macromolecules include endocrine hormones such as pituitary, insulin, insulin-related growth factor, thyroid, growth hormones; heat shock proteins; immunological response modifiers such as muramyl dipeptide, cyclosporins, interferons (including alpha-, beta- and gamma-interferons), interleukin-2, cytokines, FK506 (an epoxy-pyrido-oxaazcyclotricosine-tetrone, also known as Tacrolimus), tumor necrosis factor, pentostatin, thymopentin, transforming factor beta2, erythropoetin; antineogenesis proteins (e.g. anti VEGF, interferons), antibodies (monoclonal, polyclonal, humanized, etc.) or antibodies fragments, oligoaptamers, aptamers and gene fragments (oligonucleotides, plasmids, ribozymes, small interference RNA (SiRNA), nucleic acid fragments, peptides), immunomodulators such as endoxan, thalidomide, tamoxifene; antithrombolytic and vasodilator agents such as rtPA, urokinase, plasmin; nitric oxide donors, nucleic acids, dexamethasone, cyclosporin A, azathioprine, brequinar, gusperimus, 6-mercaptopurine, mizoribine, rapamycin, tacrolimus (FK-506), folic acid analogs (e.g., denopterin, edatrexate, methotrexate, piritrexim, pteropterin, Tomudex®, trimetrexate), purine analogs (e.g., cladribine, fludarabine, 6-mercaptopurine, thiamiprine, thiaguanine), pyrimidine analogs (e.g., ancitabine, azacitidine, 6-azauridine, carmofur, cytarabine, doxifluridine, emitefur, enocitabine, floxuridine, fluorouracil, gemcitabine, tegafur) fluocinolone, triaminolone, anecortave acetate, fluorometholone, medrysone, and prednislone. In some variations the immunosuppressive agent is dexamethasone. In some variations the immunosuppressive agent is cyclosporin A.

According to the present description the formulation may comprise a combination of one or more therapeutic agents.

Other examples of therapeutic agents that may be used in the formulations described herein include antibacterial antibiotics, aminoglycosides (e.g., amikacin, apramycin, arbekacin, bambermycins, butirosin, dibekacin, dihydrostreptomycin, fortimicin(s), gentamicin, isepamicin, kanamycin, micronomicin, neomycin, neomycin undecylenate, netilmicin, paromomycin, ribostamycin, sisomicin, spectinomycin, streptomycin, tobramycin, trospectomycin), amphenicols (e.g., azidamfenicol, chloramphenicol, florfenicol, thiamphenicol), ansamycins (e.g., rifamide, rifampin, rifamycin sv, rifapentine, rifaximin), P-lactams (e.g., carbacephems (e.g., loracarbef), carbapenems (e.g., biapenem, imipenem, meropenem, panipenem), cephalosporins (e.g., cefaclor, cefadroxil, cefamandole, cefatrizine, cefazedone, cefazolin, cefcapene pivoxil, cefclidin, cefdinir, cefditoren, cefepime, cefetamet, cefixime, cefinenoxime, cefodizime, cefonicid, cefoperazone, ceforanide, cefotaxime, cefotiam, cefozopran, cefpimizole, cefpiramide, cefpirome, cefpodoxime proxetil, cefprozil, cefroxadine, cefsulodin, ceftazidime, cefteram, ceftezole, ceftibuten, ceftizoxime, ceftriaxone, cefuroxime, cefuzonam, cephacetrile sodium, cephalexin, cephaloglycin, cephaloridine, cephalosporin, cephalothin, cephapirin sodium, cephradine, pivcefalexin), cephamycins (e.g., cefbuperazone, cefinetazole, cefminox, cefotetan, cefoxitin), monobactams (e.g., aztreonam, carumonam, tigemonam), oxacephems, flomoxef, moxalactam), penicillins (e.g., amdinocillin, amdinocillin pivoxil, amoxicillin, ampicillin, apalcillin, aspoxicillin, azidocillin, azlocillin, bacampicillin, benzylpenicillinic acid, benzylpenicillin sodium, carbenicillin, carindacillin, clometocillin, cloxacillin, cyclacillin, dicloxacillin, epicillin, fenbenicillin, floxacillin, hetacillin, lenampicillin, metampicillin, methicillin sodium, mezlocillin, nafcillin sodium, oxacillin, penamecillin, penethamate hydriodide, penicillin g benethamine, penicillin g benzathine, penicillin g benzhydrylamine, penicillin g calcium, penicillin g hydrabamine, penicillin g potassium, penicillin g procaine, penicillin n, penicillin o, penicillin v, penicillin v benzathine, penicillin v hydrabamine, penimepicycline, phenethicillin potassium, piperacillin, pivampicillin, propicillin, quinacillin, sulbenicillin, sultamicillin, talampicillin, temocillin, ticarcillin), ritipenem, lincosamides (e.g., clindamycin, lincomycin), macrolides (e.g., azithromycin, carbomycin, clarithromycin, dirithromycin, erythromycin, erythromycin acistrate, erythromycin estolate, erythromycin glucoheptonate, erythromycin lactobionate, erythromycin propionate, erythromycin stearate, josamycin, leucomycins, midecamycins, miokamycin, oleandomycin, primycin, rokitamycin, rosaramicin, roxithromycin, spiramycin, troleandomycin), polypeptides (e.g., amphomycin, bacitracin, capreomycin, colistin, enduracidin, enviomycin, fusafungine, gramicidin s, gramicidin(s), mikamycin, polymyxin, pristinamycin, ristocetin, teicoplanin, thiostrepton, tuberactinomycin, tyrocidine, tyrothricin, vancomycin, viomycin, virginiamycin, zinc bacitracin), tetracyclines (e.g., apicycline, chlortetracycline, clomocycline, demeclocycline, doxycycline, guamecycline, lymecycline, meclocycline, methacycline, minocycline, oxytetracycline, penimepicycline, pipacycline, rolitetracycline, sancycline, tetracycline), and others (e.g., cycloserine, mupirocin, tuberin); synthetic antibacterials, 2.4-Diaminopyrimidines (e.g., brodimoprim, tetroxoprim, trimethoprim), nitrofurans (e.g., furaltadone, furazolium chloride, nifuradene, nifuratel, nifurfoline, nifurpirinol, nifurprazine, nifurtoinol, nitrofurantoin), quinolones and analogs (e.g., cinoxacin, ciprofloxacin, clinafloxacin, difloxacin, enoxacin, fleroxacin, flumequine, grepafloxacin, lomefloxacin, miloxacin, nadifloxacin, nalidixic acid, norfloxacin, ofloxacin, oxolinic acid, pazufloxacin, pefloxacin, pipemidic acid, piromidic acid, rosoxacin, rufloxacin, sparfloxacin, temafloxacin, tosufloxacin, trovafloxacin), sulfonamides (e.g., acetyl sulfamethoxypyrazine, benzylsulfamide, chloramine-b, chloramine-t, dichloramine t, n2-formylsulfisomidine, n4-β-d-glucosylsulfanilamide, mafenide, 4'-(methylsulfamoyl)sulfanilanilide, noprylsulfamide, phthalylsulfacetamide, phthalylsulfathiazole, salazosulfadimidine, succinylsulfathiazole, sulfabenzamide, sulfacetamide, sulfachlorpyridazine, sulfachrysoidine, sulfacytine, sulfadiazine, sulfadicramide, sulfadimethoxine, sulfadoxine, sulfaethidole, sulfaguanidine, sulfaguanol, sulfalene, sulfaloxic acid, sulfamerazine, sulfameter, sulfamethazine, sulfamethizole, sulfamethomidine, sulfamethoxazole, sulfamethoxypyridazine, sulfametrole, sulfamidochrysoidine, sulfamoxole, sulfanilamide, 4-sulfanilamidosalicylic acid, n4-sulfanilylsulfanilamide, sulfanilylurea, n-sulfanilyl-3,4-xylamide, sulfanitran, sulfaperine, sulfaphenazole, sulfaproxyline, sulfapyrazine, sulfapyridine, sulfasomizole, sulfasymazine, sulfathiazole, sulfathiourea, sulfatolamide, sulfisomidine, sulfisoxazole) sulfones (e.g., acedapsone, acediasulfone, acetosulfone sodium, dapsone, diathymosulfone, glucosulfone sodium, solasulfone, succisulfone, sulfanilic acid, p-sulfanilylbenzylamine, sulfoxone sodium, thiazolsulfone), and others (e.g., clofoctol, hexedine, methenamine, methenamine anhydromethylene-citrate, methenamine hippurate, methenamine mandelate, methenamine sulfosalicylate, nitroxoline, taurolidine, xibomol), antifungal antibiotics, polyenes (e.g., amphotericin b, candicidin, dermostatin, filipin, fungichromin, hachimycin, hamycin, lucensomycin, mepartricin, natamycin, nystatin, pecilocin, perimycin), azaserine, griseofulvin, oligomycins, neomycin undecylenate, pyrrolnitrin, siccanin, tubercidin, viridin, synthetic antifungals, allylamines (e.g., butenafine, naftifine, terbinafine), imidazoles (e.g., bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole), thiocarbamates (e.g., tolciclate, tolindate, tolnaftate), triazoles (e.g., fluconazole, itraconazole, saperconazole, terconazole), acrisorcin, amorolfine, biphenamine, bromosalicylchloranilide, buclosamide, calcium propionate, chlorphenesin, ciclopirox, cloxyquin, coparaffinate, diamthazole dihydrochloride, exalarnide, flucytosine, halethazole, hexetidine, loflucarban, nifuratel, potassium iodide, propionic acid, pyrithione, salicylanilide, sodium propionate, sulbentine, tenonitrozole, triacetin, ujothion, undecylenic acid, zinc propionate, antineoplastics, antibiotics and analogs (e.g., aclacinomycins, actinomycin f1, anthramycin, azaserine, bleomycins, cactinomycin, carubicin, carzinophilin, chromomycins, dactinomycin, daunorubicin, 6-diazo-5-oxo-L-norleucine, doxorubicin, epirubicin, idarubicin, menogaril, mitomycins, mycophenolic acid, nogalamycin, olivomycines, peplomycin, pirarubicin, plicamycin, porfiromycin, puromycin, streptonigrin, streptozocin, tubercidin, zinostatin, zorubicin), antimetabolites (e.g. folic acid analogs (e.g., denopterin, edatrexate, methotrexate, piritrexim, pteropterin, Tomudex®, trimetrexate), purine analogs (e.g., cladribine, fludarabine, 6-mercaptopurine, thiamiprine, thioguanine), pyrimidine analogs (e.g., ancitabine, azacitidine, 6-azauridine, carmofur, cytarabine, doxifluridine, emitefur, enocitabine, floxuridine, fluorouracil, gemcitabine, tagafur), antiinflammatory agents, steroidal antiinflammatory agents, acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortamate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, and triamcinolone hexacetonide, non-steroidal antiinflammatory agents, aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lomoxicam, piroxicam, tenoxicam), ε-acetamidocaproic acid, s-adenosylmethionine, 3-amino-4-hydroxybutyric acid, amixetrine, bendazac, benzydamine, a-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, and zileuton.

According to the present description the therapeutic agents may also be used in combination with other therapeutic agents and therapies, including for example agents and therapies useful for the treatment, prevention, inhibition, delaying onset of, or causing regression of angiogenesis or neovascularization, particularly CNV. According to the present description the additional agent or therapy may be used to treat regression of angiogenesis or neovascularization, particularly CNV. Examples of such additional agents and therapies include pyrrolidine, dithiocarbamate (NFκB inhibitor); squalamine; TPN 470 analogue and fumagillin; PKC (protein kinase C) inhibitors; Tie-1 and Tie-2 kinase inhibitors; inhibitors of VEGF receptor kinase; proteosome inhibitors such as Velcade™ (bortezomib, for injection; ranibuzumab (Lucentis™) and other antibodies directed to the same target; pegaptanib (Macugen™); vitronectin receptor antagonists, such as cyclic peptide antagonists of vitronectin receptor-type integrins; α-v/β-3 integrin antagonists; α-v/β-1 integrin antagonists; thiazolidinediones such as rosiglitazone or troglitazone; interferon, including γ-interferon or interferon targeted to CNV by use of dextran and metal coordination; pigment epithelium derived factor (PEDF); endostatin; angiostatin; tumistatin; canstatin; anecortave acetate; acetonide; triamcinolone; tetrathiomolybdate; RNA silencing or RNA interference (RNAi) of angiogenic factors, including ribozymes that target VEGF expression; Accutane™ (13-*cis* retinoic acid); ACE inhibitors, including quinopril, captopril, and perindozril; inhibitors of mTOR (mammalian target of rapamycin); 3-aminothalidomide; pentoxifylline; 2-methoxyestradiol; colchicines; AMG-1470; cyclooxygenase inhibitors such as nepafenac, rofecoxib, diclofenac, rofecoxib, NS398, celecoxib, vioxx, and (E)-2-alkyl-2(4-methanesulfonylphenyl)-1-phenylethene; t-RNA synthase modulator; metalloprotease 13 inhibitor; acetylcholinesterase inhibitor; potassium channel blockers; endorepellin; purine analog of 6-thioguanine; cyclic peroxide ANO-2; (recombinant) arginine deiminase; epigallocatechin-3-gallate; cerivastatin; analogues of suramin; VEGF trap molecules; inhibitors of hepatocyte growth factor (antibodies to the growth factor or its receptors, small molecular inhibitors of the c-met tyrosine kinase, truncated versions of HGF e.g. NK4); apoptosis inhibiting agents; Visudyne™, snET2 and other photo sensitizers with photodynamic therapy (PDT); and laser photocoagulation.

According to the present description the therapeutic agent may be used in combination with other therapeutic agents. According to the present description the therapeutic agent may be used in combination with verteporfin (Visudyne™). According to the present description the therapeutic agent may be used in combination with an inhibitor of Vascular Endothelial Growth Factor (VEGF). According to the present description the therapeutic agent may be used in combination with an inhibitor of Vascular Endothelial Growth Factor-A (VEGF-A). According to the present description the inhibitor of VEGF is a VEGF trap molecule including for example the VEGF trap under development by Regeneron Pharmaceuticals. According to the present description the inhibitor of VEGF may be an antibody or fragment thereof directed to VEGF. According to the present description the antibody or fragment thereof directed to VEGF may be bevacizumab (*i.e.,* Avastin™). According to the present description the antibody or fragment thereof directed to VEGF maybe ranibizumab (*i.e.,* Lucentis™). According to the present description the therapeutic agent may be a limus compound. According to the present description the therapeutic agent may be rapamycin or an analog or derivative thereof including those described in the *Therapeutic Agents* section. In some variations, the therapeutic agent is rapamycin. According to the present description the therapeutic agent may be dasatinib.

According to the present description the therapeutic agent may be a limus compound such as rapamycin or an analog or derivative thereof including those described in the *Therapeutic Agents* section and another therapeutic agent such as ranibizumab (*i.e.,* Lucentis™), bevacizumab (*i.e.,* Avastin™), or a VEGF trap. According to the present description the therapeutic agent may be a limus compound such as rapamycin or an analog or derivative thereof including those described in the *Therapeutic Agents* section and the other therapeutic agent is ranibizumab (*i.e.,* Lucentis™) or bevacizumab (*i.e.,* Avastin™). Also disclosed are methods for treating or preventing wet-AMD in a human subject comprising administration of a liquid formulation comprising a therapeutic agent such as rapamycin or an analog or derivative thereof including those described in the *Therapeutic Agents* section and another therapeutic agent such as ranibizumab (*i.e.,* Lucentis™), bevacizumab *(i.e.,* Avastin™), or a VEGF trap. According to the present description the methods for treating or preventing wet-AMD in a human subject may comprise administration of a liquid formulation comprising rapamycin and ranibizumab (*i.e.,* Lucentis™), bevacizumab (*i.e.,* Avastin™), or a VEGF trap. According to the present description the liquid formulation may comprise rapamycin and ranibizumab (*i.e.,* Lucentis™), bevacizumab (*i.e.,* Avastin™), or a VEGF trap is administered intravitreally, subconjunctivally, subtenonally, or topically. According to the present description the liquid formulation may be administered intravitreally. According to the present description the total amount of Lucentis or Avastin may be any of about 0.1 mg, about 0.2 mg, about 0.3 mg, about 0.4 mg, about 0.5 mg, about 0.6 mg, or about 0.7 mg. According to the present description the total amount of Lucentis or Avastin is 0.3 mg or 0.5 mg.

According to the present description the therapeutic agent used in combination with the other therapeutic agent may be administered simultaneously (*i.e.,* simultaneous administration), sequentially (*i.e.,* sequential administration), or independently of the other therapeutic agents. According to the present description the therapeutic agent and the other therapeutic agent may be administered by the same route of administration. According to the present description, the therapeutic agent and other therapeutic agent are both administered during one visit to the physician. According to the present description, the therapeutic agent and other therapeutic agent may both be administered during the same physician visit. According to the present description the therapeutic agent and other therapeutic agent may be administered during more than one visit to the physician.

In some variations the therapeutic agent and the other therapeutic agent are administered by the same route of administration. In some variations the therapeutic agent and the other therapeutic agent are administered by different routes of administration. In some variations the therapeutic agent are administered by subconjunctival administration such as subconjunctival injection, and the other therapeutic agent are administered by intravitreal administration such as intravitreal injection. In some variations the therapeutic agent are administered by intravitreal administration such as intravitreal injection, and the other therapeutic agent are administered by subconjunctival administration such as subconjunctival injection. In some variations the therapeutic agent are administered by subconjunctival administration such as subconjunctival injection, and the other therapeutic agent are administered by subtenon administration such as subtenon injection. In some variations the therapeutic agent are administered by subtenon administration such as subtenon injection, and the other therapeutic agent are administered by subconjunctival administration such as subconjunctival injection. In some variations the therapeutic agent are administered by intravitreal administration including such as intravitreal injection, and the other therapeutic agent are administered by subtenon administration such as subtenon injection. In some variations the therapeutic agent are administered by subtenon administration such as subtenon injection, and the other therapeutic agent are administered by intravitreal administration such as intravitreal injection. In some variations, the therapeutic agent is administered topically and the other therapeutic agent is administered by subconjunctival, subtenon, and/or intravitreal administration. In some variations, the other therapeutic agent is administered topically and the therapeutic agent is administered by subconjunctival, subtenon, and/or intravitreal administration. In some variations the formulation of the therapeutic agent and the other therapeutic agent are the same (*i.e.,* identical).

In some variations the combination of administration of therapeutic agent and administration of other therapeutic agent reduces the dose administered (*e.g*., dose volume, dose concentration, or amount of therapeutic agent) of one or both therapeutic agents. In some variations the combination of therapeutic agent and other therapeutic agent increases or prolongs the time between administrations and/or decreases the frequency of administrations of one or both therapeutic agents.

In some variations, the therapeutic agent is rapamycin. According to the present description the therapeutic agent may be dasatinib. According to the present description the other therapeutic agent may be verteporfin, bevacizumab, and/or ranibizumab.

By way of an example, administration of rapamycin may be used to decrease the frequency of administration of bevacizumab or ranibizumab. In one example, wet AMD is treated by administering one, two, or three doses of Avastin or Lucentis followed by subsequent doses of rapamycin, alone or in combination with Avastin or Lucentis.

### Diseases and conditions that may be treated, prevented, inhibited, onset delayed, or regression caused

Herein are described diseases and conditions that may be treated, prevented, inhibited, onset delayed, or regression caused using the therapeutic agents and the formulations, liquid formulations, and methods described herein. In some variations, the diseases or conditions are treated using the therapeutic agents and the formulations, liquid formulations, and methods described herein. Unless the context indicates otherwise, it is envisioned that the subjects on whom all of the methods of treatment may be performed include human subjects.

Generally, any diseases or condition of the eye susceptible to treatment, prevention, inhibition, delaying the onset of, or causing the regression of using the therapeutic agents and the formulations, liquid formulations and methods described herein may be treated, prevented, inhibited, onset delayed, or regression caused treated or prevented. Examples of diseases or conditions of the eye include for example diseases or conditions associated with neovascularization including retinal and/or choroidal neovascularization.

Diseases or conditions associated with retinal and/or choroidal neovascularization that can be treated, prevented inhibited, have onset delayed, or be caused to regress using the formulations, liquid formulations, and methods described herein include, for example, diabetic retinopathy, macular degeneration, wet and dry AMD, retinopathy of prematurity (retrolental fibroplasia), infections causing a retinitis or choroiditis, presumed ocular histoplasmosis, myopic degeneration, angioid streaks, and ocular trauma. Other examples of diseases and conditions of the eye that may be treated, prevented inhibited, have onset delayed, or be caused to regress using the formulations, liquid formulations, and methods described herein include, pseudoxanthoma elasticum, vein occlusion, artery occlusion, carotid obstructive disease, Sickle Cell anemia, Eales disease, myopia, chronic retinal detachment, hyperviscosity syndromes, toxoplasmosis, trauma, polypoidal choroidal vasculopathy, post-laser complications, complications of idiopathic central serous chorioretinopathy, complications of choroidal inflammatory conditions, rubeosis, diseases associated with rubeosis (neovascularization of the angle), neovascular glaucoma, uveitis and chronic uveitis, macular edema, proliferative retinopathies and diseases or conditions caused by the abnormal proliferation of fibrovascular or fibrous tissue, including all forms of proliferative vitreoretinopathy (including post-operative proliferative vitreoretinopathy), whether or not associated with diabetes.

According to the present description the formulations and pharmaceutical formulations described herein may be used to prevent or delay onset of a disease or condition of the eye where the human subject, is at heightened risk of developing the disease or condition of the eye. A subject with a heightened risk of developing a disease or condition is a subject with one or more indications that the disease or condition is likely to develop in the particular subject. In some variations the subject with a heightened risk of developing wet AMD is a subject with dry AMD in at least one eye. In some variations the subject with a heightened risk of developing wet AMD in a fellow eye is a subject with wet AMD in the other eye. According to the present description the formulations and pharmaceutical formulations described herein may be used to prevent or delay onset of CNV in a subject at heightened risk of developing CNV, including for example prevention or delaying onset of CNV in the fellow eye of a such as a human subject with AMD in one eye. According to the present description, the formulations and pharmaceutical formulations described herein may be used to prevent or delay onset of CNV in the fellow eye of a subject with wet AMD in one eye. According to the present description the formulations and pharmaceutical formulations may comprise a limus compound, such as to rapamycin. In some variations the formulations and pharmaceutical formulations are administered periocularly, (e.g., subconjunctivally), to a human subject with vision of 20/40 or better. In some variations, the formulations and pharmaceutical formulations are administered periocularly, including for example subconjunctivally, to the eye of a human subject where the eye to which the formulation is administered has vision of 20/40 or better.

According to the present description the formulations and pharmaceutical formulations described herein may be used to treat, prevent, or delay onset of AMD. According to the present description the formulations and pharmaceutical formulations described herein may be used to treat, prevent, or delay onset of dry AMD. According to the present description human subjects with non-central geographic atrophy may be administered a formulation or pharmaceutical formulations described herein to treat, prevent, or delay onset of central geographic atrophy. According to the present description the formulations and pharmaceutical formulations may comprise a limus compound, such as rapamycin. In some variations the formulations and pharmaceutical formulations are administered periocularly, such as subconjunctivally, to a human subject with vision of 20/40 or better. According to the present description the formulations and pharmaceutical formulations described herein may be administered and the human subject may also be treated with a second therapy for treating the disease or disorder. According to the present description the formulations and pharmaceutical formulations described herein may be used to treat, prevent, or delay onset of wet or dry AMD and the human subject may also be treated with laser therapy such as photodynamic laser therapy, either before, during, or after treatment with the formulations or pharmaceutical formulations described herein.

According to the present description the formulations and pharmaceutical formulations described herein may be used to treat one or more of uveitis, allergic conjunctivitis, macular edema, glaucoma, or dry eye.

According to the present description a formulations or pharmaceutical formulation may comprise a limus compound such as rapamycin, and is administered to treat, prevent, or delay onset of dry eye. According to the present description formulations or pharmaceutical formulations may comprise a limus compound such as rapamycin, and may be administered to treat, prevent, or delay onset of allergic conjunctivitis.

According to the present description the formulations and pharmaceutical formulations described herein may be used to treat glaucoma. According to the present description the formulations and pharmaceutical formulations described herein for treating glaucoma may comprise a limus compound such as rapamycin, and are used as a surgical adjuvant to prevent, reduce or delay surgical complications. According to the present description the formulations and pharmaceutical formulations described herein for treating glaucoma may comprise a limus compound such as rapamycin, and are used to improve or prolong surgical implant success. According to the present description the formulations and pharmaceutical formulations described herein for treating glaucoma may comprise a limus compound such as rapamycin, and are used to improve or prolong success of an argon laser trabeculectomy or other glaucoma-related surgery. According to the present description the formulations and pharmaceutical formulations described herein may have a neuroprotective effect and are used to treat glaucoma.

According to the present description the formulations and pharmaceutical formulations described herein may be used to treat retinitis pigmentosa. According to the present description the formulations and pharmaceutical formulations described herein for treating glaucoma may comprise a limus compound such as rapamycin, and are used to treat, prevent, or delay onset of retinitis pigmentosa. According to the present description the formulations and pharmaceutical formulations described herein may have a neuroprotective effect and are used to treat retinitis pigmentosa.

According to the present description the formulations and pharmaceutical formulations described herein may be used to treat one or more of central retinal vein occlusive diseases (CRVO), branch retinal venous occlusion (BRVO), retinal vascular diseases and conditions, macular edema, diabetic macular edema, iris neovascularization, diabetic retinopathy, or corneal graft rejection. According to the present description a formulations or pharmaceutical formulation may comprise a limus compound such as rapamycin, and may be administered to treat, prevent, or delay onset of one or more of these diseases or conditions. According to the present description the formulations and pharmaceutical formulations are administered subconjunctivally to an eye with vision of 20/40 or better.

When used to treat, prevent, inhibit, delay the onset of, or cause regressions of uveitis, the formulations and pharmaceutical formulations described herein may be administered by a variety of routes as is known in the art, such as by ocular or oral administration. Other routes of administration are known and are routine in the art. According to the present description the formulations described herein may comprise rapamycin and may be used to treat uveitis.

One disease that may be treated, prevented, inhibited, have onset delayed, or be caused to regress using the formulation, liquid formulations and methods described herein is the wet form of AMD. In some variations wet AMD is treated using the formulations, liquid formulations and methods described herein. The wet form of AMD is characterized by blood vessels growing from their normal location in the choroid into an undesirable position under the retina. Leakage and bleeding from these new blood vessels results in vision loss and possibly blindness.

The formulations, liquid formulations, and methods described herein may also be used to prevent or slow the transition from the dry form of AMD (wherein the retinal pigment epithelium or RPE degenerates and leads to photoreceptor cell death and the formation of yellow deposits called drusen under the retina) to the wet form of AMD.

"Macular degeneration" is characterized by the excessive buildup of fibrous deposits in the macula and retina and the atrophy of the retinal pigment epithelium. As used herein, an eye "afflicted" with macular degeneration is understood to mean that the eye exhibits at least one detectable physical characteristic associated with the disease of macular degeneration. The administration of rapamycin appears to limit and regress angiogenesis, such as choroidal neovascularization in age-related macular degeneration (AMD), which may occur without treatment. As used herein, the term "angiogenesis" means the generation of new blood vessels ("neovascularization") into a tissue or organ. An "angiogenesis-mediated disease or condition" of the eye or retina is one in which new blood vessels are generated in a pathogenic manner in the eye or retina, resulting in diminution or loss of vision or other problem, e.g., choroidal neovascularization associated with AMD.

The formulations and liquid formulations described herein, including rapamycin-containing formulations and liquid formulations, may also be used to treat, prevent, inhibit, delay the onset of, or cause regression of various immune-related diseases and conditions, such as organ transplant rejection in a host, graft vs. host disease, autoimmune diseases, diseases of inflammation, hyperproliferative vascular disorders, solid tumors, and fungal infections. The formulations and liquid formulations described herein, such as rapamycin-containing formulations and liquid formulations, are used to treat various immune-related diseases and conditions, such as organ transplant rejection in a host, graft vs. host disease, autoimmune diseases, diseases of inflammation, hyperproliferative vascular disorders, solid tumors, and fungal infections. The formulations and liquid formulations described herein, such as rapamycin-containing formulations and liquid formulations, may be used as immunosuppressants. The formulations and liquid formulations described herein, such as rapamycin-containing formulations and liquid formulations, may be used to treat, prevent, inhibit, or delay the onset of rejection of transplanted organs or tissues (e.g., transplanted heart, liver, kidney, spleen, lung, small bowel, pancreas, and bone marrow). In some variations, the formulations and liquid formulations described herein may be used to treat the onset of rejection of transplanted organs or tissues such as transplanted heart, liver, kidney, spleen, lung, small bowel, pancreas, and bone marrow. When used to treat, prevent, inhibit, delay the onset of, or cause regressions of immune-related diseases, such as transplant rejection, the formulations and liquid formulations described herein may be administered by a variety of routes as is known in the art, such as by oral administration.

Systemic administration may be achieved by oral administration of the liquid formulation. Other systemic routes of administration are known and are routine in the art. Some examples thereof are listed in the Detailed Description section.

As used herein, to "inhibit" a disease or condition by administration of a therapeutic agent means that the progress of at least one detectable physical characteristic or symptom of the disease or condition is slowed or stopped following administration of the therapeutic agent as compared to the progress of the disease or condition without administration of the therapeutic agent.

As used herein, to "prevent" a disease or condition by administration of a therapeutic agent means that the detectable physical characteristics or symptom of the disease or condition do not develop following administration of the therapeutic agent.

As used herein, to "delay onset of" a disease or condition by administration of a therapeutic agent means that at least one detectable physical characteristic or symptom of the disease or condition develops later in time following administration of the therapeutic agent as compared to the progress of the disease or condition without administration of the therapeutic agent.

As used herein, to "treat" a disease or condition by administration of a therapeutic agent means that the progress of at least one detectable physical characteristic or symptom of the disease or condition is slowed, stopped, or reversed following administration of the therapeutic agent as compared to the progress of the disease or condition without administration of the therapeutic agent.

As used herein, to "cause regression of" a disease or condition by administration of a therapeutic agent means that the progress of at least one detectable physical characteristic or symptom of the disease or condition is reversed to some extent following administration of the therapeutic agent.

A subject, such as a human subject, having a predisposition for or in need of prevention may be identified by the skilled practitioner by established methods and criteria in the field given the teachings herein. The skilled practitioner may also readily diagnose individuals as in need of inhibition or treatment based upon established criteria in the field for identifying angiogenesis and/or neovascularization given the teachings herein.

As used herein, a "subject" is generally any animal that may benefit from administration of the therapeutic agents described herein. According to some examples herein the therapeutic agents are administered to a mammalian subject. In some variations the therapeutic agents are administered to a human subject. According to some examples herein the therapeutic agents may be administered to a veterinary animal subject. According to some examples herein the therapeutic agents may be administered to a model experimental animal subject.

Other diseases and conditions that may be treated, prevented, inhibited, have the onset delayed, or be caused to regress using the methods described herein include those disclosed in the following patents and publications: PCT Publication WO 2004/027027, assigned to Trustees of the University of Pennsylvania; US Patent 5,387,589, assigned to University of Louisville Research Foundation; US Patent 6,376,517, assigned to GPI NIL Holdings, Inc; PCT Publication WO 2004/028477, assigned to Innorx, Inc; US Patent 6,416,777, assigned to Alcon Universal Ltd; US Patent 6,713,081, assigned to Department of Health and Human Services; US Patent 5,536,729, assigned to American Home Products Corp., and US Patent 7,585,517.

### Liquid formulations

The liquid formulations described herein contain a therapeutic agent and may generally be any liquid formulation, including solutions, suspensions, and emulsions. According to the present description the liquid formulations may form a non-dispersed mass relative to a surrounding medium when placed in the vitreous of a rabbit eye.

When a certain volume of a liquid formulation is administered, it is understood that there is some imprecision in the accuracy of various devices that may be used to administer the liquid formulation. Where a certain volume is specified, it is understood that this is the target volume. However, certain devices such as insulin syringes are inaccurate to greater than 10%, and sometimes inaccurate to greater than 20% or more. Hamilton HPLC type syringes are generally considered precise to within 10%, and are recommended for volumes below 10 µl are to be injected.

As described herein, a volume of a liquid formulation described herein may be administered to the vitreous of a rabbit eye or a subject's, such as a human subject's eye that is less than about 500 µl, less than about 400 µl, less than about 300 µl, less than about 200 µl, less than about 100 µl, less than about 90 µl, less than about 80 µl, less than about 70 µl, less than about 60 µl, less than about 50 µl, less than about 40 µl, less than about 30 µl, less than about 20 µl, less than about 10 µl, less than about 5 µl, less than about 3 µl, or less than about 1 µl. In some variations, a volume of a liquid formulation described herein is administered to the vitreous of a rabbit eye or subject's, such as a human subject's eye that is less than about 20 µl. A volume of a liquid formulation described herein may be administered to the vitreous that is less than about 10 µl. In some variations, a volume of a liquid formulation described herein is administered to the vitreous of a rabbit eye or a subject's, such as a human subject's eye that is between about 0.1 µl and about 200 µl, between about 50 µl and about 200 µl, between about 50 µl and about 150 µl, between about 0.1µl and about 100 µl, between about 0.1µl and about 50 µl, between about 1µl and about 40 µl, between about 1µl and about 30 µl, between about 1µl and about 20 µl, between about 1µl and about 10 µl, or between about 1µl and about 5 µl. A volume of a liquid formulation described herein may be administered to the vitreous of a rabbit eye or a subject's, such as a human subject's eye that is between about 1µl and about 10 µl. A volume of a liquid formulation described herein may be administered to the vitreous of a rabbit eye or a subject's, such as a human subject's eye that is between about 1µl and about 5 µl. A volume of a liquid formulation described herein may be administered to the vitreous of a rabbit eye or a subject's eye that is between about 1µl and about 5 µl. A volume of a liquid formulation described herein may be administered to the vitreous of a rabbit eye or a subject's, such as a human subject's eye that is between about 0.1µl and about 200 µl.

A total volume of a liquid formulation described herein may be subconjunctivally administered to a rabbit eye or a subject's, such as a human subject's eye that is less than about 1000 µl, less than about 900 µl, less than about 800 µl, less than about 700 µl, less than about 600 µl, less than about 500 µl, less than about 400 µl, less than about 300 µl, less than about 200 µl, less than about 100 µl, less than about 90 µl, less than about 80 µl, less than about 70 µl, less than about 60 µl, less than about 50 µl, less than about 40 µl, less than about 30 µl, less than about 20 µl, less than about 10 µl, less than about 5 µl, less than about 3 µl, or less than about 1 µl. A volume of a liquid formulation described herein may be subconjunctivally administered to a rabbit eye or a subject's, such as a human subject's eye that is less than about 20 µl. A volume of a liquid formulation described herein may be subconjunctivally administered to a rabbit eye or a subject's, such as a human subject's eye that is less than about 10 µl. A volume of a liquid formulation described herein may be subconjunctivally administered to a rabbit eye or a subject's, such as a human subject's eye that is between about 0.1µl and about 200 µl, between about 50 µl and about 200 µl, between about 200 µl and about 300 µl, between about 300 µl and about 400 µl, between about 400 µl and about 500 µl, between about 600 µl and about 700 µl, between about 700 µl and about 800 µl, between about 800 µl and about 900 µl, between about 900 µl and about 1000 µl, between about 50 µl and about 150 µl, between about 0.1µl and about 100 µl, between about 0.1µl and about 50 µl, between about 1µl and about 40 µl, between about 1µl and about 30 µl, between about 1µl and about 20 µl, between about 1µl and about 10 µl, or between about 1µl and about 5 µl. may be volume of a liquid formulation described herein is subconjunctivally administered to a rabbit eye or a subject's, such as a human subject's eye that is between about 1µl and about 10 µl. A volume of a liquid formulation described herein may be subconjunctivally administered to a rabbit eye or a subject's, such as a human subject's eye that is between about 1µl and about 5 µl. A volume of a liquid formulation described herein may be administered to subconjunctivally administered to a rabbit eye or a subject's, such as a human subject's eye that is between about 1µl and about 5 µl. In some variations, a volume of a liquid formulation described herein is administered to subconjunctivally administered to a rabbit eye or a subject's, such as a human subject's eye that is between about 0.1µl and about 200 µl.

A volume of a liquid formulation described herein may be administered to the vitreous of a rabbit eye or a subject's, such as a human subject's eye that is any of about 2 µl, about 4 µl, about 5 µl, about 6 µl, about 8 µl, about 10 µl, about 12 µl, about 14 µl, about 16 µl, about 18 µl, or about 20 µl. In some variations, a volume of a liquid formulation described herein is administered to the vitreous of a rabbit eye or a subject's, such as a human subject's eye that is about 2 µl. A volume of a liquid formulation described herein is administered to the vitreous of a rabbit eye or a subject's, such as a human subject's eye that is about 5 µl. A volume of a liquid formulation described herein may be administered to the vitreous of a rabbit eye or a subject's, such as a human subject's eye that is about 8 µl.

A total volume of a liquid formulation may be subconjunctivally administered to a rabbit eye or a subject's, such as a human subject's eye that is less than about 150 µl. A volume of a liquid formulation described herein may be subconjunctivally administered to a rabbit eye or a subject's, such as a human subject's eye that is any of about 10 µl, about 20 µl, about 30 µl, about 40 µl, about 50 µl, about 60 µl, about 70 µl, about 80 µl, about 90 µl, or about 100 µl. A volume of a liquid formulation described herein may be subconjunctivally administered to a rabbit eye or a subject's, such as a human subject's eye that is about 10 µl. A volume of a liquid formulation described herein may be subconjunctivally administered to a rabbit eye or a subject's, such as a human subject's eye that is about 20 µl. A volume of a liquid formulation described herein is subconjunctivally administered to a rabbit eye or a subject's, such as a human subject's eye that is about 30 µl. A volume of a liquid formulation described herein is subconjunctivally administered to a rabbit eye or a subject's, such as a human subject's eye that is 40 µl. A volume of a liquid formulation described herein is subconjunctivally administered to a rabbit eye or a subject's, such as a human subject's eye that is any of between about 10 µl and about 50 µl, between about 15 µl and about 45 µl, between about 20 µl and about 40 µl, or between about 25 µl and about 35 µl.

A total volume of a liquid formulation described herein is subtenonally administered to a rabbit eye or a subject's, such as a human subject's eye that is less than about 1000 µl, less than about 900 µl, less than about 800 µl, less than about 700 µl, less than about 600 µl, less than about 500 µl, less than about 400 µl, less than about 300 µl, less than about 200 µl, less than about 150 µl, less than about 100 µl, less than about 90 µl, less than about 80 µl, less than about 70 µl, less than about 60 µl, less than about 50 µl, less than about 40 µl, less than about 30 µl, less than about 20 µl, less than about 10 µl, less than about 5 µl, less than about 3 µl, or less than about 1 µl. A volume of a liquid formulation described herein may be subtenonally administered to a rabbit eye or a subject's, such as a human subject's eye that is less than about 200 µl. A volume of a liquid formulation described herein may be subtenonally administered to a rabbit eye or a subject's, such as a human subject's eye that is less than about 100 µl. A volume of a liquid formulation described herein may be subtenonally administered to a rabbit eye or a subject's, such as a human subject's eye that is between about 0.1µl and about 200 µl, between about 50 µl and about 200 µl, between about 200 µl and about 300 µl, between about 300 µl and about 400 µl, between about 400 µl and about 500 µl, between about 600 µl and about 700 µl, between about 700 µl and about 800 µl, between about 800 µl and about 900 µl, between about 900 µl and about 1000 µl, between about 50 µl and about 150 µl, between about 0.1µl and about 100 µl, between about 0.1µl and about 50 µl, between about 1 µl and about 40 µl, between about 1µl and about 30 µl, between about 1µl and about 20 µl, between about 1µl and about 10 µl, or between about 1µl and about 5 µl. A volume of a liquid formulation described herein may be subtenonally administered to a rabbit eye or a subject's, such as a human subject's eye that is between about 10µl and about 200 µl. A volume of a liquid formulation described herein may be administered subtenonally to a rabbit eye or a subject's, such as a human subject's eye that is between about 0.1 µl and about 200 µl. A volume of a liquid formulation described herein may be subtenonally administered to a rabbit eye or a subject's, such as a human subject's eye that is between about 50 µl and about 150 µl. A volume of a liquid formulation described herein may be subtenonally administered to a rabbit eye or a subject's, such as a human subject's eye that is about 30 µl. A volume of a liquid formulation described herein may be subtenonally administered to a rabbit eye or a subject's, such as a human subject's eye that is about 120 µl.

In some variations, a total amount of therapeutic agent less than about 5 mg is administered subtenonally. In some variations, a total amount of therapeutic agent less than about 5.0 mg is administered subtenonally. In some variations, a total amount of therapeutic agent less than about 4.5 mg is administered subtenonally. In some variations, a total amount of therapeutic agent less than about 4.0 mg is administered subtenonally. In some variations, a total amount of therapeutic agent less than about 3.5 mg is administered subtenonally. In some variations, a total amount of therapeutic agent less than about 3.0 mg is administered subtenonally. In some variations, a total amount of therapeutic agent less than about 2.5 mg is administered subtenonally. In some variations, a total amount of therapeutic agent less than about 2 mg is administered subtenonally. In some variations, a total amount of therapeutic agent less than about 1.2 mg is administered subtenonally. In some variations, a total amount of therapeutic agent less than about 1.0 mg is administered subtenonally. In some variations, a total amount of therapeutic agent less than about 0.8 mg is administered subtenonally. In some variations, a total amount of therapeutic agent less than about 0.6 mg is administered subtenonally. In some variations, a total amount of therapeutic agent less than about 0.4 mg is administered subtenonally. In some variations, a volume of a formulation is administered that contains an amount of therapeutic agent described herein.

In some variations, a total amount of therapeutic agent less than about 5 mg is administered subconjunctivally. In some variations, a total amount of therapeutic agent less than about 5.0 mg is administered subconjunctivally. In some variations, a total amount of therapeutic agent less than about 4.5 mg is administered subconjunctivally. In some variations, a total amount of therapeutic agent less than about 4.0 mg is administered subconjunctivally. In some variations, a total amount of therapeutic agent less than about 3.5 mg is administered subconjunctivally. In some variations, a total amount of therapeutic agent less than about 3.0 mg is administered subconjunctivally. In some variations, a total amount of therapeutic agent less than about 2.5 mg is administered subconjunctivally. In some variations, a total amount of therapeutic agent less than about 2 mg is administered subconjunctivally. In some variations, a total amount of therapeutic agent less than about 1.2 mg is administered subconjunctivally. In some variations, a total amount of therapeutic agent less than about 1.0 mg is administered subconjunctivally. In some variations, a total amount of therapeutic agent less than about 0.8 mg is administered subconjunctivally. In some variations, a total amount of therapeutic agent less than about 0.6 mg is administered subconjunctivally. In some variations, a total amount of therapeutic agent less than about 0.4 mg is administered subconjunctivally. In some variations, a volume of a formulation is administered that contains an amount of therapeutic agent described herein.

In some variations, a total amount of therapeutic agent less than about 200 µg is administered intravitreally. In some variations, a total amount of therapeutic agent less than about 200 µg is administered intravitreally. In some variations, a total amount of therapeutic agent less than about 300 µg is administered intravitreally. In some variations, a total amount of therapeutic agent less than about 400 µg is administered intravitreally. In some variations, a total amount of therapeutic agent less than about 500 µg is administered intravitreally. In some variations, a total amount of therapeutic agent less than about 600 µg is administered intravitreally. In some variations, a total amount of therapeutic agent less than about 800 µg is administered intravitreally. In some variations, a total amount of therapeutic agent less than about 1 mg is administered intravitreally. In some variations, a total amount of therapeutic agent less than about 2 mg is administered intravitreally. In some variations, a total amount of therapeutic agent less than about 2.5 mg is administered intravitreally. In some variations, a total amount of therapeutic agent less than about 3 mg is administered intravitreally. In some variations, a total amount of therapeutic agent less than about 3.5 mg is administered intravitreally. In some variations, a total amount of therapeutic agent less than about 4 mg is administered intravitreally. In some variations, a volume of a formulation is administered that contains an amount of therapeutic agent described herein.

In some variations, a total amount of therapeutic agent administered subconjunctivally is any of between about 50 µg and about 3 mg, between about 150 µg and about 750 µg, between about 300 µg and about 1000 µg, between about 300 µg and about 950 µg, between about 400 µg and about 900 µg, between about 450 µg and about 850 µg, between about 500 µg and about 800 µg, between about 550 µg and about 750 µg, or between about 600 µg and about 700 µg. In some variations, a total amount of therapeutic agent administered subconjunctivally is any of about 220 µg, about 440 µg, about 587 µg, about 630 µg, about 660 µg, about 880 µg, about 1320 µg, about 1760 µg, or about 2200 µg. In some variations, a total amount of therapeutic agent administered subconjunctivally is about 220 µg. In some variations, a total amount of therapeutic agent administered subconjunctivally is about 440 µg. In some variations, a total amount of therapeutic agent administered subconjunctivally is about 660 µg. In some variations, a total amount of therapeutic agent administered subconjunctivally is about 880 µg. In some variations, a liquid formulation containing an amount of therapeutic agent of 220 µg is subconjunctivally administered to a human subject by administering about 10 µl of a liquid formulation described herein. In some variations, a liquid formulation containing an amount of therapeutic agent of 440 µg is subconjunctivally administered to a human subject by administering about 20 µl of a liquid formulation described herein. In some variations, a liquid formulation containing an amount of therapeutic agent of 660 µg is subconjunctivally administered to a human subject by administering about 30 µl of a liquid formulation described herein. In some variations, a liquid formulation containing an amount of therapeutic agent of 880 µg is subconjunctivally administered to a human subject by administering about 40 µl of a liquid formulation described herein.

In some variations, a total amount of therapeutic agent administered intravitreally is any of between about 20 µg and about 750 µg, between about 20 µg and about 500 µg, or between about 30 µg and about 200 µg. In some variations, a total amount of therapeutic agent administered intravitreally is any of about 44 µg, about 110 µg, about 132 µg, about 133.5 µg, about 176 µg, about 264 µg, about 352 µg, about 440 µg or about 976 µg. In some variations, a total amount of therapeutic agent administered intravitreally is about 44 µg. In some variations, a total amount of therapeutic agent administered intravitreally is about 110 µg. In some variations, a total amount of therapeutic agent administered intravitreally is about 967 µg. In some variations, a liquid formulation containing an amount of therapeutic agent of 44 µg is intravitreally administered to a human subject by administering about 2 µl of a liquid formulation described herein. In some variations, a liquid formulation containing an amount of therapeutic agent of 110 µg is intravitreally administered to a human subject by administering about 5 µl of a liquid formulation described herein. In some variations, a liquid formulation containing an amount of therapeutic agent of 176 µg is intravitreally administered to a human subject by administering about 8 µl of a liquid formulation described herein.

A total volume of a liquid formulation described herein may be topically administered to a rabbit eye or a subject's, such as a human subject's eye that is less than about 1000 µl, less than about 900 µl, less than about 800 µl, less than about 700 µl, less than about 600 µl, less than about 500 µl, less than about 400 µl, less than about 300 µl, less than about 200 µl, less than about 150 µl, less than about 100 µl, less than about 90 µl, less than about 80 µl, less than about 70 µl, less than about 60 µl, less than about 50 µl, less than about 40 µl, less than about 30 µl, less than about 20 µl, less than about 10 µl, less than about 5 µl, less than about 3 µl, or less than about 1 µl. A volume of a liquid formulation described herein may be topically administered to a rabbit eye or a subject's, such as a human subject's eye that is less than about 200 µl. A volume of a liquid formulation described herein may be topically administered to a rabbit eye or a subject's, such as a human subject's eye that is less than about 100 µl. A volume of a liquid formulation described herein may be topically administered to a rabbit eye or a subject's, such as a human subject's eye that is between about 0.1µl and about 200 µl, between about 50 µl and about 200 µl, between about 200 µl and about 300 µl, between about 300 µl and about 400 µl, between about 400 µl and about 500 µl, between about 600 µl and about 700 µl, between about 700 µl and about 800 µl, between about 800 µl and about 900 µl, between about 900 µl and about 1000 µl, between about 50 µl and about 150 µl, between about 0.1µl and about 100 µl, between about 0.1µl and about 50 µl, between about 1 µl and about 40 µl, between about 1µl and about 30 µl, between about 1µl and about 20 µl, between about 1µl and about 10 µl, or between about 1µl and about 5 µl. A total volume of a liquid formulation described herein may be topically administered to a rabbit eye or a subject's, such as a human subject's eye that is between about 10µl and about 200 µl. A total volume of a liquid formulation described herein may be administered topically to a rabbit eye or a subject's, such as a human subject's eye that is between about 0.1µl and about 200 µl. A total volume of a liquid formulation described herein may be topically administered to a rabbit eye or a subject's, such as a human subject's eye that is between about 40 µl and about 160 µl. A total volume of a liquid formulation described herein may be topically administered to a rabbit eye or a subject's, such as a human subject's eye that is about 40 µl. A total volume of a liquid formulation described herein may be topically administered to a rabbit eye or a subject's, such as a human subject's eye that is about 80 µl.

In some variations, a total amount of therapeutic agent less than about 5 mg is administered topically. In some variations, a total amount of therapeutic agent less than about 5.0 mg is administered topically. In some variations, a total amount of therapeutic agent less than about 4.5 mg is administered topically. In some variations, a total amount of therapeutic agent less than about 4.0 mg is administered topically. In some variations, a total amount of therapeutic agent less than about 3.5 mg is administered topically. In some variations, a total amount of therapeutic agent less than about 3.0 mg is administered topically. In some variations, a total amount of therapeutic agent less than about 2.5 mg is administered topically. In some variations, a total amount of therapeutic agent less than about 2 mg is administered topically. In some variations, a total amount of therapeutic agent less than about 1.2 mg is administered topically. In some variations, a total amount of therapeutic agent less than about 1.0 mg is administered topically. In some variations, a total amount of therapeutic agent less than about 0.8 mg is administered topically. In some variations, a total amount of therapeutic agent less than about 0.6 mg is administered topically. In some variations, a total amount of therapeutic agent less than about 0.4 mg is administered topically. In some variations, a volume of a formulation is administered that contains an amount of therapeutic agent described herein. In some variations, a total amount of therapeutic agent administered topically is any of between about 20 µg and about 4000 µg, between about 10 µg and about 2000 µg, between about 10 µg and 1750 µg, between about 1500 µg and 1000 µg, or between about 10 µg and 1000 µg. In some variations, a total amount of therapeutic agent administered topically is about 1660 µg. In some variations, a total amount of therapeutic agent administered topically is about 880 µg. In some variations, a total amount of therapeutic agent administered topically is 40 µg. In some variations, a total amount of therapeutic agent administered topically is about 28 µg.

In some variations, the therapeutic agent may be a limus compound. In some variations, the therapeutic agent may be rapamycin or an analog or derivative thereof including those described in the *Therapeutic Agents* section. In some variations, the therapeutic agent is rapamycin. According to the present description, the therapeutic agent may be dasatinib.

"Total amount of a therapeutic agent" as used herein refers to the total amount of a therapeutic agent administered during a single administration session by a patient and/or physician and/or other medical professional. In some variations, a single administration session will involve a single administration of the therapeutic agent. In some variations, one administration session will include more than one administration of the therapeutic agent. In some variations, one administration session will include a single route of administration. In some variations, one administration session will include multiple routes of administration. Thus, in some variations, portions of the total amount of the therapeutic agent are administered separately during a single administration session. In such variations, the portions of the total amount that are administered separately may be administered by the same and/or different routes of administration. In addition, in some variations, portions of the total amount that are administered separately may be administered in the same and/or different formulations.

"Total volume of a liquid formulation" as used herein refers to the total volume of a liquid formulation administered during a single administration session by a patient and/or physician and/or other medical professional. In some variations, a single administration session will involve a single administration of the liquid formulation. In some variations, one administration session will include more than one administration of the liquid formulation. In some variations, one administration session will include a single route of administration. In some variations, one administration session will include multiple, different routes of administration. Thus, in some variations, portions of the total volume are administered separately during a single administration session. In such variations, the portions of the total volume that are administered separately may be administered by the same and/or by different routes of administration.

In some variations, the liquid formulation is administered in multiple ocular locations. In some variations, the liquid formulation is administered intravitreally and subconjunctivally. In some variations, the liquid formulation is administered intravitreally and subtenonally. In some variations, the liquid formulation is administered subtenonally and subconjunctivally. In some variations, the liquid formulation is first administered intravitreally and at least one subsequent administration is administered subconjunctivally. In some variations, the liquid formulation is first administered subconjunctivally and at least one subsequent administration is administered intravitreally. In some variations, the liquid formulation is first administered intravitreally and at least one subsequent administration is administered subtenonally. In some variations, the liquid formulation is first administered subconjunctivally and at least one subsequent administration is administered subtenonally. In some variations, the liquid formulation is first administered subtenonally and at least one subsequent administration is administered subconjunctivally. In some variations, the liquid formulation is first administered subtenonally and at least one subsequent administration is administered intravitreally. In some variations, the liquid formulation is first administered intravitreally, subconjunctivally, or subtenonally and at least on subsequent administration is topically. In some variations, the first administration induces a drug response, and subsequent administrations maintain the drug response. In some variations, the same (*i.e.,* identical) liquid formulation is used for the different routes of administration, as an example for intravitreal and subconjunctival administration. In some variations, administration to multiple ocular locations occurs during one visit to the physician. In some variations, administration to multiple ocular locations occurs during separate visits to the physician.

In some variations, a therapeutic agent is administered in multiple ocular locations. In some variations, a therapeutic agent is administered intravitreally and subconjunctivally. In some variations, a therapeutic agent is first administered intravitreally and at least one subsequent administration is administered subconjunctivally. In some variations, a therapeutic agent is first administered subconjunctivally and at least one subsequent administration is administered intravitreally. In some variations, a therapeutic agent is first administered intravitreally, subconjunctivally, or subtenonally and at least on subsequent administration is topically. In some variations, the same (*i.e.,* identical) liquid formulation of the therapeutic agent is used for the first and subsequent administrations. In some variations, a different liquid formulation is used for intravitreal and subconjunctival administration for the first and subsequent administrations. In some variations, administration to multiple ocular locations occurs during one visit to the physician. In some variations, administration to multiple ocular locations occurs during separate visits to the physician.

In some variations the liquid formulations described herein are administered in multiple subconjunctival locations within a period of time, including for example within an hour of one another. Without being bound by theory, it is thought that such multiple administrations, such as multiple injections, allow for a greater total dose to be administered subconjunctivally than a single dose due to a potentially limited ability of the local ocular tissues to absorb larger volumes.

One liquid formulation described herein is an in situ gelling formulation. In situ gelling formulations, as described herein, comprise a therapeutic agent and a plurality of polymers which give a formulation that forms a gel or a gel-like substance when placed in an aqueous medium, such as an aqueous medium of the eye.

In some variations of the liquid formulations described herein, the therapeutic agent is a solution or suspension of rapamycin in a liquid medium. Liquid media include for example solvents, including those described in the *Solubilization of Therapeutic Agents* section.

The liquid formulations described herein may comprise a solubilizing agent component. In some variations the solubilizing agent component is a surfactant. Note that there is some overlap between components that may be solvents and solubilizing agents, and therefore the same component may in some systems be used as either a solvent or a solubilizing agent. A liquid formulation that comprises a therapeutic agent and a component that may be considered either a solvent or a solubilizing agent or surfactant will be considered a solvent if it is playing the role of a solvent; if the component is not playing the role of the solvent, the component may be considered a solubilizing agent or surfactant.

Liquid formulations may optionally further comprise stabilizers, excipients, gelling agents, adjuvants, antioxidants, and/or other components as described herein.

As disclosed herein all components in the liquid formulation, other than the therapeutic agent, may be liquid at room temperature.

The liquid formulation disclosed herein may comprise a release modifying agent. The release modifying agent may be a film-forming polymer component. The film-forming polymer component may comprise one or more film-forming polymers. Any film-forming polymer may be used in the excipient component. The film-forming polymer component may comprise a water insoluble film forming polymer. The release modifying agent component may comprise an acrylic polymer, such as polymethacrylate, exemplified by Eudragit RL.

Described herein are compositions and liquid formulations for delivery of the therapeutic agents described in the *Therapeutic Agents* section. Delivery of therapeutic agents using the compositions and liquid formulations described herein may be used to treat, prevent, inhibit, delay the onset of, or cause the regression of the diseases and conditions described in the *Diseases and Conditions* section. The compositions and liquid formulations described herein may comprise any of the therapeutic agents described in the *Therapeutic Agents* section, such as rapamycin. The compositions and liquid formulations described herein may comprise one or more than one therapeutic agent. Other compositions and liquid formulations in addition to those explicitly described herein may be used.

When the therapeutic agent is rapamycin, the compositions and liquid formulations may be used to maintain an amount of rapamycin in the vitreous effective to treat wet AMD. In one example, it is believed that a liquid formulation delivering rapamycin to maintain a concentration of rapamycin of about 10 pg/ml to about 2 µg/ml in the vitreous over a period of time may be used for the treatment of wet AMD. When the rapamycin is in a liquid formulation that forms a non-dispersed mass, the stated concentration of rapamycin represents the amount that is effectively treating the disease or condition of the eye, and not merely present in the form of the non-dispersed mass. In another example, it is believed that a delivery system delivering rapamycin to maintain a concentration of rapamycin of about 0.01 pg/mg to about 10 ng/mg in the retina choroid tissues over a period of time may be used for treatment of wet AMD. Other therapeutically effective amounts of therapeutic agent are also possible, and can be readily determined by one of skill in the art given the teachings herein.

When the therapeutic agent is rapamycin, the compositions and liquid formulations described herein may be used to deliver a dose of rapamycin to a subject, such as a human subject or to the eye of a human subject. In one example, it is believed that a liquid formulation containing a dose of about 20 µg to about 4 mg may be used for the treatment of wet AMD.

According to the present description, the therapeutic agent in the liquid formulation comprises between about 0.01 to about 30% of the total weight of the composition; between about 0.05 to about 15%; between about 0.1 to about 10%; between about 1 to about 5%; or between about 5 to about 15%; between about 8 to about 10%; between about 0.01 to about 1%; between about 0.05 to about 5%; between about 0.1 to about 0.2%; between about 0.2 to about 0.3%; between about 0.3 to about 0.4%; between about 0.4 to about 0.5%; between about 0.5 to about 0.6%; between about 0.6 to about 0.7%; between about 0.7 to about 1%; between about 1 to about 5%; between about 5 to about 10%; between about 15 to about 30%, between about 20 to about 30%; or between about 25 to about 30%.

The therapeutic agent in the liquid formulation may comprise between about 0.001 to about 1.00% of the total weight of the composition. The therapeutic agent in the liquid formulation may comprise any of about 0.07%, about 0.08%, 0.09%, 0.17%, 1.38%, 1.47%, 2%, 4%, 4.84%, or 5% of the total weight of the composition. The therapeutic agent may be a limus compound. In some variations, the therapeutic agent may be rapamycin or an analog or derivative thereof including those described in the *Therapeutic Agents* section. In some variations, the therapeutic agent is rapamycin. According to the present description, the therapeutic agent may be dasatinib.

Those of skill in the art, based on the teachings herein can determine what amount or concentration of a given therapeutic agent is equivalent to an amount or concentration of rapamycin by, for example, administering the therapeutic agent at various amounts or concentrations to a disease model system, such as an in vivo or in vivo model system, and comparing the results in the model system relative to the results of various amounts or concentrations of rapamycin. Those of skill in the art, based on the teachings herein can also determine what amount or concentration of a given therapeutic agent is equivalent to an amount or concentration of rapamycin by reviewing the scientific literature for experiments performed comparing rapamycin to other therapeutic agents. It is understood that even the same therapeutic agent may have a different equivalent level of rapamycin when, for example, a different disease or disorder is being evaluated, or a different type of formulation is used. Examples of scientific references with comparative studies of rapamycin and other therapeutic agents on ocular disease are Ohia et al., Effects ofsteroids and immunosuppressive drugs on endotoxin-uveitis in rabbits, J. Ocul. Pharmacol. 8(4):295-307 (1992); Kulkarni, Steroidal and nonsteroidal drugs in endotoxin-induced uveitis, J. Ocul. Pharmacol. 10(1):329-34 (1994); Hafizi et al., Differential effects of rapamycin, cyclosporine A, and FK506 on human coronary artery smooth muscle cell proliferation and signaling, Vascul Pharmacol. 41(4-5):167-76 (2004); and US 2005/0187241.

For example, in a model for wet AMD, if a therapeutic agent is found to be approximately 10-fold less potent or efficacious than rapamycin in the treatment of wet AMD, a concentration of 10 ng/ml of the therapeutic agent would be equivalent to a 1 ng/ml concentration of rapamycin. Or if a therapeutic agent is found to be approximately 10-fold less potent or efficacious than rapamycin in the treatment of wet AMD, a 10-fold amount of the therapeutic agent would be administered relative to the amount of rapamycin.

The solvent component may comprise, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%.

The solubilizing agent component may comprise, for instance, between about 0.01 to about 30 % of the total weight of the composition; between about 0.1 to about 20%; between about 2.5 to about 15%; between about 10 to about 15%; or between about 5 to about 10%; between about 8 to about 12%; between about 10 to about 20%; between about 20 to about 30%.

The liquid formulations described herein may have a viscosity of between 40% and 120% centipoise. The liquid formulations described herein may have a viscosity of between 60% and 80% centipoise.

The liquid formulations described herein may comprise a therapeutic agent and a solvent component. The solvent component may comprise a single solvent or a combination of solvents. The therapeutic agent component may comprise a single therapeutic agent or a combination of therapeutic agents. The solvent may be glycerin, dimethylsulfoxide, N-methylpyrrolidone, dimethyl acetamide (DMA), dimethyl formamide, glycerol formal, ethoxy diglycol, triethylene glycol dimethyl ether, triacetin, diacetin, corn oil, acetyl triethyl citrate (ATC), ethyl lactate, polyglycolated capryl glyceride, γ butyrolactone, dimethyl isosorbide, benzyl alcohol, ethanol, isopropyl alcohol, polyethylene glycol of various molecular weights, such as PEG 300 and PEG 400, or propylene glycol, or a mixture of one or more thereof.

The liquid formulations described herein may be solutions, and comprise a therapeutic agent and a solvent component. The solvent component may comprise ethanol. The solvent component may comprise ethanol and a polyethylene glycol, such as polyethylene glycol, exemplified by PEG 300 or PEG 400.

The liquid formulations described herein may contain no greater than about 250 µl of polyethylene glycol. The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of polyethylene glycol. Formulations containing polyethylene glycol may contain, for example, PEG 300 or PEG 400.

The liquid formulations described herein may be suspensions, and comprise a therapeutic agent and a diluent component. the diluent component comprises one or more components listed herein as solvents or solubilizing agents, wherein the resulting mixture is a suspension.

The liquid formulation may be partly a solution and partly a suspension.

The liquid formulation may be an in situ gelling formulation, and comprises a therapeutic agent and a polymer component, wherein the polymer component may comprise a plurality of polymers. In some variations, the liquid formulation comprises a polymethacrylate polymer. The liquid formulation comprises a polyvinylpyrrolidone polymer.

Some examples of liquid formulations include a therapeutic agent or agents such as rapamycin between about 0.01% and about 20% by weight of the total, a solvent between about 5% and about 15% by weight of the total, a solubilizing agent such as a surfactant between about 5% and about 15% by weight of the total, with water as the primary remaining component. In some examples the formulations further comprise stabilizing agents, excipients, adjuvants, or antioxidants, between about 0 and about 40% by weight of the total.

A liquid formulation may comprise up to about 5% therapeutic agent, such as rapamycin, per weight of the total; and up to about 99.9% of a solvent component, by weight of the total. The liquid formulation may comprise up to about 5% therapeutic agent, such as rapamycin, per weight of the total; and up to about 99.9% of a diluent component.

A liquid formulation may comprise up to about 5% therapeutic agent, such as rapamycin, per weight of the total; up to about 10% solvent by weight of the total; and up to about 85% of a solubilizing component, by weight of the total. The solubilizing component may be an aqueous solution of a surfactant.

A plurality of polymers component may comprise, for instance, between about 0.01 to about 30 % of the total weight of the composition; between about 0.1 to about 20%; between about 2.5 to about 15%; between about 10 to about 15%; between about 3 to about 5%; between about 5 to about 10%; between about 8 to about 12%; between about 10 to about 20%; or between about 20 to about 30%.

Some examples of liquid formulations includes a therapeutic agent or agents such as to rapamycin between about 0.01% and about 20% by weight of the total, a solvent component between about 60% and about 98% by weight of the total, and a plurality of polymers, whose combined percentage is between about 0.1% and about 15% by weight of the total. The formulations may further comprise stabilizing agents, excipients, adjuvants, or antioxidants, between about 0 and about 40% by weight of the total.

In some variations, a liquid formulation may comprise about 4% therapeutic agent, such as rapamycin, per weight of the total; about 91% solvent by weight of the total; and about 5% polymeric component, per weight of the total.

In some variations, the liquid formulation comprises about 2% (w/w) rapamycin, about 4% (w/w) ethanol, and about 94% (w/w) PEG 400. In some variations, liquid formulation is administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. According to the present description, the ocular disease may be macular edema.

In some variations, the liquid formulation comprises about 1.69% (w/w) rapamycin, about 3.39% (w/w) ethanol, about 79.54% (w/w) PEG 400, and about 15.38% (w/w) aqueous liquid (*e.g*., water or saline). In some variations, liquid formulation is administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. According to the present description , the ocular disease may be macular edema.

In some variations, the liquid formulation comprises about 1.47% (w/w) rapamycin, about 2.93% (w/w) ethanol, about 94% (w/w) PEG 400, and about 26.6% (w/w) aqueous liquid (e.g., water or saline). In some variations, liquid formulation is administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. According to the present description , the ocular disease may be macular edema.

The liquid formulation may comprise about 1.38% (w/w) rapamycin, about 2.75% (w/w) ethanol, about 64.62% (w/w) PEG 400, and about 31.25% (w/w) aqueous liquid (*e.g*., water or saline). The liquid formulation may be administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. According to the present description, the ocular disease may be macular edema.

In some variations the liquid formulations described herein comprise a therapeutic agent and a solvent component. In some variations, the solvent component may comprise a solvent having a hygroscopicity that is equal to or greater than that of polyethylene glycol 400 (PEG 400). In some variations, the solvent component may comprise a solvent having a hygroscopicity that is greater than that of polyethylene glycol 400 (PEG 400). In some variations, the solvent component may comprise a solvent having a hygroscopicity that is about equal to that of polyethylene glycol 400 (PEG 400). In some variations, the solvent component may comprise a solvent having a hygroscopicity that is less than that of polyethylene glycol 400 (PEG 400). In some variations, the liquid formulation is about as hygroscopic as a liquid formulation consisting of 2% rapamycin, 4% ethanol, and 94% PEG 400. In some variations, the liquid formulation is less hygroscopic as a liquid formulation consisting of 2% rapamycin, 4% ethanol, and 94% PEG 400.

The parameters that nay be used to measure hygroscopicity of a material include critical relative humidity (which may be measured by "moisture meters" (*e.g*., Hygrometers (*i.e*., Thermo Hygrometer Model 625, manufactured by BK Precision), hygroscopicity potential, hygroscopicity coefficient, or heats of absorption (which may be measured by a Differential Scanning Calorimetry (DSC).Those of ordinary skill in the art will find it routine to identify solvents having a hygroscopicity that is about equal to, less than, or greater than that of polyethylene glycol 400 (PEG 400) given the teachings herein.

The solvent component may comprise a single solvent or a combination of solvents. The therapeutic agent component may comprise a single therapeutic agent or a combination of therapeutic agents. The solvent may be N-methyl pyrrolidone (NMP), dimethyl-acetamine (DMA), dimethyl sulfoxide (DMSO), propylene glycol (PG), polyethylene glycol 600 (PEG 600), polyethylene glycol 400 (PEG 400), ethanol, or a mixture of one or more thereof. The solvent component may comprise a combination of solvents including N-methyl pyrrolidone (NMP), dimethyl-acetamine (DMA), or dimethyl sulfoxide (DMSO). The solvent component may comprise a combination of solvents including propylene glycol (PG), polyethylene glycol 600 (PEG 600), or polyethylene glycol 400 (PEG 400). The solvent component may comprise a combination of at least two solvents. The at least two solvents may comprise a first solvent such as N-methyl pyrrolidone (NMP), dimethyl-acetamine (DMA), or dimethyl sulfoxide (DMSO) and a second solvent such as propylene glycol (PG), polyethylene glycol 600 (PEG 600), or polyethylene glycol 400 (PEG 400). The solvent component may comprise N-methyl pyrrolidone (NMP) and propylene glycol (PG). The solvent component may comprise N-methyl pyrrolidone (NMP) and polyethylene glycol 600 (PEG 600). The solvent component may comprise dimethyl-acetamine (DMA) and propylene glycol (PG). The solvent component may comprise dimethyl-acetamine (DMA) and polyethylene glycol 400 (PEG 400). The solvent component may comprise dimethyl sulfoxide (DMSO) and propylene glycol (PG). The solvent component may comprise N-methyl pyrrolidone (NMP) and polyethylene glycol 400 (PEG 400). In some variations, the solvent component may further comprise ethanol. In some variations, the solvent component may further comprise water. In some variations, the solvent component may have a hygroscopicity that is about equal to, less than, or greater than that of polyethylene glycol 400 (PEG 400). In some variations, liquid formulation is administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. According to the present description, the ocular disease may be macular edema.

The solvent may be polyethoxylated castor oil (*e.g*., Cremophor (PEG 35 castor oil)), monoglycerides and/or diglycerides of caprylic acid (*e.g*., Capmul MCM (C8)), nonionic polymer of the alkyl aryl polyether alcohol (*e.g*., tyloxapol (ethoxylated p-tert-octylphenol formaldehyde polymer)), 50% phosphatidylcholine in propylene glycol/ethanol carrier (*e.g*., Phosal® 50PG), propylene glycol monolaurate, propylene glycol dicaprylate/dicaprate, macrogol 15 hydroxystearate, ethanol, or a mixture of one or more thereof. The solvent may comprise a combination of at least two solvents. The at least two solvents may comprise a first solvent such as polyethoxylated castor oil (*e.g*., Cremophor (PEG 35 castor oil)), propylene glycol monolaurate, propylene glycol dicaprylate/dicaprate, macrogol 15 hydroxystearate, or nonionic polymer of the alkyl aryl polyether alcohol (*e.g*., tyloxapol (ethoxylated p-tert-octylphenol formaldehyde polymer)) and a second solvent such as monoglycerides and/or diglycerides of caprylic acid *(e.g.,* Capmul MCM (C8)). The solvent may further comprise ethanol. The solvent may further comprise water. The liquid formulation may be topically administered. In some variations, the liquid formulation is administered as eye drops. In some variations, the liquid formulation is used to treat or prevent an ocular disease. In some variations, the ocular disease is wet AMD. In some variations, the ocular disease is dry AMD. According to the present description, the ocular disease may be macular edema.

The solvent component may comprise water, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Water may comprise between about 15 to about 30% (w/w) of the liquid formulation. Water may comprise between about 80 to about 90% (w/w) of the liquid formulation. Water may comprise at least 15% (w/w). Water may comprise at least 20% (w/w) of the liquid formulation. Water may comprise at least 25% (w/w) of the liquid formulation. Water may comprise at least about 5 percent (w/w) of the liquid formulation. Water may comprise about 16% (w/w) of the liquid formulation. Water may comprise about 22% (w/w). Water may comprise about 28% (w/w) of the liquid formulation. Water may comprise about 83% (w/w) of the liquid formulation.

The liquid formulations described herein may contain no greater than about 250 µl of water. The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of water.

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of water.

The solvent component may comprise N-methyl pyrrolidone (NMP), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. NMP may comprise between about 10 to about 40% (w/w) of the liquid formulation. NMP may comprise at least 10% (w/w) of the liquid formulation. NMP may comprise at least 11% (w/w) of the liquid formulation. NMP may comprise at least 25% (w/w) of the liquid formulation. NMP may comprise at least 35% (w/w) of the liquid formulation. NMP may comprise about 10% (w/w) of the liquid formulation. NMP may comprise about 28% (w/w) of the liquid formulation. NMP may comprise about 39% (w/w) of the liquid formulation.

The liquid formulations described herein may contain no greater than about 250 µl of N-methyl pyrrolidone (NMP). The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of N-methyl pyrrolidone (NMP).

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of N-methyl pyrrolidone (NMP).

The solvent component may comprise dimethyl-acetamine (DMA), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. DMA may comprise between about 10 to about 40% (w/w) of the liquid formulation. DMA may comprise at least 10% (w/w) of the liquid formulation. DMA may comprise about 15% (w/w) of the liquid formulation. DMA may comprise about 11% (w/w) of the liquid formulation. DMA may comprise about 10% (w/w) of the liquid formulation.

The liquid formulations described herein may contain no greater than about 250 µl of dimethyl-acetamine (DMA). The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of dimethyl-acetamine (DMA).

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of dimethyl-acetamine (DMA).

The solvent component may comprise dimethyl sulfoxide (DMSO), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. In some variations, DMSO comprises between about 10 to about 40% (w/w) of the liquid formulation. DMSO may comprise at least 10% (w/w) of the liquid formulation. DMSO may comprise at least 11% (w/w) of the liquid formulation. DMSO may comprise about 10% (w/w) of the liquid formulation.

According to the present description, the liquid formulations described herein may contain no greater than about 250 µl of dimethyl sulfoxide (DMSO). In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of dimethyl sulfoxide (DMSO).

For example, the liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of dimethyl sulfoxide (DMSO).

The solvent component may comprise propylene glycol (PG), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. PG may comprise between about 35 to about 75% (w/w) of the liquid formulation. PG may comprise at least 35% (w/w) of the liquid formulation. PG may comprise at least 70% (w/w) of the liquid formulation. PG may comprise less than about 90% (w/w) of the liquid formulation. PG may comprise about 39% (w/w) of the liquid formulation. PG may comprise about 74% (w/w) of the liquid formulation.

According to the present description, the liquid formulations described herein may contain no greater than about 250 µl of propylene glycol (PG). In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of propylene glycol (PG).

For example, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of propylene glycol (PG).

The solvent component may comprise polyethylene glycol (PEG), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. PEG may comprise between about 35 to about 75% (w/w) of the liquid formulation. PEG may comprise at least 35% (w/w) of the liquid formulation. PEG may comprise at least 70% (w/w) of the liquid formulation. PEG may comprise less than about 90% (w/w) of the liquid formulation. PEG may comprise about 39% (w/w) of the liquid formulation. PEG may comprise about 74% (w/w) of the liquid formulation. PEG may comprise about 80% (w/w) of the liquid formulation. In some variations, the PEG is polyethylene glycol 400. The PEG maybe polyethylene glycol 600.

In some variations the liquid formulations described herein contain no greater than about 250 µl of polyethylene glycol. In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of polyethylene glycol. In some variations, the PEG is polyethylene glycol 400. The PEG may be polyethylene glycol 600.

In some variations, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of polyethylene glycol. In some variations, the PEG is polyethylene glycol 400. The PEG may be polyethylene glycol 600.

The solvent component may comprise ethanol, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. In some variations, ethanol comprises less than about 10% (w/w) of the liquid formulation. In some variations, ethanol comprises less than about 5% (w/w) of the liquid formulation. In some variations, ethanol comprises less than about 1% (w/w) of the liquid formulation. In some variations, ethanol comprises less than about 0.5% (w/w) of the liquid formulation. In some variations, ethanol comprises about 5% (w/w) of the liquid formulation. In some variations, ethanol comprises about 2.9% (w/w) of the liquid formulation. In some variations, ethanol comprises about 0.35% (w/w) of the liquid formulation.

In some variations the liquid formulations described herein contain no greater than about 250 µl of ethanol. In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of ethanol.

In some variations, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of ethanol.

The solvent component may comprise polyethoxylated castor oil *(e.g.,* Cremophor (PEG 35 castor oil)), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Polyethoxylated castor oil (*e.g*., Cremophor (PEG 35 castor oil)) may comprise less than about 10% (w/w) of the liquid formulation. Polyethoxylated castor oil (*e.g*., Cremophor (PEG 35 castor oil)) may comprise about 8.35% (w/w) of the liquid formulation. Polyethoxylated castor oil (*e.g*., Cremophor (PEG 35 castor oil)) may comprise about 7.18% (w/w) of the liquid formulation.

The liquid formulations described herein may contain no greater than about 250 µl of polyethoxylated castor oil (*e.g*., Cremophor (PEG 35 castor oil)). The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of polyethoxylated castor oil (*e.g*., Cremophor (PEG 35 castor oil)).

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of polyethoxylated castor oil (*e.g*., Cremophor (PEG 35 castor oil)).

The solvent component may comprise monoglycerides and/or diglycerides of caprylic acid (*e.g*., Capmul MCM (C8)), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Monoglycerides and/or diglycerides of caprylic acid (*e.g*., Capmul MCM (C8)) may comprise less than about 10% (w/w) of the liquid formulation. Monoglycerides and/or diglycerides of caprylic acid (*e.g*., Capmul MCM (C8)) may comprise about 9.47% (w/w) of the liquid formulation. Monoglycerides and/or diglycerides of caprylic acid (*e.g*., Capmul MCM (C8)) may comprise about 8.37% (w/w) of the liquid formulation. Monoglycerides and/or diglycerides of caprylic acid (*e.g.,* Capmul MCM (C8)) may comprise about 8.15% (w/w) of the liquid formulation. Monoglycerides and/or diglycerides of caprylic acid (*e.g*., Capmul MCM (C8)) may comprise about 6.81 % (w/w) of the liquid formulation.

The liquid formulations described herein may contain no greater than about 250 µl of monoglycerides and/or diglycerides of caprylic acid (*e.g*., Capmul MCM (C8)). In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of monoglycerides and/or diglycerides of caprylic acid (*e.g*., Capmul MCM (C8)).

For example, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of monoglycerides and/or diglycerides of caprylic acid (*e.g*., Capmul MCM (C8)).

The solvent component may comprise nonionic polymer of the alkyl aryl polyether alcohol (*e.g*., tyloxapol (ethoxylated p-tert-octylphenol formaldehyde polymer)), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. The nonionic polymer of the alkyl aryl polyether alcohol (*e.g*., tyloxapol (ethoxylated p-tert-octylphenol formaldehyde polymer)) may comprise less than about 10% (w/w) of the liquid formulation. The nonionic polymer of the alkyl aryl polyether alcohol (e.g., tyloxapol (ethoxylated p-tert-octylphenol formaldehyde polymer)) may comprise about 8.12% (w/w) of the liquid formulation. The nonionic polymer of the alkyl aryl polyether alcohol (*e.g*., tyloxapol (ethoxylated p-tert-octylphenol formaldehyde polymer)) may comprise about 6.81% (w/w) of the liquid formulation.

The liquid formulations described herein may contain no greater than about 250 µl of nonionic polymer of the alkyl aryl polyether alcohol (*e.g*., tyloxapol (ethoxylated p-tert-octylphenol formaldehyde polymer)). For example, the liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of nonionic polymer of the alkyl aryl polyether alcohol (*e.g*., tyloxapol (ethoxylated p-tert-octylphenol formaldehyde polymer)).

For example, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of nonionic polymer of the alkyl aryl polyether alcohol (*e.g*., tyloxapol (ethoxylated p-tert-octylphenol formaldehyde polymer)).

The solvent component may comprise 50% phosphatidylcholine in propylene glycol/ethanol carrier (*e.g*., Phosal® 50PG), for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. 50% phosphatidylcholine in propylene glycol/ethanol carrier (*e.g*., Phosal® 50PG) may comprise less than about 10% (w/w) of the liquid formulation. 50% phosphatidylcholine in propylene glycol/ethanol carrier (*e.g*., Phosal® 50PG) may comprise less than about 5% (w/w) of the liquid formulation. 50% phosphatidylcholine in propylene glycol/ethanol carrier (*e.g*., Phosal® 50PG) may comprise about 3.12% (w/w) of the liquid formulation.

The liquid formulations described herein may contain no greater than about 250 µl of 50% phosphatidylcholine in propylene glycol/ethanol carrier (*e.g*., Phosal® 50PG). In some variations the liquid formulations described herein contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of 50% phosphatidylcholine in propylene glycol/ethanol carrier (*e.g*., Phosal® 50PG).

For example, the liquid formulations described herein contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of 50% phosphatidylcholine in propylene glycol/ethanol carrier (*e.g*., Phosal® 50PG).

The solvent component may comprise propylene glycol monolaurate, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Propylene glycol monolaurate may comprise less than about 10% (w/w) of the liquid formulation. Propylene glycol monolaurate may comprise less than about 5% (w/w) of the liquid formulation. Propylene glycol monolaurate may comprise about 3.12% (w/w) of the liquid formulation.

The liquid formulations described herein may contain no greater than about 250 µl of propylene glycol monolaurate. The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of propylene glycol monolaurate.

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of propylene glycol monolaurate.

The solvent component may comprise propylene glycol dicaprylate/dicaprate, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Propylene glycol dicaprylate/dicaprate may comprise less than about 10% (w/w) of the liquid formulation. Propylene glycol dicaprylate/dicaprate may comprise less than about 5% (w/w) of the liquid formulation. Propylene glycol dicaprylate/dicaprate may comprise about 3.12% (w/w) of the liquid formulation.

The liquid formulations described herein may contain no greater than about 250 µl of propylene glycol dicaprylate/dicaprate. The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of propylene glycol dicaprylate/dicaprate.

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of propylene glycol dicaprylate/dicaprate.

The solvent component may comprise macrogol 15 hydroxystearate, for instance, between about 0.01 to about 99.9 % of the total weight of the composition; between about 0.1 to about 99%; between about 25 to about 55%; between about 30 to about 50%; or between about 35 to about 45%; between about 0.1 to about 10%; between about 10 to about 20%; between about 20 to about 30%; between about 30 to about 40%; between about 40 to about 45%; between about 40 to about 45%; between about 45 to about 50%; between about 50 to about 60%; between about 50 to about 70%; between about 70 to about 80%; between about 80 to about 90%; or between about 90 to about 100%. Macrogol 15 hydroxystearate may comprise less than about 10% (w/w) of the liquid formulation. Macrogol 15 hydroxystearate may comprise less than about 5% (w/w) of the liquid formulation. Macrogol 15 hydroxystearate may comprise about 3.12% (w/w) of the liquid formulation.

The liquid formulations described herein may contain no greater than about 250 µl of macrogol 15 hydroxystearate. The liquid formulations described herein may contain no greater than about 250 µl, no greater than about 200 µl, no greater than about 150 µl, no greater than about 125 µl, no greater than about 100 µl, no greater than about 75 µl, no greater than about 50 µl, no greater than about 25 µl, no greater than about 20 µl, no greater than about 15 µl, no greater than about 10 µl, no greater than about 7.5 µl, no greater than about 5 µl, no greater than about 2.5 µl, no greater than about 1.0 µl, or no greater than about 0.5 µl of macrogol 15 hydroxystearate.

The liquid formulations described herein may contain no greater than about 250 mg, no greater than about 200 mg, no greater than about 150 mg, no greater than about 125 mg, no greater than about 100 mg, no greater than about 75 mg, no greater than about 50 mg, no greater than about 25 mg, no greater than about 20 mg, no greater than about 15 mg, no greater than about 10 mg, no greater than about 7.5 mg, no greater than about 5 mg, no greater than about 2.5 mg, no greater than about 1.0 mg, or no greater than about 0.5 mg of macrogol 15 hydroxystearate.

The liquid formulation may contain a solvent component comprising about 10.5% of NMP, about 73.7% of propylene glycol, and about 15.8% of water. The liquid formulation may contain a solvent component comprising about 38.9% ofNMP, about 38.9% of propylene glycol, and about 22.2% of water. The liquid formulation may contain a solvent component comprising about 10.5% of NMP, about 73.7% of PEG600, and about 15.8% of water. The liquid formulation may contain a solvent component comprising about 10.5% of DMA, about 73.7% of propylene glycol, and about 15.8% of water. The liquid formulation may contain a solvent component comprising about 10.5% of DMSO, about 73.7% of propylene glycol, and about of 15.8% water. The liquid formulation may contain a solvent component comprising about 27.9% of NMP, about 39% of PEG400, and about 28.4 % of water. The liquid formulation further may contain a therapeutic agent. The therapeutic agent may be less than or equal to about 5% of the total weight of the liquid formulation. The therapeutic agent may be less than any of about 5%, 4%, 3%, 2%, or 1% of the total weight of the liquid formulation. The liquid formulation may be administered by subconjunctival, subtenon, and/or intravitreal injection. In some variations, the liquid formulation is used to treat or prevent an ocular disease. In some variations, the ocular disease is wet AMD. In some variations, the ocular disease is dry AMD. The ocular disease may be macular edema.

The liquid formulation may contain about 8.35% of polyethoxylated castor oil (*e.g*., Cremophor (PEG 35 castor oil)), about 8.37% of monoglycerides and/or diglycerides of caprylic acid (*e.g*., Capmul MCM (C8)), about 0.07% of therapeutic agent, and about 83.21% of aqueous liquid (*e.g*., water or saline). The liquid formulation may contain about 8.15% of monoglycerides and/or diglycerides of caprylic acid (*e.g*., Capmul MCM (C8)), about 8.12% of nonionic polymer of the alkyl aryl polyether alcohol (*e.g*., tyloxapol (ethoxylated p-tert-octylphenol formaldehyde polymer)), about 0.09% of therapeutic agent, about 0.35% of Ethanol, and about 83.29% of aqueous liquid (*e.g*., water or saline). The liquid formulation may contain about 6.81 % of nonionic polymer of the alkyl aryl polyether alcohol (*e.g*., tyloxapol (ethoxylated p-tert-octylphenol formaldehyde polymer)), about 6.81 % of monoglycerides and/or diglycerides of caprylic acid (*e.g*., Capmul MCM (C8)), about 0.08% therapeutic agent, about 3.12% of 50% phosphatidylcholine in propylene glycol/ethanol carrier (*e.g*., Phosal® 50PG), and about 83.21% aqueous liquid (*e.g*., water or saline). The liquid formulation may contain about 7.18% of polyethoxylated castor oil (*e.g*., Cremophor (PEG 35 castor oil)), about 9.47% of monoglycerides and/or diglycerides of caprylic acid (*e.g*., Capmul MCM (C8)), about 0.17% of therapeutic agent, and about 83.18% of aqueous liquid (*e.g*., water or saline). The therapeutic agent may be rapamycin. The therapeutic agent may be dasatinib. The liquid formulation may be topically administered. The liquid formulation may be administered as eye drops. The liquid formulation may be used to treat or prevent an ocular disease. The ocular disease may be wet AMD. The ocular disease may be dry AMD. According to the present description, the ocular disease may be macular edema.

In some variations, any of the liquid formulation described herein may be diluted. In some variations, the liquid formulation may be diluted any of about 5, about 4, about 3, about 2, about 1.9, about 1.8, about 1.7, about 1.6, about 1.5, about 1.4, about 1.3, about 1.2, about 1.1 fold. In some variations, the liquid formulation is diluted 1.5 fold. In some variations, the liquid formulation is dilute about 1.4 fold. In some variations, the liquid formulation is diluted with an aqueous liquid (such as water or saline). In some variations, the liquid formulation is diluted with saline. In some variations, the liquid formulation is diluted with water. Fold dilution, as used herein, refers to the final total volume of the diluted liquid formulation divided by the total volume of undiluted liquid formulation added to make the dilution. For example, a 1.5 fold dilution would result if 500 µl of an undiluted liquid formulation was added to 250 µl of saline to result in a final total volume of the diluted liquid formulation of 750 µl (750 µl of final total volume of the diluted liquid formulation/ 500 µl of the total volume of undiluted liquid formulation = 1.5). In some variations, the dilution is a solution. In some variations, the solution is a clear solution. In some variations, the dilution is a suspension. In some variations, the suspension is a milky suspension. In some variations, the dilution is made prior to distribution to the patient or physician. In some variations, the dilution is made by the patient or physician. In some variations, the dilution is made prior to administration. In some variations, the entire diluted liquid formulation is administered to the subject. In some variations, a portion of the diluted liquid formulation is administered to the subject. In some variations, a portion of the diluted liquid formulation equal to the volume of the undiluted liquid formulation is administered to the subject.

Some examples and variations of liquid formulations described herein were prepared and are listed in Table 1. Depending on their type, the listed formulations are denoted one or more of solutions ("S"), suspensions ("SP"), emulsions ("E") or in situ gelling ("ISG"). Median particle size is listed for some of the suspensions. As described herein, some liquid formulations form a non-dispersed mass after, for example, injection into an aqueous environment such as the vitreous of an eye. For those formulations injected into the vitreous of a rabbit eye, the right-hand column of Table 1 indicates whether or not a non-dispersed mass (NDM) formed after a specified volume was injected into the vitreous of the rabbit eye.

### Liquid formulations which form a non-dispersed mass

One class of liquid formulations described herein forms a non-dispersed mass when placed in an aqueous medium. As used herein, a "non-dispersed mass" refers to the structure formed or shape assumed when the liquid formulation is placed into an environment, relative to the environment in which it is placed.

Generally, a non-dispersed mass of a liquid formulation is anything other than a homogeneous distribution of the liquid formulation in the surrounding medium. The non-dispersed mass may, for instance, be indicated by visually inspecting the administered liquid formulation and characterizing its appearance relative to the surrounding medium.

According to the present description, the aqueous medium may be water. The water may be deionized, distilled, sterile, or tap water, such as tap water available at the place of business of MacuSight in Union City, California.

According to the present description, the aqueous medium may be an aqueous medium of a subject. The aqueous medium may be an aqueous medium of the eye of a subject, such as the vitreous of an eye of a subject. The subject may be a human subject. The subject may be a rabbit.

The liquid formulation may form a non-dispersed mass when exposed to a certain temperature or range of temperatures, such as room temperature, about ambient temperature, about 30°C, about 37°C, or about the temperature of the aqueous medium of the subject.

The liquid formulation may form a non-dispersed mass when exposed to a certain pH or range of pH, such as a pH between about 6 and about 8.

The non-dispersed mass may comprise a gel or gel-like substance.

The non-dispersed mass may comprise a polymer matrix. The non-dispersed mass may comprise a polymer matrix in which a therapeutic agent is dispersed.

The liquid formulations described herein may generally be of any geometry or shape after administration to a subject or the eye of a subject, such as a human subject. The non-dispersed mass may be between about 0.1 and about 5 mm. The non-dispersed mass may be between about 1 and about 3 mm. The non-dispersed mass-forming liquid formulations may, for instance, appear as a compact spherical mass when administered to the vitreous. In some instances, the liquid formulation may appear as a non-dispersed mass relative to the surrounding medium, wherein the non-dispersed mass is less clearly defined and the geometry is more amorphous than spherical.

The non-dispersed mass-forming liquid formulations described herein may form a non-dispersed mass immediately upon placement in the medium or the non-dispersed mass may form some period of time after placement of the liquid formulation. In some variations the non-dispersed mass forms over the course of about 1, about 2, about 3, about 4, about 5, about 6, or about 7 days. In some variations the non-dispersed mass forms over the course of about 1 week, about 2 weeks, or about 3 weeks.

The liquid formulations described herein that form a non-dispersed mass may appear as a milky or whitish colored semi-contiguous or semi-solid non-dispersed mass relative to the medium in which it is placed.

One liquid formulation described herein forms a non-dispersed mass which has the form of a solid depot when the formulation is injected into any or all of water, the vitreous of a rabbit eye, or between the sclera and the conjunctiva of a rabbit eye. One liquid formulation described herein forms a non-dispersed mass which has the form of a semi-solid when the formulation is injected into any or all of water, the vitreous of a rabbit eye, or between the sclera and the conjunctiva of a rabbit eye. One liquid formulation described herein forms a non-dispersed mass which has the form of a polymeric matrix when the formulation is injected into any or all of water, the vitreous of a rabbit eye, or between the sclera and the conjunctiva of a rabbit eye. One liquid formulation described herein forms a non-dispersed mass which has the form of a gel, a hydrogel, or a gel-like substance when the formulation is injected into any or all of water, the vitreous of a rabbit eye, or between the sclera and the conjunctiva of a rabbit eye.

In some examples described herein the liquid formulation forms a non-dispersed mass relative to a surrounding medium where the surrounding medium is aqueous. An "aqueous medium" or "aqueous environment" is one that contains at least about 50% water. Examples of aqueous media include water, the vitreous, extracellular fluid, conjunctiva, sclera, between the sclera and the conjunctiva, aqueous humor, gastric fluid, and any tissue or body fluid comprised of at least about 50% of water. Aqueous media include gel structures, such as the conjunctiva and sclera.

The liquid formulations described herein may form a non-dispersed mass when a test volume of the liquid formulation is placed in the vitreous of a rabbit eye. The test volume may be administered to a rabbit eye, and the test volume is equal to the volume of the liquid formulation administered to a subject's, such as a human subject's eye.

The test volume administered to a rabbit eye may be equal to the volume administered to the subject's eye multiplied by a scale factor, and the scale factor is equal to the average volume of a rabbit eye divided by the average volume of a subject eye. The "average volume" of an eye, as used herein, refers to the average volume of an eye of a member of similar age of the species under consideration generally, as opposed to the average volume of any particular individual's eye.

The test volume administered to the rabbit eye may be between about 10 µl and about 50 µl The test volume administered to the rabbit eye may be between about 1 µl and about 30 µl. The test volume administered to the rabbit eye may be between about 50 µl and about 100 µl. The test volume administered to the rabbit eye may be between about 25 µl and about 75 µl. The test volume administered to the rabbit eye may be about 30 µl.

The liquid formulation that forms a non-dispersed mass when placed in the medium may comprises a therapeutic agent or agents with a concentration of between about 0.01 % and about 10% by weight of the total, and a solvent between about 10 % and about 99 % by weight of the total. The formulation may further comprise a solubilizing agent such as a surfactant. The liquid formulation may further comprise a stabilizing agent, excipient, adjuvant, or antioxidant, etc., between about 0 and about 40% by weight of the total. The therapeutic agent may be about 5% by weight of the total, and the solvent component is about 95% by weight of the total.

Whether a liquid formulation exhibits a non-dispersed mass relative to a surrounding medium when present in a subject, such as a human subject or the eye of a subject may be determined by, for instance, mixing a therapeutic agent with a solvent, administering it to the vitreous of an eye of a subject, such as a human subject, and comparing the liquid formulation to the surrounding medium.

One liquid formulation that may be used for treating, preventing, inhibiting, delaying the onset of, or causing the regression of the diseases and conditions of a subject, such as a human subject, is a liquid formulation that forms a non-dispersed mass when placed into the vitreous of a rabbit eye. When used for treating, preventing, inhibiting, delaying the onset of, or causing the regression of the disease or condition of the subject, the liquid formulation is administered to the subject. The liquid formulation may or may not form a non-dispersed mass in the subject. One liquid formulation described herein forms a non-dispersed mass when administered to a subject and forms a non-dispersed mass when administered to a rabbit eye.

Without being bound by theory, it is believed that the low solubility of rapamycin in the vitreous contributes to the formation of a non-dispersed mass by some rapamycin-containing liquid formulations described herein. The vitreous is a clear gel composed almost entirely of water (up to 99%). Without being bound by theory, it is believed that as rapamycin in an injected formulation contacts the vitreous, the rapamycin precipitates.

Without being bound by theory, factors believed to affect the formation of and geometry of a non-dispersed mass include the concentration of rapamycin in the formulation, the viscosity of the formulation, ethanol content of the formulation, and the volume of injection. It is believed that maintaining a higher local concentration of rapamycin after injection of the formulation favors formation of a non-dispersed mass, as opposed to a lower local concentration of rapamycin after injection of the formulation. As volume is increased for a given dose, formation of a non-dispersed mass may become less favorable. Formation of a non-dispersed mass may become more favorable as rapamycin concentration is increased and/or as viscosity is increased. Ethanol content affects both the solubility of the rapamycin in the formulation and the viscosity of the formulation.

In one comparison, 100 µl of a solution of 0.4 % rapamycin, 4.0% ethanol, and 95.6% PEG 400 (a 400 µg dose) did not form a non-dispersed mass after injection into a rabbit eye. In contrast, 20 µl of a solution of 2.00 % rapamycin, 4.0% ethanol, and 94% PEG 400 (also a 400 µg dose) formed a compact spherical non-dispersed mass after injection into a rabbit eye.

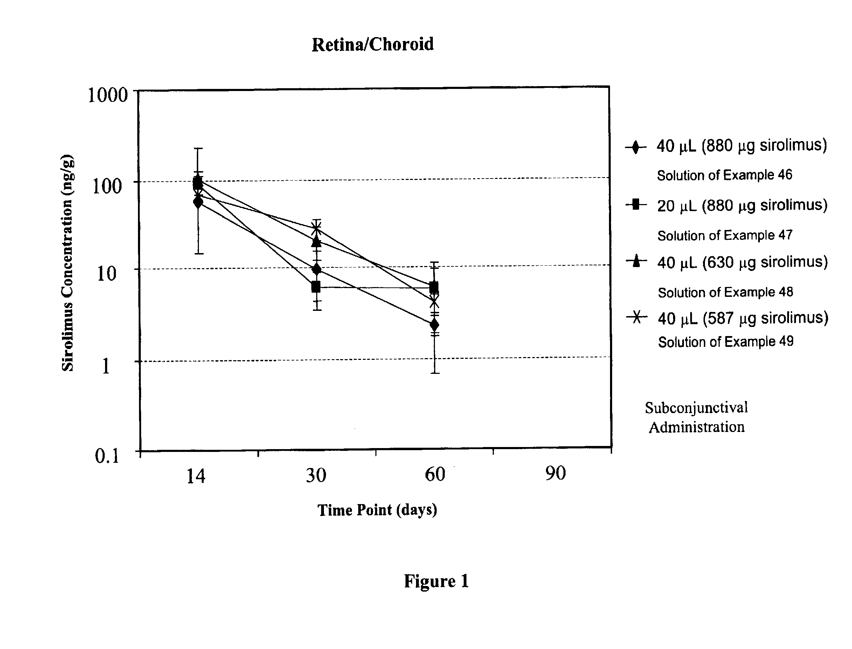

Without being bound by theory, in the latter example, it is hypothesized that formation of the non-dispersed mass occurred as depicted in Figures 1A-1C and described as follows. Upon injection, due to its viscosity the liquid formulation formed a spherical globule 100 within the vitreous 110. Ethanol then diffused out of this globule, resulting in localized precipitation 120 of the rapamycin within the globule. Eventually, the polyethylene glycol also diffused out of the globule to leave a solid, compact non-dispersed mass of rapamycin 130.

The non-dispersed masses described herein may consist of at least about 20%, at least about 30%, at least about 40%, at least about 50%, at least about 60%, at least about 70%, at least about 80%, at least about 90%, or at least about 95% by volume of therapeutic agent when injected into the vitreous of a rabbit eye.

Upon formation of a non-dispersed mass comprising rapamycin, for example, may deliver the drug continuously at approximately a constant rate for an extended period of time. Without being bound by theory, it is believed that delivery of rapamycin from a non-dispersed mass in the vitreous depends on dissolution of the rapamycin in the vitreous, which depends in turn on clearance of the drug from the vitreous to other tissues. Without being bound by theory, this release process is believed to maintain a steady-state concentration of rapamycin in the vitreous.

Formation of a non-dispersed mass may reduce the toxicity of the injected liquid formulation compared to an equivalent dose that did not form a non-dispersed mass. In alternatives in which a liquid formulation injected into the vitreous does not form a non-dispersed mass, the drug (e.g., rapamycin) appears to disperse in the vitreous body. This may interfere with vision.

The liquid formulations that are suspensions may form a non-dispersed mass upon injection into the vitreous. Formation of a non-dispersed mass from an injected suspension may become more favorable as the suspension particle size increases.

According to the present description, it is believed that the liquid formulations will form a visually observable non-dispersed mass when injected into the eye of a subject, such as a human subject.

The liquid formulations are believed to form non-dispersed masses when injected subconjunctivally. It is believed that when subconjunctivally administered the liquid formulation may form a depot in the scleral tissue. That is, it is believed that the therapeutic agent is absorbed into the sclera proximate to the injection site and forms a local concentration of drug in the sclera.

### In situ Gelling Formulations

Described herein are non-dispersed mass-forming liquid formulations which form a gel or gel-like substance when placed in an aqueous medium. The non-dispersed mass may comprise a gel; in some variations the gel is a hydrogel.

An "in situ gelling formulation," as used herein, refers to a liquid formulation which forms a gel-like non-dispersed mass when the liquid formulation is placed in an aqueous medium, such as water, the vitreous of a rabbit eye, and between the sclera and the conjunctiva of a rabbit eye. An in situ gelling formulation may form a gel-like non-dispersed mass when placed in tap water.

The in situ gelling formulation may be a suspension prior to placement in an aqueous medium, and forms a gel in situ upon placement in an aqueous medium. The in situ gelling formulation may be a solution prior to placement in an aqueous medium, and forms a gel in situ upon placement in an aqueous medium. The in situ gelling formulation may be an emulsion prior to placement in an aqueous medium, and forms a gel in situ upon placement in an aqueous medium. In some alternatives a gel-like non-dispersed mass forms after placement of the in situ gelling formulation into an aqueous medium, such as water, the vitreous, or between the sclera and the conjunctiva of an eye. The in situ gel may be formed of a polymer matrix. A therapeutic agent may be dispersed in the polymer matrix.

Described herein are in situ gelling formulations which may be used for treating, preventing, inhibiting, delaying the onset of, or causing the regression of the diseases and conditions of a subject such as a human subject. When used for treating, preventing, inhibiting, delaying the onset of, or causing the regression of the disease or condition of the subject, the in situ gelling formulation is administered to the subject. One liquid formulation described herein comprises an in situ gelling formulation which forms a non-dispersed mass when administered to a subject and forms a non-dispersed mass when administered to a rabbit eye.

In some examples, the in situ gelling formulation may comprise one or more polymers. Described herein are various types of polymers, including polymers which are solvents, polymers which are solubilizing agents, polymers which are release modifying agents, polymers which are stabilizing agents, etc. In some variations, any combination of polymers is used wherein the polymers when combined with the therapeutic agent form any or all of a non-dispersed mass, a gel, a hydrogel, or polymeric matrix when placed in an aqueous medium, such as water, the vitreous, or between the sclera and the conjunctiva.

The in situ gelling formulation may deliver extended release of therapeutic agents to a subject when administered to the subject.

The liquid formulation may comprise a therapeutic agent and a plurality of polymers, wherein one of the polymers is a polymethacrylate. Polymethacrylates are known by various names and are available in various preparations, such as polymeric methacrylates, methacrylic acid-ethyl acrylate copolymer (1:1), methacrylic acid-ethyl acrylate copolymer (1:1) dispersion 30 per cent, methacrylic acid-methyl methacrylate copolymer (1:1), methacrylic acid-methyl methacrylate copolymer (1:2), acidum methacrylicum et ethylis acrylas polymerisatum 1:1, acidum methacrylicum et ethylis acrylas polymerisatum 1:1 dispersio 30 per centum, acidum methacrylicum et methylis methacrylas polymerisatum 1:1, acidum methacrylicum et methylis methacrylas polymerisatum 1:2, USPNF: ammonio methacrylate copolymer, methacrylic acid copolymer, methacrylic acid copolymer dispersion.

In some examples, one of the polymers is polyvinylpyrrolidone. Polyvinylpyrrolidone is known by various names and is available in various preparations, such as to povidone, povidonum, kollidon; plasdone; poly[1-(2-oxo-1-pyrrolidinyl)ethylene]; polyvidone; PVP; 1-vinyl-2- pyrrolidinone polymer, and 1-Ethenyl-2-pyrrolidinone homopolymer.

One liquid formulation described herein comprises a therapeutic agent and a solvent component. The solvent component may comprise a single solvent or a combination of solvents.

In some examples, the solvent is glycerin, dimethylsulfoxide, N-methylpyrrolidone, ethanol, isopropyl alcohol, polyethylene glycol of various molecular weights, such as PEG 300 and PEG 400, or propylene glycol, or a mixture of one or more thereof.

In some variations, the solvent is polyethylene glycol. Polyethylene glycol is known by various names and is available in various preparations (e.g., macrogel 400, macrogel 1500, macrogel 4000, macrogel 6000, macrogel 20000, macrogola, breox PEG; carbowax; carbowax sentry; Hodag PEG; Lipo; Lipoxol; Lutrol E; PEG; Pluriol E; polyoxyethylene glycol, and α-Hydro-co-hydroxy-poly(oxy-1,2-ethanediyl)).

### Compositions and liquid formulations for delivery of therapeutic agents

The compositions and liquid formulations described herein may be used to deliver amounts of the therapeutic agents effective for treating, preventing, inhibiting, delaying on set of, or causing the regression of the diseases and conditions described in the *Diseases and Conditions* section. In some variations the compositions and liquid formulations described herein deliver one or more therapeutic agents over an extended period of time.

An "effective amount," which is also referred to herein as a "therapeutically effective amount," of a therapeutic agent for administration as described herein is that amount of the therapeutic agent that provides the therapeutic effect sought when administered to the subject, such as a human subject. The achieving of different therapeutic effects may require different effective amounts of therapeutic agent. For example, the therapeutically effective amount of a therapeutic agent used for preventing a disease or condition may be different from the therapeutically effective amount used for treating, inhibiting, delaying the onset of, or causing the regression of the disease or condition. In addition, the therapeutically effective amount may depend on the age, weight, and other health conditions of the subject as is well known to those versed in the disease or condition being addressed. Thus, the therapeutically effective amount may not be the same in every subject to which the therapeutic agent is administered.

An effective amount of a therapeutic agent for treating, preventing, inhibiting, delaying the onset of, or causing the regression of a specific disease or condition is also referred to herein as the amount of therapeutic agent effective to treat, prevent, inhibit, delay the onset of, or cause the regression of the disease or condition.

To determine whether a level of therapeutic agent is a "therapeutically effective amount" to treat, prevent, inhibit, delay on set of, or cause the regression of the diseases and conditions described in the *Diseases and Conditions* section, liquid formulations may be administered in animal models for the diseases or conditions of interest, and the effects may be observed. In addition, dose ranging human clinical trials may be conducted to determine the therapeutically effective amount of a therapeutic agent.

Generally, the therapeutic agent may be formulated in any composition or liquid formulation capable of delivery of a therapeutically effective amount of the therapeutic agent to a subject or to the eye of a subject for the required delivery period. Compositions include liquid formulations.

### Solubilization of therapeutic agents

One composition or liquid formulation that may be used is a composition or liquid formulation in which the therapeutic agent is dissolved in a solvent component. Generally, any solvent which has the desired effect may be used in which the therapeutic agent dissolves. According to some examples the solvent is aqueous. In some examples the solvent is non-aqueous. An "aqueous solvent" is a solvent that contains at least about 50% water.

Generally, any concentration of solubilized therapeutic agent that has the desired effect can be used. The solvent component may be a single solvent or may be a mixture of solvents. The solvent component may be a single solvent or may be a mixture of solvents. Solvents and types of solutions are well known to those versed in such drug delivery technologies. See for example, Remington: The Science and Practice of Pharmacy, Twentieth Edition, Lippincott Williams & Wilkins; 20th edition (December 15, 2000); Ansel's Pharmaceutical Dosage Forms and Drug Delivery Systems, Eighth Edition, Lippincott Williams & Wilkins (August 2004); Handbook Of Pharmaceutical Excipients 2003, American Pharmaceutical Association, Washington, DC, USA and Pharmaceutical Press, London, UK; and Strickley, solubilizing Excipients in Oral and Injectable Formulations, Pharmaceutical Research, Vol. 21, No. 2, February 2004.

As noted previously, some solvents may also serve as solubilizing agents.

Exemplary solvents that may be used include DMSO, ethanol, methanol, isopropyl alcohol; castor oil, propylene glycol, glycerin, polysorbate 80, benzyl alcohol, dimethyl acetamide (DMA), dimethyl formamide (DMF), triacetin, diacetin, corn oil, acetyl triethyl citrate (ATC), ethyl lactate, glycerol formal, ethoxy diglycol (Transcutol, Gattefosse), tryethylene glycol dimethyl ether (Triglyme), dimethyl isosorbide (DMI), γ-butyrolactone, N-Methyl-2-pyrrolidinone (NMP), polyethylene glycol of various molecular weights, such as PEG 300 and PEG 400, and polyglycolated capryl glyceride (Labrasol, Gattefosse), combinations of any one or more of the foregoing, or analogs or derivatives of any one or more of the foregoing.

In some variations, the solvent is a polyethylene glycol. Polyethylene glycol is known by various names and is available in various preparations (e.g., macrogel 400, macrogel 1500, macrogel 4000, macrogel 6000, macrogel 20000, macrogola, breox PEG; carbowax; carbowax sentry; Hodag PEG; Lipo; Lipoxol; Lutrol E; PEG; Pluriol E; polyoxyethylene glycol, and α-Hydro-ω-hydroxy-poly(oxy-1,2-ethanediyl)).

In some variations the polyethylene glycol is a liquid PEG, and is one or more of PEG 300 or PEG 400.

Other solvents may include an amount of a C₆-C₂₄ fatty acid sufficient to solubilize a therapeutic agent.

Phospholipid solvents may also be used, such as lecithin, phosphatidylcholine, or a mixture of various diglycerides of stearic, palmitic, and oleic acids, linked to the choline ester of phosphoric acid; hydrogenated soy phosphatidylcholine (HSPC), distearoylphosphatidylglycerol (DSPG), L-α-dimyristoylphosphatidylcholine (DMPC), L-α-dimyristoylphosphatidylglycerol (DMPG).

Further examples of solvents include, for example, components such as alcohols, propylene glycol, polyethylene glycol of various molecular weights, propylene glycol esters, propylene glycol esterified with fatty acids such as oleic, stearic, palmic, capric, linoleic, etc.; medium chain mono-, di-, or triglycerides, long chain fatty acids, naturally occurring oils, and a mixture thereof. The oily components for the solvent system include commercially available oils as well as naturally occurring oils. The oils may further be vegetable oils or mineral oils. The oils can be characterized as non-surface active oils, which typically have no hydrophile lipophile balance value. Exemplary commercially available substances comprising medium chain triglycerides include Captex 100, Captex 300, Captex 355, Miglyol 810, Miglyol 812, Miglyol 818, Miglyol 829, and Dynacerin 660. Propylene glycol ester compositions that are commercially available encompass Captex 200 and Miglyol 840, and the like. The commercial product, Capmul MCM, comprises one of many possible medium chain mixtures comprising monoglycerides and diglycerides.

Other solvents include naturally occurring oils such as peppermint oil, and seed oils. Exemplary natural oils include oleic acid, castor oil, safflower seed oil, soybean oil, olive oil, sunflower seed oil, sesame oil, and peanut oil. Soy fatty acids may also be used. Examples of fully saturated non-aqueous solvents include esters of medium to long chain fatty acids (such as fatty acid triglycerides with a chain length of about C₆ to about C₂₄). Hydrogenated soybean oil and other vegetable oils may also be used. Mixtures of fatty acids may be split from the natural oil (for example coconut oil, palm kernel oil, babassu oil, or the like) and refined. Medium chain (about C₈ to about C₁₂) triglycerides, such as caprilyic/capric triglycerides derived from coconut oil or palm seed oil, may be used. Medium chain mono- and diglycerides may also be used. Other fully saturated non-aqueous solvents include saturated coconut oil (which typically includes a mixture of lauric, myristic, palmitic, capric and caproic acids), including those sold under the Miglyol™ from Huls and bearing trade designations 810, 812, 829 and 840. Also noted are the NeoBee™ products sold by Drew Chemicals. Non-aqueous solvents include isopropyl myristate. Examples of synthetic oils include triglycerides and propylene glycol diesters of saturated or unsaturated fatty acids having 6 to 24 carbon atoms such as, for example hexanoic acid, octanoic (caprylic), nonanoic (pelargonic), decanoic (capric), undecanoic, lauric, tridecanoic, tetradecanoic (myristic), pentadecanoic, hexadecanoic (palmitic), heptadecanoic, octadecanoic (stearic), nonadecanoic, heptadecanoic, eicosanoic, heneicosanoic, docosanoic and lignoceric acids, and the like. Examples of unsaturated carboxylic acids include oleic, linoleic and linolenic acids, and the like. The non-aqueous solvent can comprise the mono-, di- and triglyceryl esters of fatty acids or mixed glycerides and/or propylene glycol mono- or diesters wherein at least one molecule of glycerol has been esterified with fatty acids of varying carbon atom length. An example of a "non-oil" useful as a solvent is polyethylene glycol.

Exemplary vegetable oils include cottonseed oil, corn oil, sesame oil, soybean oil, olive oil, fractionated coconut oil, peanut oil, sunflower oil, safflower oil, almond oil, avocado oil, palm oil, palm kernel oil, babassu oil, beechnut oil, linseed oil, rape oil and the like. Mono-, di-, and triglycerides of vegetable oils, including such as corn, may also be used.

Polyvinyl pyrrolidone (PVP), cross-linked or not, may also be used as a solvent. Further solvents include C₆-C₂₄ fatty acids, oleic acid, Imwitor 742, Capmul, F68, F68 (Lutrol), PLURONICS such as PLURONICS F108, F127, and F68, Poloxamers, Jeffamines), Tetronics, F127; cyclodextrins such as α-cyclodextrin, β-cyclodextrin, hydroxypropyl- β-cyclodextrin, sulfobutylether- β-cyclodextrin (Captisol); CMC, polysorbitan 20, Cavitron, polyethylene glycol of various molecular weights such as PEG 300 and PEG 400.

Beeswax and *d*-α-tocopherol (Vitamin E) may also be used as solvents.

In some examples, the solvent is N-methyl pyrrolidone (NMP), dimethyl-acetamine (DMA), dimethyl sulfoxide (DMSO), propylene glycol (PG), polyethylene glycol 600 (PEG 600), polyethylene glycol 400 (PEG 400), ethanol, or a mixture of one or more thereof. In some examples, the solvent comprises a combination of solvents including N-methyl pyrrolidone (NMP), dimethyl-acetamine (DMA), or dimethyl sulfoxide (DMSO). In some examples, the solvent comprises a combination of solvents including propylene glycol (PG), polyethylene glycol 600 (PEG 600), or polyethylene glycol 400 (PEG 400). In some examples, the solvent may comprise a combination of at least two solvents. In some examples, the at least two solvents comprising a first solvent such as N-methyl pyrrolidone (NMP), dimethyl-acetamine (DMA), or dimethyl sulfoxide (DMSO) and a second solvent such as propylene glycol (PG), polyethylene glycol 600 (PEG 600), or polyethylene glycol 400 (PEG 400). In some examples, the solvent may comprise N-methyl pyrrolidone (NMP) and propylene glycol (PG). In some examples, the solvent may comprise N-methyl pyrrolidone (NMP) and polyethylene glycol 600 (PEG 600). In some variations, the solvent may comprise dimethyl-acetamine (DMA) and propylene glycol (PG). In some examples, the solvent component may comprise dimethyl-acetamine (DMA) and polyethylene glycol 400 (PEG 400). In some examples, the solvent may comprise dimethyl sulfoxide (DMSO) and propylene glycol (PG). In some examples, the solvent may comprise N-methyl pyrrolidone (NMP) and polyethylene glycol 400 (PEG 400). In some examples, the solvent may further comprise ethanol. In some examples, the solvent may further comprise water. In some examples, the solvent may have a hygroscopicity that is about equal to, less than, or greater than that of polyethylene glycol 400 (PEG 400).

In some examples, the solvent is polyethoxylated castor oil (*e.g*., Cremophor (PEG 35 castor oil)), monoglycerides and/or diglycerides of caprylic acid (*e.g*., Capmul MCM (C8)), nonionic polymer of the alkyl aryl polyether alcohol (*e.g*., tyloxapol (ethoxylated p-tert-octylphenol formaldehyde polymer)), Phosal® 50PG, ethanol, or a mixture of one or more thereof. In some variation, the solvent may comprise a combination of at least two solvents. In some examples, the at least two solvents comprising a first solvent such as polyethoxylated castor oil (*e.g*., Cremophor (PEG 35 castor oil)) or nonionic polymer of the alkyl aryl polyether alcohol (*e.g*., tyloxapol (ethoxylated p-tert-octylphenol formaldehyde polymer)) and a second solvent such as monoglycerides and/or diglycerides of caprylic acid (*e.g*., Capmul MCM (C8)). In some variations, the solvent may further comprise 50% phosphatidylcholine in propylene glycol/ethanol carrier (*e.g*., Phosal® 50PG).

Solvents for use in the liquid formulations can be determined by a variety of methods known in the art, such as (1) theoretically estimating their solubility parameter values and choosing the ones that match with the therapeutic agent, using standard equations in the field; and (2) experimentally determining the saturation solubility of therapeutic agent in the solvents, and choosing the ones that exhibit the desired solubility.

### Solubilization of rapamycin

Where the therapeutic agent is rapamycin, solvents that may be used for making solutions or suspensions of rapamycin include solvents containing any one or more of DMSO, glycerin, ethanol, methanol, isopropyl alcohol; castor oil, propylene glycol, polyvinylpropylene, glycerin, polysorbate 80, benzyl alcohol, dimethyl acetamide (DMA), dimethyl formamide (DMF), glycerol formal, ethoxy diglycol (Transcutol, Gattefosse), tryethylene glycol dimethyl ether (Triglyme), dimethyl isosorbide (DMI), γ-butyrolactone, N-Methyl-2-pyrrolidinone (NMP), polyethylene glycol of various molecular weights, such as PEG 300 and PEG 400, and polyglycolated capryl glyceride (Labrasol, Gattefosse).

Further solvents include C₆-C₂₄ fatty acids, oleic acid, Imwitor 742, Capmul, F68, F68 (Lutrol), PLURONICS such as PLURONICS F108, F127, and F68, Poloxamers, Jeffamines, Tetronics, F127, beta-cyclodextrin, CMC, polysorbitan 20, Cavitron, softigen 767, captisol, and sesame oil.

Other methods that may be used to dissolve rapamycin are described in Solubilization of Rapamycin, P. Simamora et al. Int'l J. Pharma 213 (2001) 25-29.

As an example, rapamycin can be dissolved in 5% DMSO or methanol in a balanced salt solution. The rapamycin solution can be unsaturated, a saturated or a supersaturated solution of rapamycin. The rapamycin solution can be in contact with solid rapamycin. In one example, rapamycin can be dissolved in a concentration of up to about 400 mg/ml. Rapamycin can also, for example, be dissolved in propylene glycol esterified with fatty acids such as oleic, stearic, palmic, capric, linoleic, etc.

According to some examples, the solvent is N-methyl pyrrolidone (NMP), dimethyl-acetamine (DMA), dimethyl sulfoxide (DMSO), propylene glycol (PG), polyethylene glycol 600 (PEG 600), polyethylene glycol 400 (PEG 400), ethanol, or a mixture of one or more thereof. According to some examples, the solvent comprises a combination of solvents including N-methyl pyrrolidone (NMP), dimethyl-acetamine (DMA), or dimethyl sulfoxide (DMSO). According to some examples, the solvent comprises a combination of solvents including propylene glycol (PG), polyethylene glycol 600 (PEG 600), or polyethylene glycol 400 (PEG 400). According to some examples, the solvent may comprise a combination of at least two solvents. According to some examples, the at least two solvents comprising a first solvent such as N-methyl pyrrolidone (NMP), dimethyl-acetamine (DMA), or dimethyl sulfoxide (DMSO) and a second solvent such as propylene glycol (PG), polyethylene glycol 600 (PEG 600), or polyethylene glycol 400 (PEG 400). According to some examples, the solvent may comprise N-methyl pyrrolidone (NMP) and propylene glycol (PG). In some variations, the solvent may comprise N-methyl pyrrolidone (NMP) and polyethylene glycol 600 (PEG 600). According to some examples, the solvent may comprise dimethyl-acetamine (DMA) and propylene glycol (PG). According to some examples, the solvent component may comprise dimethyl-acetamine (DMA) and polyethylene glycol 400 (PEG 400). According to some examples, the solvent may comprise dimethyl sulfoxide (DMSO) and propylene glycol (PG). According to some examples, the solvent may comprise N-methyl pyrrolidone (NMP) and polyethylene glycol 400 (PEG 400). According to some examples, the solvent may further comprise ethanol. According to some examples, the solvent may further comprise water. In some variations, the solvent may have a hygroscopicity that is about equal to, less than, or greater than that of polyethylene glycol 400 (PEG 400).

According to some examples, the solvent is polyethoxylated castor oil *(e.g.,* Cremophor (PEG 35 castor oil)), monoglycerides and/or diglycerides of caprylic acid *(e.g.,* Capmul MCM (C8)), nonionic polymer of the alkyl aryl polyether alcohol (*e.g*., tyloxapol (ethoxylated p-tert-octylphenol formaldehyde polymer)), Phosal® 50PG, ethanol, or a mixture of one or more thereof. According to some examples, the solvent may comprise a combination of at least two solvents. According to some examples, the at least two solvents comprising a first solvent such as polyethoxylated castor oil (*e.g*., Cremophor (PEG 35 castor oil)) or nonionic polymer of the alkyl aryl polyether alcohol (*e.g*., tyloxapol (ethoxylated p-tert-octylphenol formaldehyde polymer)) and a second solvent such as monoglycerides and/or diglycerides of caprylic acid (*e.g*., Capmul MCM (C8)). According to some examples, the solvent may further comprise 50% phosphatidylcholine in propylene glycol/ethanol carrier (*e.g*., Phosal® 50PG). According to some examples, the solvent may further comprise ethanol. According to some examples, the solvent may further comprise water.

Many other solvents are possible. Those of ordinary skill in the art will find it routine to identify solvents for rapamycin given the teachings herein.

### Solubilizing Agents

Generally, any solubilizing agent or combination of solubilizing agents may be used in the liquid formulations described herein.

According to some examples, the solubilizing agent is a surfactant or combination of surfactants. Many surfactants are possible. Combinations of surfactants, including combinations of various types of surfactants, may also be used. For instance, surfactants which are nonionic, anionic (i.e. soaps, sulfonates), cationic (i.e. CTAB), zwitterionic, polymeric or amphoteric may be used.

Surfactants that can be used may be determined by mixing a therapeutic agent of interest with a putative solvent and a putative surfactant, and observing the characteristics of the formulation after exposure to a medium.

Examples of surfactants include fatty acid esters or amides or ether analogues, or hydrophilic derivatives thereof; monoesters or diesters, or hydrophilic derivatives thereof; or mixtures thereof; monoglycerides or diglycerides, or hydrophilic derivatives thereof; or mixtures thereof; mixtures having enriched mono- or/and diglycerides, or hydrophilic derivatives thereof; surfactants with a partially derivatized with a hydrophilic moiety; monoesters or diesters or multiple-esters of other alcohols, polyols, saccharides or oligosaccharides or polysaccharides, oxyalkylene oligomers or polymers or block polymers, or hydrophilic derivatives thereof, or the amide analogues thereof; fatty acid derivatives of amines, polyamines, polyimines, aminoalcohols, aminosugars, hydroxyalkylamines, hydroxypolyimines, peptides, polypeptides, or the ether analogues thereof.

Hydrophilic Lipophilic Balance ("HLB") is an expression of the relative simultaneous attraction of a surfactant for water and oil (or for the two phases of the emulsion system being considered).

Surfactants are characterized according to the balance between the hydrophilic and lipophilic portions of their molecules. The hydrophilic-lipophilic balance (HLB) number indicates the polarity of the molecule in an arbitrary range of 1 - 40, with the most commonly used emulsifiers having a value between 1 - 20. The HLB increases with increasing hydrophilicity.

Surfactants that may be used include those with an HLB greater than 10, 11, 12, 13 or 14. Examples of surfactants include polyoxyethylene products of hydrogenated vegetable oils, polyethoxylated castor oils or polyethoxylated hydrogenated castor oil, polyoxyethylene-sorbitan-fatty acid esters, polyoxyethylene castor oil derivatives and the like, for example, Nikkol HCO-50, Nikkol HCO-35, Nikkol HCO-40, Nikkol HCO-60 (from Nikko Chemicals Co. Ltd.); Cremophor (from BASF) such as Cremophor RH40, Cremophor RH60, Cremophor EL, TWEENs (from ICI Chemicals) e.g., TWEEN 20, TWEEN 21, TWEEN 40, TWEEN 60, TWEEN 80, TWEEN 81, Cremophor RH 410, Cremophor RH 455 and the like.

The surfactant component may be selected from compounds having at least one ether formed from at least about 1 to 100 ethylene oxide units and at least one fatty alcohol chain having from at least about 12 to 22 carbon atoms; compounds having at least one ester formed from at least about 1 to 100 ethylene oxide units and at least one fatty acid chain having from at least about 12 to 22 carbon atoms; compounds having at least one ether, ester or amide formed from at least about 1 to 100 ethylene oxide units and at least one vitamin or vitamin derivative; and combinations thereof consisting of no more than two surfactants.

Other examples of surfactants include Lumulse GRH-40, TGPS, Polysorbate-80 (TWEEN-80), Polysorbate-20 (TWEEN-20), polyoxyethylene (20) sorbitan mono-oleate), glyceryl glycol esters, polyethylene glycol esters, polyglycolyzed glycerides, and the like, or mixtures thereof; polyethylene sorbitan fatty acid esters, polyoxyethylene glycerol esters, such as Tagat TO, Tagat L, Tagat I, tagat I2 and Tagat 0 (commercially available from Goldschmidt Chemical Co., Essen, Germany); ethylene glycol esters, such as glycol stearate and distearate; propylene glycol esters, such as propylene glycol myristate; glyceryl esters of fatty acids, such as glyceryl stearates and monostearates; sorbitan esters, such as spans and TWEENs; polyglyceryl esters, such as polyglyceryl 4-oleate; fatty alcohol ethoxylates, such as Brij type emulsifiers; ethoxylated propoxylated block copolymers, such as poloxamers; polyethylene glycol esters of fatty acids, such as PEG 300 linoleic glycerides or Labrafil 2125 CS, PEG 300 oleic glycerides or Labrafil M 1944 CS, PEG 400 caprylic/capric glycerides or Labrasol, and PEG 300 caprylic/capric glycerides or Softigen 767; cremophors, such as Cremophor E, polyoxyl 35 castor oil or Cremophor EL, Cremophor EL-P, Cremophor RH 4OP, polyoxyl 40 hydrogenated castor oil, Cremophor RH40; polyoxyl 60 hydrogenated castor oil or Cremophor RH 60, glycerol monocaprylate/caprate, such as Campmul CM 10; polyoxyethylated fatty acids (PEG-stearates, PED-laurates, Brij®), polyoxylated glycerides of fatty acid, polyoxylated glycerol fatty acid esters i.e. Solutol HS-15; PEG-ethers (Mirj®), sorbitan derivatives (TWEENs), sorbitan monooleate or Span 20, aromatic compounds (Tritons®), PEG-glycerides (PECEOL™), PEG-PPG (polypropylene glycol) copolymers (PLURONICS such as PLURONICS F108, F127, and F68, Poloxamers, Jeffamines, Tetronics, Polyglycerines, PEG-tocopherols, PEG-LICOL 6-oleate; propylene glycol derivatives, sugar and polysaccharide alkyl and acyl derivatives (octylsucrose, sucrose stearate, laurolydextran etc.) and/or a mixture thereof; surfactants based on an oleate or laureate ester of a polyalcohol copolymerized with ethylene oxide; Labrasol Gelucire 44/14; polyoxytheylene stearates; saturated polyglycolyzed glycerides; or poloxamers; all of which are commercially available. Polyoxyethylene sorbitan fatty acid esters can include polysorbates, for example, polysorbate 20, polysorbate 40, polysorbate 60, and polysorbate 80. Polyoxyethylene stearates can include polyoxyl 6 stearate, polyoxyl 8 stearate, polyoxyl 12 stearate and polyoxyl 20 stearate. Saturated polyglycolyzed glycerides are, for example, GELUCIRE 44/14 or GELUCIRE™ 50/13 (Gattefosse, Westwood, N.J., U.S.A.). Poloxamers used herein include poloxamer 124 and poloxamer 188.

Surfactants include *d*-α-tocopheryl polyethylene glycol 1000 succinate (TPGS), polyoxyl 8 stearate (PEG 400 monostearate), polyoxyl 40 stearate (PEG 1750 monostearate) and peppermint oil.

According to some examples, the surfactant is polyethoxylated castor oil (*e.g.,* Cremophor (PEG 35 castor oil)).

According to some examples, surfactants having an HLB lower than 10 are used. Such surfactants may optionally be used in combination with other surfactants as co-surfactants. Examples of some surfactants, mixtures, and other equivalent compositions having an HLB less than or equal to 10 are propylene glycols, glyceryl fatty acids, glyceryl fatty acid esters, polyethylene glycol esters, glyceryl glycol esters, polyglycolyzed glycerides and polyoxyethyl steryl ethers. Propylene glycol esters or partial esters form the composition of commercial products, such as Lauroglycol FCC, which contains propylene glycol laureate. The commercially available excipient Maisine 35-1 comprises long chain fatty acids, for example glyceryl linoleate. Products, such as Acconon E, which comprise polyoxyethylene stearyl ethers, may also be used. Labrafil M 1944 CS is one example of a surfactant wherein the composition contains a mixture of glyceryl glycol esters and polyethylene glycol esters.

### Solubilizing agents for rapamycin

Many solubilizing agents may be used for rapamycin, such as those described in the solubilizing agents section above.

According to some examples the solubilizing agent is a surfactant. Examples of surfactants that may be used for rapamycin include surfactants with an HLB greater than 10, 11, 12, 13 or 14. One example is Cremophor EL. In some variations, the surfactant is polyethoxylated castor oil (*e.g.*, Cremophor (PEG 35 castor oil)). In some variations, the surfactant may be a polymeric surfactant such as PLURONICS F108, F127, and F68, and Tetronics. As noted herein, some solvents may also serve as surfactants. Those of ordinary skill in the art will find it routine to identify which solubilizing agents and surfactants may be used for rapamycin given the teachings herein.

### Viscosity Modifying Agents

The liquid formulations described herein may be administered with or further comprise a viscosity modifying agent.

One exemplary viscosity modifying agent that may be used is hyaluronic acid. Hyaluronic acid is a glycosaminoglycan. It is made of a repetitive sequence of glucuronic acid and glucosamine. Hyaluronic acid is present in many tissues and organs of the body, and contributes to the viscosity and consistency of such tissues and organs. Hyaluronic acid is present in the eye, including the vitreous of the eye, and along with collagen contributes to the viscosity thereof. The liquid formulations described herein may further comprise or be administered with hyaluronic acid.

Other examples of viscosity modifying agents include polyalkylene oxides, glycerol, carboxymethyl cellulose, sodium alginate, chitosan, dextran, dextran sulfate and collagen. These viscosity modifying agents can be chemically modified.

Other viscosity modifying agents that may be used include carrageenan, cellulose gel, colloidal silicon dioxide, gelatin, propylene carbonate, carbonic acid, alginic acid, agar, carboxyvinyl polymers or carbomers and polyacrylamides, acacia, ester gum, guar gum, gum arabic, ghatti, gum karaya, tragacanth, terra, pectin, tamarind seed, larch arabinogalactan, alginates, locust bean, xanthan gum, starch, veegum, tragacanth, polyvinyl alcohol, gellan gum, hydrocolloid blends, and povidone. Other viscosity modifying agents known in the art can also be used, such as sodium carboxymethyl cellulose, algin, carageenans, galactomannans, hydropropyl methyl cellulose, hydroxypropyl cellulose, polyethylene glycol, polyvinylpyrrolidone, sodium carboxymethyl chitin, sodium carboxymethyl dextran, sodium carboxymethyl starch, xanthan gum, and zein.

### Other components of liquid formulations

The formulations described herein may further comprise various other components such as stabilizers, for example. Stabilizers that may be used in the formulations described herein include agents that will (1) improve the compatibility of excipients with the encapsulating materials such as gelatin, (2) improve the stability (e.g. prevent crystal growth of a therapeutic agent such as rapamycin) of a therapeutic agent such as rapamycin and/or rapamycin derivatives, and/or (3) improve formulation stability. Note that there is overlap between components that are stabilizers and those that are solvents, solubilizing agents or surfactants, and the same component can carry out more than one role.

Stabilizers may be selected from fatty acids, fatty alcohols, alcohols, long chain fatty acid esters, long chain ethers, hydrophilic derivatives of fatty acids, polyvinylpyrrolidones, polyvinylethers, polyvinyl alcohols, hydrocarbons, hydrophobic polymers, moisture-absorbing polymers, and combinations thereof Amide analogues of the above stabilizers can also be used. The chosen stabilizer may change the hydrophobicity of the formulation (e.g. oleic acid, waxes), or improve the mixing of various components in the formulation (e.g. ethanol), control the moisture level in the formula (e.g. PVP), control the mobility of the phase (substances with melting points higher than room temperature such as long chain fatty acids, alcohols, esters, ethers, amides etc. or mixtures thereof; waxes), and/or improve the compatibility of the formula with encapsulating materials (e.g. oleic acid or wax). Some of these stabilizers may be used as solvents/co-solvents (e.g. ethanol). Stabilizers may be present in sufficient amount to inhibit the therapeutic agent's (such as rapamycin's) crystallization.

Examples of stabilizers include saturated, monoenoic, polyenoic, branched, ring-containing, acetylenic, dicarboxylic and functional-group-containing fatty acids such as oleic acid, caprylic acid, capric acid, caproic acid, lauric acid, myristic acid, palmitic acid, stearic acid, behenic acid, linoleic acid, linolenic acid, eicosapentaenoic acid (EPA), DHA; fatty alcohols such as stearyl alcohol, cetyl alcohol, ceteryl alcohol; other alcohols such as ethanol, isopropyl alcohol, butanol; long chain fatty acid esters, ethers or amides such as glyceryl stearate, cetyl stearate, oleyl ethers, stearyl ethers, cetyl ethers, oleyl amides, stearyl amides; hydrophilic derivatives of fatty acids such as polyglyceryl fatty acids, polyethylene glycol fatty acid esters; polyvinylpyrrolidones, polyvinylalcohols (PVAs), waxes, docosahexaenoic acid and de-hydroabietic acid etc.

The formulations described may further contain a gelling agent that alters the texture of the final formulation through formation of a gel.

The therapeutic agents for use as described herein, such as rapamycin, may be subjected to conventional pharmaceutical operations, such as sterilization and compositions containing the therapeutic agent may also contain conventional adjuvants, such as preservatives, stabilizers, wetting agents, emulsifiers, buffers etc. The therapeutic agents may also be formulated with pharmaceutically acceptable excipients for clinical use to produce a pharmaceutical composition. Formulations for ocular administration may be presented as a solution, suspension, particles of solid material, a discrete mass of solid material, incorporated within a polymer matrix, liquid formulations or in any other form for ocular administration. The therapeutic agents may be used to prepare a medicament to treat, prevent, inhibit, delay onset, or cause regression of any of the conditions described herein. The therapeutic agents may be used to prepare a medicament to treat any of the conditions described herein.

A composition containing a therapeutic agent such as rapamycin may contain one or more adjuvants appropriate for the indicated route of administration. Adjuvants with which the therapeutic agent may be admixed with include lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol. When a solubilized formulation is required the therapeutic agent may be in a solvent such as polyethylene glycol of various molecular weights, propylene glycol, carboxymethyl cellulose colloidal solutions, methanol, ethanol, DMSO, corn oil, peanut oil, cottonseed oil, sesame oil, tragacanth gum, and/or various buffers. Other adjuvants and modes of administration are well known in the pharmaceutical art and may be used in the practice of the methods, compositions and liquid formulations described herein. The carrier or diluent may include time delay material, such as glyceryl monostearate or glyceryl distearate alone or with a wax, or other materials well known in the art. The formulations for use as described herein may also include gel formulations, erodible and non-erodible polymers, microspheres, and liposomes.

Other adjuvants and excipients that may be used include C₈-C₁₀ fatty acid esters such as softigen 767, polysorbate 80, PLURONICS, Tetronics, Miglyol, and Transcutol.

Additives and diluents normally utilized in the pharmaceutical arts can optionally be added to the pharmaceutical composition and the liquid formulation. These include thickening, granulating, dispersing, flavoring, sweetening, coloring, and stabilizing agents, including pH stabilizers, other excipients, anti-oxidants (e.g., tocopherol, BHA, BHT, TBHQ, tocopherol acetate, ascorbyl palmitate, ascorbic acid propyl gallate, and the like), preservatives (e.g., parabens), and the like. Exemplary preservatives include benzylalcohol, ethylalcohol, benzalkonium chloride, phenol, chlorobutanol, and the like. Some useful antioxidants provide oxygen or peroxide inhibiting agents for the formulation and include butylated hydroxytoluene, butylhydroxyanisole, propyl gallate, ascorbic acid palmitate, α-tocopherol, and the like. Thickening agents, such as lecithin, hydroxypropylcellulose, aluminum stearate, and the like, may improve the texture of the formulation.

In some variations, the therapeutic agent is rapamycin, and the rapamycin is formulated as rapamune in solid or liquid form. In some variations, the rapamune is formulated as an oral dosage.

In addition, a viscous polymer may be added to the suspension, assisting the localization and ease of placement and handling. In some uses of the liquid formulation, a pocket in the sclera may be surgically formed to receive an injection of the liquid formulations. The hydrogel structure of the sclera can act as a rate-controlling membrane. Particles of therapeutic agent substance for forming a suspension can be produced by known methods, such as via ball milling, for example by using ceramic beads. For example, a Cole Parmer ball mill such as Labmill 8000 may be used with 0.8 mm YTZ ceramic beads available from Tosoh or Norstone Inc.

The formulations may conveniently be presented in unit dosage form and may be prepared by conventional pharmaceutical techniques. Such techniques include the step of bringing into association the therapeutic agent and the pharmaceutical carrier(s) or excipient(s). The formulations may be prepared by uniformly and intimately bringing into associate the active ingredient with liquid carriers or finely divided solid carriers or both, and then, if necessary, shaping the product.

According to the present description, the formulations described herein may be provided in one or more unit dose forms, wherein the unit dose form contains an amount of a liquid formulation described herein that is effective to treat or prevent the disease or condition for which it is being administered. The formulations described herein may be provided in one or more unit dose forms, wherein the unit dose form contains an amount of a liquid rapamycin formulation described herein that is effective to treat or prevent the disease or condition for which it is being administered.

In some examples, the unit dose form is prepared in the concentration at which it will be administered. In some examples, the unit dose form is diluted prior to administration to a subject. In some examples, a liquid formulation described herein is diluted in an aqueous medium prior to administration to a subject. In some examples the aqueous medium is an isotonic medium. In some examples, a liquid formulation described herein is diluted in a non-aqueous medium prior to administration to a subject.

Disclosed herein are kits comprising one or more unit dose forms as described herein. The kit may comprise one or more of packaging and instructions for use to treat one or more diseases or conditions. The kit may comprise a diluent which is not in physical contact with the formulation or pharmaceutical formulation. The kit may comprise any of one or more unit dose forms described herein in one or more sealed vessels. The kit may comprise any of one or more sterile unit dose forms.

According to the present description, the unit dose form may be in a container, such as a sterile sealed container. In some variations the container is a vial, ampule, or low volume applicator, such as a syringe. A low-volume applicator may be pre-filled with rapamycin for treatment of an ophthalmic disease or condition such as a limus compound for treatment of age-related macular degeneration. Described herein is a pre-filled low-volume applicator pre-filled with a formulation comprising a therapeutic agent such as rapamycin. In some examples a low-volume applicator is pre-filled with a solution comprising a therapeutic agent, such as rapamycin and a polyethylene glycol, and optionally further comprises one or more additional components such as ethanol. In some examples a pre-filled low-volume applicator is pre-filled with a solution comprising about 2% rapamycin, about 94% PEG-400, about 4% ethanol.

Described herein are kits comprising one or more containers. A kit may comprise one or more low-volume applicators is pre-filled with a formulation described herein comprising a therapeutic agent, such as rapamycin (e.g., formulations comprising rapamycin and a polyethylene glycol, and optionally further comprises one or more additional components such as ethanol, and formulations in liquid form comprising about 2% rapamycin, about 94% PEG-400, about 4% ethanol). The kit may comprise one or more containers, such as pre-filled low-volume applicators, with instructions for its use. In a further example a kit comprises one or more low-volume applicators pre-filled with rapamycin, with instructions for its use in treating a disease or condition of the eye. The containers described herein may be in a secondary packaging.

### Routes of Administration

The compositions, methods, and liquid formulations described herein deliver one or more therapeutic agents to a subject, such as a human subject.

In some variations, the compositions, methods, and liquid formulations described herein deliver one or more therapeutic agents to an aqueous medium of a human subject.

The compositions, methods, and liquid formulations described herein may deliver one or more therapeutic agents to an aqueous medium in or proximal to an area where a disease or condition is to be treated, prevented, inhibited, onset delayed, or regression caused.

The compositions, methods, and liquid formulations described herein may deliver one or more therapeutic agents to an eye of a subject, including the macula and the retina choroid tissues, in an amount and for a duration effective to treat, prevent, inhibit, delay the onset of, or cause the regression of the diseases and conditions described in the *Diseases and Conditions* section.

"Retina choroid" and "retina choroid tissues," as used herein, are synonymous and refer to the combined retina and choroid tissues of the eye.

As an example, the compositions, liquid formulations, and methods described in herein may be administered to the vitreous, aqueous humor, sclera, conjunctiva, between the sclera and conjunctiva, the retina choroid tissues, macula, or other area in or proximate to the eye of a subject, either by direct administration to these tissues or by periocular routes, in amounts and for a duration effective to treat, prevent, inhibit, delay the onset of, or cause the regression of CNV and wet AMD. The effective amounts and durations may be different for each of treating, preventing, inhibiting, delaying the onset of, or causing the regression of CNV and wet AMD, and for each of the different sites of delivery.

Intravitreal administration is more invasive than some other types of ocular procedures. Because of the potential risks of adverse effects, intravitreal administration may not be optimal for treatment of relatively healthy eyes. By contrast, periocular administration, such as subconjunctival administration, is much less invasive than intravitreal administration. When a therapeutic agent is delivered by a periocular route, it may be possible to treat patients with healthier eyes than could be treated using intravitreal administration. In some variations, subconjunctival injection is used to prevent or delay onset of a disease or condition of the eye, where the eye of the subject has visual acuity of 20/40 or better.

"Subconjunctival" placement or injection, as used herein, refers to placement or injection between the sclera and conjunctiva. Subconjunctival is sometimes referred to herein as "sub-conj" administration.

Routes of administration that may be used to administer a liquid formulation include placement of the liquid formulation, for example by injection, into an aqueous medium in the subject, (e.g., by injection into the eye of a subject, such as a human subject). The liquid formulation may be administered systemically, such as by the following delivery routes: rectal, vaginal, infusion, intramuscular, intraperitoneal, intraarterial, intrathecal, intrabronchial, intracisternal, cutaneous, subcutaneous, intradermal, transdermal, intravenous, intracervical, intraabdominal, intracranial, intraocular, intrapulmonary, intrathoracic, intratracheal, nasal, buccal, sublingual, oral, parenteral, or nebulised or aerosolized using aerosol propellants.

Compositions and liquid formulations comprising therapeutic agent can be administered directly to the eye using a variety of procedures, including procedures in which (1) the therapeutic agent is administered by injection using a syringe and hypodermic needle, (2) a specially designed device is used to inject the therapeutic agent, (3) prior to injection of the therapeutic agent, a pocket is surgically formed within the sclera to serve as a receptacle for the therapeutic agent or therapeutic agent composition. For example, in one administration procedure a surgeon forms a pocket within the sclera of the eye followed by injection of a solution or liquid formulation comprising the therapeutic agent into the pocket.

Other administration procedures include procedures in which (1) a formulation of the therapeutic agent is injected through a specially designed curved cannula to place the therapeutic agent directly against a portion of the eye, (2) a compressed form of the therapeutic agent is placed directly against a portion of the eye, (3) the therapeutic agent is inserted into the sclera by a specially designed injector or inserter, (4) the liquid formulation comprising the therapeutic agent is incorporated within a polymer, (5) a surgeon makes a small conjunctival incision through which to pass a suture and any therapeutic agent delivery structure so as to secure the structure adjacent to the sclera, (6) a needle is used for injection directly into the vitreous of an eye, or into any other site described.

The liquid formulations described herein may be used directly, for example, by injection, as an elixir, for topical administration such as via eye drops, or in hard or soft gelatin or starch capsules. The capsules may be banded to prevent leakage.

In some variations, the liquid formulations described herein may be administered by topical administration. In some variations, the topical administration is ocular topical administration. In some variations, the ocular topical administration includes administration via eye drops, contacts, punctual plugs, or other ocular devices. In some variations, the liquid formulation is applied topically to the eye, any of about 1, 2, 3, 4, or 5 times per day. In some variations, the liquid formulation is applied topically to the eye, about once or less any of about every 1, 2, 3, 4, 5, 6, 7, 10, 14, 21, or 28 day(s). In some variations, the liquid formulation is applied topically to the eye, about once or less a day. In some variations, the liquid formulation is applied topically to the eye, about once or less every 5 days. In some variations, the liquid formulation is applied topically to the eye, about once or less of every 10 days.

### Delivery by injection

One method that may be used to deliver the compositions and liquid formulations described herein is delivery by injection. In this method compositions and liquid formulations may be injected into a subject, such as a human subject, or into a position in or proximate to an eye of the subject for delivery to a subject or to the eye of a subject. Injection includes intraocular and periocular injection. Examples of positions that are in or proximate to an eye of a subject are as follows.

Injection of therapeutic agent into the vitreous may provide a high local concentration of therapeutic agent in the vitreous and retina. Further, it has been found that in the vitreous the clearance half-lives of drugs increases with molecular weight.

Intracameral injection, or injection into the anterior chamber of the eye, may also be used. In one example, up to about 100 µl may be injected intracamerally.

Periocular routes of delivery may deliver therapeutic agent to the retina without some of the risks of intravitreal delivery. Periocular routes include for example, subconjunctival, subtenon, retrobulbar, peribulbar and posterior juxtascleral delivery. A "periocular" route of administration means placement near or around the eye. For a description of exemplary periocular routes for retinal drug delivery, see Periocular routes for retinal drug delivery, Raghava et al. (2004), Expert Opin. Drug Deliv. 1(1):99-114.

In some variations the liquid formulations described herein are administered intraocularly. Intraocular administration includes placement or injection within the eye, including in the vitreous.

Subconjunctival injection may be by injection of therapeutic agent underneath the conjunctiva, or between the sclera and conjunctiva. In one example, up to about 500 µl may be injected subconjunctivally. As one example, a needle of up to about 25 to about 30 gauge and about 30 mm long may be used. Local pressure to the subconjunctival site of therapeutic agent administration may elevate delivery of the therapeutic agent to the posterior segment by reducing local choroidal blood flow.

Subtenon injection may be by injection of therapeutic agent into the tenon's capsule around the upper portion of the eye and into the "belly" of the superior rectus muscle. In one example, up to about 4 ml may be injected subtenon. As one example, a blunt-tipped cannula about 2.5 cm long may be used.

Retrobulbar injection refers to injection into the conical compartment of the four rectus muscles and their intermuscular septa, behind the globe of the eye. In one example, up to about 5 ml may be injected retrobulbarly. As one example, a blunt needle of about 25 - or about 27-gauge may be used.

Peribulbar injection may be at a location external to the confines of the four rectus muscles and their intramuscular septa, i.e., outside of the muscle cone. A volume of, for example, up to about 10 ml may be injected peribulbarly. As one example, a blunt-tipped cannula about 1.25 inches long and about 25-gauge may be used.

Posterior juxtascleral delivery refers to placement of a therapeutic agent near and above the macula, in direct contact with the outer surface of the sclera, and without puncturing the eyeball. In one example, up to about 500 ml may be injected posterior juxtasclerally. As one example, a blunt-tipped curved cannula, specially designed at 56°, is used to place the therapeutic agent in an incision in the sclera.

In some variations the liquid formulations described herein are injected intraocularly. Intraocular injection includes injection within the eye.

Sites to which the compositions and liquid formulations may be administered include for example, the vitreous, aqueous humor, sclera, conjunctiva, between the sclera and conjunctiva, the retina choroid tissues, macula, or other area in or proximate to the eye of a subject. Methods that may be used for placement of the compositions and liquid formulations include injection.

In one method that may be used, the therapeutic agent is dissolved in an solvent or solvent mixture and then injected into or proximate to the vitreous, aqueous humor, sclera, conjunctiva, between the sclera and conjunctiva, the retina choroid tissues, macula, other area in or proximate to the eye of a subject, or other medium of a subject, according to any of the procedures mentioned above. In one such method that may be used, the therapeutic agent is rapamycin in a liquid formulation.

When the therapeutic agent is rapamycin, the compositions and liquid formulations may be used to deliver or maintain an amount of rapamycin in tissues of the eye, such as the retina, choroid, or the vitreous, which amount is effective to treat AMD. In one example, it is believed that a liquid formulation delivering rapamycin in an amount capable of providing a concentration of rapamycin of about 0.1 µg/ml to about 2 µg/ml in the vitreous may be used for treatment of wet AMD. In some examples, it is believed that a liquid formulation delivering a concentration of rapamycin of about 0.1 pg/mg to about 1 µg/mg in the retina choroid tissues may be used for treatment of wet AMD. Other effective concentrations are readily ascertainable by those of skill in the art based on the teachings described herein.

"Subtenonally administered", as used herein, means administration to the subtenon. In some variations, a therapeutic agent or liquid formulation is subtenonally administered by subtenon injection. In some variations, a therapeutic agent or liquid formulation is subtenonally administered by a means other than subtenon injection.

### Method of preparing liquid formulations

One method that may be used for preparing the liquid formulations described herein, such as liquid formulations comprising rapamycin, is by mixing a solvent and a therapeutic agent together at room temperature or at slightly elevated temperature until a solution or suspension is obtained, with optional use of a sonicator, and then cooling the formulation. Other components including those described above may then be mixed with the formulation. Other preparation methods that may be used are described herein including in the examples, and those of skill in the art will be able to select other preparation methods based on the teachings herein.

### Extended delivery of therapeutic agents

Described herein are compositions and liquid formulations showing in vivo delivery or clearance profiles with one or more of the following characteristics. The delivery or clearance profiles are for clearance of the therapeutic agent in vivo after injection of the composition or liquid formulations subconjunctivally or into the vitreous of a rabbit eye. The delivery or clearance profiles may be for clearance of rapamycin in vivo after injection of the composition or liquid formulations subconjunctivally or into the vitreous of a rabbit eye. The volume of the rabbit vitreous is approximately 30-40% of the volume of the human vitreous. The amount of therapeutic agent is measured using techniques such as those as described in Example 2.

The therapeutic agents with the in vivo delivery or clearance profiles described herein include those described in the *Therapeutic Agents* section. In some variations the therapeutic agent is rapamycin. The liquid formulations described herein may be used to deliver therapeutic agents in a concentration equivalent to rapamycin. The liquid formulations described herein may comprise any therapeutic agent including those described in the *Therapeutic Agents* section, in a concentration equivalent to rapamycin such as those concentrations described herein.

"Average percentage in vivo" level means that an average concentration of therapeutic agent is obtained across multiple rabbit eyes for a given timepoint, and the average concentration of therapeutic agent at one timepoint is divided by the average concentration of therapeutic agent at another timepoint. In some variations of the average percentage in vivo levels, the therapeutic agent is rapamycin.

The average concentration of a therapeutic agent in the tissue of a rabbit eye at a given time after administration of a formulation containing the therapeutic agent may be measured according to the following method. Where volumes below 10 µl are to be injected, a Hamilton syringe is used.

The liquid formulations are stored at a temperature of 2-8°C prior to use.

The experimental animals are specific pathogen free (SPF) New Zealand White rabbits. A mixed population of about 50% male, about 50% female is used. The rabbits are at least 12 weeks of age, usually at least 14 weeks of age, at the time of dosing. The rabbits each weigh at least 2.2 kg, usually at least 2.5 kg, at the time of dosing. Prior to the study, the animals are quarantined for at least one week and examined for general health parameters. Any unhealthy animals are not used in the study. At least 6 eyes are measured and averaged for a given timepoint.

Housing and sanitation are performed according to standard procedures used in the industry. The animals are provided approximately 150 grams of Teklad Certified Hi-Fiber Rabbit Diet daily, and are provided tap water ad libitum. No contaminants are known to exist in the water and no additional analysis outside that provided by the local water district is performed. Environmental Conditions are monitored.

Each animal undergoes a pre-treatment ophthalmic examination (slit lamp and ophthalmoscopy), performed by a board certified veterinary ophthalmologist. Ocular findings are scored according to the McDonald and Shadduck scoring system as described in Dermatoxicology, F. N. Marzulli and H.I. Maibach, 1977 "Eye Irritation," T.O. McDonald and J.A. Shadduck (pages 579-582). Observations are recorded using a standardized data collection sheet. Acceptance criteria for placement on study are as follows: scores of ≤ 1 for conjunctival congestion and swelling; scores of 0 for all other observation variables.

Gentamicin ophthalmic drops are placed into both eyes of each animal twice daily on the day prior to dosing, on the day of dosing (Day 1), and on the day after dosing (Day 2). Dosing is performed in two phases, the first including one set of animals and the second including the other animals. Animals are randomized separately into masked treatment groups prior to each phase of dosing according to modified Latin squares. Animals are fasted at least 8 hours prior to injection. The start time of the fast and time of injection are recorded.

Animals are weighed and anesthetized with an intravenous injection of a ketamine/xylazine cocktail (87 mg/mL ketamine, 13 mg/mL xylazine) at a volume of 0.1-0.2 mL/kg. Both eyes of each animal are prepared for injection as follows: approximately five minutes prior to injection, eyes are moistened with an ophthalmic Betadine solution. After five minutes, the Betadine is washed out of the eyes with sterile saline. Proparacaine hydrochloride 0.5% (1-2 drops) is delivered to each eye. For eyes to be intravitreally injected, 1% Tropicamide (1 drop) is delivered to each eye.

On Day 1, both eyes of each animal receive an injection of test or control article. Animals in selected groups are dosed a second time on Day 90 ± 1. Dosing is subconjunctival or intravitreal. Actual treatments, injection locations, and dose volumes are masked and revealed at the end of the study.

Subconjunctival injections are given using an insulin syringe and 30 gauge x 1/2-inch needle. The bulbar conjunctiva in the dorsotemporal quadrant is elevated using forceps. Test article is injected into the subconjunctival space.

Intravitreal injections are given using an Insulin syringe and 30 gauge x 1/2-inch needle. For each injection, the needle is introduced through the ventral-nasal quadrant of the eye, approximately 2-3 mm posterior to the limbus, with the bevel of the needle directed downward and posteriorly to avoid the lens. Test article is injected in a single bolus in the vitreous near the retina.

Animals are observed for mortality/morbidity twice daily. An animal determined to be moribund is euthanized with an intravenous injection of commercial euthanasia solution. Both eyes are removed and stored frozen at -70°C for possible future evaluation. If an animal is found dead prior to onset of rigor mortis, both eyes are removed and stored frozen at -70°C for possible future evaluation. Animals found after the onset of rigor mortis are not necropsied.

Animals are weighed at randomization, on Day 1 prior to dosing, and prior to euthanasia.

Ophthalmic observations (slit lamp and indirect ophthalmoscopy) are performed on all animals on Days 5 ± 1, 30 ± 1, 60 ± 1, 90 ± 1, and at later dates in some variations. Observations are performed by a board certified veterinary ophthalmologist. For animals to be dosed on Day 90 ± 1, ophthalmic observations are performed prior to dosing. Ocular findings are scored according to the McDonald and Shadduck scoring system as described in Dermatoxicology, F.N. Marzulli and H.I. Maibach, 1977 "Eye Irritation", T.O. McDonald and J.A. Shadduck (pages 579-582), and observations are recorded using a standardized data collection sheet.

Whole blood samples (1-3 mL per sample) are collected from each animal prior to necropsy in vacutainer tubes containing EDTA. Each tube is filled at least 2/3 full and thoroughly mixed for at least 30 seconds. Tubes are stored frozen until shipped on dry ice.

Animals are euthanized with an intravenous injection of commercial euthanasia solution. Euthanasia is performed according to standard procedures used in the industry.

For treatment groups dosed intravitreally or subconjunctivally with placebo, all eyes from each of these groups are placed into Davidsons solution for approximately 24 hours. Following the 24-hour period, the eyes are transferred to 70% ethanol; these globes are submitted for masked histopathological evaluation by a board certified veterinary pathologist. The time that eyes are placed into Davidsons and the time of removal are recorded.

For treatment groups dosed intravitreally or subconjunctivally with test article, some eyes from each of these groups are frozen at -70°C and submitted for pharmacokinetic analysis. The remaining eyes from each of these groups are placed into Davidsons solution for approximately 24 hours. Following the 24-hour period, the eyes are transferred to 70% ethanol; these globes are submitted for masked histopathological evaluation by a board certified veterinary pathologist. The time that eyes are placed into Davidsons and the time of removal are recorded.

Frozen samples submitted for pharmacokinetic analysis are dissected with disposable instruments. One set of instruments is used per eye, and then discarded. The samples are thawed at room temperature for 1 to 2 minutes to ensure that the frost around the tissue has been removed. The sclera is dissected into 4 quadrants, and the vitreous is removed. If a non-dispersed mass (NDM) is clearly visible within the vitreous, the vitreous is separated into two sections. The section with the NDM is approximately two-thirds of the vitreous. The section without the NDM is the portion of the vitreous that is the most distant from the NDM. The aqueous humor, lens, iris, and cornea are separated. The retina choroid tissue is removed using a forceps and collected for analysis. The conjunctiva is separated from the sclera.

The various tissue types are collected into separate individual pre-weighed vials which are then capped and weighed. The vials of tissue are stored at -80°C until analyzed.

The sirolimus content of the retina choroid, sclera, vitreous humor, and whole anti-coagulated blood is determined by high-pressure liquid chromatography/tandem mass spectroscopy (HPLC/MS/MS) using 32-O-desmethoxyrapamycin as an internal standard. Where an NDM was observed in the vitreous, the section of the vitreous containing the NDM and the section of the vitreous not containing the NDM are analyzed separately.

The average concentration of a therapeutic agent over a period of time means for representative timepoints over the period of time the average concentration at each time point. For example, if the time period is 30 days, the average concentration may be measured at 5 day intervals: for the average concentration at day 5, the average of a number of measurements of concentration at day 5 would be calculated; for the average concentration at day 10, the average of a number of measurements of the concentration at day 10 would be calculated, etc.

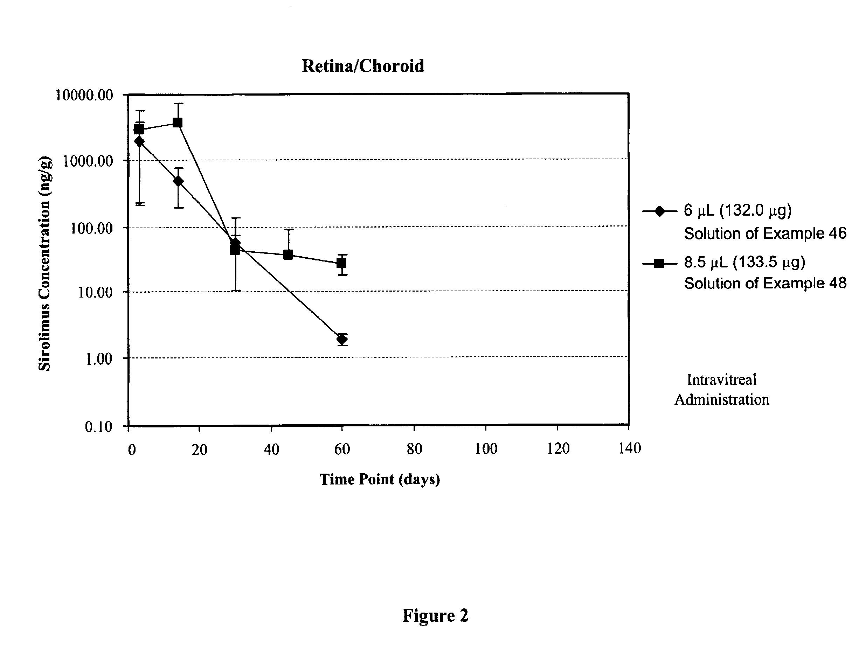

In some variations, the liquid formulations described herein may have in vivo delivery to the vitreous profiles with the following described characteristics, where the delivery profiles are for delivery of therapeutic agent in vivo after injection of the liquid formulation between the sclera and the conjunctiva of a rabbit eye. One variation of in vivo delivery to the vitreous profiles is shown in Fig. 2.

At day 40 after injection, the average percentage in vivo vitreal level may be between about 70% and about 100%, and more usually between about 80% and about 90%, relative to the level present at day 20 after injection. At day 40 after injection, the average percentage in vivo vitreal level may be greater than about 70%, and more usually greater than about 80%, relative to the level present at day 20 after injection.

At day 67 after injection, the average percentage in vivo vitreal level may be between about 75% and about 115%, and more usually between about 85% and about 105%, relative to the level present at day 20 after injection. At day 67 after injection, the average percentage in vivo vitreal level may be greater than about 75%, and more usually greater than about 85%, relative to the level present at day 20 after injection.

At day 90 after injection, the average percentage in vivo vitreal level may be between about 20% and about 50%, and more usually between about 30% and about 40%, relative to the level present at day 20 after injection. At day 90 after injection, the average percentage in vivo vitreal level may be greater than about 20%, and more usually greater than about 30%, relative to the level present at day 20 after injection.

The average percentage in vivo vitreal level may have the following characteristics relative to the level present at day 20 after injection: at 40 days after injection it is less than about 100%; at 67 days after injection it is less than about 115%; and 90 days after injection it is less than about 50%.

The liquid formulation when injected between the sclera and conjunctiva of a rabbit eye delivers therapeutic agent giving an average concentration of therapeutic agent in the vitreous of the rabbit eye of at least about 0.01 ng/mL for at least about 30, at least about 60, or at least about 90 days after administration of the liquid formulation to the rabbit eyes. The liquid formulation when injected between the sclera and conjunctiva of a rabbit eye delivers therapeutic agent giving an average concentration of therapeutic agent in the vitreous of the rabbit eye of at least about 0.1 ng/mL for at least about 30, at least about 60, or at least about 90 days after administration of the liquid formulation to the rabbit eyes. The liquid formulation when injected between the sclera and conjunctiva of a rabbit eye delivers therapeutic agent giving an average concentration of therapeutic agent in the vitreous of the rabbit eye of at least about 1 ng/mL for at least about 30, at least about 60, or at least about 90 days after administration of the liquid formulation to the rabbit eyes.

The liquid formulations described herein may have in vivo delivery to the retina choroid profiles with the following described characteristics, where the delivery profiles are for delivery of therapeutic agent in vivo after injection of the liquid formulation between the sclera and the conjunctiva of a rabbit eye.

At day 40 after injection, the average percentage in vivo retina choroid level may be between about 350% and about 410%, and more usually between about 360% and about 400%, relative to the level present at day 20 after injection. At day 40 after injection, the average percentage in vivo retina choroid level may be greater than about 350%, and more usually greater than about 360%, relative to the level present at day 20 after injection.

At day 67 after injection, the average percentage in vivo retina choroid level may be between about 125% and about 165%, and more usually between about 135% and about 155%, relative to the level present at day 20 after injection. At day 67 after injection, the average percentage in vivo retina choroid level may be greater than about 125%, and more usually greater than about 135%, relative to the level present at day 20 after injection.

At day 90 after injection, the average percentage in vivo retina choroid level may be between about 10% and about 50%, and more usually between about 20% and about 40%, relative to the level present at day 20 after injection. At day 90 after injection, the average percentage in vivo retina choroid level may be greater than about 10%, and more usually greater than about 20%, relative to the level present at day 20 after injection.

The average percentage in vivo retina choroid level may have the following characteristics relative to the level present at day 20 after injection: at 40 days after injection it is less than about 410%; at 67 days after injection it is less than about 165%; and 90 days after injection it is less than about 50%.

The liquid formulation when injected between the sclera and conjunctiva of a rabbit eye may deliver therapeutic agent giving an average concentration of therapeutic agent in the retina choroid tissues of the rabbit eye of at least about 0.001 ng/mg for at least about 30, at least about 60, or at least about 90 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of at least about 0.01 ng/mg for at least about 30, at least about 60, or at least about 90 days after administration of the liquid formulation to the rabbit eyes.

The level of therapeutic agent present in the retina choroid first may increase, then peaks and decreases. The peak may, for instance, occur at about day 40 after injection.

The liquid formulations described herein may have in vivo clearance from the sclera profiles with the following described characteristics, where the clearance profiles are for clearance of therapeutic agent in vivo after injection of the liquid formulation between the sclera and the conjunctiva of a rabbit eye. Where injection is between the sclera and the conjunctiva, the scleral level is thought to include the injected liquid formulation.

At day 40 after injection, the average percentage in vivo scleral level may be between about 150% and about 230%, and more usually between about 170% and about 210%, relative to the level present at day 20 after injection. At day 40 after injection, the average percentage in vivo scleral level may be greater than about 150%, and more usually greater than about 170%, relative to the level present at day 20 after injection.

At day 67 after injection, the average percentage in vivo scleral level may be between about 30% and about 70%, and more usually between about 40% and about 60%, relative to the level present at day 20 after injection. At day 67 after injection, the average percentage in vivo scleral level may be greater than about 30%, and more usually greater than about 40%, relative to the level present at day 20 after injection.

At day 90 after injection, the average percentage in vivo scleral level may be between about 110% and about 160%, and more usually between about 125% and about 145%, relative to the level present at day 20 after injection. At day 90 after injection, the average percentage in vivo scleral level may be greater than about 110%, and more usually greater than about 125%, relative to the level present at day 20 after injection.

The average percentage in vivo scleral level may have the following characteristics relative to the level present at day 20 after injection: at 40 days after injection it is less than about 230%; at 67 days after injection it is less than about 70%; and 90 days after injection it is less than about 160%.

The level of therapeutic agent present in the sclera may first increase, then peak and decrease. The peak may, for instance, occur at about day 40 after injection.

The liquid formulations described herein may have in vivo delivery to the vitreous profiles with the following described characteristics, where the delivery profiles are for delivery of therapeutic agent in vivo after injection of the liquid formulation between the sclera and the conjunctiva of a rabbit eye.

At day 14 after injection, the average percentage in vivo vitreal level may be between about 1350% and about 1650%, and more usually between about 1450% and about 1550%, relative to the level present at day 2 after injection. At day 14 after injection, the average percentage in vivo vitreal level may be greater than about 1350%, and more usually greater than about 1450%, relative to the level present at day 2 after injection.

At day 35 after injection, the average percentage in vivo vitreal level may be between about 200% and about 300%, and more usually between about 225% and about 275%, relative to the level present at day 2 after injection. At day 35 after injection, the average percentage in vivo vitreal level may be greater than about 200%, and more usually greater than about 225%, relative to the level present at day 2 after injection.

At day 62 after injection, the average percentage in vivo vitreal level may be between about 100% and about 160%, and more usually between about 115% and about 145%, relative to the level present at day 2 after injection. At day 62 after injection, the average percentage in vivo vitreal level may be greater than about 100%, and more usually greater than about 115%, relative to the level present at day 2 after injection.

At day 85 after injection, the average percentage in vivo vitreal level may be between about 5% and about 30%, and more usually between about 10% and about 25%, relative to the level present at day 2 after injection. At day 85 after injection, the average percentage in vivo vitreal level may be greater than about 5%, and more usually greater than about 10%, relative to the level present at day 2 after injection.

The average percentage in vivo vitreal level may have the following characteristics relative to the level present at day 2 after injection: at 14 days after injection it is less than about 1600%; at 35 days after injection it is less than about 300%; at 62 days after injection it is less than about 160% and 85 days after injection it is less than about 30%.

The liquid formulation when injected between the sclera and conjunctiva of a rabbit eye may deliver therapeutic agent giving an average concentration of therapeutic agent in the vitreous of the rabbit eye of at least about 0.01 ng/mL for at least about 30, at least about 60, or at least about 85 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of at least about 0.1 ng/mL for at least about 30, at least about 60, or at least about 85 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of at least about 1 ng/mL for at least about 30, or at least about 60 days after administration of the liquid formulation to the rabbit eyes.

The level of therapeutic agent present in the vitreous may first increase, then peak and decrease. The peak may, for instance, occur at about day 14 after injection.

The liquid formulations described herein may have in vivo delivery to the retina choroid profiles with the following described characteristics, where the delivery profiles are for delivery of therapeutic agent in vivo after injection of the liquid formulation between the sclera and the conjunctiva of a rabbit eye.

At day 35 after injection, the average percentage in vivo retina choroid level may be between about 320% and about 400%, and more usually between about 340% and about 380%, relative to the level present at day 14 after injection. At day 35 after injection, the average percentage in vivo retina choroid level may be greater than about 320%, and more usually greater than about 340%, relative to the level present at day 14 after injection.

At day 62 after injection, the average percentage in vivo retina choroid level may be between about 3% and about 25%, and more usually between about 6% and about 20%, relative to the level present at day 14 after injection. At day 62 after injection, the average percentage in vivo retina choroid level may be greater than about 3%, and more usually greater than about 6%, relative to the level present at day 14 after injection.

At day 85 after injection, the average percentage in vivo retina choroid level may be between about 0.1% and about 6%, and more usually between about 0.5% and about 4%, relative to the level present at day 14 after injection. At day 85 after injection, the average percentage in vivo retina choroid level may be greater than about 0.1%, and more usually greater than about 0.5%, relative to the level present at day 14 after injection.

The average percentage in vivo retina choroid level may have the following characteristics relative to the level present at day 14 after injection: at 35 days after injection it is less than about 400%; at 62 days after injection it is less than about 25%; and 85 days after injection it is less than about 6%.

The liquid formulation when injected between the sclera and conjunctiva of a rabbit eye may deliver therapeutic agent giving an average concentration of therapeutic agent in the retina choroid tissues of the rabbit eye of at least about 0.001 ng/mg for at least about 30, at least about 60, or at least about 85 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of at least about 0.01 ng/mg for at least about 30, at least about 60, or at least about 85 days after administration of the liquid formulation to the rabbit eyes.

The liquid formulations described herein may have in vivo clearance from the sclera profiles with the following described characteristics, where the clearance profiles are for clearance of therapeutic agent in vivo after injection of the liquid formulation between the sclera and the conjunctiva of a rabbit eye. For injection between the sclera and conjunctiva, the scleral level is thought to include the injected liquid formulation.

At day 35 after injection, the average percentage in vivo scleral level may be between about 0.1% and about 0.7%, and more usually between about 0.2% and about 0.6%, relative to the level present at day 14 after injection. At day 35 after injection, the average percentage in vivo scleral level may be greater than about 0.1%, and more usually greater than about 0.2%, relative to the level present at day 14 after injection.

At day 62 after injection, the average percentage in vivo scleral level may be between about 0.05% and about 0.35%, and more usually between about 0.07% and about 0.3%, relative to the level present at day 14 after injection. At day 62 after injection, the average percentage in vivo scleral level may be greater than about 0.05%, and more usually greater than about 0.07%, relative to the level present at day 14 after injection.

At day 85 after injection, the average percentage in vivo scleral level may be between about 0.1% and about 0.9%, and more usually between about 0.3% and about 0.7%, relative to the level present at day 14 after injection. At day 85 after injection, the average percentage in vivo scleral level may be greater than about 0.1%, and more usually greater than about 0.3%, relative to the level present at day 14 after injection.

The average percentage in vivo scleral level may have the following characteristics relative to the level present at day 14 after injection: at 35 days after injection it is less than about 0.7%; at 62 days after injection it is less than about 0.35%; and 85 days after injection it is less than about 0.9%.

The liquid formulations described herein may have in vivo clearance from the vitreous profiles with the following described characteristics, where the clearance profiles are for clearance of therapeutic agent in vivo after injection of the liquid formulation into the vitreous of a rabbit eye. Where injection is into the vitreous, the measured vitreous level is thought to include the injected formulation.

At day 35 after injection, the average percentage in vivo vitreal level may be between about 1% and about 40%, and more usually between about 1% and about 10%, relative to the level present at day 14 after injection. At day 35 after injection, the average percentage in vivo vitreal level may be greater than about 1% relative to the level present at day 14 after injection.

At day 62 after injection, the average percentage in vivo vitreal level may be between about 1% and about 40%, and more usually between about 5% and about 25%, relative to the level present at day 14 after injection. At day 62 after injection, the average percentage in vivo vitreal level may be greater than about 1% relative to the level present at day 14 after injection, and more usually greater than about 5% relative to the level present at day 14 after injection.

At day 90 after injection, the average percentage in vivo vitreal level may be between about 1% and about 40%, and more usually between about 10% and about 30%, relative to the level present at day 14 after injection. At day 90 after injection, the average percentage in vivo vitreal level may be greater than about 1% relative to the level present at day 14 after injection, and more usually greater than about 10% relative to the level present at day 14 after injection.

The level of therapeutic agent present in the vitreous may first increase, then peak and decrease. The peak may, for instance, occur at about day 14 after injection.

The liquid formulations described herein may have in vivo delivery to the retina choroid profiles with the following described characteristics, where the delivery profiles are for delivery of therapeutic agent in vivo after injection of the liquid formulation into the vitreous of a rabbit eye.

At day 35 after injection, the average percentage in vivo retina choroid level may be between about 3400% and about 5100%, and more usually between about 3750% and about 4750%, relative to the level present at day 14 after injection. At day 35 after injection, the average percentage in vivo retina choroid level may be greater than about 3400%, and more usually greater than about 3750%, relative to the level present at day 14 after injection.

At day 62 after injection, the average percentage in vivo retina choroid level may be between about 0.1% and about 5%, and more usually between about 1% and about 3%, relative to the level present at day 14 after injection. At day 62 after injection, the average percentage in vivo retina choroid level may be greater than about 0.1%, and more usually greater than about 1%, relative to the level present at day 14 after injection.

At day 90 after injection, the average percentage in vivo retina choroid level may be between about 10% and about 50%, and more usually between about 20% and about 40%, relative to the level present at day 14 after injection. At day 90 after injection, the average percentage in vivo retina choroid level may be greater than about 10%, and more usually greater than about 20%, relative to the level present at day 14 after injection.

The average percentage in vivo retina choroid level may have the following characteristics relative to the level present at day 14 after injection: at 35 days after injection it is less than about 5100%; at 62 days after injection it is less than about 5%; and 90 days after injection it is less than about 50%.

The liquid formulations described herein may have in vivo delivery to the sclera profiles with the following described characteristics, where the delivery profiles are for delivery of therapeutic agent in vivo after injection of the liquid formulation into the vitreous of a rabbit eye.

At day 35 after injection, the average percentage in vivo scleral level may be between about 1700% and about 2600%, and more usually between about 1900% and about 2400%, relative to the level present at day 14 after injection. At day 35 after injection, the average percentage in vivo scleral level may be greater than about 1700%, and more usually greater than about 1900%, relative to the level present at day 14 after injection.

At day 62 after injection, the average percentage in vivo scleral level may be between about 120% and about 180%, and more usually between about 140% and about 160%, relative to the level present at day 14 after injection. At day 62 after injection, the average percentage in vivo scleral level may be greater than about 120%, and more usually greater than about 140%, relative to the level present at day 14 after injection.

At day 90 after injection, the average percentage in vivo scleral level may be between about 95% and about 155%, and more usually between about 115% and about 135%, relative to the level present at day 14 after injection. At day 90 after injection, the average percentage in vivo scleral level may be greater than about 95%, and more usually greater than about 115%, relative to the level present at day 14 after injection.

In some examples the average percentage in vivo scleral level has the following characteristics relative to the level present at day 14 after injection: at 35 days after injection it is less than about 2600%; at 62 days after injection it is less than about 180%; and 90 days after injection it is less than about 155%.

The liquid formulation when injected into the vitreous of a rabbit eye may deliver therapeutic agent giving an average concentration of therapeutic agent in the sclera of the rabbit eye of at least about 0.001 ng/mg for at least about 30, at least about 60, or at least about 90 days after administration of the liquid formulation to the rabbit eyes. The liquid formulation when injected into the vitreous of a rabbit eye may deliver therapeutic agent giving an average concentration of therapeutic agent in the sclera of the rabbit eye of at least about 0.01 ng/mg for at least about 30, at least about 60, or at least about 90 days after administration of the liquid formulation to the rabbit eyes. The liquid formulation when injected into the vitreous of a rabbit eye may deliver therapeutic agent giving an average concentration of therapeutic agent in the sclera of the rabbit eye of at least about 0.1 ng/mg for at least about 30, at least about 60, or at least about 90 days after administration of the liquid formulation to the rabbit eyes.

The level of therapeutic agent present in the vitreous may first increase, then peak and decrease. The peak may, for instance, occur at about day 35 after injection.

In situ gelling liquid formulations described herein may have in vivo delivery to the vitreous profiles with the following described characteristics, where the delivery profiles are for delivery of therapeutic agent in vivo after injection of the liquid formulation between the sclera and the conjunctiva of a rabbit eye.

At day 32 after injection, the average percentage in vivo vitreal level may be between about 25% and about 85%, and more usually between about 45% and about 65%, relative to the level present at day 7 after injection. At day 40 after injection, the average percentage in vivo vitreal level may be greater than about 25%, and more usually greater than about 45%, relative to the level present at day 7 after injection.

At day 45 after injection, the average percentage in vivo vitreal level may be between about 2% and about 50%, and more usually between about 8% and about 20%, relative to the level present at day 7 after injection. At day 67 after injection, the average percentage in vivo vitreal level may be greater than about 2%, and more usually greater than about 5%, relative to the level present at day 7 after injection.

At day 90 after injection, the average percentage in vivo vitreal level may be between about 40% and about 100%, and more usually between about 60% and about 80%, relative to the level present at day 7 after injection. At day 90 after injection, the average percentage in vivo vitreal level may be greater than about 40%, and more usually greater than about 60%, relative to the level present at day 7 after injection.

The average percentage in vivo vitreal level may have the following characteristics relative to the level present at day 7 after injection: at 32 days after injection it is less than about 80%; at 45 days after injection it is less than about 30%; and 90 days after injection it is less than about 100%.

In some examples, the liquid formulation when injected between the sclera and conjunctiva of a rabbit eye delivers therapeutic agent giving an average concentration of therapeutic agent in the vitreous of the rabbit eye of at least about 0.1 pg/mL for at least about 30, at least about 60, or at least about 90 days after administration of the liquid formulation to the rabbit eye. The liquid formulation when injected between the sclera and conjunctiva of a rabbit eye delivers therapeutic agent giving an average concentration of therapeutic agent in the vitreous of the rabbit eye of at least about 0.01 ng/mL for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 0.1 ng/mL for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 1 ng/mL for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 10 ng/mL for at least about 30, at least about 60, or at least about 90 days after administration of the liquid formulation to the rabbit eye.

The liquid formulation when injected between the sclera and conjunctiva of a rabbit eye may deliver therapeutic agent giving an average concentration of therapeutic agent in the vitreous of the rabbit eye of at least 0.001 ng/mL for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of at least 0.01 ng/mL for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of at least 0.1 ng/mL for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of at least 0.5 ng/mL for at least 30, at least 60, at least 90, or at least 120 days after administration of the liquid formulation to the rabbit eyes.

The liquid formulation when injected between the sclera and conjunctiva of a rabbit eye may deliver therapeutic agent giving an average concentration of therapeutic agent in the vitreous of the rabbit eye of between 0.001 ng/mL and 10.0 ng/mL for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of between 0.01 ng/mL and 10 ng/mL for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of the rabbit eye of between 0.1 ng/mL and 10 ng/mL for at least 30, at least 60, at least 90, or at least 120 days after administration of the liquid formulation to the rabbit eyes.

The liquid formulation when injected between the sclera and conjunctiva of a rabbit eye may deliver therapeutic agent giving an average concentration of therapeutic agent in the vitreous of the rabbit eye of between 0.5 ng/mL and 10.0 ng/mL for at least 30, at least 60, at least 90, or at least 120 days after administration of the liquid formulation to the rabbit eyes.

The liquid formulation when injected between the sclera and conjunctiva of a rabbit eye may deliver therapeutic agent giving a ratio of a maximum average concentration of therapeutic agent in the vitreous of a rabbit eye to a minimum average concentration of therapeutic agent in the vitreous of a rabbit eye less than 100 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the vitreous of a rabbit eye to a minimum average concentration of therapeutic agent in the vitreous of a rabbit eye less than 50 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the vitreous of a rabbit eye to a minimum average concentration of therapeutic agent in the vitreous of a rabbit eye less than 10 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the vitreous of a rabbit eye to a minimum average concentration of therapeutic agent in the vitreous of a rabbit eye less than 5 for days 30 to at least 60, at least 90, or at least 120 days after administration of the liquid formulation to the rabbit eyes.

"Approximately constant," as used herein, means that the average level does not vary by more than one order of magnitude over the extended period of time, i.e., the difference between the maximum and minimum is less than a 10-fold difference for measurements of the average concentration at times in the relevant period of time.

The liquid formulation when injected between the sclera and conjunctiva of a rabbit eye may deliver therapeutic agent giving an average concentration of therapeutic agent in the vitreous of a rabbit eye that is approximately constant at a value greater than 0.001 ng/mL for days 30 to at least 60, at least 90, or at least 120 days after administration of the solution to the rabbit eyes. The liquid formulation when injected between the sclera and conjunctiva of a rabbit eye may deliver therapeutic agent giving an average concentration of therapeutic agent in the vitreous of a rabbit eye that is approximately constant at a value greater than 0.01 ng/mL for days 30 to at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the vitreous of a rabbit eye that is approximately constant at a value greater than 0.1 ng/mL for days 30 to at least 60, at least 90, or at least 120 days after administration of the liquid formulation to the rabbit eyes. In some variations, the liquid formulation when injected between the sclera and conjunctiva of a rabbit eye delivers therapeutic agent giving an average concentration of therapeutic agent in the vitreous of a rabbit eye that is approximately constant at a value of 1.0 ng/mL for days 30 to at least 60, at least 90, or at least 120 days after administration of the liquid formulation to the rabbit eyes.

The liquid formulation when injected between the sclera and conjunctiva of a rabbit eye may deliver therapeutic agent giving an average concentration of therapeutic agent in the retina choroid tissues of the rabbit eye of at least 0.001 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of at least 0.005 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of at least 0.01 ng/mg for at least 30, at least 60, at least 90, or at least 120 days after administration of the liquid formulation to the rabbit eyes.

According to the present description, the liquid formulation when injected between the sclera and conjunctiva of a rabbit eye delivers therapeutic agent giving an average concentration of therapeutic agent in the retina choroid tissues of the rabbit eye of between 0.001 ng/mg and 1.0 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.001 ng/mg and 0.50 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.001 ng/mg and 0.15 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.001 ng/mg and 0.1 ng/mg for at least 30, at least 60, at least 90, or at least 120 days after administration of the liquid formulation to the rabbit eyes.

According to the present description, the liquid formulation when injected between the sclera and conjunctiva of a rabbit eye delivers therapeutic agent giving an average concentration of therapeutic agent in the retina choroid tissues of the rabbit eye of between 0.005 ng/mg and 1.0 ng/mg for at least 30, at least 60, at least 90, or at least 120 days after administration of the liquid formulation to the rabbit eyes. The liquid formulation when injected between the sclera and conjunctiva of a rabbit eye delivers therapeutic agent giving an average concentration of therapeutic agent in the retina choroid tissues of the rabbit eye of between 0.005 ng/mg and 0.50 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.005 ng/mg and 0.15 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.005 ng/mg and 0.1 ng/mg for at least 30, at least 60, at least 90, or at least 120 days after administration of the liquid formulation to the rabbit eyes.

According to the present description, the liquid formulation when injected between the sclera and conjunctiva of a rabbit eye delivers therapeutic agent giving an average concentration of therapeutic agent in the retina choroid tissues of the rabbit eye of between 0.01 ng/mg and 1.0 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.01 ng/mg and 0.50 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.01 ng/mg and 0.15 ng/mg for at least 30, at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of the rabbit eye of between 0.01 ng/mg and 0.1 ng/mg for at least 30, at least 60, at least 90, or at least 120 days after administration of the liquid formulation to the rabbit eyes.

According to the present description, the liquid formulation when injected between the sclera and conjunctiva of a rabbit eye delivers therapeutic agent giving a ratio of a maximum average concentration of therapeutic agent in the retina choroid tissues of a rabbit eye to a minimum average concentration of therapeutic agent in the retina choroid tissues of a rabbit eye less than 100 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the retina choroid tissues of a rabbit eye to a minimum average concentration of therapeutic agent in the retina choroid tissues of a rabbit eye less than 50 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the retina choroid tissues of a rabbit eye to a minimum average concentration of therapeutic agent in the retina choroid tissues of a rabbit eye less than 10 for days 30 to at least 60, at least 90, or at least 120 days after administration of the liquid formulation to the rabbit eyes. The liquid formulation when injected between the sclera and conjunctiva of a rabbit eye may deliver therapeutic agent giving a ratio of a maximum average concentration of therapeutic agent in the retina choroid tissues of a rabbit eye to a minimum average concentration of therapeutic agent in the retina choroid tissues of a rabbit eye less than 5 for days 30 to at least 60, at least 90, or at least 120 days after administration of the liquid formulation to the rabbit eyes.

According to the present description, the liquid formulation when injected between the sclera and conjunctiva of a rabbit eye delivers therapeutic agent giving an average concentration of therapeutic agent in the retina choroid tissues of a rabbit eye that is approximately constant at a value greater than 0.001 ng/mg for days 30 to at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of a rabbit eye that is approximately constant at a value greater than 0.005 ng/mg for days 30 to at least 60, at least 90, or at least 120 days after administration of the liquid formulation to the rabbit eyes. The liquid formulation when injected between the sclera and conjunctiva of a rabbit eye may deliver therapeutic agent giving an average concentration of therapeutic agent in the retina choroid tissues of a rabbit eye that is approximately constant at a value greater than 0.01 ng/mg for days 30 to at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the vitreous of the rabbit eye of at least 100 ng/mL for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the vitreous of the rabbit eye of at least 1000 ng/mL for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the vitreous of the rabbit eye of at least 10,000 ng/mL for at least 30, at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the vitreous of the rabbit eye between 100 ng/mL and 100,000 ng/mL for day 30 to at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the vitreous of the rabbit eye between 100 ng/mL and 50,000 ng/mL for day 30 to at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the vitreous of the rabbit eye between 1000 ng/mL and 100,000 ng/mL for day 30 to at least 60, at least 90, or at least 120 days after administration of the liquid formulation to the rabbit eyes. In some variations, the liquid formulation when injected into the vitreous of a rabbit eye delivers therapeutic agent giving an average concentration of therapeutic agent in the vitreous of the rabbit eye between 1000 ng/mL and 50,000 ng/mL for day 30 to at least 60, at least 90, or at least 120 days after administration of the liquid formulation to the rabbit eyes.

According to the present description, the liquid formulation when injected into the vitreous of a rabbit eye delivers therapeutic agent giving a ratio of a maximum average concentration of therapeutic agent in the vitreous of the rabbit eye to a minimum average concentration of therapeutic agent in the vitreous of the rabbit eye less than 100 for days 30 to at least 60, at least 90, or at least 120 days after administration of the liquid formulation to the rabbit eyes. The liquid formulation when injected into the vitreous of a rabbit eye may deliver therapeutic agent giving a ratio of a maximum average concentration of therapeutic agent in the vitreous of the rabbit eye to a minimum average concentration of therapeutic agent in the vitreous of the rabbit eye less than 50 for days 30 to at least 60, at least 90, or at least 120 days after administration of the liquid formulation to the rabbit eyes. The liquid formulation when injected into the vitreous of a rabbit eye may deliver therapeutic agent giving a ratio of a maximum average concentration of therapeutic agent in the vitreous of the rabbit eye to a minimum average concentration of therapeutic agent in the vitreous of the rabbit eye less than 10 for days 30 to at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the vitreous of the rabbit eye that is approximately constant at a value greater than 100 ng/mL for days 30 to at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the vitreous of the rabbit eye that is approximately constant at a value greater than 1000 ng/mL for days 30 to at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the vitreous of the rabbit eye that is approximately constant at a value greater than 10,000 ng/mL for days 30 to at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the retina choroid tissues of the rabbit eye of at least 0.001 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye of at least 0.01 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye of at least 0.05 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye of at least 0.10 ng/mg for at least 30, at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the retina choroid tissues of the rabbit eye between 0.001 ng/mg and 10.00 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye between 0.001 ng/mg and 5.00 ng/mg for at least 30, at least 60, at least 90, or at least 120 days after administration of the liquid formulation to the rabbit eyes. In some variations, the liquid formulation when injected into the vitreous of a rabbit eye delivers therapeutic agent giving an average concentration of therapeutic agent in the retina choroid tissues of the rabbit eye between 0.001 ng/mg and 1.00 ng/mg for at least 30, at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the retina choroid tissues of the rabbit eye between 0.01 ng/mg and 10.00 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye between 0.01 ng/mg and 5.00 ng/mg for at least 30, at least 60, at least 90, or at least 120 days after administration of the liquid formulation to the rabbit eyes. In some variations, the liquid formulation when injected into the vitreous of a rabbit eye delivers therapeutic agent giving an average concentration of therapeutic agent in the retina choroid tissues of the rabbit eye between 0.01 ng/mg and 1.00 ng/mg for at least 30, at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the retina choroid tissues of the rabbit eye between 0.05 ng/mg and 10.00 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye between 0.05 ng/mg and 5.00 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye between 0.05 ng/mg and 1.00 ng/mg for at least 30, at least 60, at least 90, or at least 120 days after administration of the liquid formulation to the rabbit eyes.

According to the present description, the liquid formulation when injected into the vitreous of a rabbit eye delivers therapeutic agent giving an average concentration of therapeutic agent in the retina choroid tissues of the rabbit eye between 0.10 ng/mg and 10.00 ng/mg for at least 30, at least 60, at least 90, or at least 120 days after administration of the liquid formulation to the rabbit eyes. According to the present description, the liquid formulation when injected into the vitreous of a rabbit eye may deliver therapeutic agent giving an average concentration of therapeutic agent in the retina choroid tissues of the rabbit eye between 0.10 ng/mg and 5.00 ng/mg for at least 30, at least 60, at least 90, or at least 120 days after administration of the liquid formulation to the rabbit eyes. The liquid formulation when injected into the vitreous of a rabbit eye may deliver therapeutic agent giving an average concentration of therapeutic agent in the retina choroid tissues of the rabbit eye between 0.10 ng/mg and 1.00 ng/mg for at least 30, at least 60, at least 90, or at least 120 days after administration of the liquid formulation to the rabbit eyes.

According to the present description, the liquid formulation when injected into the vitreous of a rabbit eye delivers therapeutic agent giving a ratio of a maximum average concentration of therapeutic agent in the retina choroid tissues of the rabbit eye to a minimum average concentration of therapeutic agent in the retina choroid tissues of the rabbit eye less than 100 for days 30 to at least 60, at least 90, or at least 120 days after administration of the liquid formulation to the rabbit eyes. The liquid formulation when injected into the vitreous of a rabbit eye may deliver therapeutic agent giving a ratio of a maximum average concentration of therapeutic agent in the retina choroid tissues of the rabbit eye to a minimum average concentration of therapeutic agent in the retina choroid tissues of the rabbit eye less than 50 for days 30 to at least 60, at least 90, or at least 120 days after administration of the liquid formulation to the rabbit eyes.

According to the present description, in situ gelling liquid formulations described herein may have in vivo delivery to the retina choroid tissue profiles with the following described characteristics, where the delivery profiles are for delivery of therapeutic agent in vivo after injection of the liquid formulation between the sclera and the conjunctiva of a rabbit eye.

At day 32 after injection, the percentage in vivo vitreal level may be between about 20% and about 80%, and more usually between about 40% and about 60%, relative to the level present at day 7 after injection. At day 40 after injection, the percentage in vivo vitreal level may be greater than about 20%, and more usually greater than about 40%, relative to the level present at day 7 after injection.

At day 45 after injection, the percentage in vivo vitreal level may be between about 15% and about 55%, and more usually between about 25% and about 45%, relative to the level present at day 7 after injection. At day 67 after injection, the percentage in vivo vitreal level may be greater than about 15%, and more usually greater than about 25%, relative to the level present at day 7 after injection.

At day 90 after injection, the percentage in vivo vitreal level may be between about 60% and about 100%, and more usually between about 70% and about 90%, relative to the level present at day 7 after injection. At day 90 after injection, the percentage in vivo vitreal level may be greater than about 60%, and more usually greater than about 70%, relative to the level present at day 7 after injection.

According to the present description, the percentage in vivo vitreal level has the following characteristics relative to the level present at day 7 after injection: at 32 days after injection it is less than about 80%; at 45 days after injection it is less than about 60%; and 90 days after injection it is less than about 100%.

According to the present description, the liquid formulation when injected between the sclera and conjunctiva of a rabbit eye delivers therapeutic agent giving an average concentration of therapeutic agent in the retina choroid tissues of the rabbit eye of at least about 0.1 pg/mg for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 0.01 ng/mg for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 0.1 ng/mg for at least about 30, at least about 60, or at least about 90 days after administration of the liquid formulation to the rabbit eye. The liquid formulation when injected between the sclera and conjunctiva of a rabbit eye may deliver therapeutic agent giving an average concentration of therapeutic agent in the vitreous of the rabbit eye of at least about 1 ng/mL for at least about 30, at least about 60, or at least about 90 days after administration of the liquid formulation to the rabbit eye.

According to the present description, the ratio of the base ten logarithms of the average levels of a therapeutic agent in two or more of the retina choroid tissues, the sclera, and the vitreous is approximately constant over an extended period of time after placement of the in situ gelling formulation in or proximate to the eye. The ratio of the base ten logarithms of the average levels of a therapeutic agent in two or more of the retina choroid tissues, the sclera, and the vitreous may be approximately constant over an extended period of time after placement of the in situ gelling formulation between the sclera and the conjunctiva of an eye. The ratio of the base ten logarithms of the average levels of a therapeutic agent in the vitreous and the sclera may be approximately constant over an extended period of time after placement of the in situ gelling formulation between the sclera and the conjunctiva of an eye.

According to the present description, the ratio of the base ten logarithms of the average levels of a therapeutic agent in the vitreous and the retina choroid tissues is approximately constant over an extended period of time. Put another way, as the level of therapeutic agent in the vitreous rises, the level of therapeutic agent in the retina choroid tissues rises to a similar degree when considered on the logarithmic scale, and vice versa.

According to the present description, the ratio of the base ten logarithms of the average levels of a therapeutic agent in the vitreous versus the retina choroid tissues is approximately constant over an extended period of time of about 7, about 30, about 60, or about 90 days. The ratio of the average level of therapeutic agent in the vitreous relative to the level of therapeutic agent in the retina choroid tissues after placement of the in situ gelling formulation between the sclera and the conjunctiva of an eye may be constant at about 37:1 at day 7, about 40:1 at day 32, about 10:1 at day 45, and about 34:1 at day 90.

According to the present description, the ratio of the average level of therapeutic agent in the vitreous relative to the level of therapeutic agent in the retina choroid tissues is constant at about 40:1 over a period of about 7, about 32, about 45, or about 90 days.

According to the present description, the average level of the therapeutic agent in any or all of the retina choroid tissues, the sclera, and the vitreous is approximately constant over an extended period of time after placement of the in situ gelling formulation in or proximate to the eye.

According to the present description, after placement of an in situ gelling formulation between the sclera and the conjunctiva, the average level of therapeutic agent in the vitreous is approximately constant at about 8.1 ng/ml. In some variations, after placement of an in situ gelling formulation between the sclera and the conjunctiva, the average level of therapeutic agent in the retina choroid tissues is approximately constant at about 0.25 ng/mg. After placement of an in situ gelling formulation between the sclera and the conjunctiva, the average level of therapeutic agent in the sclera is approximately constant at about 1930 ng/mg.

According to the present description, the in situ gelling formulation when injected between the sclera and conjunctiva of a rabbit eye maintains an average level of therapeutic agent in the vitreous that is approximately constant at about 0.1 pg/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 0.001 ng/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 0.01 ng/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 0.1 ng/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 1 ng/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 10 ng/mL for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the vitreous that may be approximately constant at about 100 ng/mL for at least about 30, at least about 60, or at least about 90 days after administration of the liquid formulation to the rabbit eye.

According to the present description, the in situ gelling formulation when injected between the sclera and conjunctiva of a rabbit eye maintains an average level of therapeutic agent in the retina choroid tissues that is approximately constant at about 0.1 pg/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the retina choroid tissues that may be approximately constant at about 0.001 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the retina choroid tissues that may be approximately constant at about 0.01 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the retina choroid tissues that may be approximately constant at about 0.1 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the retina choroid tissues that may be approximately constant at about 1 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the retina choroid tissues that may be approximately constant at about 10 ng/mg for at least about 30, at least about 60, or at least about 90 days after administration of the liquid formulation to the rabbit eye.

According to the present description, the in situ gelling formulation when injected between the sclera and conjunctiva of a rabbit eye maintains an average level of therapeutic agent in the sclera that is approximately constant at about 0.1 pg/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 0.001 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 0.01 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 0.1 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 1 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 10 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 100 ng/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 1 µg/mg for at least about 30, at least about 60, or at least about 90 days after administration of the liquid formulation to the rabbit eye. The in situ gelling formulation when injected between the sclera and conjunctiva of a rabbit eye maintains an average level of therapeutic agent in the sclera that may be approximately constant at about 10 µg/mg for at least about 30, at least about 60, or at least about 90 days after administration of the liquid formulation to the rabbit eye.

For treatment, prevention, inhibition, delaying the onset of, or causing the regression of certain diseases or conditions, it may be desirable to maintain delivery of a therapeutically effective amount of the therapeutic agent for an extended period of time. Depending on the disease or condition being treated, prevented, inhibited, having onset delayed, or being caused to regress this extended period of time may be at least about 1 week, at least about 2 weeks, at least about 3 weeks, at least about 1 month, at least about 3 months, at least about 6 months, at least about 9 months, or at least about 1 year. Generally, however, any extended period of delivery may be possible. A therapeutically effective amount of agent may be delivered for an extended period by a liquid formulation or composition that maintains for the extended period a concentration of agent in a subject or an eye of a subject sufficient to deliver a therapeutically effective amount of agent for the extended time.

Delivery of a therapeutically effective amount of the therapeutic agent for an extended period may be achieved via placement of one composition or liquid formulation or may be achieved by application of two or more doses of composition or liquid formulations. As an example of such multiple applications, maintenance of the therapeutic amount of rapamycin for 3 months for treatment, prevention, inhibition, delay of onset, or cause of regression of wet AMD may be achieved by application of one liquid formulation or composition delivering a therapeutic amount for 3 months or by sequential application of a plurality of liquid formulations or compositions. The optimal dosage regime will depend on the therapeutic amount of the therapeutic agent needing to be delivered, and the period over which it need be delivered. Those versed in such extended therapeutic agent delivery dosing will understand how to identify dosing regimens that may be used based on the teachings provided herein.

When using certain therapeutic agents or for the treatment, prevention, inhibition, delaying the onset of, or causing the regression of certain diseases, it may be desirable for delivery of the therapeutic agent not to commence immediately upon placement of the liquid formulation or composition into the eye region, but for delivery to commence after some delay. For example, such delayed release may be useful where the therapeutic agent inhibits or delays wound healing and delayed release is desirable to allow healing of any wounds occurring upon placement of the liquid formulation or composition. Depending on the therapeutic agent being delivered and/or the diseases and conditions being treated, prevented, inhibited, onset delayed, and regression caused this period of delay before delivery of the therapeutic agent commences may be about 1 hour, about 6 hours, about 12 hours, about 18 hours, about 1 day, about 2 days, about 3 days, about 4 days, about 5 days, about 6 days, about 7 days, about 8 days, about 9 days, about 10 days, about 11 days, about 12 days, about 13 days, about 14 days, about 21 days, about 28 days, about 35 days, or about 42 days. Other delay periods may be possible. Delayed release formulations that may be used are known to people versed in the technology.

### Intravitreal, subconjunctival, subtenonal, and topical delivery of rapamycin for treatment, prevention, inhibition, delay of onset, or cause of regression of AMD

In one method described herein, a liquid formulation comprising rapamycin is delivered subconjunctivally or to the vitreous of an eye to prevent, treat, inhibit, delay onset of, or cause regression of angiogenesis in the eye, such as treating CNV as observed, for example, in AMD. The liquid formulation may be used to treat angiogenesis in the eye, such as treating CNV as observed, for example, in AMD.

Rapamycin has been shown to inhibit CNV in rat and mice models, as described in U.S. Application No. 10/665,203. Rapamycin has been observed to inhibit Matrigel^{™} and laser-induced CNV when administered systemically and subretinally. Also, periocular injection of rapamycin inhibits laser-induced CNV.

As described herein, the dosage of the therapeutic agent will depend on the condition being addressed, whether the condition is to be treated, prevented, inhibited, have onset delayed, or be caused to regress, the particular therapeutic agent, and other clinical factors such as weight and condition of the subject and the route of administration of the therapeutic agent. It is to be understood that the methods, liquid formulations, and compositions described herein have application for both human and veterinary use, as well as uses in other possible animals. As described herein, tissue concentrations of therapeutic agents expressed in units of mass per volume generally refer to tissues that are primarily aqueous such as the vitreous, for example. Tissue concentrations of therapeutic agents expressed in unit of mass per mass generally refer to other tissues such as the sclera or retina choroid tissues, for example.

One concentration of rapamycin that may be used in the methods described herein is one that provides about 0.01 pg/ml or pg/mg or more of rapamycin at the tissue level. Another concentration that may be used is one that provides about 0.1 pg/ml or ng/mg or more at the tissue level. Another concentration that may be used is one that provides about 1 pg/ml or ng/mg or more at the tissue level. Another concentration that may be used is one that provides about 0.01 ng/ml or ng/mg or more at the tissue level. Another concentration that may be used is one that provides about 0.1 ng/ml or ng/mg or more at the tissue level. Another concentration that may be used is one that provides about 0.5 ng/ml or ng/mg or more at the tissue level. Another concentration that may be used is one that provides about 1 ng/ml or more at the tissue level. Another concentration that may be used is one that provides about 2 ng/ml or more at the tissue level. Another concentration that may be used is one that provides about 3 ng/ml or more at the tissue level. Another concentration that may be used is one that provides about 5 ng/ml or more at the tissue level. Another concentration that may be used is one that provides about 10 ng/ml or more at the tissue level. Another concentration that may be used is one that provides about 15 ng/ml or more at the tissue level. Another concentration that may be used is one that provides about 20 ng/ml or more at the tissue level. Another concentration that may be used is one that provides about 30 ng/ml or more at the tissue level. Another concentration that may be used is one that provides about 50 ng/ml or more at the tissue level. One of ordinary skill in the art would know how to arrive at an appropriate concentration depending on the route and duration of administration utilized, given the teachings herein.

Generally, the amount of rapamycin administered in a liquid formulation is an amount sufficient to treat, prevent, inhibit, delay the onset, or cause regression of the disease or condition of the eye for the required amount of time. In some variations the amount of rapamycin administered in the liquid formulation is an amount sufficient to treat the disease or condition of the eye for the required amount of time.

In some variations, a total amount of rapamycin less than about 5 mg is administered subconjunctivally. In some variations, a total amount of rapamycin less than about 5.0 mg is administered subconjunctivally. In some variations, a total amount of rapamycin less than about 4.5 mg is administered subconjunctivally. In some variations, a total amount of rapamycin less than about 4.0 mg is administered subconjunctivally. In some variations, a total amount of rapamycin less than about 3.5 mg is administered subconjunctivally. In some variations, a total amount of rapamycin less than about 3.0 mg is administered subconjunctivally. In some variations, a total amount of rapamycin less than about 2.5 mg is administered subconjunctivally. In some variations, a total amount of rapamycin less than about 2 mg is administered subconjunctivally. In some variations, a total amount of rapamycin less than about 1.2 mg is administered subconjunctivally. In some variations, a total amount of rapamycin less than about 1.0 mg is administered subconjunctivally. In some variations, a total amount of rapamycin less than about 0.8 mg is administered subconjunctivally. In some variations, a total amount of rapamycin less than about 0.6 mg is administered subconjunctivally. In some variations, a total amount of rapamycin less than about 0.4 mg is administered subconjunctivally. In some variations, a volume of a formulation is administered that contains an amount of rapamycin described herein.

In some variations, a liquid formulation containing a concentration of rapamycin by weight of the total of between about 0.5% and about 6% is subconjunctivally administered to a human subject by administering between about 0.1 µl and about 200 µl of a liquid formulation described herein. In some variations, a liquid formulation containing a concentration of rapamycin by weight of the total of between about 0.5% and about 4% is subconjunctivally administered to a human subject by administering between about 1 µl and about 50 µl of a liquid formulation described herein. In some variations, a liquid formulation containing a concentration of rapamycin by weight of the total of between about 1.5% and about 3.5% is subconjunctivally administered to a human subject by administering between about 1 µl and about 15 µl of a liquid formulation described herein. In some variations, a liquid formulation containing a concentration of rapamycin by weight of the total of about 2% is subconjunctivally administered to a human subject by administering between about 1 µl and about 15 µl of a liquid formulation described herein.

In some variations, a liquid formulation containing an amount of rapamycin of between about 0.2 µg and about 4 mg is subconjunctivally administered to a human subject by administering between about 0.1 µl and about 200 µl of a liquid formulation described herein. In some variations, a liquid formulation containing an amount of rapamycin of between about 20 µg and about 2 mg is subconjunctivally administered to a human subject by administering between about 1 µl and about 100 µl of a liquid formulation described herein. In some variations, a liquid formulation containing an amount of rapamycin of between about 20 µg and about 1 mg is subconjunctivally administered to a human subject by administering between about 1 µl and about 50 µl of a liquid formulation described herein. In some variations, a liquid formulation containing an amount of rapamycin of between about 20 µg and about 500 µg is subconjunctivally administered to a human subject by administering between about 1 µl and about 25 µl of a liquid formulation described herein. In some variations, a liquid formulation containing an amount of rapamycin of between about 20 µg and about 300 µg is subconjunctivally administered to a human subject by administering between about 1 µl and about 15 µl of a liquid formulation described herein.

In some variations, a total amount of rapamycin less than about 200 µg is administered intravitreally. In some variations, a total amount of rapamycin less than about 200 µg is administered intravitreally. In some variations, a total amount of rapamycin less than about 300 µg is administered intravitreally. In some variations, a total amount of rapamycin less than about 400 µg is administered intravitreally. In some variations, a total amount of rapamycin less than about 500 µg is administered intravitreally. In some variations, a total amount of rapamycin less than about 600 µg is administered intravitreally. In some variations, a total amount of rapamycin less than about 800 µg is administered intravitreally. In some variations, a total amount of rapamycin less than about 1 mg is administered intravitreally. In some variations, a total amount of rapamycin less than about 2 mg is administered intravitreally. In some variations, a total amount of rapamycin less than about 2.5 mg is administered intravitreally. In some variations, a total amount of rapamycin less than about 3 mg is administered intravitreally. In some variations, a total amount of rapamycin less than about 3.5 mg is administered intravitreally. In some variations, a total amount of rapamycin less than about 4 mg is administered intravitreally. In some variations, a volume of a formulation is administered that contains an amount of rapamycin described herein.

In some variations, a liquid formulation containing a concentration of rapamycin by weight of the total of between about 0.5% and about 6% is intravitreally administered to a human subject by administering between about 0.1 µl and about 200 µl of a liquid formulation described herein. In some variations, a liquid formulation containing a concentration of rapamycin by weight of the total of between about 0.5% and about 4% is intravitreally administered to a human subject by administering between about 1 µl and about 50 µl of a liquid formulation described herein. In some variations, a liquid formulation containing a concentration of rapamycin by weight of the total of between about 1.5% and about 3.5% is intravitreally administered to a human subject by administering between about 1 µl and about 15 µl of a liquid formulation described herein. In some variations, a liquid formulation containing a concentration of rapamycin by weight of the total of about 2% is intravitreally administered to a human subject by administering between about 1 µl and about 15 µl of a liquid formulation described herein.

In some variations, a liquid formulation containing an amount of rapamycin of between about 0.2 µg and about 4 mg is intravitreally administered to a human subject by administering between about 0.1 µl and about 200 µl of a liquid formulation described herein. In some variations, a liquid formulation containing an amount of rapamycin of between about 20 µg and about 2 mg is intravitreally administered to a human subject by administering between about 1 µl and about 100 µl of a liquid formulation described herein. In some variations, a liquid formulation containing an amount of rapamycin of between about 20 µg and about 1 mg is intravitreally administered to a human subject by administering between about 1 µl and about 50 µl of a liquid formulation described herein. In some variations, a liquid formulation containing an amount of rapamycin of between about 20 µg and about 500 µg is intravitreally administered to a human subject by administering between about 1 µl and about 25 µl of a liquid formulation described herein. In some variations, a liquid formulation containing an amount of rapamycin of between about 20 µg and about 300 µg is intravitreally administered to a human subject by administering between about 1 µl and about 15 µl of a liquid formulation described herein.

In some variations, a total amount of rapamycin less than about 5 mg is administered subtenonally. In some variations, a total amount of rapamycin less than about 5.0 mg is administered subtenonally. In some variations, a total amount of rapamycin less than about 4.5 mg is administered subtenonally. In some variations, a total amount of rapamycin less than about 4.0 mg is administered subtenonally. In some variations, a total amount of rapamycin less than about 3.5 mg is administered subtenonally. In some variations, a total amount of rapamycin less than about 3.0 mg is administered subtenonally. In some variations, a total amount of rapamycin less than about 2.5 mg is administered subtenonally. In some variations, a total amount of rapamycin less than about 2 mg is administered subtenonally. In some variations, a total amount of rapamycin less than about 1.2 mg is administered subtenonally. In some variations, a total amount of rapamycin less than about 1.0 mg is administered subtenonally. In some variations, a total amount of rapamycin less than about 0.8 mg is administered subtenonally. In some variations, a total amount of rapamycin less than about 0.6 mg is administered subtenonally. In some variations, a total amount of rapamycin less than about 0.4 mg is administered subtenonally. In some variations, a volume of a formulation is administered that contains an amount of therapeutic agent described herein.

In some variations, a total amount of rapamycin less than about 5 mg is administered topically. In some variations, a total amount of rapamycin less than about 5.0 mg is administered topically. In some variations, a total amount of rapamycin less than about 4.5 mg is administered topically. In some variations, a total amount of rapamycin less than about 4.0 mg is administered topically. In some variations, a total amount of rapamycin less than about 3.5 mg is administered topically. In some variations, a total amount of rapamycin less than about 3.0 mg is administered topically. In some variations, a total amount of rapamycin less than about 2.5 mg is administered topically. In some variations, a total amount of rapamycin less than about 2 mg is administered topically. In some variations, a total amount of rapamycin less than about 1.2 mg is administered topically. In some variations, a total amount of rapamycin less than about 1.0 mg is administered topically. In some variations, a total amount of rapamycin less than about 0.8 mg is administered topically. In some variations, a total amount of rapamycin less than about 0.6 mg is administered topically. In some variations, a total amount of rapamycin less than about 0.4 mg is administered topically. In some variations, a volume of a formulation is administered that contains an amount of rapamycin described herein. In some variations, a total amount of therapeutic agent administered topically is any of between about 20 µg and about 4000 µg, between about 10 µg and about 2000 µg, between about 10 µg and 1750 µg, between about 1500 µg and 1000 µg, or between about 10 µg and 1000 µg. In some variations, a total amount of therapeutic agent administered topically is about 1660 µg. In some variations, a total amount of therapeutic agent administered topically is about 880 µg. In some variations, a total amount of therapeutic agent administered topically is 40 µg. In some variations, a total amount of therapeutic agent administered topically is about 28 µg.

According to the present description, the liquid formulation when topically administered to a rabbit eye delivers therapeutic agent giving an average concentration of therapeutic agent in the cornea of the rabbit eye of at least any of about 0.001 ng/mg, 0.01 ng/mg, 0.1 ng/mg, or 1 ng/mg. The liquid formulation when topically administered to a rabbit eye may deliver therapeutic agent giving an average concentration of therapeutic agent in the cornea of the rabbit eye of at least 0.01 ng/mg. The liquid formulation when topically administered to a rabbit eye may deliver therapeutic agent giving an average concentration of therapeutic agent in the retina choroid tissues of the rabbit eye of at least any of about 0.0001 ng/mg, 0.001 ng/mg, 0.01 ng/mg, 0.1 ng/mg, or 1 ng/mg. The liquid formulation when topically administered to a rabbit eye may deliver therapeutic agent giving an average concentration of therapeutic agent in the retina choroid tissues of the rabbit eye of at least 0.001 ng/mg.

In some variations, a total amount of rapamycin administered subconjunctivally is any of between about 50 µg and about 3 mg, between about 150 µg and about 750 µg, between about 300 µg and about 1000 µg, between about 300 µg and about 950 µg, between about 400 µg and about 900 µg, between about 450 µg and about 850 µg, between about 500 µg and about 800 µg, between about 550 µg and about 750 µg, or between about 600 µg and about 700 µg. In some variations, a total amount of rapamycin administered subconjunctivally is any of about 220 µg, about 440 µg, about 587 µg, about 630 µg, about 660 µg, about 880 µg, about 1320 µg, about 1760 µg, or about 2200 µg. In some variations, a total amount of rapamycin administered subconjunctivally is about 220 µg. In some variations, a total amount ofrapamycin administered subconjunctivally is about 440 µg. In some variations, a total amount of rapamycin administered subconjunctivally is about 660 µg. In some variations, a total amount of rapamycin administered subconjunctivally is about 880 µg. In some variations, a liquid formulation containing an amount of rapamycin of 220 µg is subconjunctivally administered to a human subject by administering about 10 µl of a liquid formulation described herein. In some variations, a liquid formulation containing an amount of rapamycin of 440 µg is subconjunctivally administered to a human subject by administering about 20 µl of a liquid formulation described herein. In some variations, a liquid formulation containing an amount of rapamycin of 660 µg is subconjunctivally administered to a human subject by administering about 30 µl of a liquid formulation described herein. In some variations, a liquid formulation containing an amount of rapamycin of 880 µg is subconjunctivally administered to a human subject by administering about 40 µl of a liquid formulation described herein.

In some variations, a total amount of rapamycin administered intravitreally is any of between about 20 µg and about 750 µg, between about 20 µg and about 500 µg, or between about 30 µg and about 200 µg. In some variations, a total amount of rapamycin administered intravitreally is any of about 44 µg, about 110 µg, about 132 µg, about 133.5 µg, about 176 µg, about 264 µg, about 352 µg, or about 440 µg. In some variations, a total amount of rapamycin administered intravitreally is about 44 µg. In some variations, a total amount of rapamycin administered intravitreally is about 110 µg. In some variations, a liquid formulation containing an amount of rapamycin of 44 µg is intravitreally administered to a human subject by administering about 2 µl of a liquid formulation described herein. In some variations, a liquid formulation containing an amount of rapamycin of 110 µg is intravitreally administered to a human subject by administering about 5 µl of a liquid formulation described herein. In some variations, a liquid formulation containing an amount of rapamycin of 176 µg is intravitreally administered to a human subject by administering about 8 µl of a liquid formulation described herein.

In some variations a liquid formulation as described herein containing an amount of rapamycin of between about 1 µg and about 5 mg is administered to a human subject for treatment of wet AMD. In some variations a liquid formulation as described herein containing an amount of rapamycin of between about 20 µg and about 4 mg is administered to a human subject for treatment of wet AMD. In some variations a liquid formulation as described herein containing an amount of rapamycin of between about 20 µg and about 1.2 mg is administered to a human subject for treatment of wet AMD. In some variations an amount of rapamycin of between about 10 µg and about 0.5 mg is administered to a human subject for treatment of wet AMD. In some variations an amount of rapamycin of between about 10 µg and 90 µg is administered to a human subject for treatment of wet AMD. In some variations an amount of rapamycin of between about 60 µg and about 120 µg is administered to a human subject for treatment of wet AMD. In some variations an amount of rapamycin of between about 100 µg and about 400 µg is administered to a human subject for treatment of wet AMD. In some variations an amount of rapamycin of between about 400 µg and about 1 mg is administered to a human subject for treatment of wet AMD. In some variations an amount of rapamycin of between about 1 mg and about 5 mg is administered to a human subject for treatment of wet AMD. In some variations, an amount of rapamycin of between about 3 mg and about 7 mg is administered to a human subject for treatment of wet AMD. In some variations, an amount of rapamycin of between about 5 mg and about 10 mg is administered to a human subject for treatment of wet AMD.

In some variations a liquid formulation as described herein containing an amount of rapamycin of between about 1 µg and about 5 mg is administered to a human subject for prevention of wet AMD. In some variations a liquid formulation as described herein containing an amount of rapamycin of between about 20 µg and about 4 mg is administered to a human subject for prevention of wet AMD. In some variations a liquid formulation as described herein containing an amount of rapamycin of between about 20 µg and about 1.2 mg is administered to a human subject for prevention of wet AMD. In some variations an amount of rapamycin of between about 10 µg and about 0.5 mg is administered to a human subject for prevention of wet AMD. In some variations an amount of rapamycin of between about 10 µg and 90 µg is administered to a human subject for prevention of wet AMD. In some variations an amount of rapamycin of between about 60 µg and about 120 µg is administered to a human subject for prevention of wet AMD. In some variations an amount of rapamycin of between about 100 µg and about 400 µg is administered to a human subject for prevention of wet AMD. In some variations an amount of rapamycin of between about 400 µg and about 1 mg is administered to a human subject for prevention of wet AMD. In some variations an amount of rapamycin of between about 1 mg and about 5 mg is administered to a human subject for prevention of wet AMD. In some variations, an amount of rapamycin of between about 3 mg and about 7 mg is administered to a human subject for prevention of wet AMD. In some variations, an amount of rapamycin of between about 5 mg and about 10 mg is administered to a human subject for prevention of wet AMD.

In some variations a liquid formulation as described herein containing an amount of rapamycin of between about 1 µg and about 5 mg is administered to a human subject for treatment of dry AMD. In some variations a liquid formulation as described herein containing an amount of rapamycin of between about 20 µg and about 4 mg is administered to a human subject for treatment of dry AMD. In some variations a liquid formulation as described herein containing an amount of rapamycin of between about 20 µg and about 1.2 mg is administered to a human subject for treatment of dry AMD. In some variations an amount of rapamycin of between about 10 µg and about 0.5 mg is administered to a human subject for treatment of dry AMD. In some variations an amount of rapamycin of between about 10 µg and 90 µg is administered to a human subject for treatment of dry AMD. In some variations an amount of rapamycin of between about 60 µg and about 120 µg is administered to a human subject for treatment of dry AMD. In some variations an amount of rapamycin of between about 100 µg and about 400 µg is administered to a human subject for treatment of dry AMD. In some variations an amount of rapamycin of between about 400 µg and about 1 mg is administered to a human subject for treatment of dry AMD. In some variations an amount of rapamycin of between about 1 mg and about 5 mg is administered to a human subject for treatment of dry AMD. In some variations, an amount of rapamycin of between about 3 mg and about 7 mg is administered to a human subject for treatment of dry AMD. In some variations, an amount of rapamycin of between about 5 mg and about 10 mg is administered to a human subject for treatment of dry AMD.

In some variations, a liquid formulation as described herein containing an amount of rapamycin of between about 1 µg and about 5 mg is administered to a human subject for treatment of angiogenesis, such as choroidal neovascularization. In some variations, an amount of rapamycin of between about 20 µg and about 4 mg is administered to the human subject; between about 20 µg and about 1.2 mg; between about 10 µg and about 0.5 mg is administered to a human subject for treatment of wet AMD, between about 10 µg and 90 µg, between about 60 µg and 120 µg is administered to the human subject; between about 100 µg and 400 µg, between about 400 µg and 1 mg is administered to the human subject; in some variations, an amount of rapamycin of between about 1 mg and 5 mg is administered to the human subject; in some variations, an amount of rapamycin of between about 3 mg and 7 mg is administered to the human subject; in some variations, an amount of rapamycin of between about 5 mg and 10 mg is administered to the human subject for treatment of angiogenesis, such as choroidal neovascularization.

In one method according to the present disclosure, a liquid formulation as described herein contains an amount of a therapeutic agent equivalent to an amount of rapamycin.

In one method according to the present disclosure, a liquid formulation as described herein containing an amount of a therapeutic agent equivalent to an amount of rapamycin of between about 1 µg and about 5 mg is administered to a human subject for treatment of wet AMD. An amount of a therapeutic agent equivalent to an amount of rapamycin of between about 1 µg and about 5 mg may be administered to the human subject; between about 20 µg and about 1.2 mg; between about 10 µg and about 0.5 mg is administered to a human subject for treatment of wet AMD, between about 10 µg and 90 µg, between about 60 µg and 120 µg is administered to the human subject; between about 100 µg and 400 µg between about 400 µg and 1 mg is administered to the human subject is administered to the human subject;, an amount of a therapeutic agent equivalent to an amount of rapamycin of between about 1 mg and 5 mg may be administered to the human subject; an amount of a therapeutic agent equivalent to an amount of rapamycin of between about 3 mg and 7 mg may be administered to the human subject; an amount of a therapeutic agent equivalent to an amount of rapamycin of between about 5 mg and 10 mg may be administered to the human subject.

In some variations, a liquid formulation as described herein containing an amount of a therapeutic agent equivalent to an amount of rapamycin of between about 1 µg and about 5 mg is administered to a human subject for treatment of dry AMD. In some variations, an amount of a therapeutic agent equivalent to an amount of rapamycin of between about 20 µg and about 4 mg is administered to the human subject; between about 20 µg and about 1.2 mg; between about 10 µg and about 0.5 mg is administered to a human subject for treatment of wet AMD, between about 10 µg and 90 µg, between about 60 µg and 120 µg is administered to the human subject; between about 100 µg and 400 µg, between about 400 µg and 1 mg is administered to the human subject; in some variations, an amount of a therapeutic agent equivalent to an amount of rapamycin of between about 400 µg and 1 mg is administered to the human subject; in some variations, an amount of a therapeutic agent equivalent to an amount of rapamycin of between about 1 mg and 5 mg is administered to the human subject; in some variations, an amount of a therapeutic agent equivalent to an amount of rapamycin of between about 3 mg and 7 mg is administered to the human subject; in some variations, an amount of a therapeutic agent equivalent to an amount of rapamycin of between about 5 mg and 10 mg is administered to the human subject to treat dry AMD.

In some variations, a liquid formulation as described herein containing an amount of a therapeutic agent equivalent to an amount of rapamycin of between about 1 µg and about 5 mg is administered to a human subject for prevention of wet AMD. In some variations, an amount of a therapeutic agent equivalent to an amount of rapamycin of between about 20 µg and about 4 mg is administered to the human subject; between about 20 µg and about 1.2 mg; between about 10 µg and about 0.5 mg is administered to a human subject for prevention of wet AMD, between about 10 µg and 90 µg, between about 60 µg and 120 µg is administered to the human subject; between about 100 µg and 400 µg, between about 400 µg and 1 mg is administered to the human subject; in some variations, an amount of a therapeutic agent equivalent to an amount of rapamycin of between about 400 µg and 1 mg is administered to the human subject; in some variations, an amount of a therapeutic agent equivalent to an amount of rapamycin of between about 1 mg and 5 mg is administered to the human subject; in some variations, an amount of a therapeutic agent equivalent to an amount of rapamycin of between about 3 mg and 7 mg is administered to the human subject; in some variations, an amount of a therapeutic agent equivalent to an amount of rapamycin of between about 5 mg and 10 mg is administered to the human subject to prevent wet AMD.

According to the present description, any one or more of the formulations described herein are administered intravitreally every 3 or more months, every 6 or more months, every 9 or more months, or every 12 or more months, or longer, to treat one or more of choroidal neovascularization, wet AMD, dry AMD, to prevent wet AMD, or to prevent progression of dry AMD to wet AMD. Any one or more of the formulations described herein may be administered subconjunctivally every 3 or more months, every 6 or more months, every 9 or more months, or every 12 or more months, or longer, to treat one or more of choroidal neovascularization, wet AMD, dry AMD, or to prevent wet AMD.

According to the present description, any one or more of the rapamycin formulations described herein are administered intravitreally every 3 or more months, every 6 or more months, every 9 or more months, or every 12 or more months, or longer, to treat one or more of choroidal neovascularization, wet AMD, dry AMD, to prevent wet AMD, or to prevent progression of dry AMD to wet AMD. Any one or more of the rapamycin formulations described herein may be administered subconjunctivally every 3 or more months, every 6 or more months, every 9 or more months, or every 12 or more months, or longer, to treat one or more of choroidal neovascularization, wet AMD, dry AMD, or to prevent wet AMD. The effect of the rapamycin may persist beyond the period during which it is present in the ocular tissues.

Delivery of the therapeutic agents described herein may, for example, be delivered at a dosage range between about 1 ng/day and about 100 µg/day, or at dosages higher or lower than this range, depending on the route and duration of administration. In some examples of liquid formulation or composition used in the methods described herein, the therapeutic agents are delivered at a dosage range of between about 0.1 µg/day and about 10 µg/day. In some examples of liquid formulation or composition used in the methods described herein, the therapeutic agents are delivered at a dosage range of between about 1 µg/day and about 5 µg/day. Dosages of various therapeutic agents for treatment, prevention, inhibition, delay of onset, or cause of regression of various diseases and conditions described herein can be refined by the use of clinical trials.

The liquid formulations, such as solutions, suspensions, emulsions and situ gelling formulations, and compositions described herein may be used for delivery to the eye, for example by ocular or periocular administration, of therapeutically effective amounts of rapamycin for extended periods of time to treat, prevent, inhibit, delay the onset of, or cause regression of CNV, and thus may be used to treat, prevent, inhibit, delay the onset of, or cause regression of wet AMD, or transition of dry AMD to wet AMD. It is believed that by changing certain characteristics of the liquid formulations described herein, including components of the liquid formulations, the location in the eye to which the liquid formulation is delivered, such as subconjunctival or intravitreal placement, the liquid formulations may be used to deliver therapeutically effective amounts of rapamycin to the eye for a variety of extended time periods including delivery of therapeutic amounts for greater than about 1 week, for greater than about 2 weeks, for greater than about 3 weeks, for greater than about 1 month, for greater than about 3 months, for greater than about 6 months, for greater than about 9 months, for greater than about 1 year.

When a therapeutically effective amount of rapamycin is administered to a subject suffering from wet AMD, the rapamycin may treat, inhibit, or cause regression of the wet AMD. Different therapeutically effective amounts may be required for treatment, inhibition or causing regression. A subject suffering from wet AMD may have CNV lesions, and it is believed that administration of a therapeutically effective amount of rapamycin may have a variety of effects, including causing regression of the CNV lesions, stabilizing the CNV lesion, and preventing progression of an active CNV lesion.

When a therapeutically effective amount of rapamycin is administered to a subject suffering from dry AMD, it is believed that the rapamycin may prevent or slow the progression of dry AMD to wet AMD.

### EXAMPLES

Unless the context indicates otherwise, the error bars in the charts show one standard deviation. Where ethanol is used, it is 200 proof ethanol from Gold Shield Distributors, Hayward, CA. Where rapamycin is used, it is from LC laboratories, Woburn, MA, or Chunghwa Chemical Synthesis & Biotech Co., LTD (CCSB), Taipei Hsien, Taiwan, ROC. Where PEG 400 is used, it is from The Dow Chemical Company, New Milford, CT. Some of the graphs use the expression "uL" or "ug" to refer to µL or µg, respectively. Where a volume of 10 µL or less is administered, Hamilton HPLC syringes were used.

### Example 1-45 - Preparation and Administration of Rapamycin-Containing Compositions

Preparation and characterization of exemplary rapamycin-containing compositions, and administration thereof to New Zealand white rabbits is described in US 2006/0258698 A1 and WO 2006/102378A2 of Mudumba et al. (Examples 1-45 and associated figures).

### Example 46 - Preparation and Characterization of a Rapamycin-Containing Solution

About 320 g of ethanol was sparged with N₂ for about 10 minutes, and then about 40 g of sirolimus was added to the ethanol. The mixture was sonicated for about 20 minutes, by the end of which all of the sirolimus had gone into solution to form a sirolimus stock solution. A diluent solvent was prepared by sonicating about 1880 g of PEG 400 for about 60 minutes, and then sparging the solvent with nitrogen for about 10 minutes.

The sirolimus stock solution and the PEG 400 were then rotated at room temperature in a rotary evaporator for about 10 minutes to mix the stock solution with the diluent solvent. After mixing, the solution was sparged with nitrogen for about 10 minutes and blanketed with nitrogen for about 5 minutes. After the solution was sparged and filled with nitrogen, about 240 g of excess ethanol was evaporated from the solution by increasing the solution temperature, maintaining a temperature that did not exceed 40°C for an extended period of time and continuing to rotate the solution for about 2.5 hours.

The resulting solution comprised about 40 g of sirolimus (about 2% by weight), about 80 g of ethanol (about 4% by weight), and about 1880 g of PEG 400 (about 94% by weight). This solution was sparged with nitrogen for about 10 minutes and blanketed with nitrogen for about 5 minutes. The solution was then filtered through a 0.2 micron filter. USP type I glass vials were filled with 0.5 ml each of the filtered solution to leave a head space in each container of about 2 ml. This head space was filled with nitrogen gas and capped. This solution is listed as formulation #340 in Table 1.

### Example 47 - Preparation and Characterization of a Rapamycin-Containing Solution

About 32 g of ethanol was sparged with N₂ for about 10 minutes, and then about 4 g of sirolimus was added to the ethanol. The mixture was sonicated for about 20 minutes, by the end of which all of the sirolimus had gone into solution to form a sirolimus stock solution. A diluent solvent was prepared by sonicating about 92 g of PEG 400 for about 60 minutes, and then sparging the solvent with Nitrogen for about 10 minutes.

The sirolimus stock solution and the PEG 400 were then rotated at about room temperature in a rotary evaporator for about 10 minutes to mix the stock solution with the diluent solvent. After mixing, the solution was sparged with nitrogen for about 10 minutes and blanketed with nitrogen for about 5 minutes. After the solution was sparged and filled with nitrogen, about 28 g of excess ethanol was evaporated from the solution by increasing the solution temperature, maintaining a temperature that did not exceed 40°C for an extended period of time and continuing to rotate the solution for about 2.5 hours.

The resulting solution comprised about 4 g of sirolimus (about 4% by weight), about 4 g of ethanol (about 4% by weight), and about 92 g of PEG 400 (about 92% by weight). This solution was sparged with nitrogen for about 10 minutes and blanketed with nitrogen for about 5 minutes. The solution was then filtered through a 0.2 micron filter. USP type I glass vials were filled with 0.5 ml each of the filtered solution to leave a head space in each container of about 2 ml. This head space was filled with nitrogen gas and capped. This solution is listed as formulation #326 in Table 1.

### Example 48 - Preparation and Characterization of a Rapamycin-Containing Solution

About 200 µL of normal saline (sodium chloride, 0.9%) was added to 0.5 mL of the solution of Example 46 (previously filled in 2 mL USP type I glass vials) using a 0.5 cc insulin syringe, and mixed by gently swirling. The resulting mixture is a clear solution and has an approximate composition of 1.47% (w/w) of sirolimus, 2.93% (w/w) of ethanol, 26.6% (w/w) of normal saline, and 69% (w/w) of PEG 400. This solution is listed as formulation #327 in Table 1.

### Example 49 - Preparation and Characterization of a Rapamycin-Containing Solution

About 250 µL of normal saline (sodium chloride, 0.9%) was added to 0.5 mL of the solution of Example 46 (previously filled in 2 mL USP type I glass vials) using a 0.5 cc insulin syringe, and mixed by gently swirling. The resulting mixture is a dispersed form and has an approximate composition of 1.38% (w/w) of sirolimus, 2.75% (w/w) of ethanol, 31.25% (w/w) of normal saline, and 64.62% (w/w) of PEG 400. This solution is listed as formulation #328 in Table 1.

### Example 50 - Subconjunctival Injection of a Rapamycin-Containing Solution

Either 40 al of the solution described in Example 46, Example 48, or Example 49, or 20 µL of the solution described in Example 47 was injected between the sclera and the conjunctiva of the eye of New Zealand White rabbits. **Fig. 1** depicts the average concentration of rapamycin present in the retina choroid (ng/g) on a logarithmic scale at 14, 30, 60, and 90 days after injection.

The analysis was by liquid chromatography mass spectroscopy (LCMS) using an internal standard. At each time point, the average concentration of rapamycin was calculated by adding the concentrations of rapamycin obtained for each eye from each rabbit, and dividing the total by the number of eyes analyzed. In this experiment, each time point represents the average of either two eyes of each of two rabbits (four eyes at that time point).

The full retina choroid was homogenized and analyzed. The average concentration of the retina choroid was calculated by dividing the mass of rapamycin measured by the mass of retina choroid analyzed. The sample did not include the site of administration; thus, this measurement indicated the level of rapamycin delivered to the retina choroid via test article (in this case, the solutions of Examples 46, 47, 48, and 49).

The average level of rapamycin in the retina choroid at 14, 30, 60, and 90 days after subconjunctival injection of the solution of Example 46 was about 56.94, 9.79, 2.26, and 0.00 ng/g, respectively. The average level of rapamycin in the retina choroid at 14, 30, 60, and 90 days after subconjunctival injection of the solution of Example 47 was about 90.19, 6.0, 5.72, and 0.98 ng/g, respectively. The average level of rapamycin in the retina choroid at 14, 30, 60, and 90 days after subconjunctival injection of the solution of Example 48 was about 103.86, 20.54, 6.07, and 12.76 ng/g, respectively. The average level of rapamycin in the retina choroid at 14, 30, 60, and 90 days after subconjunctival injection of the solution of Example 49 was about 70.12, 27.83, 3.99, and 5.94 ng/g, respectively.

### Example 51 - Intravitreal Injection of a Rapamycin-Containing Solution

6 µl of the solution of Example 46 or 8.5 µL of the solution of Example 48 was injected into the vitreous of the eye of New Zealand White rabbits using a 10 µL Hamilton syringe. Fig. 2 depicts the level of rapamycin present in the retina choroid (ng/g) on a logarithmic scale at 3, 14, 30, 45 and 60 days after injection.

Full vitreous of each eye was homogenized and analyzed by liquid chromatography tandem mass spectrometry (LC/MS/MS). The average level of rapamycin in the vitreous at 3, 14, 30 and 60 days after intravitreal injection of the solution of Example 46 was about 63,064.3, 6,095.56, 58.21, and 0.15 ng/ml, respectively. The average level of rapamycin in the vitreous at 3, 14, 30, 45 and 60 days after intravitreal injection of the solution of Example 48 was about 58,930.4, 18,573.1, 6,793.21, 799.18, and 4116.17 ng/ml, respectively.

The full retina choroid was homogenized and analyzed. The average concentration of the retina choroid was calculated by dividing the mass of rapamycin measured by the mass of retina choroid analyzed. The sample did not include the site of administration; thus, this measurement indicated the level of rapamycin delivered to the retina choroid via test article (in this case, the solution of Example 46 or 48). The average level of rapamycin in the retina choroid at 3, 14, 30, and 60 days after intravitreal injection of the solution of Example 46 was about 1971.84, 478.02, 57.70, and 1.90 ng/g, respectively. The average level of rapamycin in the retina choroid at 3, 14, 30, 45 and 60 days after intravitreal injection of the solution of Example 48 was about 2869.05, 3633.64, 43.38, 37.09 and 27.36 ng/g, respectively.

### Example 52 - Intravitreal and Subtenon Injection of a Rapamycin-Containing Solution

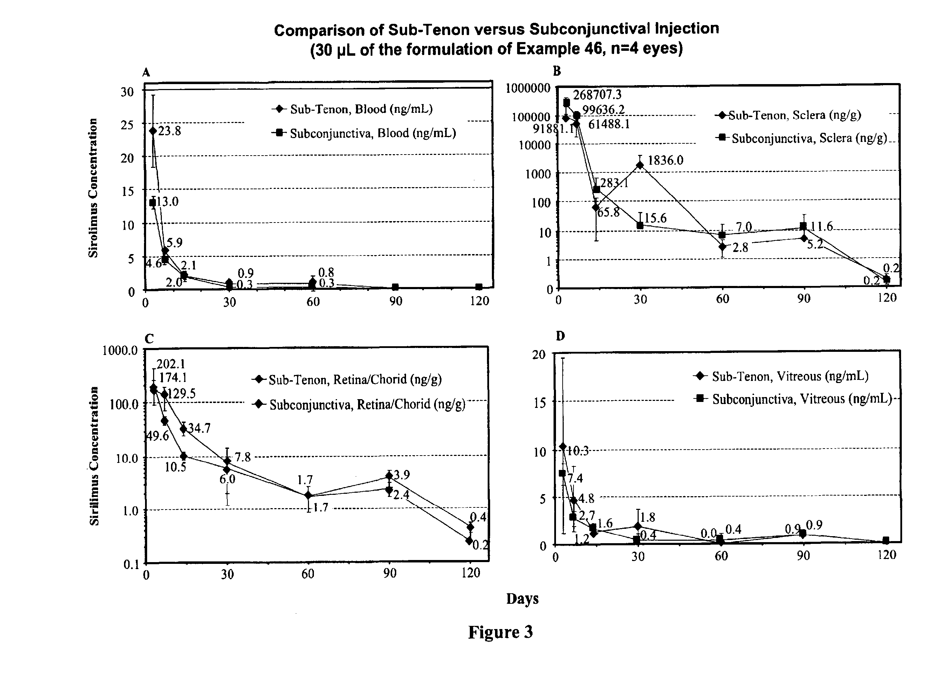

30 gl of the solution of Example 46 was injected between the sclera and the conjunctiva of the eye of New Zealand White rabbits. **Fig. 3** depicts the average concentration of rapamycin present in the retina choroid (ng/g) and whole blood (ng/mL) on a logarithmic scale at 3, 7, 14, 30, 60 and 90 days after injection.

The full retina choroid was processed and analyzed as described earlier. The average level of rapamycin in the retina choroid at 3, 7, 14, 30, 60 and 90 days after subconjunctival injection of the solution of Example 46 was about 174.1, 129.5, 34.7, 7.8, 1.7, and 1.7 ng/g, respectively. The whole blood samples are precipitated by addition of known amount organic solvents, homogenized, centrifuged, and supernatant was collected to analyze rapamycin levels using LC/MS/MS. The average level of rapamycin in the blood at 3, 7, 14, 30 and 60 days after subconjunctival injection of the solution of Example 46 was about 13.0, 4.6, 2.0, 0.3, and 0.3 ng/mL, respectively.

For the 30 uL subtenon injections, a guide hole was made by inserting and removing a 20 gauge x ½ inch sharp BD needle though the conjunctiva in the dorsotemporal quadrant 3 mm posterior to the limbus and temporal to the dorsal rectus muscle attachment. A 24 G fukasaku anesthesia cannula attached to a sterile 0.5 cc BD syringe was then guided through the guide hole posteriorly into the subtenon space and the solution of Example 46 was injected slowly on the posterior surface of the sclera near the optic nerve. **Fig. 3** depicts the average concentration of rapamycin present in the retina choroid (ng/g) and whole blood (ng/mL) on a logarithmic scale at 3, 7, 14, 30, 60 and 90 days after injection.

The full retina choroid was processed and analyzed as described earlier. The average level of rapamycin in the retina choroid at 3, 7, 14, 30, 60 and 90 days after subtenon injection of the solution of Example 46 was about 202.1, 49.6, 10.5, 6.0, 1.7, and 3.9 ng/g, respectively. The whole blood samples are precipitated by addition of known amount organic solvents, homogenized, centrifuged, and supernatant was collected to analyze rapamycin levels using LC/MS/MS. The average level of rapamycin in the blood at 3, 7, 14, 30 and 60 days after subtenon injection of the solution of Example 46 was about 23.8, 5.9, 2.1, 0.4, and 0.8 ng/mL, respectively.

### Example 53 - Preparation and Characterization of a Rapamycin-Containing Solution

The following solvent preparation allows up to 5% (w/w) of rapamycin. N-methyl pyrrolidone (NMP) and propylene glycol (PG) are mixed in 10.5 : 73.7 ratio, desired amount of sirolimus (up to 5% w/w of final composition) is dissolved in the mix, and subsequently water (up to 15.8% w/w of final composition) is added slowly to obtain the final solution. This solution is listed as formulation #333 in Table 1.

### Example 54 - Preparation and Characterization of a Rapamycin-Containing Solution

The following solvent preparation allows up to 5% (w/w) of rapamycin. N-methyl pyrrolidone (NMP) and propylene glycol (PG) are mixed in 38.9 : 38.9 (1:1) ratio, desired amount of sirolimus (up to 5% w/w of final composition) is dissolved in the mix, and subsequently water (up to 22.2% w/w of final composition) is added slowly to obtain the final solution. This solution is listed as formulation #334 in Table 1.

### Example 55 - Preparation and Characterization of a Rapamycin-Containing Solution

The following solvent preparation allows up to 5% (w/w) of rapamycin. N-methyl pyrrolidone (NMP) and polyethylene glycol 600 (PEG 600) are mixed in 10.5 : 73.7 ratio, desired amount of sirolimus (up to 5% w/w of final composition) is dissolved in the mix, and subsequently water (up to 15.8% w/w of final composition) is added slowly to obtain the final solution. This solution is listed as formulation #335 in Table 1.

### Example 56 - Preparation and Characterization of a Rapamycin-Containing Solution

The following solvent preparation allows up to 5% (w/w) of rapamycin. Dimethyl acetamide (DMA) and propylene glycol (PG) are mixed in 10.5 : 73.7 ratio, desired amount of sirolimus (up to 5% w/w of final composition) is dissolved in the mix, and subsequently water (up to 15.8% w/w of final composition) is added slowly to obtain the final solution. This solution is listed as formulation #336 in Table 1.

### Example 57 - Preparation and Characterization of a Rapamycin-Containing Solution

The following solvent preparation allows up to 5% (w/w) of rapamycin. Dimethyl sulfoxide (DMSO) and propylene glycol (PG) are mixed in 10.5 : 73.7 ratio, desired amount of sirolimus (up to 5% w/w of final composition) is dissolved in the mix, and subsequently water (up to 15.8% w/w of final composition) is added slowly to obtain the final solution. This solution is listed as formulation #337 in Table 1.

### Example 58 - Preparation and Characterization of a Rapamycin-Containing Solution

N-methyl pyrrolidone (NMP), polyethylene glycol 400 (PEG 400) and ethanol are mixed in 27.9 : 28.4 : 4.7 ratio (respectively), desired amount of sirolimus (up to 5% w/w of final composition) is dissolved in the mix, and subsequently water (up to 28.4% w/w of final composition) is added slowly to obtain the final solution. This solution is listed as formulation #338 in Table 1.

### Example 59 - Preparation and Characterization of a Rapamycin-Containing Solution

8.35% of Cremophor (percentage of total weight) is mixed with 8.37% of Capmul MCM (percentage of total weight). 0.07% rapamycin (percentage of the total weight) was dissolved in the Cremophor-Capmul MCM solution using an ultrasound bath. 83.21 % (percentage of total weight) of sterile water is added to the former solution and the resulting solution is vortexed creating a milky microemulsion. The solution was placed at 2 - 8°C until use. This solution is listed as formulation #329 in Table 1.

### Example 60 - Preparation and Characterization of a Rapamycin-Containing Solution

8.15% of Capmul MCM (percentage of total weight) is mixed with 8.12% of Tyloxapol (percentage of total weight). 0.09% rapamycin (percentage of the total weight) was dissolved in the Tyloxapol-Capmul MCM solution in which 0.35% of Ethanol was added. The final dissolution is completed by using an ultrasound bath. 83.29% (percentage of total weight) of sterile water is added to the former solution and the resulting solution is vortexed creating a milky microemulsion. The solution was placed at 2 - 8°C until use. This solution is listed as formulation #330 in Table 1.

### Example 61 - Preparation and Characterization of a Rapamycin-Containing Solution

6.81% of Tyloxapol (percentage of total weight) is mixed with 6.81% of Capmul MCM (percentage of total weight) and 3.12% of PHOSAL® 50PG (percentage of total weight). 0.08% rapamycin (percentage of the total weight) was dissolved in the Tyloxapol-Capmul MCM-PHOSAL® 50PG solution using an ultrasound bath. 83.21% (percentage of total weight) of sterile water is added to the former solution and the resulting solution is vortexed creating a milky microemulsion. The solution was placed at 2 - 8°C until use. This solution is listed as formulation #331 in Table 1.

### Example 62 - Preparation and Characterization of a Rapamycin-Containing Solution

7.18% of Cremophor (percentage of total weight) is mixed with 9.47% of Capmul MCM (percentage of total weight). 0.17% rapamycin (percentage of the total weight) was dissolved in the Cremophor-Capmul MCM solution in which 0.35% of Ethanol was added. 83.18% (percentage of total weight) of sterile water was added to the former solution and the resulting solution is vortexed creating a milky microemulsion. The solution was placed at 2 - 8°C until use. This solution is listed as formulation #332 in Table 1.

### Example 63 - Topical Administration of Rapamycin-Containing Formulations

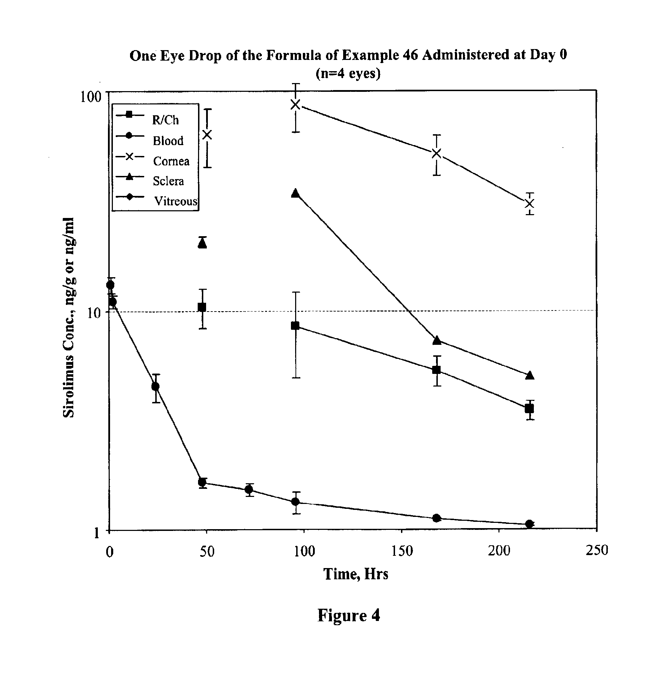

Both eyes (or one eye depending on the study) of each animal received one or two drops (40 gl per drop) of the studied formulations using a calibrated pipette on day 0 (day of dosing). Results are shown in Tables 3-6 and **Figs. 4-9****.**

For Tables 3-6, the solution of Example 46 (formulation #340) was administered two drops twice a day on both eyes, one time for Group I, two drops once a day on one eye, one time for Group II, and two drops once a day on both eyes for 6 days in Group III. In Group II, the right eye (OD) but not the left eye (OS) received eye drops. In a further study, one drop of the formulation #340 (40 µL containing 880 µg rapamycin) was administered on day zero. As shown in **Fig. 4****,** after administration of a single eye drop, the rapamycin concentration in the cornea remained above 0.01 ng/g nine days (e.g., 216 hrs) after administration, while the rapamycin concentration in retina choroid remained above 0.001 ng/g nine days (e.g., 216 hrs) after administration. In fact after administration of a single eye drop, the rapamycin concentration in the cornea remained above 10.0 ng/g (above 0.01 ng/mg) nine days (e.g., 216 hrs) after administration, while the rapamycin concentration in retina choroid remained above 1.0 ng/g (above 0.001 ng/mg) nine days (e.g., 216 hrs) after administration.

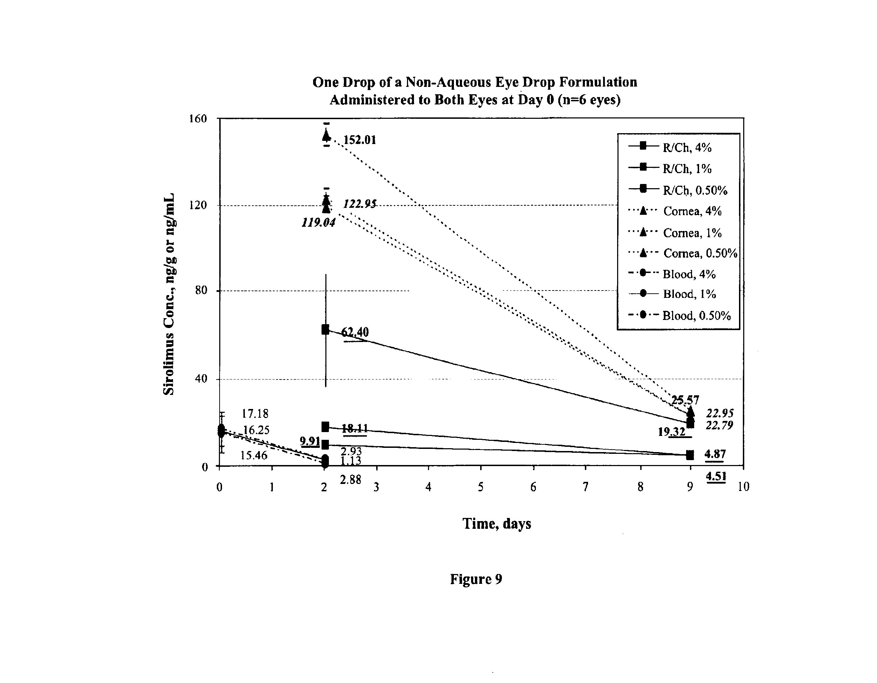

Moreover as shown in **Fig. 9** in a further study, following administration of a single non-aqueous eye drop formulation containing 4%, 1% or 0.5% rapamycin, sustained levels of rapamycin were detected in the cornea and retina choroid. In particular, after administration of a single eye drop, the rapamycin concentration in the cornea remained above 0.01 ng/g (above 0.01 ng/mg) for at least nine days after administration, while the rapamycin concentration in retina choroid remained above 0.001 ng/g (above 0.001 ng/mg) for at least 9 days after administration. In contrast, the rapamycin concentration in the blood was substantially lower than that observed in the cornea and retina choroid indicating that the non-aqueous eye drop formulations did not result in significant systemic exposure to rapamycin.

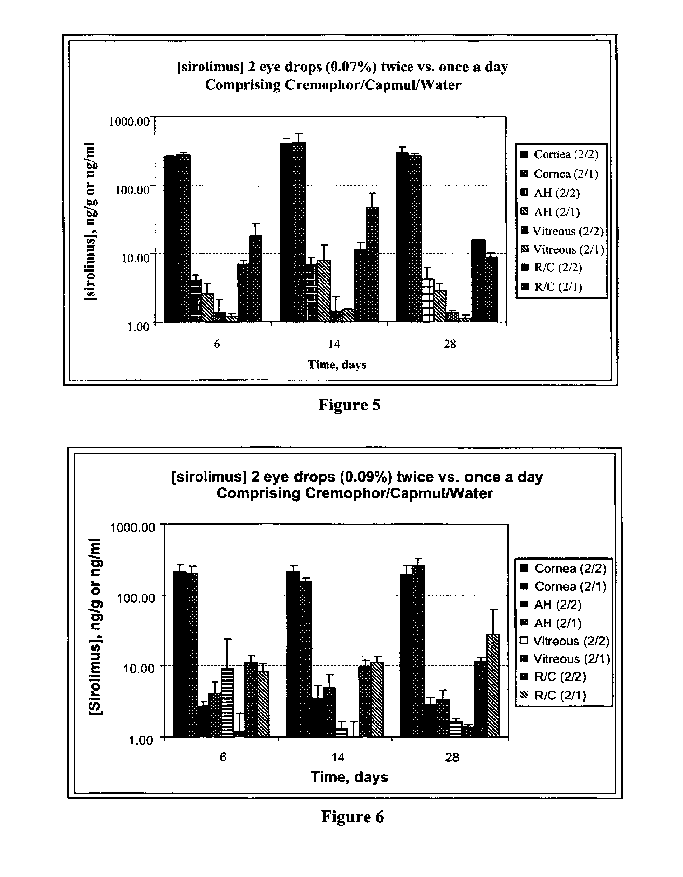

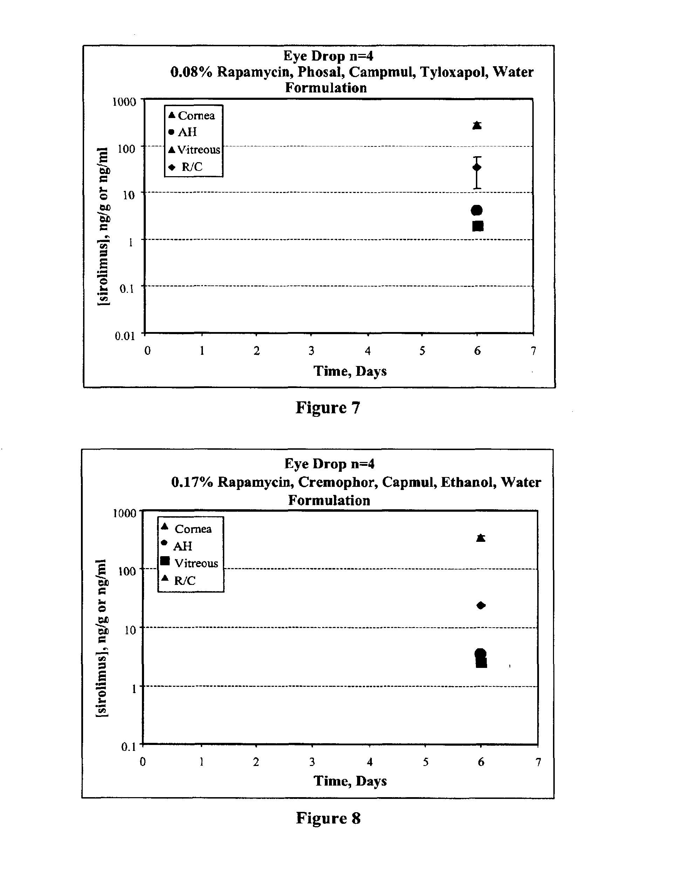

For **Figs. 5 and 6****,** the aqueous rapamycin-containing formulations #329 and #330 of Table 1, were administered two drops once or twice a day for up to 28 days. For **Figs. 7 and 8****,** the aqueous rapamycin-containing formulations #331 and #332 of Table 1 were administered two drops twice a day for 6 days. In short, the aqueous rapamycin-containing formulations tested achieved an average concentration of rapamycin in the cornea of at least 0.01 ng/g (greater than 0.01 ng/mg to 0.1 ng/mg in **Figs. 5-8**), in the retina choroid of at least 0.01 ng/g (greater than 0.001 ng/mg to 0.01 ng/mg in some measurements of **Figs. 5-8**), in the aqueous humor of at least 0.1 ng/mL (greater than 1.0 ng/mL), and in the vitreous humor of at least 0.1 ng/mL (greater than 1.0 ng/mL).

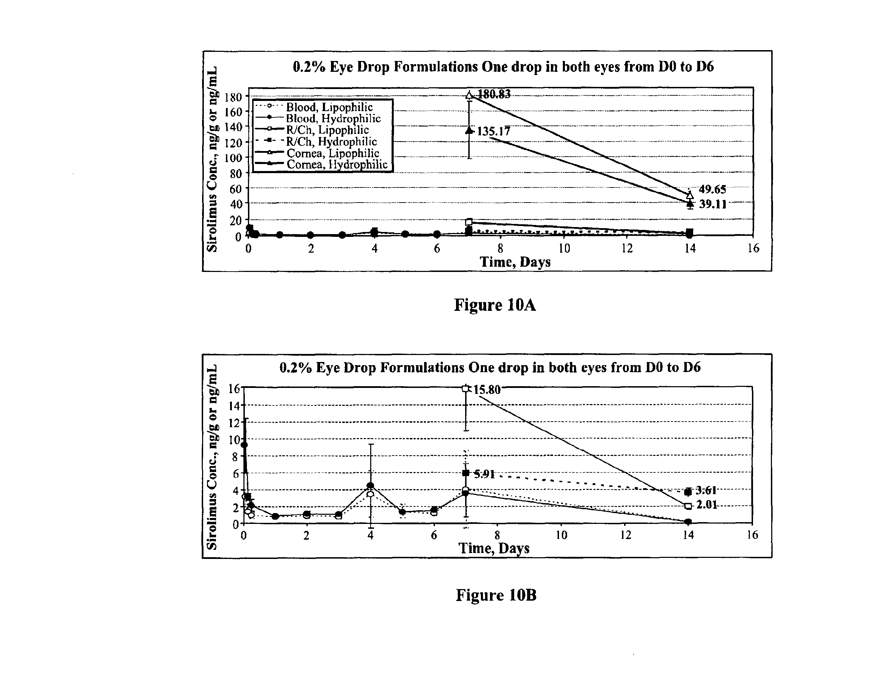

For **Fig. 10A and 10B** one drop of an aqueous or a non-aqueous formulation containing about 0.2% rapamycin were administered to both eyes of rabbit subjects once a day for six days (day 0 to day 6). As used herein, the terms "aqueous" and "hydrophilic" refer to formulations in which the dominant component is an aqueous compound such as water (e.g., formulations #342 and #342 of Table 1), while the terms "non-aqueous" and "lipophilic" refer to formulation in which the dominant component is a non-aqueous compound such as PEG 400 (e.g., formulations #344 and #345 of Table 1). Rapamycin concentrations in the cornea, retina choroid and blood were measured at day 7 and day 14. Both formulations tested achieved an average concentration of rapamycin in the cornea of at least 0.01 ng/g (at least 10 ng/g) on days 7 and 14, and in the retina choroid of at least 0.01 ng/g (at least 1 ng/g) on days 7 and 14.

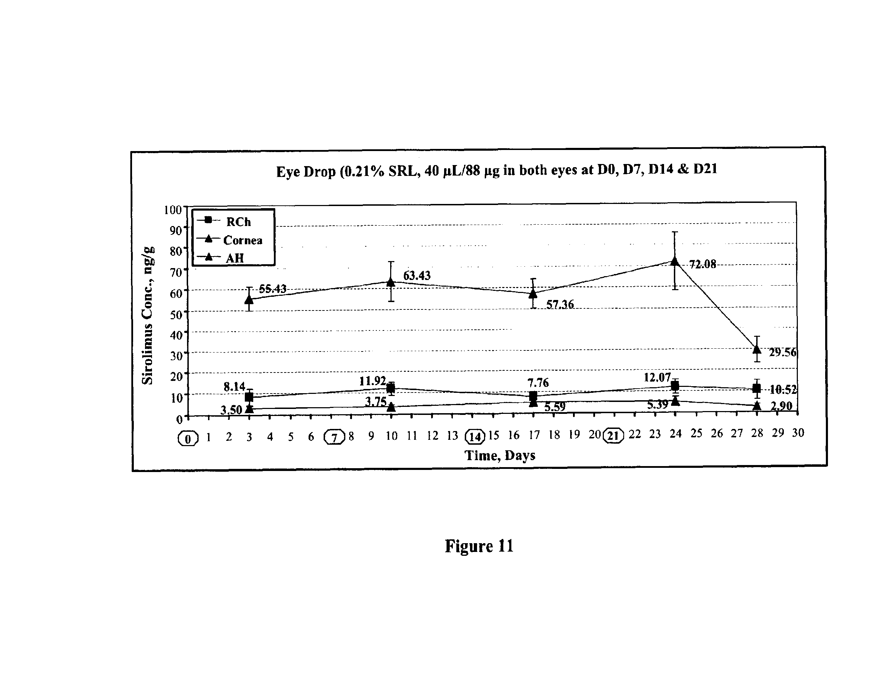

For **Fig. 11** one drop of a non-aqueous formulation containing about 0.2% rapamycin (e.g., formulations #344 or #345) was administered to both eyes of rabbit subjects once a day on day 0, 7, 14 and 21. Rapamycin concentrations in the cornea, retina choroid and aqueous humor were measured on days 3, 10, 17, 24 and 28. Infrequent administration of the non-aqueous eye drops achieved sustained therapeutic levels of rapamycin. In particular a sustained rapamycin concentration was achieved in the cornea of at least 0.01 ng/g (above 10 ng/g) and in the retina choroid and aqueous humor of at least 0.01 ng/mL (above 1 ng/mL).

### Example 64 - Preparation and Intravitreal Administration of a Rapamycin-Containing Formulation

An exemplary rapamycin containing formulation was prepared by adding 0.2003 g rapamycin to 5.9989 g NMP. After mixing, 7.8030 g PG is added, followed by 6.010 g sterile water. This recipe yielded a formulation having final concentrations as a percentage of the total weight of approximately: rapamycin 1.0%, NMP 30.0%, PG 39.0% and sterile water 30.0%. This formulation is listed as #346 in Table 1.

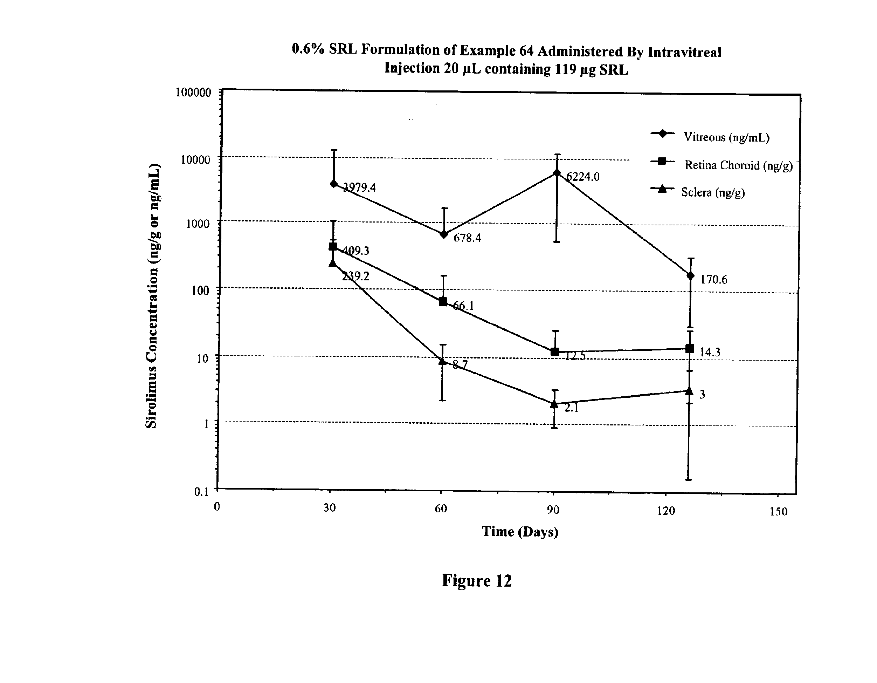

The formulation of this example, however, was not stable. As such at the time of the in vivo study (ten days after this preparation was made) the rapamycin potency was determined to be 0.6%. Twenty µl of the 0.6% rapamycin-containing formulation was injected into the vitreous of the eye of New Zealand White rabbits. Rapamycin concentrations in vitreous, retina choroid and sclera were determined as described in Example 51. **Fig. 12** depicts the level of rapamycin present in the vitreous, retina choroid and sclera 30 days or more (e.g., 30, 60, 90 and 120 days or more) after injection.

### Example 65 - Preparation and Subconjunctival Administration of Rapamycin Formulations

An exemplary 2% rapamycin suspension was prepared by dispersing 106.9 mg of rapamycin (2.07% by weight) in 5056.3 mg of 1% carboxymethylcellulose (CMC) low viscosity (97.93% by weight) aqueous solution. Briefly, 106.9 mg of rapamycin and 5056.3 mg of 1% carboxymethylcellulose low viscosity were placed in an amber vial. High wear resistant Ziconia grinding media (beads) of 3 mm diameter were added, up to ¾ of the total volume. The vial was sealed and placed in a Cole-Parmer milling apparatus for at least 24 hrs. The median and mean particle sizes for rapamycin were 3.2957 µm and 3.5487 µm, respectively. The formulation was kept at 4°C until use. Final concentrations as a percentage of the total weight were approximately: rapamycin: 2.07%, CMC 1%, and sterile water 97.93%. This formulation is listed as #347 in Table 1.

Another exemplary 2% rapamycin in situ gelling formulation was prepared by dissolving 459.9 mg of PLGA (75/25) in 1678.0 mg of NMP overnight. Then 44.8 mg of rapamycin is added until complete dissolution. The formulation was kept at 4°C until use. Final concentrations as a percentage of the total weight were approximately: rapamycin 2.05%, PLGA 75/25 21.07%, and NMP 76.88%. This formulation is listed as #348 in Table 1.

A further exemplary 2% rapamycin formulation was prepared by dissolving rapamycin in NMP. Then PEG 400 is added under continuous agitation, followed by sterile water also under continuous agitation. The formulation was kept at 4°C until use. Final concentrations as a percentage of the total weight were approximately: rapamycin 2.02%, NMP 44.47%, PEG 400 29.04%, and sterile water 24.47%. This formulation is listed as #349 in Table 1.

Thirty µl of the 2% rapamycin-containing formulations are injected between the sclera and the conjunctiva of the eye of New Zealand White rabbits. Rapamycin concentrations in vitreous, retina/choroid and sclera are determined as described in Example 50, at days 30, 60 and 90 days post-administration.

### Example 66 - Preparation and Topical Administration of a Raparnycin-Containing SDF

An exemplary rapamycin-containing solid dosage form (SDF) was prepared using a solvent cast technique. Briefly, the following components were mixed together: 10.0% rapamycin, 57.9% KOLLIDON 90F (PVP 90), 20.0% phosal 50 SD, 10.1% PEG 400, 1.0% vitamin E (gamma tocopherol), and 1.0% ascorbyl palmitate (e.g., equivalent to the formulation #351 in Table 1). A volume of ethanol was added sufficient to obtain 30% total solids (e.g., 70% ethanol) as a final percentage. The mixture was then poured onto a release liner (polyester film with a silicone release coating, and spread to a thickness of 50 mm using a gardner knife. The ethanol was evaporated off in a dark, nitrogen rich environment (i.e., glove box) for about 24 hrs. After drying, the concentration of rapamycin in the SDF was determined by liquid chromatography to be 9.1 ± 0.1 % (e.g., 0.9% rapamycin lost). The dried formulation was punched to the desired diameter. The SDF of this example had a diameter of 4 mm, with a thickness of 0.15-0.20 mm, yielding a rapamycin dosage of about z 21 jug.

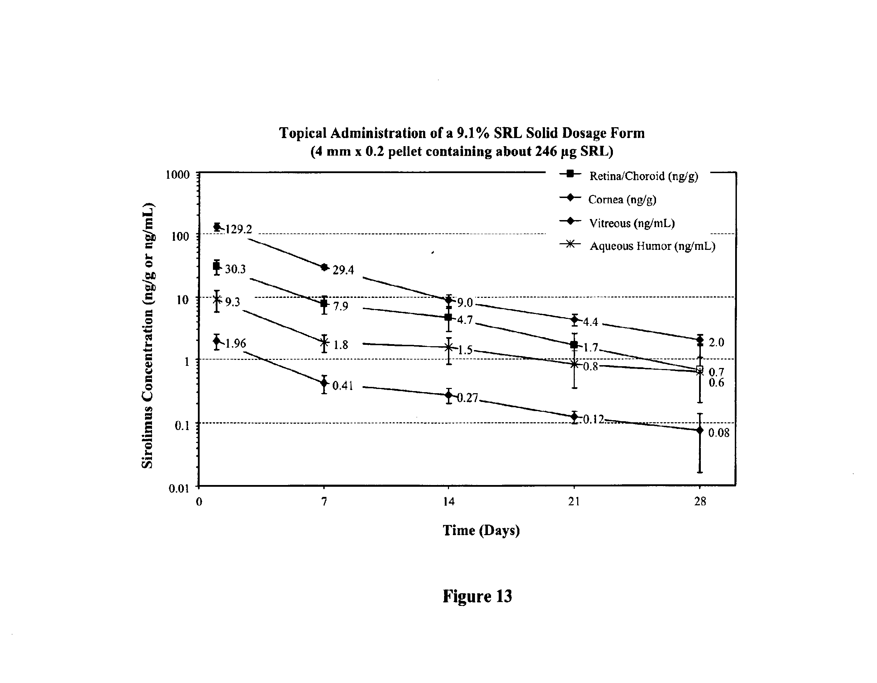

The SDF was administered topically to the eyes of New Zealand White rabbits by placement in the inferior cul-de-sac. Briefly, the lower lid of the eye was pulled out, permitting the placement at the bottom of the cul-de-sac on the inferior side (not sclera side) with a pair of tweezers. Rapamycin concentrations in cornea, retina choroid, vitreous humor, aqueous humor and blood were determined. **Fig. 13** depicts the levels of rapamycin in various tissues on days l, 7, 14, 21 and 28 post-administration.

### Example 67 - Preparation of a Dexamethasone-Containing SDF (illustration only)

An exemplary dexamethasone-containing solid dosage form (SDF) is prepared using a solvent cast technique. The SDF is prepared with concentrations as a percentage of the total weight of approximately: 25.3% dexamethasone, 33.2% hydroxypropyl cellulose (HPC), 32.0% eudragit RS 30D, and 9.5% xanthan gum. The SDF of less than or equal to 3 mm in diameter is inserted subconjunctivally, for controlled release in the retina / choroid for up to six months (1 to 100 ng/g dexamethasone). This formulation is listed as #352 in Table 1.

### Example 68 - Preparation and Invitreal Administration of a Dasatanib Formulation (illustration only)

An exemplary dasatanib-containing solution was prepared by dissolving dasatanib in DMA. PEG 400 was then added under continuous agitation. Final concentrations as a percentage of the total weight were approximately: dasatanib 4.8%, dimethylacetamide 16.0%, and PEG 400 79.2% (e.g., equivalent to the formulation #339 in Table 1).

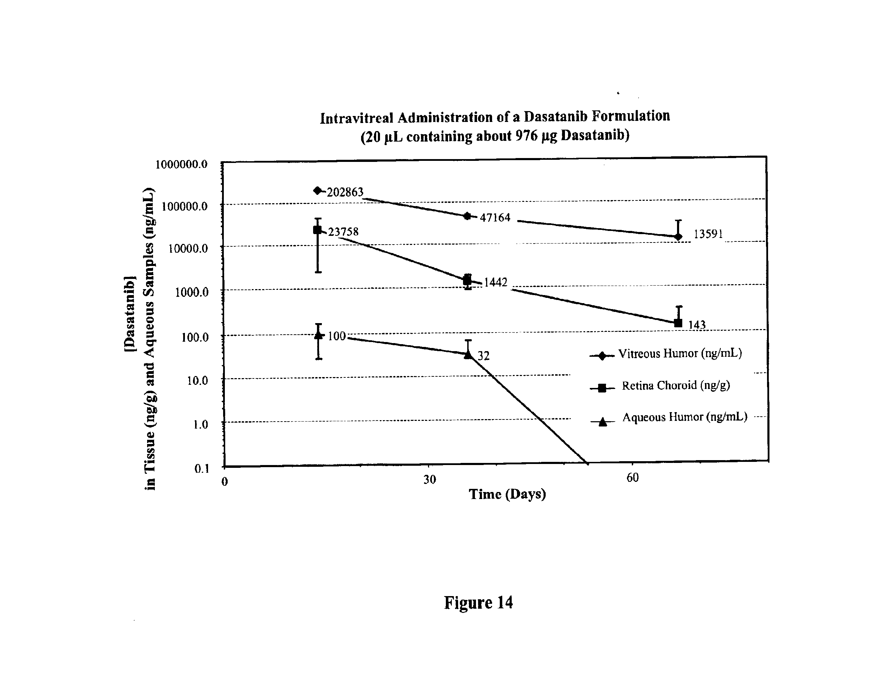

Twenty µl of the 4.84% dasatanib-containing formulation was injected into the vitreous of the eye of New Zealand White rabbits. Dasatanib concentrations in the vitreous and retina choroid were determined. **Fig. 14** depicts the level of dasatanib present in the vitreous and retina choroid about 2 weeks, 5 weeks and 10 weeks after injection.

**Table 1 Formulations**

| **Form .#** | **Composition (mg), % (w/w)** | **Type** | **Median particle size** | **NDM, Injection volume** |
|---|---|---|---|---|
| 1 | DMSO = 2000mg (20%) | S | | |
| | Water = 8000mg (80%) | | | |
| 2 | F68 = 1000mg (10%) | S | | |
| | Water = 9000mg (90%) | | | |
| 3 | F68 = 3000mg (30%) | S | | |
| | Water = 7000mg (70%) | | | |
| 4 | F127 = 1000mg (10%) | S | | |
| | Water = 9000mg (90%) | | | |
| 5 | F127 = 1500mg (15%) | S | | |
| | Water = 8500mg (85%) | | | |
| 6 | Beta-cyclodextrin = 250mg (2.5%) | S | | |
| | Water = 9750mg (97.5%) | | | |
| 7 | Rapa = 10.2mg (0.101%) | S | | No, 50 µL |
| | Pluronic, F68 = 1010mg (9.99%) | | | |
| | Water = 9090mq (89.909%) | | | |
| 8 | Rapa = 10.2mg (0.102%) | S | | No, 50 µL |
| | Pluronic, F68 = 3000mg (29.969%) | | | |
| | Water = 7000mg (69.929%) | | | |
| 9 | Rapa = 10.5mg (0.104%) | S | | No, 50 µL |
| | Pluronic, F127 = 1010mg (9.99%) | | | |
| | Water = 9090mg (89.907%) | | | |
| 10 | Rapa = 10.5mg (0.105%) | S | | No, 50 µL |
| | Pluronic, F127 = 1500mg (14.984%) | | | |
| | Water = 8925mg (84.9%) | | | |
| 11 | Rapa = 10.7mg (0.105%) | S | | No, 50 µL |
| | Beta-cyclodextrin = 255mg (2.497%) | | | |
| | Water = 9945mg (97.398%) | | | |
| 12 | Rapa = 6.4mg (0.0999%) | SP | | |
| | CMC = 48mg (0.7493%) | | | |
| | Polysorbitan 20 = 2.56mg (0.04%) | | | |
| | Water = 6349.44mg (99.111 %) | | | |
| 13 | Rapa = 6.5mg (0.0999%) | S | | |
| | DMSO = 325mg (4.995%) | | | |
| | Water = 6175mg (94.905%) | | | |
| 14 | Rapa = 13.5mg (0.0999%) | SP | | |
| | CMC = 101.25mg (0.7493%) | | | |
| | Polysorbitan 20 = 5.4mg (0.04%) | | | |
| | Water = 13393.35mg (99.112%) | | | |
| 15 | Rapa = 11.0mg (0.2%) | S | | |
| | EtOH = 5500mg (99.8%) | | | |
| 16 | Rapa = 6.6mg (0.1%) | S | | |
| | EtOH = 1054.6mg (15.933%) | | | |
| | F127 = 833.64mg (12.595%) | | | |
| | Water = 4723.96mg (71.372%) | | | |
| 17 | Rapa = 5 mg (0.1%) | S | | |
| | Cavitron = 0.25 g (5%) | | | |
| | Ethanol, 95% = 57 mg (1.1 %) | | | |
| | Sterile water = 4.753 8 (93.8%) | | | |
| 18 | Rapa = 5 mg (0.1%) | S | | |
| | Ethanol, 95% = 150 mg (2.9%) | | | |
| | PEG400 = 1.0 g (19.4%) | | | |
| | Sterile water = 4.01 g (77.6%) | | | |
| 19 | Rapa = 5 mg (0.1%) | S | | Yes, 50 µL |
| | Ethanol, 95% =152 mg (3.2%) | | | |
| | PEG400 = 1.5227 g (30.2%) | | | |
| | Sterile water = 3.3592 g (66.67%) | | | |
| 20 | Rapa = 6.6mg (0.1%) | S | | |
| | EtOH = 505.1 mg (7.618%) | | | |
| | F127 = 917.8mg (13.843%) | | | |
| | Water = 5200.6mg (78.44%) | | | |
| 21 | Rapa = 6.6mg (0.1%) | S | | No, 50 µL |
| | EtOH = 536mg (7.5%) | | | |
| | Pluronic, F127 = 983.75mg (14.0%) | | | |
| | Water = 5574.56mg (78.4%) | | | |
| 22 | Rapa = 5.2mg (0.1023%) | S | | |
| | EtOH = 56.6mg (1.127%) | | | |
| | Captisol = 2008.9mg (39.5%) | | | |
| | Water = 3013.3mg (59.3%) | | | |
| 23 | Rapa = 6.9mg (0.201%) | S | | |
| | EtOH = 3418.0mg (99.799%) | | | |
| 24 | Rapa = 9.1 mg (0.491%) | S | | |
| | EtOH = 90.9mg (4.908%) | | | |
| | F127 = 262.8mg (14.191%) | | | |
| | Water = 1489.1 mg (80.409%) | | | |
| 25 | Rapa = 0mg (0%) . | S | | |
| | EtOH = 310.2mg (5.144%) | | | |
| | F127 = 858.1mg (14.228%) | | | |
| | Water = 4862.6mg (80.628%) | | | |
| 26 | Rapa = 0mg (0%) | S | | |
| | EtOH = 613.1mg (10.19%) | | | |
| | F127 = 810.6mg (13.471 %) | | | |
| | Water = 4593.6mg (76.339%) | | | |
| 27 | Rapa = 53.5mg (1.095%) | S | | Yes, 50 µL |
| | EtOH = 414.8mg (8.488%) | | | |
| | F127 = 662.8mg (13.563%) | | | |
| | Water = 3755.7mg (76.854%) | | | |
| 28 | Rapa = 0.3 g (10%) | ISG, SP | | |
| | PVP K90 = 0.35 g (12%) | | | |
| | Eudragit RS30D = 2.35 g (78%) | | | |
| 29 | Rapa = 0.2154 g (7.31%) | ISG, SP | | |
| | PVP K90 = 0.25 g (8.5%) | | | |
| | Eudraqit RS30D = 2.48 g (84.19%) | | | |
| 30 | Rapa = 53.9mg (1.103%) | S | | No, 50 µL |
| | EtOH = 413.6mg (8.463%) | | | |
| | Sterile water = 3843.5mg (78.647%) | | | |
| | F127 (Lutrol) = 576.0mg (11.786%) | | | |
| 31 | Rapa = 0mg (0%) | S | | |
| | EtOH = 411.9mg (8.513%) | | | |
| | Sterile Water = 3849.3mg (79.554%) | | | |
| | F127(Lutrol) = 577.4mg (11.933%) | | | |
| 32 | Rapa = 54.1mg (1.256%) | S | | |
| | EtOH = 416.8mg (9.676%) | | | |
| | Sterile Water = 3836.3mg (78.569%) | | | |
| | F127(Lutrol) = 577.5mg (10.499%) | | | |
| 33 | Rapa = 80.7g (1.964%) | S | | |
| | EtOH = 65.0mg (0.158%) | | | |
| | PEG400 = 4021.8mg (97.878%) | | | |
| 34 | Rapa = 106.9g (5.233%) | S | | Yes, 25 µL |
| | EtOH = 129.6mg (6.344%) | | | |
| | PEG400 = 1806.5mg (88.424%) | | | |
| 35 | Rapa = 0 mg (0%) | ISG, SP | | |
| | PVP K90 = 0.204 g (2.3%) | | | |
| | Ethanol, 100% = 0.4 g (4.5%) | | | |
| | Eudragit RL100 =0.201g (2.3%) | | | |
| | PEG 400 = 8.00 g (90.9%) | | | |
| 36 | Rapa = 0 mg (0%) | ISG, SP | | |
| | PVP K90 = 0.2 g (2.2%) | | | |
| | Ethanol, 100% = 0.4 g (4.4%) | | | |
| | PVAP = 0.4 g (4.4%) | | | |
| | PEG 400 = 8.00 g (88.9%) | | | |
| 37 | Rapa = 106.1 mg (4.2%) | ISG, SP | | |
| | PVP K90 = 55.2 mg (2.2%) | | | |
| | Ethanol, 100% = 108 mg (4.3%) | | | |
| | Eudragit RL100 = 55 mg (2.2%) | | | |
| | PEG 400 = 2.2 g (87.1%) | | | |
| 38 | Rapa = 399.6mg (9.965%) | S | | Yes, 20 µL |
| | F68(Lutrol) = 40.6mg (1.012%) | | | |
| | Sterile Water = 3569.7mg (89.022%) | | | |
| 39 | Rapa = 53.8mg (1.1%) | S | | |
| | EtOH = 415.2mg (8.489%) | | | |
| | Sterile Water = 3844.2mg (78.594%) | | | |
| | F127 = 578.0mg (11.817%) | | | |
| 40 | Rapa = 208.1 mg (3.148%) | S | | Yes, 20 µL |
| | PEG400 = 6403.4mg (96.852%) | | | |
| 41 | Rapa = 200.4mg (5.148%) | SP | | |
| | F68(Lutrol) = 20.8mg (0.534%) | | | |
| | PEG400 = 3569.3mg (91.697%) | | | |
| | EtOH (95%) = 102mg (2.62%) | | | |
| 42 | Rapa = 200.4g (5.259%) | SP | | |
| | PEG400 = 3561.4mg (93.46%) | | | |
| | Tween 80 = 48.8mg (1.281%) | | | |
| 43 | Rapa = 30.9 mg (1.03%) | S | | No, 50 µL |
| | PEG 400 = 2.9624 g (98.97%) | | | |
| 44 | Rapa = 61 mg (1.96%) | S | | Yes, 50 µL |
| | Ethanol, 100% = 0.1860 g (6%) | | | |
| | PEG 400 = 2.8588 g (92.04%) | | | |
| 45 | Rapa = 90.7 mg (3.02%) | S | | Yes, 50 µL |
| | Ethanol, 100% = 0.2722 g (9.06%) | | | |
| | PEG 400 = 2.6423 g (87.94%) | | | |
| 46 | Rapa = 101.6mg (4.997%) | S | | |
| | EtOH = 331.6mg (16.308%) | | | |
| | PEG400 = 1600.1mg (78.695%) | | | |
| 47 | Rapa = 120.9g (3.189%) | SP | | |
| | F68(Lutrol) = 42.4mg (1.118%) | | | |
| | Sterile Water = 3627.7mg (95.692%) | | | |
| 48 | Rapa = 100.1g (1.999%) | S | | |
| | EtOH = 305.1mg (6.092%) | | | |
| | PEG400 = 4602.9mg (91.909%) | | | |
| 49 | Rapa = 150.5mg (3.004%) | SP | | Yes, 20 µL, 40 µL |
| | PEG400 = 4860.3mg (96.996%) | | | |
| 50 | Rapa = 153.4mg (3.055%) | SP | | No, 20 µL |
| | F68(Pluronic)= 50.6mg (1.008%) | | | |
| | Sterile Water = 4816.6mg (95.937%) | | | |
| 51 | Rapa = 116.6mg (2.29%) | S | | Yes, 30 µL |
| | EtOH = 306.6mg (6.05%) | | | |
| | PEG400 = 4647.5mg (91.66%) | | | |
| 52 | Rapa = 150.4 mg (2.994%) | SP | | |
| | F68 Lutrol = 15.4 mg (0.306%) | | | |
| | Sterile water = 4859.1 mg (96.7%) | | | |
| 53 | Rapa = 306.5 mg (6.088%) | SP | | |
| | PEG 400 = 4727.7 mg (93.912%) | | | |
| 54 | Rapa = 309.3 mg (6.146%) | SP | | |
| | PEG 400 = 4723.3 mg (93.854%) | | | |
| 55 | Rapa = 303.3 mg (6.061 %) | SP | | |
| | PEG 400 = 4700.6 mg (93.939%) | | | |
| 56 | Rapa = 305.4 mg (6.088%) | SP | | |
| | PEG 400 = 4711.0 mg (93.912%) | | | |
| 57 | Rapa = 306.9 mg (6.098%) | SP | | |
| | PEG 400 = 4725.5 mg (93.902%) | | | |
| 58 | Rapa = 302.5 mg (6.021%) | SP | | |
| | PEG 400 = 4721.6 mg (93.979%) | | | |
| 59 | Rapa = 304.5 mg (6.053%) | SP | | |
| | PEG 400 = 4726.4 mg (93.947%) | | | |
| 60 | Dexamethasone = 251.4 mg (5.011 %) | SP | | |
| | PEG 400 = 4765.2 mg (94.989%) | | | |
| 61 | Dexamethasone = 252.4 mg (5%) | SP | | |
| | PEG 400 = 4600 mg (92%) | | | |
| | EtOH = 150 mg (3%) | | | |
| 62 | Rapa = 32.2 mg (0.641%) | S | | |
| | PEG 400 = 4677.9 mg (93.096%) | | | |
| | EtOH = 314.7 mg (6.263%) | | | |
| 63 | Rapa = 32.3 mg (0.6%) | S | | |
| | PEG 400 = 5516.3 mg (93.1%) | | | |
| | EtOH = 314.7 mg (6.263%) | | | |
| 64 | Rapa = 54.4 mg (1.007%) | S | | |
| | PEG 400 = 4638.9 mg (92.702%) | | | |
| | EtOH = 314.8 mg (6.291%) | | | |
| 65 | Rapa = 50.8 mg (1.013%) | S | | |
| | PEG 400 = 4963.2 mg (98.987%) | | | |
| 66 | Rapa = 52.1 mg (1.035%) | S | | |
| | PEG 400 = 4868.6 mg (96.718%) | | | |
| | EtOH = 113.1 mg (2.247%) | | | |
| 67 | Rapa = 50.5 mg (1.009%) | S | | Yes, 20 µL No, 40 µL, 100 µL |
| | PEG 400 = 4752.8 mg (94.953%) | | | |
| | EtOH = 202.1 mg (4.038%) | | | |
| 68 | Rapa = 101.8 mg (2.030%) | S | | |
| | PEG 400 = 4712.4 mg (93.970%) | | | |
| | EtOH = 200.6 mg (4.000%) | | | |
| 69 | Rapa = 102.1 mg (2.036%) | S | | |
| | PEG 400 = 4605.5mg (91.847%) | | | |
| | EtOH = 306.7 mg (6.117%) | | | |
| 70 | Rapa = 101.6 mg (2.025%) | S | | |
| | PEG 400 = 4510.6 mg (89.892%) | | | |
| | EtOH = 405.6 mg (8.083%) | | | |
| 71 | Rapa = 75.9 mg (3.019%) | SP | | |
| | PEG 400 = 2438.4 mg (96.981%) | | | |
| 72 | Rapa = 50.9 mg (2.034%) | S | | |
| | PEG 400 = 2350.1 mg (93.914%) | | | |
| | EtOH = 101.4mg (4.052%) | | | |
| 73 | Rapa = 12.5 mg (0.620%) | SP | | |
| | PEG 400 = 2004.8 mg (99.380%) | | | |
| 74 | Rapa = 1.20949 g (2.0152%) | S | | |
| | EtOH = 2.401 g (4.000%) | | | |
| | PEG 400 = 56.407 g (93.9848%) | | | |
| 75 | Rapa = 16.0 mg g (0.795%) | S | | No, 50 µL |
| | EtOH = 80.0 mg (3.976%) | | | |
| | PEG 400 = 1916.0 mg (95.2298%) | | | |
| 76 | Rapa = 8.1 mg (0.400%) | SP | | |
| | PEG 400 = 2014.5 mg (99.600%) | | | |
| 77 | Rapa = 8.6 mg (0.428%) | S | | |
| | PEG 400 = 2002.5 mg (99.572%) | | | |
| 78 | Rapa = 8.2 mg (0.410%) | S | | |
| | PEG 400 = 1992.0 mg (99.590%) | | | |
| 79 | Rapa = 8.7 mg (0.433%) | S | | |
| | PEG 400 = 1998.8 mg (99.567%) | | | |
| 80 | Rapa = 8.6 mg (0.427%) | S | | |
| | PEG 400 = 2003.2 mg (99.573%) | | | |
| 81 | Rapa = 8.6 mg (0.428%) | S | | |
| | PEG 400 = 1999.3 mg (99.572%) | | | |
| 82 | Rapa = 9.0 mg (0.448%) | S | | |
| | PEG 400 = 2000.8 mg (99.552%) | | | |
| 83 | Rapa = 8.0 mg (0.397%) | S | | |
| | PEG 400 = 2008.8 mg (99.603%) | | | |
| 84 | Rapa = 8.5 mg (0.422%) | S | | |
| | PEG 400 = 2006.8 mg (99.578%) | | | |
| 85 | Rapa = 8.0 mg (0.399%) | S | | |
| | PEG 400 = 1998.2 mg (99.601%) | | | |
| 86 | Rapa = 8.5 mg (0.422%) | S | | |
| | PEG 400 = 2004.3 mg (99.578%) | | | |
| 87 | Rapa = 8.6 mg (0.428%) | S | | |
| | PEG 400 = 2002.5 mg (99.572%) | | | |
| 88 | Rapa = 0.7 g (1.983%) | S | | |
| | EtOH = 1.4 g (3.966%) | | | |
| | PEG 400 = 33.2 g (94.051%) | | | |
| 89 | Rapa = 0 g (0%) | S | | |
| | EtOH = 0.574 g (1.995%) | | | |
| | PEG 400 = 28.2 g (98.005%) | | | |
| 90 | Rapa = 1.95 g (1.950%) | S | | |
| | EtOH = 4.05 g (4.050%) | | | |
| | PEG 400 = 94.00 g (94.000%) | | | |
| 91 | Rapa = 0.0107 g (0.534%) | S | | No, 80 µL |
| | EtOH = 0.0805 g (4.019%) | | | |
| | PEG 400 = 1.912 g (95.447%) | | | |
| 92 | Rapa = 0.0081 g (0.403%) | S | | No, 100 µL |
| | EtOH = 0.0804 g (4.003%) | | | |
| | PEG 400 = 1.920 g (95.594%) | | | |
| 93 | Rapa = 1.992 g (2%) | S | | |
| | EtOH = 3.9419 (4%) | | | |
| | PEG 400 = 93.95 g (94%) | | | |
| 94 | Rapa = 0.405 g (0.4%) | S | | |
| | EtOH = 4.24 g (4%) | | | |
| | PEG 400 = 95.6 (95,6%) | | | |
| 95 | PEG 400 = 96 g (96%) | S | | |
| | EtOH = 3.9027 (4%) | | | |
| 96 | Rapa = 0.4020 g (0.402%) | S | | |
| | EtOH = 3.970 g (3.971%) | | | |
| | PEG 400 = 95.600 g (95.627%) | | | |
| 97 | Rapa = 2.000 g (1.990%) | S | | |
| | EtOH = 4.000 g (3.980%) | | | |
| | PEG 400 = 94.500 g (94.030%) | | | |
| 98 | PEG 400 = 96 g (96%) | S | | |
| | EtOH = 3.92 g (4%) | | | |
| 99 | Rapa = 0.4036 g (0.4%) | S | | No, 100 µL |
| | EtOH = 3.9054 g (4%) | | | |
| | PEG 400 = 95.6 (95.6%) | | | |
| 100 | Rapa = 2.0025 g (2%) | S | | Yes, 1 µL, 3 µL, 20 µL, 40 µL |
| | EtOH = 3.98 g (4%) | | | |
| | PEG 400 = 94.00 g (94%) | | | |
| 101 | Rapa = 9.5 mg (0.472%) | S | | |
| | EtOH = 90.3 mg (4.485%) | | | |
| | PEG 600 = 1913.5 mg (95.043%) | | | |
| 102 | Rapa = 44.6 mg (2.21%) | S | | |
| | EtOH = 86.1.0 mg (4.26%) | | | |
| | PEG 600 = 1891.1 mg (93.53%) | | | |
| 103 | Rapa = 1.97 g (2%) | S | | |
| | EtOH = 4.10 g (4%) | | | |
| | PEG 400 = 94.15 g (94%) | | | |
| 104 | Rapa = 1.95 g (2%) | S | | |
| | EtOH = 4.00 g (4%) | | | |
| | PEG 400 = 94.0 g (94%) | | | |
| 105 | Rapa = 8.00 g (2%) | S | | |
| | PEG 400 = 376.0 g (94%) | | | |
| | EtOH = 16.0 g (4%) | | | |
| 106 | Rapa = 6.00 g (2%) | S | | |
| | PEG 400 = 282.0 g (94%) | | | |
| | EtOH = 12.0 g (4%) | | | |
| 107 | Rapa = 8.9 mg (0.4434%) | S | | |
| | EtOH = 80.3 mg (4.0006%) | | | |
| | PEG 300 = 1918.0 mg (95.556%) | | | |
| 108 | Rapa = 40.8 mg (2.00886%) | S | | |
| | EtOH = 110.0mg (5.41605%) | | | |
| | PEG 300 = 1880.2 mg (92.57509%) | | | |
| 109 | Rapa = 9.9 mg (0.488%) | S | | |
| | EtOH = 86.7mg (4.277%) | | | |
| | PEG 400/300(50/50) = 1930.3 mg | | | |
| | (95.235%) | | | |
| 110 | Dexamethasone = 142.5 mg (4.994%) | SP | 0.3305 µm | Yes, 30 µL |
| | PEG 400 = 2710.7 mg (95.006%) | | | |
| 111 | Dexamethasone = 134.3 mg (4.891%) | SP | >10 µm | |
| | PEG 400 = 2611.4 mg (95.109%) | | | |
| 112 | Triamcinolone = 139.2 mg (5.087%) | SP | 3.98 µm | Yes, 30 µL |
| | PEG 400 = 2597.4 mg (94.913%) | | | |
| 113 | Triamcinolone = 135.3 mg (5.089%) | SP | >10 µm | |
| | PEG 400 = 2523.5 mg (94.911%) | | | |
| 114 | EtOH = 206.4 mg (4.121%) | S | | No, 30 µL |
| | PEG 400 = 4801.6 mg (95.879%) | | | |
| 115 | Rapa = 43.0 mg (2.144%) | SP | 61.4390 µm | |
| | PEG 400 = 1962.3mg (97.8567%) | | | |
| 116 | Rapa = 40.0 mg (2.001%) | SP | 3.7128 µm | |
| | PEG 400 = 1959.1mg (97.999%) | | | |
| 117 | Rapa = 42.9 mg (2.142%) | SP | 2.7313 µm | |
| | PEG 400 = 1959.7mg (97.858%) | | | |
| 118 | Rapa = 100.8 mg (2.013%) | SP | 4.1063 µm | |
| | PEG 400 = 4906.0 mg (97.987%) | | | |
| 119 | Rapa = 20.9 mg (0.42%) | S | | |
| | EtOH = 209.1 mg (4.17%) | | | |
| | PEG 400 = 4784.9 mg (95.41%) | | | |
| 120 | Rapa = 20.6 mg (0.41%) | S | | |
| | EtOH = 211.5mg (4.22%) | | | |
| | Benz. Chl = 19.1 mg (0.38%) | | | |
| | PEG 400 = 4762.0 mg (94.99%) | | | |
| 121 | Rapa = 20.1 mg (0.40%) | S | | |
| | EtOH = 211.5mg (4.22%) | | | |
| | Benz. Chl = 2.3 mg (0.05%) | | | |
| | PEG 400 = 4782.3 mg (95.34%) | | | |
| 122 | Rapa = 8.0 g (2%) | S | | |
| | EtOH = 16.0 g (4%) | | | |
| | PEG 400 = 376.0 g (94%) | | | |
| 123 | Rapa = 351.3 mg (2.006%) | S | | |
| | EtOH = 2353.1 mg (4.093%) | | | |
| | PEG 400 = 16448.2 mg (93.901%) | | | |
| 124 | Rapa = 2.2035 g (2%) | S | | |
| | EtOH = 4.45 g (4%) | | | |
| | PEG 400 = 103.7 g (94%) | | | |
| 125 | Rapa = 515.5 mg (2.021 %) | SP | 18.1453 Nm | |
| | PEG 400 = 24,993.8 mg (97.979%) | | | |
| 126 | Rapa = 0.3 g (2%) | S | | |
| | EtOH = 0.6 g (4%) | | | |
| | PEG 400 = 14.1 g (94%) | | | |
| | BHT = 0.0002 (0.002%) | | | |
| 127 | Rapa = 0.3 g (2%) | S | | |
| | EtOH = 0.6 g (4%) | | | |
| | PEG 400 = 14.1 g (94%) | | | |
| | BHT = 0.00037 (0.004%) | | | |
| 128 | Rapa = 0.3 g (2%) | S | | |
| | EtOH = 0.6 g (4%) | | | |
| | PEG 400 = 14.1 g (94%) | | | |
| | BHT = 0.0081 (0.05%) | | | |
| 129 | Rapa = 243.2 mg (1.869%) | S | | |
| | EtOH = 4.88.4 mg (3.753%) | | | |
| | PEG 400 = 12283.3 mg (94.378%) | | | |
| 130 | Rapa = 0.404 g (2%) | S | | |
| | EtOH = 0.8 g (4%) | | | |
| | PEG 400 = 18.8 g (94%) | | | |
| | BHT = 0.00051 (0.002%) | | | |
| 131 | Rapa = 0.6024 g (2%) | S | | |
| | EtOH = 1.2 g (4%) | | | |
| | PEG 400 = 28.25 g (94%) | | | |
| 132 | Rapa = 2.001 g (2%) | S | | |
| | EtOH = 4.05 g (4%) | | | |
| | PEG 400 = 94.45 g (94%) | | | |
| 133 | Rapa = 0.5155 g (2.057%) | S | | |
| | EtOH = 1.0198 g (4.070%) | | | |
| | PEG 400 = 23.5225 g (93.873%) | | | |
| 134 | PEG 400 = 9.6 g (96%) | S | | |
| | EtOH = 0.4 g (4%) | | | |
| 135 | Rapa = 0.610 g (2%) | S | | |
| | EtOH = 1.2 g (4%) | | | |
| | PEG 400 = 28.2 g (94%) | | | |
| 136 | Rapa = 24.6 mg (1.193%) | S | | |
| | EtOH = 91.1mg (4.418%) | | | |
| | Tyloxapol = 219.6 mg (10.649%) | | | |
| | BSS = 1726.8 mg (83.740%) | | | |
| 137 | Rapa = 100.0 mg (1.993%) | SP | | |
| | PEG 400 = 4916.9 mg (98.007%) | | | |
| 138 | Rapa = 201.6 mg (4.005%) | SP | | |
| | PEG 400 = 4831.5 mg (95.995%) | | | |
| 139 | Rapa = 102.4 mg (2.036%) | S | | |
| | EtOH = 209.0 mg (4.154%) | | | |
| | PEG 400 = 4719.3 mg (93.810%) | | | |
| 140 | Rapa = 10.3 mg (0.205%) | S | | Yes, 10 IJL |
| | EtOH = 27.4 mg (0.544%) | | | |
| | PEG 400 = 4995.8 mg (99.251%) | | | |
| 141 | Rapa = 10.6 mg (0.211%) | S | | No, 10 µL |
| | EtOH = 208.4 mg (4.150%) | | | |
| | PEG 400 = 4802.3 mg (95.639%) | | | |
| 142 | Rapa = 31.5 mg (0.628%) | S | | Yes, 10 µL |
| | EtOH = 67.1 mg (1.337%) | | | |
| | PEG 400 = 4918.9mg (98.035%) | | | |
| 143 | Rapa = 30.8 mg (0.613%) | S | | No, 10 µL, 100 µL |
| | EtOH = 204.5 mg (4.073%) | | | |
| | PEG 400 = 4786.1 mg (95.314%) | | | |
| 144 | Rapa = 103.5 mg (2.057%) | S | | Yes, 10 µL |
| | EtOH = 207.1 mg (4.116%) | | | |
| | PEG 400 = 4720.8 mg (93.827%) | | | |
| 145 | Rapa = 283.0 mg (2.020%) | S | | |
| | EtOH = 566.1 mg (4.041%) | | | |
| | PEG 400 = 13,160.8 mg (93.939%) | | | |
| 146 | Rapa = 280.1 mg (1.998%) | S | | |
| | EtOH = 565.2 mg (4.033%) | | | |
| | PEG 400 = 13,171.7 mg (93.969%) | | | |
| 147 | Rapa = 201.6 mg (3.000%) | SP | | |
| | PEG 400 = 6518.8 mg (97.000%) | | | |
| 148 | Rapa = 31.9 mg (1.019%) | S | | |
| | Benzyl Alcohol = 1021.9 mg (20.070%) | | | |
| | Sesame Oil = 4017.9 mg (78.911%) | | | |
| 149 | Rapa = 51.5 mg (1.03%) | S | | |
| | Benzyl Alcohol = 259.9 mg (5.19%) | | | |
| | Sesame Oil = 4694.3 mg (93.78%) | | | |
| 150 | Rapa = 5.96 g (2%) | S | | |
| | EtOH = 12.0 g (4%) | | | |
| | PEG 400 = 282.0 g (94%) | | | |
| 151 | Rapa = 54.5 mg (1.07%) | S | | |
| | Benzyl Alcohol = 1014.3 mg (19.95%) | | | |
| | Olive Oil = 4014.8 mg (78.98%) | | | |
| 152 | Rapa =0 mg (0.00%) | S | | |
| | Benzyl Alcohol = 269.4 mg (5.421 %) | | | |
| | Tyloxapol = 608.2 mg (12.238%) | | | |
| | Sesame Oil = 4092.2 mg (82.341%) | | | |
| 153 | Rapa = 76.3 mg (1.75%) | S | | |
| | Benzyl Alcohol = 307.0 mg (7.06%) | | | |
| | Tyloxapol = 607.8 mg (13.97%) | | | |
| | Sesame Oil = 3000.5 mg (68.97%) | | | |
| | Span 80 = 63.1 mg (1.45%) | | | |
| | EtOH = 295.5 mg (6.79%) | | | |
| 154 | Form. # 150 = 200 g (99.998) | S | | |
| | BHT = 0.004 g (0.002%) | | | |
| 155 | Rapa = 51.0 mg (0.87%) | S | | |
| | EtOH = 642.3 mg (10.93%) | | | |
| | Benzyl Alcohol = 431.8 mg (7.34%) | | | |
| | Sesame Oil = 4753.7 mg (80.86%) | | | |
| 156 | Rapa = 51.4 mg (1.03%) | S | | |
| | Benzyl Alcohol = 518.4 mg (10.34%) | | | |
| | Olive Oil = 4444.7 mg (88.64%) | | | |
| 157 | Rapa = 8.1 g (2%) | S | | |
| | EtOH = 16.0 g (4%) | | | |
| | PEG 400 = 376.0 g (94%) | | | |
| 158 | Form. # 157 = 225.00 g (99.998%) | S | | |
| | BHT = 0.0045 g (0.002%) | | | |
| 159 | Rapa = 8.1 g (2%) | S | | |
| | EtOH = 16.0 g (4%) | | | |
| | PEG 400 = 376 g (94%) | | | |
| 160 | Form. # 159 = 112.0 g (99.998%) | S | | |
| | BHT = 0.00224 g (0.002%) | | | |
| 161 | Form. # 159 = 112.0 g (99.998%) | S | | |
| | BHT = 0.0019 g (0.002%) | | | |
| 162 | Rapa = 55.4 mg (1.10%) | S | | |
| | EtOH = 112.7 mg (2.25%) | | | |
| | Benzyl Alcohol = 157.8 mg (3.15%) | | | |
| | Cotton Seed Oil = 4688.0 mg (93.50%) | | | |
| 163 | Rapa = 5.005 g (1%) | S | | |
| | EtOH = 10.0 g (2%) | | | |
| | PEG 400 = 485.5 g (97%) | | | |
| 164 | PEG 400 = 9.82 g (98%) | S | | |
| | EtOH = 0.235 g (2%) | | | |
| 165 | Form. # 163 = 100.25 g (99.998%) | S | | |
| | BHT = 0.0026g (0.002%) | | | |
| 166 | Rapa = 203.1 mg (2.025%) | SP | 2.8651 µm | |
| | F68 = 30.3 mg (0.303%) | | | |
| | Sterile Water = 9792.6 mg (97.672%) | | | |
| 167 | Rapa = 201.4 mg (2.0005%) | SP | 1.0984 µm | |
| | Tween 20 = 43.9 mg (0.436%) | | | |
| | Sterile Water = 9822.8 mg (97.564%) | | | |
| 168 | EtOH = 0.8301 g (4.144%) | S | | |
| | PEG 400 = 19.2014 g (95.856%) | | | |
| 169 | Form. # 168 = 300 µl | S | | |
| 170 | Form. # 168 = 250 µl | S | | |
| | Form. # 154 = 50 µl | | | |
| 171 | Form. # 168 = 200 µl | S | | |
| | Form. # 154 = 100 µl | | | |
| 172 | Form. # 168 = 150 µl | S | | |
| | Form. # 154 = 150 µl | | | |
| 173 | Form. # 154 = 300 µl | S | | |
| 174 | Rapa = 102.2 mg (2.041%) | SP | 0.4165 µm | |
| | F68 = 16.0 mg (0.32%) | | | |
| | Sterile Water = 4889.0 mg (97.639%) | | | |
| 175 | Rapa = 101.1 mg (2.010%) | SP | 0.5294 µm | |
| | Tween 20 = 27.7 mg (0.551 %) | | | |
| | Sterile Water = 4901.0 mg (97.439%) | | | |
| 176 | BSS+ = 0 µl | S | | |
| | Sterile Water = 0 µl | | | |
| | Form. # 154 = 1000 µl | | | |
| 177 | BSS+ = 200 µl | SP | | |
| | Sterile Water = 0 µl | | | |
| | Form. # 154 = 800 µl | | | |
| 178 | BSS+ = 400 µl | SP | | |
| | Form. # 154 = 600 µl | | | |
| 179 | BSS+ = 500 µl | SP | | |
| | Form. # 154 = 500 µl | | | |
| 180 | BSS+ = 600 µl | SP | | |
| | Form. # 154 = 400 µl | | | |
| 181 | BSS+ = 800 µl | SP | | |
| | Form. # 154 = 200 µl | | | |
| 182 | Sterile Water = 200 µl | SP | | |
| | Form. # 154 = 800 µl | | | |
| 183 | Sterile Water = 400 µl | SP | | |
| | Form. # 154 = 600 µl | | | |
| 184 | Sterile Water = 500 µl | SP | | |
| | Form. # 154 = 500 µl | | | |
| 185 | Sterile Water = 600 µl | SP | | |
| | Form. # 154 = 400 µl | | | |
| 186 | Sterile Water = 800 µl | SP | | |
| | Form. # 154 = 200 µl | | | |
| 187 | BSS+ = 2536.9 mg (49.98%) | SP | 60.2075 µm | |
| | Form. # 154 = 2538.7 mg (50.02%) | | | |
| 188 | Sterile Water = 2515.6 mg (49.84%) | SP | 617.5157 µm | |
| | Form. # 154 = 2532.2 mg (50.16%) | | | |
| 189 | F68 = 12.6 mg (0.25%) | SP | 70.6089 µm | |
| | Sterile Water = 2524.7 mg (49.79%) | | | |
| | Form. # 154 = 2533.1 mg (49.96%) | | | |
| 190 | Rapa = 2.0225 g (2%) | S | | |
| | EtOH = 3.65 g (4%) | | | |
| | PEG 400 = 94.0 g (94%) | | | |
| | BHT = 0.002 g (0.002%) | | | |
| 191 | F68 = 12.1 mg | SP | | |
| | Sterile Water = 2558.9 mg | | | |
| | Form. # 154 = 2556.4 mg | | | |
| 192 | F68 = 19.8 mg | SP | | |
| | Sterile Water = 2564.1 mg | | | |
| | Form. # 154 = 25557.5 mg | | | |
| 193 | F68 = 25.3 mg | SP | | |
| | Sterile Water = 2575.1 mg | | | |
| | Form. # 154 = 2572.9 mg | | | |
| 194 | F68 = 32.4 mg | SP | | |
| | Sterile Water = 2572.1 mg | | | |
| | Form. # 154 = 2562.1 mg | | | |
| 195 | F68 = 38.3 mg | SP | | |
| | Sterile Water = 2563.2 mg | | | |
| | Form. # 154 = 2573.5 mg | | | |
| 196 | F68 = 43.6 mg | SP | | |
| | Sterile Water = 2541.1 mg | | | |
| | Form. # 154 = 2556.0 mg | | | |
| 197 | F68 = 51.2 mg | SP | | |
| | Sterile Water = 2594.5 mg | | | |
| | Form. # 154 = 2594.1 mg | | | |
| 198 | PEG 400 = 1920 g (96%) | S | | |
| | EtOH = 80 g (4%) | | | |
| 199 | Form. # 168 = 1000 µl | S | | |
| 200 | Form. # 168 =20 0 µl | S | | |
| | Form. # 154 = 800 µl | | | |
| 201 | Form. # 168 = 400 µl | S | | |
| | Form. # 154 = 600 µl | | | |
| 202 | Form. # 168 = 500 µl | S | | |
| | Form. # 154 = 500 µl | | | |
| 203 | Form. # 168 = 600 µl | S | | |
| | Form. # 154 = 400 µl | | | |
| 204 | Form. # 168 = 800 µl | S | | |
| | Form. # 154 = 200 µl | | | |
| 205 | PEG 400 = 200 µl | S | | |
| | Form. # 154 = 800 µl | | | |
| 206 | PEG 400 = 400 µl | S | | |
| | Form. # 154 = 600 µl | | | |
| 207 | PEG 400 = 500 µl | S | | |
| | Form. # 154 = 500 µl | | | |
| 208 | PEG 400 = 600 µl | S | | |
| | Form. # 154 = 400 µl | | | |
| 209 | PEG 400 = 800 µl | S | | |
| | Form. # 154 = 200 µl | | | |
| 210 | Phosal 50PG = 6735.0 mg (99.002%) | S | | |
| | Tween 80 = 67.9 mg (0.998%) | | | |
| 211 | Rapa = 2.0047 g (2%) | S | | |
| | EtOH = 4.00 g (4%) | | | |
| | PEG 400 = 94.05 g (94%) | | | |
| 212 | Phosal 50PG = 20.0662 g (98.999%) | S | | |
| | Tween 80 = 0.2029 g (1.001%) | | | |
| 213 | Form. # 154= 100 µl | S | | |
| | Form. # 168 = 900 µl | | | |
| 214 | Form. # 154 = 100 µl | S | | |
| | Form. # 168 = 900 µl | | | |
| 215 | Form. # 154 = 100 µl | S | | |
| | Form. # 168 = 900 µl | | | |
| 216 | Form. # 154 = 100 µl | S | | |
| | PEG 400 = 900 µl | | | |
| 217 | Form. # 154 = 100 µl | S | | |
| | PEG 400 = 900 µl | | | |
| 218 | Form. # 154 = 100 µl | S | | |
| | PEG 400 = 900 µl | | | |
| 219 | Form. # 154 = 100 µl | SP | | |
| | BSS+ = 900 µl | | | |
| 220 | Form. # 154 = 100 µl | SP | | |
| | BSS+ = 900 µl | | | |
| 221 | Form. # 154 = 100 µl | SP | | |
| | BSS+ = 900 µl | | | |
| 222 | Form. # 154 = 1000 µl | S | | |
| 223 | Form. # 154 = 1000 µl | S | | |
| 224 | Form. # 154 = 100 µl | S | | |
| | Form. # 168 = 900 µl | | | |
| 225 | Form. # 154 = 100 µl | S | | |
| | Form. # 168 = 900 µl | | | |
| 226 | Form. # 154 = 100 µl | S | | |
| | Form. # 168 = 900 µl | | | |
| 227 | Form. # 154 = 100 µl | S | | |
| | PEG 400 = 900 µl | | | |
| 228 | Form. # 154 = 100 µl | S | | |
| | PEG 400 = 900 µl | | | |
| 229 | Form. # 154 = 100 µl | S | | |
| | PEG 400 = 900 µl | | | |
| 230 | Form. # 154 = 100 µl | SP | | |
| | BSS+ =900 µl | | | |
| 231 | Form. # 154 = 100 µl | SP | | |
| | BSS+ = 900 µl | | | |
| 232 | Form. # 154 = 100 µl | SP | | |
| | BSS+ = 900 µl | | | |
| 233 | Form. # 154 = 200 µl | S | | |
| | Form. # 168 = 800 µl | | | |
| 234 | Form. # 154 = 200 µl | S | | |
| | Form. # 168 = 800 µl | | | |
| 235 | Form. # 154 = 200 µl | S | | |
| | Form. # 168 = 800 µl | | | |
| 236 | Form. # 154 = 200 µl | S | | |
| | Form. # 168 = 800 µl | | | |
| 237 | Form. # 154 = 200 µl | S | | |
| | PEG 400 = 800 µl | | | |
| 238 | Form. # 154 = 200 µl | S | | |
| | PEG 400 = 800 µl | | | |
| 239 | Form. # 154 = 200 µl | SP | | |
| | BSS+ = 800 µl | | | |
| 240 | Form. # 154 = 200 µl | SP | | |
| | BSS+ = 800 µl | | | |
| 241 | Form. # 154 = 200 µl | SP | | |
| | BSS+ = 800 µl | | | |
| 242 | Form. # 154 = 100 µl | S | | No, 10 µl |
| | Form. # 168 = 900 µl | | | |
| 243 | Form. # 154 = 100 µl | S | | Yes, 10 µl |
| | PEG 400 = 900 µl | | | |
| 244 | Form. # 154 = 100 µl | SP | | Yes, 10 µl |
| | BSS+ = 900 µl | | | |
| 245 | Form. # 154 = 100 µl | SP | | |
| | BSS+/CMC(0.5%)= 900 µl | | | |
| 246 | Form. # 154 = 400 µl | S | | No, 10 µl |
| | Form. # 168 = 900 µl | | | |
| 247 | Form. # 154 = 400 µl | S | | Yes, 10 µl |
| | PEG 400 = 900 µl | | | |
| 248 | Form. # 154 = 400 µl | SP | | Yes, 10 µl |
| | BSS+ = 900 µl | | | |
| 249 | Form. # 154 = 400 µl | SP | | |
| | BSS+/CMC(0.5%)= 900 µl | | | |
| 250 | Form. # 154 = 100 µl | SP | | |
| | BSS+/CMC(0.5%)= 900 µl | | | |
| 251 | Form. # 154 = 100 µl | SP | | |
| | BSS+/CMC(0.5%)= 900 µl | | | |
| 252 | Form. # 154 = 100 µl | SP | | |
| | BSS+/CMC(0.5%)= 900 µl | | | |
| 253 | Form. # 154 = 200 µl | SP | | |
| | BSS+/CMC(0.5%)= 800 µl | | | |
| 254 | Form. # 154 = 200 µl | SP | | |
| | BSS+/CMC(0.5%)= 800 µl | | | |
| 255 | Form. # 154 = 200 µl | SP | | |
| | BSS+/CMC(0.5%)= 800 µl | | | |
| 256 | Form. # 154 = 400 µl | SP | | |
| | BSS+/CMC(0.5%)= 900 µl | | | |
| 257 | Form. # 154 = 400 µl | SP | | |
| | BSS+/CMC(0.5%)= 900 µl | | | |
| 258 | Form. # 154 = 400 µl | SP | | |
| | BSS+/CMC(0.5%)= 900 µl | | | |
| 259 | EtOH = 17.1 mg (0.57%) | S | | |
| | PEG 400 = 2997.3 mg (99.43%) | | | |
| 260 | EtOH = 40.8 mg (1.35%) | S | | |
| | PEG 400 = 2980.2 mg (98.65%) | | | |
| 261 | EtOH = 47.1 mg (1.57%) | S | | |
| | PEG 400 = 2950.1 mg (98.43%) | | | |
| 262 | Rapa = 2.0032 g (2%) | S | | |
| | EtOH = 3.92 g (4%) | | | |
| | PEG 400 = 94.00 g (94%) | | | |
| 263 | Triamcinolone acetomide = 80.8 mg (4.04%) | SP | | |
| | PEG 400 = 1920.8 mg (95.96%) | | | |
| 264 | NFF-0007 filled in glove box | S | | |
| 265 | PEG 400 = 9.598 g (96%) | S | | |
| | EtOH = 0.4052 (4%) | | | |
| 266 | Triamcinolone acetomide = 42.2 mg (4.123%) | SP | | |
| | PEG 400 = 981.3 mg (95.877%) | | | |
| 267 | Phosal 50PG = 20.0783 g (99.00835%) | S | | |
| | Tween 80 = 0.2011 g (0.99165%) | | | |
| 268 | PEG 400 96.1 g (96%) | S | | |
| | EtOH = 4.00 g (4%) | | | |
| 269 | Rapa = 0.4001 g (2%) | S | | |
| | EtOH = 0.80 g (4%) | | | |
| | PEG 400 = 18.8 g (94%) | | | |
| 270 | Sterile Water = 9955.8 mg (99.27%) | S | | |
| | CMC High visc. = 47.8 mg (0.48%) | | | |
| | Tween 80 = 25.4 mg (0.25%) | | | |
| 271 | Sterile Water = 9947.5 mg (99.00%) | S | | |
| | CMC Medium visc. = 75 mg (0.75%) | | | |
| | Tween 80 = 25.1 mg (0.25%) | | | |
| 272 | Rapa = 41 mg (2.01%) | SP | | |
| | Form. # 270 = 2000 mg (97.99%) | | | |
| 273 | Rapa = 40.2 mg (1.97%) | SP | | |
| | MSF-03-172-07E = 2000 mg (98.03%) | | | |
| 274 | NMP (Pharmasolve^{®} = 1280.5 mg (65.89%) | S | | |
| | PLGA 75/25 = 662.9 mg (34.11%) | | | |
| 275 | NMP (Pharmasolve^{®)} = 1573.3 mg (80.50%) | S | | |
| | PLGA 75/25 = 381.0 mg (19.50%) | | | |
| 276 | NMP (Pharmasolve^{®)}= 1009.7 mg 49.8%) | S | | Yes, 10 µL |
| | PLGA 75/25 = 1001.6 mg (50.20%) | | | |
| 277 | Sterile Water = 14934.0 mg (99.25%) | S | | |
| | CMC Medium visc. = 112.4 mg (0.75%) | | | |
| 278 | Propylene Glycol = 1893.7 mg (93.85%) | S | | Yes, 10 µL |
| | EtOH = 83.8 mg (4.16%) | | | |
| | Rapa = 40.2 mg (1.99%) | | | |
| 279 | Propylene Glycol = 1946.2 mg (95.68%) | S | | Yes, 10 µL |
| | Benzyl Alcohol = 47.1 mg (2.31%) | | | |
| | Rapa = 40.8 mg (2.01%) | | | |
| 280 | PEG 300 = 1894.1 mg (93.74%) | S | | Yes, 10 µL |
| | EtOH = 40.1 mg (1.98%) | | | |
| | Rapa = 86.4 mg (4.28%) | | | |
| 281 | PEG 300 = 1925.5 mg (95.88%) | S | | Yes, 10 µL, 30 µL |
| | EtOH = 39.8 mg (1.98%) | | | |
| | Rapa = 43.0 mg (2.14%) | | | |
| 282 | Rapa = 100.6 mg (2.01 %) | SP | | Yes, 10 µL, 30 µL |
| | MSF-03-176-02 = 4910.8 mg (97.99%) | | | |
| 283 | Rapa = 11.5 mg (0.57%) | S | | |
| | PEG 300 = 2012.5 mg (99.43%) | | | |
| 284 | Rapa = 10.3 mg (0.51%) | S | | |
| | PEG 400 = 2017.2 mg (99.49%) | | | |
| 285 | Rapa = 9.8 mg (0.486%) | S | | |
| | PEG 600 = 2005.9 mg (99.51%) | | | |
| 286 | Tacrolimus = 42.7 mg (2.11%) | S | | |
| | EtOH = 46.0 mg (2.27%) | | | |
| | PG = 1938.7 mg (95.62%) | | | |
| 287 | Tacrolimus = 40.7 mg (2.01%) | S | | |
| | EtOH = 43.0 mg (2.12%) | | | |
| | PEG 300 = 1942.1 mg (95.87%) | | | |
| 288 | Tacrolimus = 40.3 mg (1.99%) | S | | |
| | EtOH = 43.8 mg (2.16%) | | | |
| | PEG 400 = 1942.3 mg (95.85%) | | | |
| 289 | Tacrolimus = 40.8 mg (2.03%) | S | | |
| | EtOH = 44.5 mg (2.21 %) | | | |
| | PEG 600 = 1924.0mg (95.76%) | | | |
| 290 | Rapa = 61.0 mg (3.17%) | S | | |
| | NMP = 1226.54 mg (63-80%) | | | |
| | PLGA 75/25 = 634.96 mg (33.03%) | | | |
| 291 | Rapa = 100.2 mg (5.13%) | S | | |
| | NMP = 1492.95 mg (76.37%) | | | |
| | PLGA 75/25 = 361.65 mg (18.50%) | | | |
| 292 | Rapa = 62.9 mg (3.04%) | S | | |
| | NMP = 1103.8g mg (53.40%) | | | |
| | PLGA 75/25 = 900.2 mg (43.56%) | | | |
| 293 | Rapa = 62.4 mg (3.00%) | S | | |
| | NMP = 1205.1 mg (58.11%) | | | |
| | PLGA 75/25 = 806.4 mg (38.89%) | | | |
| 294 | Water+1% CMC Med. = 4909.1 mg (97.99%) | SP | | |
| | Rapa = 100.5 mg (2.01%) | | | |
| 295 | Water+1% CMC high. = 4903.8 mg (97.96%) | SP | | |
| | Rapa= 101.9mg (2.04%) | | | |
| 296 | Rapa = 40.5 mg (2.03%) | S | | |
| | NMP = 1958.7 mg (97.97%) | | | |
| 297 | Rapa = 20.5mg (2.0%) | SP | | |
| | DMA = 41.4mg (4.0%) | | | |
| | PVP = 35.0mg (3.4%) | | | |
| | H2O = 934.7mg (90.6%) | | | |
| 298 | Rapa = 10.6mg (2.0%) | S | | |
| | DMA = 10.6mg (2.0%) | | | |
| | PEG 400 = 506.1mg (96%) | | | |
| 299 | Rapa = 5.2mg (2.0%) | SP | | |
| | 1% DMA in PEG 400 = 257.4 mg (98%) | | | |
| 300 | Rapa = 20.0mg (2.0%) | S | | |
| | DMA = 7.8mg (0.8%) | | | |
| | PEG 400 = 974mg (97.2%) | | | |
| 301 | Rapa = 20.1mg (1.3%) | S | | |
| | DMA = 19.5mg (1.3%) | | | |
| | PEG 400 = 1449.6mg (97.3%) | | | |
| 302 | Rapa = 20.0mg (2.0%) | SP | | |
| | PVP = 10.8mg (1.1%) | | | |
| | PEG 400 = 994.5mg (97.0%) | | | |
| 303 | Rapa = 20.4mg (2.0%) | SP | | |
| | PVP = 24.5mg (2.4%) | | | |
| | PEG 400 = 990.7mg (95.7%) | | | |
| 304 | Rapa = 25.5mg (2.4%) | SP | | |
| | PVP = 51.9mg (4.8%) | | | |
| | PEG 400 = 1000.6mg (92.8%) | | | |
| 305 | Rapa = 22.5mg (2.3%) | S | | |
| | BA = 27.5mg (2.7%) | | | |
| | PEG 400 = 950.7mg (95.0%) | | | |
| 306 | Rapa = 30.2mg (2.3%) | SP | | |
| | PVP = 240.9mg (18.6%) | | | |
| | PEG 400 = 1021.2mg (79.0%) | | | |
| 307 | Rapa = 8.7mg (3.1%) | SP | | |
| | 1% PVP in H2O = 273 mg (96.9%) | | | |
| 308 | Rapa = 12.6mg (2.53%) | SP | | |
| | 5% PVP in H2O = 501.6 mg (97.5%) | | | |
| 309 | Rapa = 20.3mg (3.8%) | SP | | |
| | 10% PVP in H2O = 513.9 mg (96.2%) | | | |
| 310 | Rapa = 100.5mg (2.0%) | S | | Yes, 10 µL |
| | DMA = 67.8mg (1.4%) | | | |
| | PEG 400 = 4838.3mg (96.6%) | | | |
| 311 | Rapa = 96.8mg (1.9%) | S | | Yes, 10 µL |
| | BA = 157.5mg (3.2%) | | | |
| | PEG 400 = 4748.7mg (94.9%) | | | |
| 312 | Rapa = 105.8mg (2.1%) | S | | |
| | DMA = 5.63mg (0.1%) | | | |
| | PEG 400 = 4888.9mg (97.8%) | | | |
| 313 | Rapa = 20.2mg (2.0%) | SP | | |
| | PVP = 99.2mg (9.9%) | | | |
| | H2O = 882.3mg (88.1%) | | | |
| 314 | Rapa = 100.3mg (2.0%) | SP | | |
| | PVP = 251.4mg (5.0%) | | | |
| | H2O = 4662.8mg (93.0%) | | | |
| 315 | Rapa = 20.3mg (2.0%) | S | | |
| | DMA = 983.9mg (98%) | | | |
| 316 | Triamcinolone = 22.8mg (2.0%) | S | | Yes, 10 µL |
| | DMA = 12.0mg (1.1%) | | | |
| | PEG 400 = 1104.5mg (96.9%) | | | |
| 317 | Triamcinolone = 1.0mg (0.1%) | S | | |
| | EtOH = 49.30mg (4.0%) | | | |
| | PEG 400 = 1191.9mg (96.0%) | | | |
| 318 | Triamcinolone = 18.7mg (0.9%) | S | | |
| | PEG 400 = 959.8mg (99.1%) | | | |
| 319 | Triamcinolone = 25.5mg (1.3%) | S | | |
| | EtOH = 83.0mg (4.1 %) | | | |
| | PEG 400 = 1905.6mg (94.6%) | | | |
| 320 | Dexamethasone = 20.4mg (1.2%) | S | | |
| | EtOH = 71.7mg (4.1%) | | | |
| | PEG 400 = 1737.6mg (98.8%) | | | |
| 321 | Dexamethasone = 27.5mg (2.0%) | S | | Yes, 10 µL |
| | DMA = 5.6mg (0.4%) | | | |
| | PEG 400 = 1347.3mg (97.6%) | | | |
| 322 | Rapa = 9.1mg (0.152%) | E | | |
| | EtOH = 90.9mg (1.514%) | | | |
| | F127 = 262.8mg (4.378%) | | | |
| | Water = 1489.1mg (24.804%) | | | |
| | Sesame oil = 4151.5mg (69.152%) | | | |
| 323 | Rapa = 24.4mg (0.625%) | E | | |
| | Phosal 50PG = 203.1mg (5.201%) | | | |
| | EtOH = 166.8mg (4.272%) | | | |
| | Labrafac CC = 1502.8mg (38.486%) | | | |
| | Sesame oil = 2007.7mg (51.416%) | | | |
| 324 | Form. # 174 with 2mm beads | SP | 0.4929 µm | |
| 325 | Form. # 175 with 2mm beads | SP | 0.4804 µm | |
| 326 | Rapa = 4 mg (4%) | S | | |
| | Ethanol = 4 mg (4%) | | | |
| | PEG 400 = 92 mg (92%) | | | |
| 327 | Rapa = 1.47% | S | | |
| | Ethanol = 2.93% | | | |
| | PEG 400 = 69% | | | |
| | Saline = 26.6% | | | |
| 328 | Rapa = 1.38% | SP | | |
| | Ethanol = 2.75% | | | |
| | PEG 400 = 64.62% | | | |
| | Saline = 31.25% | | | |
| 329 | Rapa = 0.07% | E | | |
| | Cremophor (PEG 35 castor oil) = 8.35% | | | |
| | Capmul MCM (C8) (mono/diglycerides of caprylic acid) = 8.37% | | | |
| | Sterile Water = 83.21% | | | |
| 330 | Rapa = 0.09% | E | | |
| | Tyloxapol (nonionic polymer of the alkyl aryl polyether alcohol) = 8.12% | | | |
| | Capmul MCM (C8) (mono/diglycerides of caprylic acid) = 8.15% | | | |
| | Ethanol = 0.35% | | | |
| | Sterile Water = 83.29% | | | |
| 331 | Rapa = 0.08% | E | | |
| | Tyloxapol (nonionic polymer of the alkyl aryl polyether alcohol) = 6.78% | | | |
| | Capmul MCM (C8) (mono/diglycerides of caprylic acid) = 6.81 % | | | |
| | Phosal® 50PG = 3.12% | | | |
| | Sterile Water = 83.21% | | | |
| 332 | Rapa = 0.17% | E | | |
| | Cremophor (PEG 35 castor oil) = 7.18% | | | |
| | Capmul MCM (C8) (mono/diglycerides of caprylic acid) = 9.47% | | | |
| | Sterile Water = 83.18% | | | |
| 333 | N-methyl Pyrrolidone (NMP) = 10.5% | S | | |
| | Water = up to 15.8% | | | |
| | Propylene Glycol (PG) = 73.7% | | | |
| | Rapamycin = up to 5% (w/w) | | | |
| 334 | N-methyl Pyrrolidone (NMP) = 38.9% | S | | |
| | Water = up to 22.2% | | | |
| | Propylene Glycol (PG) = 38.9% | | | |
| | Rapamycin = up to 5% (w/w) | | | |
| 335 | N-methyl Pyrrolidone (NMP) = 10.5% | S | | |
| | Water = up to 15.8% | | | |
| | PEG 600 = 73.7% | | | |
| | Rapamycin = up to 5% (w/w) | | | |
| 336 | Dimethyl Acetamine (DMA) = 10.5% | S | | |
| | Water = up to 15.8% | | | |
| | Propylene Glycol (PG) = 73.7% | | | |
| | Rapamycin = up to 5% (w/w) | | | |
| 337 | Dimethyl Sulfoxide (DMSO)= 10.5% | S | | |
| | Water = up to 15.8% | | | |
| | Propylene Glycol (PG) = 73.7% | | | |
| | Rapamycin = up to 5% (w/w) | | | |
| 338 | N-methyl Pyrrolidone (NMP) = 27.9% | S | | |
| | Water = up to 28.4% | | | |
| | PEG 400 = 39% | | | |
| | Ethanol = 4.7% | | | |
| | Rapamycin = up to 5% (w/w) | | | |
| 339 | Dasatanib = 4.84% | S | | |
| | Dimethyl Acetamine (DMA) = 15% | | | |
| | PEG 400 = 80.16% | | | |
| 340 | Rapa = 40 mg (2%) | S | | |
| | Ethanol = 80 mg (4%) | | | |
| | PEG 400 = 1880 mg (94%) | | | |
| 341 | Rapa = 0.3% | | | |
| | Tyloxapol = 4.8% | | | |
| | Lauroglycol = 2.8% | | | |
| | Labrafac = 1.8% | | | |
| | remainder Sterile Water | | | |
| 342 | Rapa = 0.2% | | | |
| | Tyloxapol = 2.8% | | | |
| | EtOH = 0.3% | | | |
| | Labrafac = 2.8% | | | |
| | remainder Sterile Water | | | |
| 343 | Rapa = 0.2% | | | |
| | Tyloxapol = 2.8% | | | |
| | EtOH = 0.3% | | | |
| | Labrafac = 2.8% | | | |
| | remainer Sterile Water | | | |
| 344 | Rapa = 0.2% | | | |
| | EtOH = 0.4% | | | |
| | PEG 400 = 69.4% | | | |
| | remainder Sterile Water | | | |
| 345 | Rapa = 0.2% | | | |
| | EtOH = 0.4% | | | |
| | PEG 400 = 69.2% | | | |
| | remainder Sterile Water | | | |
| 346 | Rapa = 0.6% (0.4% degradation products) | S | | |
| | N-methyl pyrrolidone (NMP) = 30% | | | |
| | Propylene Glycol (PG) = 39% | | | |
| | remainder Sterile Water | | | |
| 347 | Rapa = 2.1% | SP | 3.5 µm | |
| | CMC = 1 % | | | |
| | Sterile Water = 96.9% | | | |
| 348 | Rapa = 2.1% | ISG | | |
| | PLGA 75/25 = 21.1% | | | |
| | N-methyl pyrrolidone (NMP) = 76.8% | | | |
| 349 | Rapa = 2.0% | | | |
| | N-methyl pyrrolidone (NMP) = 44.5% | | | |
| | PEG 400 = 29.0% | | | |
| | Water = 24.5% | | | |
| 350 | Rapa = 1% | | | |
| | Ethanol = 4% | | | |
| | PEG 400 = 95% | | | |
| 351 | Rapa = 10.0% | SDF | | |
| | PVP 90 = 57.9% | | | |
| | Phosal 50 SD = 20.0% | | | |
| | PEG 400 = 10.1% | | | |
| | Gamma Tocopherol = 1.0% | | | |
| | Ascorbyl palmitate = 1.0% | | | |
| 352 | Dexamethasone = 25.3% | SDF | | |
| | HPC = 33.2% | | | |
| | Eudragit RS 30D = 32.0% | | | |
| | Xanthan Gum = 9.5% | | | |

| | | | | |
|---|---|---|---|---|
| Depending on their type, the listed formulations are denoted as one or more of solutions (S), suspensions (SP), emulsions (E), in situ gels (ISG) or solid dosage forms (SDF). | | | | |

**Table 2 Aqueous Humor Rapa Concentration**

| 2% Rapa-PEG-EtOH Solution | Mean [Rapa] (ng/mL) | SD (ng/mL) |
|---|---|---|
| 1.0 µL intravitreal | 0.438 (1 day after injection) | 0.141 |
| 3.0 µL intravitreal | 0.355 (1 day after injection) | 0.234 |
| 3.0 µL sub-conj | 0.338 (1 day after injection) | 0.122 |
| 5.0 µL into anterior chamber | 0.167 (14 days after injection) | 0.183 |
| 10.0 µL into anterior chamber | 0.004 (14 days after injection) | 0.006 |

**Table 3: Topical Administration Rapamycin Levels**

| | **Blood, ng/ml** | | | **Cornea, ng/g** | | | | | |
|---|---|---|---|---|---|---|---|---|---|
| | Gr. I | Gr. II | Gr. III | Gr. I | | Gr. II | | Gr. III | |
| **time, day** | | | | (n=2) | std | OD (n=1) | OS (n=1) | (n=2) | std |
| 1 | 4.63 | 2.84 | | 121.72 | 2.22 | 147.26 | 4.10 | | |
| 2 | 65.72 | 4.29 | | 229.49 | 10.03 | 209.98 | 10.46 | | |
| 3 | 37.14 | 27.29 | | 173.76 | 20.17 | 184.05 | 11.94 | | |
| 8 | | | 15.54 | | | | | 168.75 | 27.93 |
| 10 | | | 7.67 | | | | | 110.10 | 42.14 |
| 12 | | | 2.88 | | | | | 97.90 | 0.71 |
| 14 | | | 48.32 | | | | | 84.20 | 31.25 |

**Table 4: Topical Administration Rapamycin Levels**

| | **R/Ch, ng/g** | | | | | |
|---|---|---|---|---|---|---|
| | **Group I** | | **Group II** | | **Group III** | |
| **time, day** | **(n=2)** | **std** | **OD (n=1)** | **OS (n=1)** | **(n=2)** | **std** |
| 1 | 20.23 | 0.0354 | 34.51 | 11.58 | | |
| 2 | 41.53 | 4.5679 | 64.86 | 25.68 | | |
| 3 | 48.92 | 36.0695 | 22.88 | 63.67 | | |
| 8 | | | | | 107.24 | 80.5748 |
| 10 | | | | | 139.98 | 150.1895 |
| 12 | | | | | 30.05 | 12.7845 |
| 14 | | | | | 117.50 | 116.9555 |

**Table 5: Topical Administration Rapamycin Levels**

| | **Sclera, ng/g** | | | | | |
|---|---|---|---|---|---|---|
| | **Group I** | | **Group II** | | **Group III** | |
| **time, day** | **(n=2)** | **std** | **OD (n=1)** | **OS (n=1)** | **(n=2)** | **std** |
| 1 | 90.80 | 40.59 | 420.60 | 34.20 | | |
| 2 | 751.35 | 471.29 | 1353.60 | 144.50 | | |
| 3 | 350.80 | 77.22 | 540.60 | 104.20 | | |
| 8 | | | | | 895.20 | 368.83 |
| 10 | | | | | 612.65 | 530.97 |
| 12 | | | | | 130.85 | 60.17 |
| 14 | | | | | 419.65 | 515.41 |

**Table 6: Topical Administration Rapamycin Levels**

| | **Vitreous, ng/ml** | | | | | |
|---|---|---|---|---|---|---|
| | **Gr. I** | | **Gr. II** | | **Gr. III** | |
| **time, day** | **(n=2)** | **std** | **OD (n=1)** | **OS (n=1)** | **(n=2)** | **std** |
| 1 | 1.24 | 0.1697 | 2.06 | 1.00 | | |
| 2 | 5.41 | 1.2799 | 5.01 | 2.95 | | |
| 3 | 3.03 | 0.6859 | 3.96 | 4.00 | | |
| 8 | | | | | 19.69 | 22.5072 |
| 10 | | | | | 7.90 | 7.2903 |
| 12 | | | | | 1.46 | 0.5374 |
| 14 | | | | | 5.66 | 2.6870 |

## Claims

1. A first formulation comprising an amount of rapamycin in combination with a second formulation comprising an amount of ranibizumab for use in a method of treating age-related macular degeneration in a human subject, wherein the first and second formulations are each administered by intraocular or periocular delivery to the subject in volumes such that the amount of the rapamycin and the amount of the ranibizumab together are effective to treat the age-related macular degeneration.

2. A first formulation for use according to claim 1, wherein the first formulation consists essentially of about 2% (w/w) rapamycin, about 4% (w/w) ethanol, and about 94% (w/w) PEG 400.

3. A first formulation for use according to claim 1, wherein the first formulation consists essentially of about 4% (w/w) rapamycin, about 4% (w/w) ethanol, and about 92% (w/w) PEG 400.

4. A first formulation for use according to claim 1, wherein the first formulation consists essentially of about 1% (w/w) rapamycin, about 4% (w/w) ethanol, and about 95% (w/w) PEG 400.

5. A first formulation for use according to any one of claims 1-4, wherein the age-related macular degeneration is wet age-related macular degeneration.

6. A first formulation for use according to claim 1, wherein the first formulation is administered independently of the second formulation.

7. A first formulation for use according to claim 1, wherein the first formulation is administered coincident with the second formulation.

8. A first formulation for use according to claim 1, wherein the first formulation is administered subsequent to the second formulation.

9. The first formulation for use according to claim 1, wherein the second formulation contains between about 100 µg and about 500 µg of ranibizmab.

10. The first formulation for use according to claim 1, wherein the first formulation contains between about 200 µg and about 2000 µg of rapamycin.

11. The first formulation for use according to claim 1, wherein the first formulation contains between about 200 µg and about 2000 µg of rapamycin; the second formulation for use according to claim 1 contains between about 100 µg and about 500 µg of ranibizmab; and the age-related macular degeneration is wet age-related macular degeneration.

## Patentansprüche

1. Erste Formulierung, die eine Rapamycin-Menge beinhaltet, in Kombination mit einer zweiten Formulierung, die eine Ranibizumab-Menge beinhaltet, zur Verwendung in einem Verfahren zur Behandlung von altersbedingter Makuladegeneration bei einem menschlichen Subjekt, wobei die erste und die zweite Formulierung jeweils durch intraokulare oder periokulare Zuführung zu einem Subjekt in solchen Volumen verabreicht werden, dass die Rapamycin-Menge und die Ranibizumab-Menge zusammen wirksam die altersbedingte Makuladegeneration behandeln.

2. Erste Formulierung zur Verwendung nach Anspruch 1, wobei die erste Formulierung im Wesentlichen aus etwa 2 % (w/w) Rapamycin, etwa 4 % (w/w) Ethanol und etwa 94 % (w/w) PEG 400 besteht.

3. Erste Formulierung zur Verwendung nach Anspruch 1, wobei die erste Formulierung im Wesentlichen aus etwa 4 % (w/w) Rapamycin, etwa 4 % (w/w) Ethanol und etwa 92 % (w/w) PEG 400 besteht.

4. Erste Formulierung zur Verwendung nach Anspruch 1, wobei die erste Formulierung im Wesentlichen aus etwa 1 % (w/w) Rapamycin, etwa 4 % (w/w) Ethanol und etwa 95 % (w/w) PEG 400 besteht.

5. Erste Formulierung zur Verwendung nach einem der Ansprüche 1-4, wobei die altersbedingte Makuladegeneration feuchte altersbedingte Makuladegeneration ist.

6. Erste Formulierung zur Verwendung nach Anspruch 1, wobei die erste Formulierung unabhängig von der zweiten Formulierung verabreicht wird.

7. Erste Formulierung zur Verwendung nach Anspruch 1, wobei die erste Formulierung gleichzeitig mit der zweiten Formulierung verabreicht wird.

8. Erste Formulierung zur Verwendung nach Anspruch 1, wobei die erste Formulierung im Anschluss an die zweite Formulierung verabreicht wird.

9. Erste Formulierung zur Verwendung nach Anspruch 1, wobei die zweite Formulierung zwischen etwa 100 µg und etwa 500 µg Ranibizumab enthält.

10. Erste Formulierung zur Verwendung nach Anspruch 1, wobei die erste Formulierung zwischen etwa 200 µg und etwa 2000 µg Rapamycin enthält.

11. Erste Formulierung zur Verwendung nach Anspruch 1, wobei die erste Formulierung zwischen etwa 200 µg und etwa 2000 µg Rapamycin enthält; die zweite Formulierung zur Verwendung nach Anspruch 1 zwischen etwa 100 µg und etwa 500 µg Ranibizumab enthält; und die altersbedingte Makuladegeneration feuchte altersbedingte Makuladegeneration ist.

## Revendications

1. Première formulation comprenant une certaine quantité de rapamycine en combinaison avec une seconde formulation comprenant une certaine quantité de ranibizumab pour usage dans un procédé de traitement de la dégénérescence maculaire liée à l'âge chez un sujet humain, dans laquelle le première et la seconde formulation sont chacune administrées par délivrance intraoculaire ou périoculaire au sujet en volumes tels que la quantité de rapamycine et la quantité de ranibizumab soient conjointement efficaces pour traiter la dégénérescence maculaire liée à l'âge.

2. Première formulation pour usage selon la revendication 1, dans laquelle la première formulation est constituée essentiellement d'environ 2 % (en poids/poids) de rapamycine, d'environ 4 % (en poids/poids) d'éthanol et d'environ 94 % (en poids/poids) de PEG 400.

3. Première formulation pour usage selon la revendication 1, dans laquelle la première formulation est constituée essentiellement d'environ 4 % (en poids/poids) de rapamycine, d'environ 4 % (en poids/poids) d'éthanol et d'environ 92 % (en poids/poids) de PEG 400.

4. Première formulation pour usage selon la revendication 1, dans laquelle la première formulation est constituée essentiellement d'environ 1 % (en poids/poids) de rapamycine, d'environ 4 % (en poids/poids) d'éthanol et d'environ 95 % (en poids/poids) de PEG 400.

5. Première formulation pour usage selon l'une quelconque des revendications 1 à 4, dans laquelle la dégénérescence maculaire liée à l'âge est la dégénérescence maculaire liée à l'âge humide.

6. Première formulation pour usage selon la revendication 1, dans laquelle la première formulation est administrée indépendamment de la seconde formulation.

7. Première formulation pour usage selon la revendication 1, dans laquelle la première formulation est administrée simultanément avec la seconde formulation.

8. Première formulation pour usage selon la revendication 1, dans laquelle la première formulation est administrée après la seconde formulation.

9. Première formulation pour usage selon la revendication 1, dans laquelle la seconde formulation contient entre environ 100 µg et environ 500 µg de ranibizumab.

10. Première formulation pour usage selon la revendication 1, dans laquelle la première formulation contient entre environ 200 µg et environ 2000 µg de rapamycine.

11. Première formulation pour usage selon la revendication 1, dans laquelle la première formulation contient entre environ 200 µg et environ 2000 µg de rapamycine ; la seconde formulation pour usage selon la revendication 1 contient entre environ 100 µg et environ 500 µg de ranibizumab ; et la dégénérescence maculaire liée à l'âge est la dégénérescence maculaire liée à l'âge humide.
